# EUROPEAN PATENT APPLICATION

(11) **EP 4 821 926 A1**
(43) Date of publication of application: **14.10.2026**
(21) Application number: 24900013.4
(22) Date of filing: 06.12.2024
(51) Int. Cl.: C07D 401/14, C07D 471/04, C07D 407/14, A61K 31/496, A61K 31/4412, A61P 35/00, A61P 31/14, A61P 31/20, A61P 33/00

(54) **ESTROGEN RECEPTOR MODULATOR AND USE THEREOF**

(30) Priority: 08.12.2023 CN 202311681737; 12.12.2023 CN 202311700571; 13.12.2023 CN 202311711928; 26.12.2023 CN 202311808960; 25.01.2024 CN 202410106933; 27.02.2024 CN 202410214069; 29.06.2024 CN 202410863924
(71) Applicant: Jiangsu Vcare Pharmatech Co., Ltd., Nanjing, Jiangsu 210032 (CN)
(72) Inventor: WU, Yong, Nanjing, Jiangsu 210032 (CN); GONG, Yanchun, Nanjing, Jiangsu 210032 (CN); LIU, Yongqiang, Nanjing, Jiangsu 210032 (CN)
(74) Representative: Laine IP Oy
(86) International application number: PCT/CN2024/137621
(87) International publication number: WO 2025/119380

(57) **Abstract**

The present invention relates to the technical field of medicine. Provided are an estrogen receptor modulator and a use thereof, particularly a compound as represented by general formula (i) or an isomer or pharmaceutically acceptable salt thereof and a use thereof, particularly a use thereof as an estrogen receptor degrading agent, a use thereof in the preparation of a medicament for treating and/or preventing an estrogen receptor-mediated or dependent disease or condition, and a use thereof in the preparation of a medicament for treating and/or preventing a disease or condition by degrading a target protein.

## Description

The present invention claims the priority of:
the earlier application filed with the China National Intellectual Property Administration (CNIPA) on December 8, 2023, with patent application number 202311681737.X and entitled "Estrogen receptor modulator and use thereof";
the earlier application filed with the China National Intellectual Property Administration (CNIPA) on December 12, 2023, with patent application number 202311700571.1 and entitled "Estrogen receptor modulator and use thereof;
the earlier application filed with the China National Intellectual Property Administration (CNIPA) on December 13, 2023, with patent application number 202311711928.6 and entitled "Estrogen receptor modulator and use thereof;
the earlier application filed with the China National Intellectual Property Administration (CNIPA) on December 26, 2023, with patent application number 202311808960.6 and entitled "Estrogen receptor modulator and use thereof;
the earlier application filed with the China National Intellectual Property Administration (CNIPA) on January 25, 2024, with patent application number 202410106933.2 and entitled "Estrogen receptor modulator and use thereof;
the earlier application filed with the China National Intellectual Property Administration (CNIPA) on February 27, 2024, with patent application number 202410214069.8 and entitled "Estrogen receptor modulator and use thereof;
the earlier application filed with the China National Intellectual Property Administration (CNIPA) on June 29, 2024, with patent application number 202410863924.8 and entitled "Estrogen receptor modulator and use thereof;
the entire contents of the earlier applications are incorporated into the present invention by reference.

### Field of the Invention

The present invention belongs to the technical field of medicine, and relates to an estrogen receptor modulator represented by formula (1), or an isomer or pharmaceutically acceptable salt thereof, and a use thereof.

### Description of Related Art

Anti-estrogen (hormone) therapy is the first choice for treating estrogen-positive breast cancer. Anti-estrogen therapy is divided into three main types, including aromatase inhibitors (such as letrozole and anastrozole); estrogen antagonists (such as tamoxifen and raloxifene); and selective estrogen-receptor degrading agents (such as fulvestrant). These types of anti-estrogen therapies work through different mechanisms, functioning to block estrogen and inhibit tumor growth.

Proteolysis-targeting chimeras (PROTACs) represent a novel therapeutic technology that blocks a signaling pathway by degrading a target protein, thereby exerting therapeutic effects. A PROTAC molecule usually consists of three parts: a target protein binding ligand, an E3 ubiquitin ligase ligand and a linker. The PROTAC molecule tightly binds to the target protein at one end of the chimera and binds to the E3 ubiquitin ligase at the other end to form a target protein-PROTAC-E3 ubiquitin ligase ternary complex. The target protein is "marked" with ubiquitination by E3 ubiquitin ligase-mediated ubiquitination; and the ubiquitination-"marked" target protein is then transported to the proteasome and degraded by the proteasome.

Compared with traditional small molecule inhibitors, PROTAC technology has the following advantages: 1. It may require a smaller dosage of administration. The target protein degradation process is similar to a catalytic reaction, in which the PROTAC molecule can be reused, so only a catalytic amount of a drug is needed to inhibit the target protein. 2. It can avoid the feedback upregulation of target protein expression caused by inhibition of the target protein. 3. It can expand the scope of drug targeting. PROTAC technology makes proteins that cannot be directly acted upon by traditional small molecule inhibitors, such as transcription factors, regulatory proteins and other non-enzyme proteins, become regulatable proteins, turning the target from "non-drugable" to "drugable".

ER-PROTAC drugs for targeted degradation of estrogen receptors have preliminarily demonstrated efficacy and excellent safety in clinical studies and are expected to become a new type of hormone therapy, an important supplement to traditional therapies that are resistant, intolerant, and ineffective.

### Summary of the Invention

One object of the invention is to provide a class of ER-PROTAC compounds for targeted degradation of estrogen receptors, isomers thereof or pharmaceutically acceptable salts thereof.

Technical solution of the present invention is as follows:
A compound represented by formula (i), or an isomer or pharmaceutically acceptable salt thereof:

A-B-C (i)

wherein,
A is 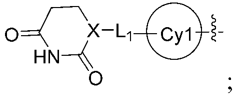
X is selected from N and CH;
Cy1 is selected from 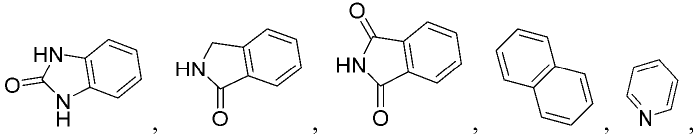 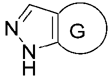 (for example 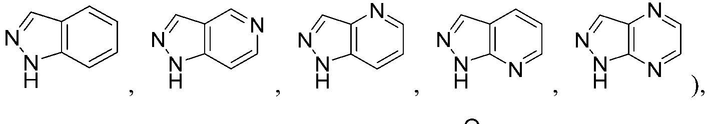 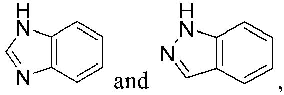 wherein the 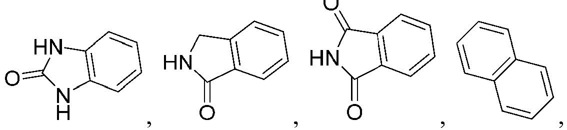 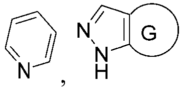 (for example 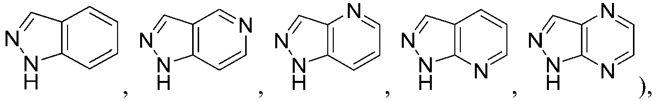 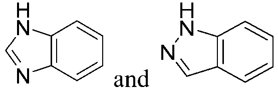 are unsubstituted, or each independently substituted with 1-3 Rₐ₁; G is selected from the following groups that are unsubstituted or optionally substituted with one, two or more Rₐ₁: C₆₋₁₀ aryl or a 5-10 membered heteroaryl group, such as phenyl, pyridyl, and pyrimidinyl;
each Rₐ₁ is independently selected from hydrogen, deuterium, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
B is -L2-Cy2-L3-Cy3-L4-Cy4-L5-; A is connected to L2; C is connected to L5;
L1, L2, L3, L4, L5, and L6 are independently selected from a bond, -(CH₂)ₙ-(NH)ₘ-, -O-, -S-, -NR₂, -CR₃₁R₃₂-, -C(O)-, -C(O)O-, -C(O)NR₄-, -NR₅-C(O)-, -S(O)-, -S(O)₂-, -P(O)(R₆)-, C₂₋₈ alkenyl and C₂₋₈ alkynyl;
n and m are independently any integer from zero to six; for example, 0, 1, 2, 3, 4, 5, or 6;
R₂, R₃₁, R₃₂, R₄, R₅, and R₆ are independently selected from hydrogen, deuterium, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
Cy2 is selected from a 3-13 membered cycloalkyl group, a 3-13 membered heterocyclyl group, a 3-13 membered cycloalkenyl group, a 3-13 membered cycloalkyl group substituted with 1-3 Rₐ₂, a 3-13 membered heterocyclyl group substituted with 1-3 Rₐ₂, a 3-13 membered cycloalkenyl group substituted with 1-3 Rₐ₂, and Cy21-Cy22, wherein the heteroatom of the 3-13 membered heterocyclyl group is selected from O, N and S; Cy21 and Cy22 are the same or different, and are independently selected from a 3-13 membered cycloalkyl group, a 3-13 membered heterocyclyl group, and a 3-13 membered cycloalkenyl group;
Cy3 is selected from a 3-13 membered cycloalkyl group, a 3-13 membered heterocyclyl group, a 6-13 membered bicyclic condensed group, or a 8-21 membered polycyclic condensed group, wherein the 3-13 membered cycloalkyl group, 3-13 membered heterocyclyl group, 6-13 membered bicyclic condensed group, or 8-21 membered polycyclic condensed group are unsubstituted or are each independently substituted with 1-5 Rₐ₃, wherein the heteroatom of the 5-6 membered heterocyclyl group is N, O, S, or Si;
Cy4 is selected from a 3-8 membered cycloalkyl group, a 3-8 membered heterocyclyl group, a 3-8 membered cycloalkenyl group, a 6-13 membered bicyclic condensed cycloalkyl group, a 6-13 membered bicyclic condensed cycloalkenyl group, a 6-13 membered bicyclic condensed heterocyclyl group, a 3-8 membered cycloalkyl group substituted with 1-3 Rₐ₄, a 3-8 membered heterocyclyl group substituted with 1-3 Rₐ₄, a 3-8 membered cycloalkenyl group substituted with 1-3 Rₐ₄, a 6-13 bicyclic condensed cycloalkyl group substituted with 1-3 Rₐ₄, a 6-13 membered bicyclic condensed cycloalkenyl group substituted with 1-3 Rₐ₄, and a 6-13 membered bicyclic condensed heterocyclyl group substituted with 1-3 Rₐ₄, wherein the heteroatom of the 3-8 membered heterocyclyl group or the 6-13 membered bicyclic condensed heterocyclyl group is selected from O, N and S;
each Rₐ₂, Rₐ₃ and Rₐ₄ at each occurrence are independently selected from hydrogen, deuterium, halogen, hydroxy, thiol, silanol, selenol, carboxyl, amino, cyano, nitro, C₁₋₆ alkyl, halogenated C₁₋₆ alkyl, C₁₋₆ alkoxy, halogenated C₁₋₆ alkoxy, C₁₋₆ alkyl-C(O)O-, C₁₋₆ alkylthio, C₁₋₆ alkylseleno, hydroxy C₁₋₆ alkyl, C₁₋₆ alkyl-C(O)NH-, C₁₋₆ alkylamino, (C₁₋₆ alkyl)₂amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, a 3-8 membered cycloalkyl group, a 3-8 membered heterocyclyl group, and a 3-8 membered cycloalkenyl group, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, 3-8 membered cycloalkyl group, 3-8 membered heterocyclyl group, and 3-8 membered cycloalkenyl group are unsubstituted or optionally substituted with one or more groups selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, halogenated C₁₋₆ alkoxy, a 3-8 membered cycloalkyl group, a 3-8 membered heterocyclyl group, and a 3-8 membered cycloalkenyl group;
C is 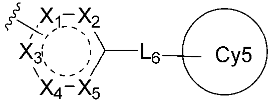
X₁ is N or CR_{b1};
X₂ is N or CR_{b2};
X₃ is N or CR_{b3};
X₄ is N or CR_{b4};
X₅ is N or CR_{b5};
R_{b1}, R_{b2}, R_{b3}, R_{b4} and R_{b5} are independently selected from hydrogen, deuterium, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
Cy5 is selected from a 6-13 membered bicyclic condensed group and an 8-21 membered polycyclic condensed group; wherein the 6-13 membered bicyclic condensed group and the 8-21 membered polycyclic condensed group are unsubstituted or substituted with 1-3 Rₐ₅; and
each Rₐ₅ is independently selected from hydrogen, deuterium, hydroxy, amino, carboxyl, cyano, nitro, halogen, C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, a 4-6 membered heterocyclyl group, a 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, a 4-6 membered heterocyclylcarbonyl group and a 5-6 membered heteroaryl-oxy group, wherein the C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, 4-6 membered heterocyclyl group, 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, 4-6 membered heterocyclylcarbonyl group and 5-6 membered heteroaryl-oxy group are unsubstituted or optionally substituted with one or more groups independently selected from hydroxy, amino, carboxyl, cyano, nitro, halogen, C₁₋₆ alkyl,
C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
provided that when Cy3 is 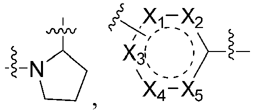 is not

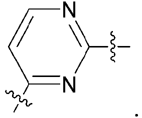

In another embodiment, the compound represented by formula (i), or the isomer or pharmaceutically acceptable salt thereof:
wherein,
A is 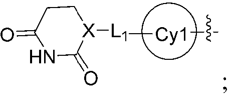
X is selected from N and CH;
Cy1 is selected from 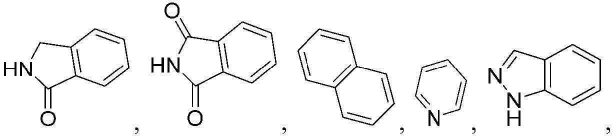 and phenyl, wherein the 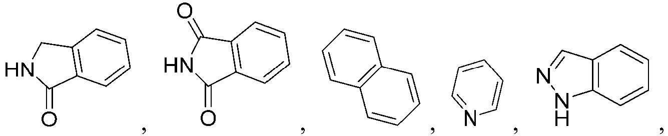 and phenyl are unsubstituted or each independently substituted with 1-3 Rₐ₁;
each Rₐ₁ is independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
B is -L2-Cy2-L3-Cy3-L4-Cy4-L5-;
L1, L2, L3, L4, L5 and L6 are independently selected from a bond, -(CH₂)ₙ-(NH)ₘ-, -O-, -S-, -NR₂, -CR₃₁R₃₂-, -C(O)-, -C(O)O-, -C(O)NR₄-, -NR₅-C(O)-, -CH₂NR₄-, -NR₅-CH₂-, C₂₋₈ alkenyl and C₂₋₈ alkynyl;
n and m are independently any integer from zero to six;
R₂, R₃₁, R₃₂, R₄ and R₅ are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
Cy2 is selected from a 4-6 membered cycloalkyl group, a 4-6 membered heterocyclyl group, a 4-6 membered cycloalkenyl group, a 4-6 membered cycloalkyl group substituted with 1-3 Rₐ₂, a 4-6 membered heterocyclyl group substituted with 1-3 Rₐ₂, and a 4-6 membered cycloalkenyl group substituted with 1-3 Rₐ₂, wherein the heteroatom of the 4-6 membered heterocyclyl group is selected from O, N and S;
Cy3 is selected from a 5-6 membered cycloalkyl group and a 5-6 membered heterocyclyl group, wherein the 5-6 membered cycloalkyl group and 5-6 membered heterocyclyl group are unsubstituted or each independently substituted with 1-3 Rₐ₃, wherein the heteroatom of the 5-6 membered heterocyclyl group is N;
Cy4 is selected from a 3-8 membered cycloalkyl group, a 3-8 membered heterocyclyl group, a 3-8 membered cycloalkenyl group, a 6-13 membered bicyclic condensed cycloalkyl group, a 6-13 membered bicyclic condensed cycloalkenyl group, a 6-13 membered bicyclic condensed heterocyclyl group, a 3-8 membered cycloalkyl group substituted with 1-3 Rₐ₄, a 3-8 membered heterocyclyl group substituted with 1-3 Rₐ₄, a 3-8 membered cycloalkenyl group substituted with 1-3 Rₐ₄, a 6-13 bicyclic condensed cycloalkyl group substituted with 1-3 Rₐ₄, a 6-13 membered bicyclic condensed cycloalkenyl group substituted with 1-3 Rₐ₄, and a 6-13 membered bicyclic condensed heterocyclyl group substituted with 1-3 Rₐ₄, wherein the heteroatom of the 3-8 membered heterocyclyl group or the 6-13 membered bicyclic condensed heterocyclyl group is selected from O, N and S;
each Rₐ₂, Rₐ₃ and Rₐ₄ at each occurrence are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, a 3-8 membered cycloalkyl group, a 3-8 membered heterocyclyl group, and a 3-8 membered cycloalkenyl group, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, 3-8 membered cycloalkyl group, 3-8 membered heterocyclyl group, and 3-8 membered cycloalkenyl group are unsubstituted or optionally substituted with one or more groups selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, halogenated C₁₋₆ alkoxy, a 3-8 membered cycloalkyl group, a 3-8 membered heterocyclyl group, and a 3-8 membered cycloalkenyl group;
C is 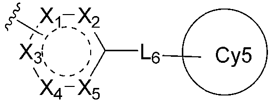
X₁ is N or CR_{b1};
X₂ is N or CR_{b2};
X₃ is N or CR_{b3};
X₄ is N or CR_{b4};
X₅ is N or CR_{b5};
R_{b1}, R_{b2}, R_{b3}, R_{b4} and R_{b5} are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
Cy5 is selected from a 6-13 membered bicyclic condensed group and an 8-21 membered polycyclic condensed group; wherein the 6-13 membered bicyclic condensed group and the 8-21 membered polycyclic condensed group are unsubstituted or substituted with 1-3 Rₐ₅; and
each Rₐ₅ is independently selected from hydroxy, amino, carboxyl, cyano, nitro, halogen, C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, a 4-6 membered heterocyclyl group, a 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, a 4-6 membered heterocyclylcarbonyl group and a 5-6 membered heteroaryl-oxy group, wherein the C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, 4-6 membered heterocyclyl group, 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, 4-6 membered heterocyclylcarbonyl group and 5-6 membered heteroaryl-oxy group are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
provided that when Cy3 is 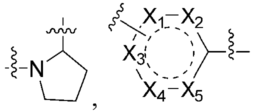 is not

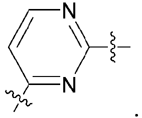

Another embodiment of the present invention relates to a compound represented by formula (i), or an isomer or pharmaceutically acceptable salt thereof,
wherein,
A is 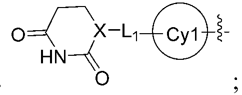
X is selected from N and CH;
Cy1 is selected from 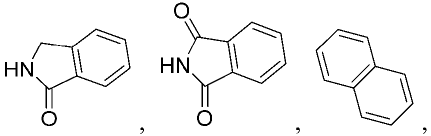 and phenyl, wherein the 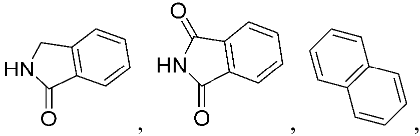 and phenyl are unsubstituted or each independently substituted with 1-3 Rₐ₁;
each Rₐ₁ is independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
B is -L2-Cy2-L3-Cy3-L4-Cy4-L5-;
L1, L2, L3, L4, L5 and L6 are independently selected from a bond, -(CH₂)ₙ-(NH)ₘ-, -O-, -S-, -NR₂, -CR₃₁R₃₂-, -C(O)-, -C(O)O-, -C(O)NR₄-, -NR₅-C(O)-, C₂₋₈ alkenyl and C₂₋₈ alkynyl;
n and m are independently any integer from zero to six;
R₂, R₃₁, R₃₂, R₄ and R₅ are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
Cy2 is selected from a 4-6 membered cycloalkyl group, a 4-6 membered heterocyclyl group, a 4-6 membered cycloalkenyl group, a 4-6 membered cycloalkyl group substituted with 1-3 Rₐ₂, a 4-6 membered heterocyclyl group substituted with 1-3 Rₐ₂, and a 4-6 membered cycloalkenyl group substituted with 1-3 Rₐ₂, wherein the heteroatom of the 4-6 membered heterocyclyl group is selected from O, N and S;
Cy3 is selected from a 5-6 membered cycloalkyl group and a 5-6 membered heterocyclyl group, wherein the 5-6 membered cycloalkyl group and 5-6 membered heterocyclyl group are unsubstituted or each independently substituted with 1-3 Rₐ₃, wherein the heteroatom of the 5-6 membered heterocyclyl group is N;
Cy4 is selected from a 3-8 membered cycloalkyl group, a 3-8 membered heterocyclyl group, a 3-8 membered cycloalkenyl group, a 6-13 membered bicyclic condensed cycloalkyl group, a 6-13 membered bicyclic condensed cycloalkenyl group, a 6-13 membered bicyclic condensed heterocyclyl group, a 3-8 membered cycloalkyl group substituted with 1-3 Rₐ₄, a 3-8 membered heterocyclyl group substituted with 1-3 Rₐ₄, a 3-8 membered cycloalkenyl group substituted with 1-3 Rₐ₄, a 6-13 bicyclic condensed cycloalkyl group substituted with 1-3 Rₐ₄, a 6-13 membered bicyclic condensed cycloalkenyl group substituted with 1-3 Rₐ₄, and a 6-13 membered bicyclic condensed heterocyclyl group substituted with 1-3 Rₐ₄, wherein the heteroatom of the 3-8 membered heterocyclyl group or the 6-13 membered bicyclic condensed heterocyclyl group is selected from O, N and S;
each Rₐ₂, Rₐ₃ and Rₐ₄ at each occurrence are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, a 3-8 membered cycloalkyl group, a 3-8 membered heterocyclyl group, and a 3-8 membered cycloalkenyl group, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, 3-8 membered cycloalkyl group, 3-8 membered heterocyclyl group, and 3-8 membered cycloalkenyl group are unsubstituted or optionally substituted with one or more groups selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, halogenated C₁₋₆ alkoxy, a 3-8 membered cycloalkyl group, a 3-8 membered heterocyclyl group, and a 3-8 membered cycloalkenyl group;
C is 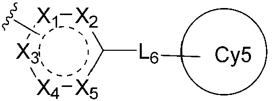
X₁ is N or CR_{b1};
X₂ is N or CR_{b2};
X₃ is N or CR_{b3};
X₄ is N or CR_{b4};
X₅ is N or CR_{b5};
R_{b1}, R_{b2}, R_{b3}, R_{b4} and R_{b5} are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
Cy5 is selected from a 6-13 membered bicyclic condensed group and an 8-21 membered polycyclic condensed group; wherein the 6-13 membered bicyclic condensed group and the 8-21 membered polycyclic condensed group are unsubstituted or substituted with 1-3 Rₐ₅; and
each Rₐ₅ is independently selected from hydroxy, amino, carboxyl, cyano, nitro, halogen, C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, a 4-6 membered heterocyclyl group, a 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, a 4-6 membered heterocyclylcarbonyl group and a 5-6 membered heteroaryl-oxy group, wherein the C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, 4-6 membered heterocyclyl group, 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, 4-6 membered heterocyclylcarbonyl group and 5-6 membered heteroaryl-oxy group are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
provided that when Cy3 is 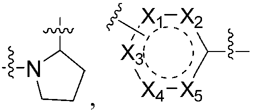 is not

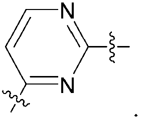

Another embodiment of the present invention relates to a compound represented by formula (i), or an isomer or pharmaceutically acceptable salt thereof,
wherein,
A is 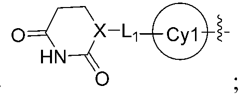
X is selected from N and CH;
Cy1 is selected from 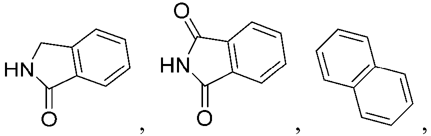 and phenyl, wherein the 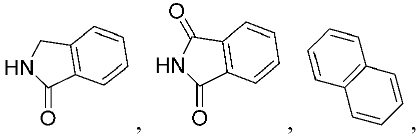 and phenyl are unsubstituted or each independently substituted with 1-3 Rₐ₁;
each Rₐ₁ is independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
B is -L2-Cy2-L3-Cy3-L4-Cy4-L5-;
L1, L2, L3, L4, L5, and L6 are independently selected from a bond, -(CH₂)ₙ-(NH)ₘ-, -O-, -S-, -NR₂, -CR₃₁R₃₂-, -C(O)-, -C(O)O-, -C(O)NR₄-, -NR₅-C(O)-, C₂₋₈ alkenyl and C₂₋₈ alkynyl;
n and m are independently any integer from zero to six;
R₂, R₃₁, R₃₂, R₄ and R₅ are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
Cy2 is selected from a 4-6 membered cycloalkyl group, a 4-6 membered heterocyclyl group, a 4-6 membered cycloalkenyl group, a 4-6 membered cycloalkyl group substituted with 1-3 Rₐ₂, a 4-6 membered heterocyclyl group substituted with 1-3 Rₐ₂, and a 4-6 membered cycloalkenyl group substituted with 1-3 Rₐ₂, wherein the heteroatom of the 4-6 membered heterocyclyl group is selected from O, N and S;
Cy3 is selected from 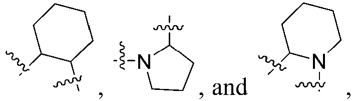 wherein the 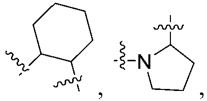 and 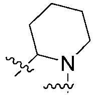 are unsubstituted or each independently substituted with 1-3 Rₐ₃;
Cy4 is selected from a 3-8 membered cycloalkyl group, a 3-8 membered heterocyclyl group, a 3-8 membered cycloalkenyl group, a 6-13 membered bicyclic condensed cycloalkyl group, a 6-13 membered bicyclic condensed cycloalkenyl group, a 6-13 membered bicyclic condensed heterocyclyl group, a 3-8 membered cycloalkyl group substituted with 1-3 Rₐ₄, a 3-8 membered heterocyclyl group substituted with 1-3 Rₐ₄, a 3-8 membered cycloalkenyl group substituted with 1-3 Rₐ₄, a 6-13 bicyclic condensed cycloalkyl group substituted with 1-3 Rₐ₄, a 6-13 membered bicyclic condensed cycloalkenyl group substituted with 1-3 Rₐ₄, and a 6-13 membered bicyclic condensed heterocyclyl group substituted with 1-3 Rₐ₄, wherein the heteroatom of the 3-8 membered heterocyclyl group or the 6-13 membered bicyclic condensed heterocyclyl group is selected from O, N and S;
each Rₐ₂, Rₐ₃ and Rₐ₄ at each occurrence are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, a 3-8 membered cycloalkyl group, a 3-8 membered heterocyclyl group, and a 3-8 membered cycloalkenyl group, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, 3-8 membered cycloalkyl group, 3-8 membered heterocyclyl group, and 3-8 membered cycloalkenyl group are unsubstituted or optionally substituted with one or more groups selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, halogenated C₁₋₆ alkoxy, a 3-8 membered cycloalkyl group, a 3-8 membered heterocyclyl group, and a 3-8 membered cycloalkenyl group;
C is 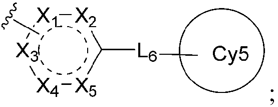
X₁ is N or CR_{b1};
X₂ is N or CR_{b2};
X₃ is N or CR_{b3};
X₄ is N or CR_{b4};
X₅ is N or CR_{b5};
R_{b1}, R_{b2}, R_{b3}, R_{b4} and R_{b5} are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
Cy5 is selected from a 6-13 membered bicyclic condensed group and an 8-21 membered polycyclic condensed group; wherein the 6-13 membered bicyclic condensed group and the 8-21 membered polycyclic condensed group are unsubstituted or optionally substituted with 1-3 Rₐ₅; and
each Rₐ₅ is independently selected from hydroxy, amino, carboxyl, cyano, nitro, halogen, C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, a 4-6 membered heterocyclyl group, a 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, a 4-6 membered heterocyclylcarbonyl group and a 5-6 membered heteroaryl-oxy group, wherein the C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, 4-6 membered heterocyclyl group, 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, 4-6 membered heterocyclylcarbonyl group and 5-6 membered heteroaryl-oxy group are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
provided that when Cy3 is 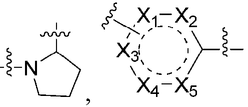 is not

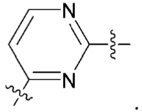

Another embodiment of the present invention relates to a compound represented by formula (i), or an isomer or pharmaceutically acceptable salt thereof,
A is 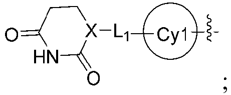
X is selected from N and CH;
Cy1 is selected from 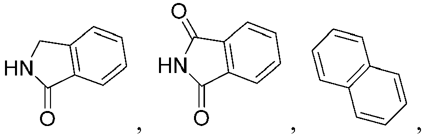 and phenyl, wherein the 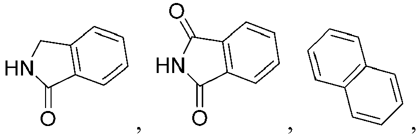 and phenyl are unsubstituted or each independently substituted with 1-3 Rₐ₁;
each Rₐ₁ is independently selected from hydrogen, halogen, hydroxy, amino, C₁₋₆ alkyl, halogenated C₁₋₆ alkyl, C₁₋₆ alkoxy, halogenated C₁₋₆ alkoxy, and hydroxy C₁₋₆ alkyl;
B is -L2-Cy2-L3-Cy3-L4-Cy4-L5-;
L1, L2, L3, L4, L5, and L6 are independently selected from a bond, -(CH₂)ₙ-(NH)ₘ-, -O-, and -C(O)-;
n and m are independently 0, 1 or 2;
Cy2 is selected from a 4-6 membered cycloalkyl group, a 4-6 membered heterocyclyl group, a 4-6 membered cycloalkenyl group, a 4-6 membered cycloalkyl group substituted with 1-3 Rₐ₂, a 4-6 membered heterocyclyl group substituted with 1-3 Rₐ₂, and a 4-6 membered cycloalkenyl group substituted with 1-3 Rₐ₂, wherein the heteroatom of the 4-6 membered heterocyclyl group is selected from O, N and S;
Cy3 is selected from 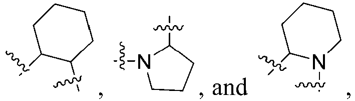 wherein the 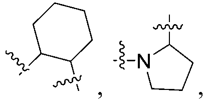 and 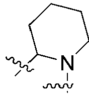 are unsubstituted or each independently substituted with 1-3 Rₐ₃;
Cy4 is selected from a 3-8 membered cycloalkyl group, a 3-8 membered heterocyclyl group, a 3-8 membered cycloalkenyl group, a 6-13 membered bicyclic condensed cycloalkyl group, a 6-13 membered bicyclic condensed cycloalkenyl group, a 6-13 membered bicyclic condensed heterocyclyl group, a 3-8 membered cycloalkyl group substituted with 1-3 Rₐ₄, a 3-8 membered heterocyclyl group substituted with 1-3 Rₐ₄, a 3-8 membered cycloalkenyl group substituted with 1-3 Rₐ₄, a 6-13 bicyclic condensed cycloalkyl group substituted with 1-3 Rₐ₄, a 6-13 membered bicyclic condensed cycloalkenyl group substituted with 1-3 Rₐ₄, and a 6-13 membered bicyclic condensed heterocyclyl group substituted with 1-3 Rₐ₄, wherein the heteroatom of the 3-8 membered heterocyclyl group or the 6-13 membered bicyclic condensed heterocyclyl group is selected from O, N and S;
each Rₐ₂,Rₐ₃ and Rₐ₄ at each occurrence are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, a 3-8 membered cycloalkyl group, a 3-8 membered heterocyclyl group, and a 3-8 membered cycloalkenyl group, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, 3-8 membered cycloalkyl group, 3-8 membered heterocyclyl group, and 3-8 membered cycloalkenyl group are unsubstituted or optionally substituted with one or more groups selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, halogenated C₁₋₆ alkyl, halogenated C₁₋₆ alkoxy, a 3-8 membered cycloalkyl, a 3-8 membered heterocyclyl, and a 3-8 membered cycloalkenyl;
C is 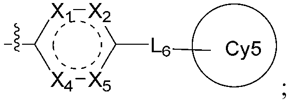
X₁ is N or CR_{b1};
X₂ is N or CR_{b2};
X₄ is N or CR_{b4};
X₅ is N or CR_{b5};
R_{b1}, R_{b2}, R_{b3}, R_{b4}, and R_{b5} are independently selected from hydrogen, halogen, hydroxy, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, and (C₁₋₆ alkyl)₂ amino;
Cy5 is selected from a 6-13 membered bicyclic fused cycloalkenyl group, a 6-13 membered bicyclic fused heterocyclyl group, a 6-13 membered bicyclic fused heteroaryl group, an 8-21 membered polycyclic fused heterocyclyl group, and an 8-21 membered polycyclic fused heteroaryl group; wherein the 6-13 membered bicyclic fused cycloalkenyl group, 6-13 membered bicyclic fused heterocyclyl group, 6-13 membered bicyclic fused heteroaryl group, 8-21 membered polycyclic fused heterocyclyl group, and 8-21 membered polycyclic fused heteroaryl group are unsubstituted or optionally substituted with 1-3 Rₐ₅; and
each Rₐ₅ is independently selected from hydroxy, amino, carboxyl, cyano, nitro, halogen, C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₃₋₆ cycloalkyl, a 4-6 membered heterocyclyl group, a 5-6 membered heteroaryl group, and aryl, wherein the C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₃₋₆ cycloalkyl, 4-6 membered heterocyclyl group, 5-6 membered heteroaryl group, and aryl are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy.

**Another embodiment of the present invention relates to a compound represented by formula (i), or an isomer or pharmaceutically acceptable salt thereof, having a structure represented by formula (ii),** 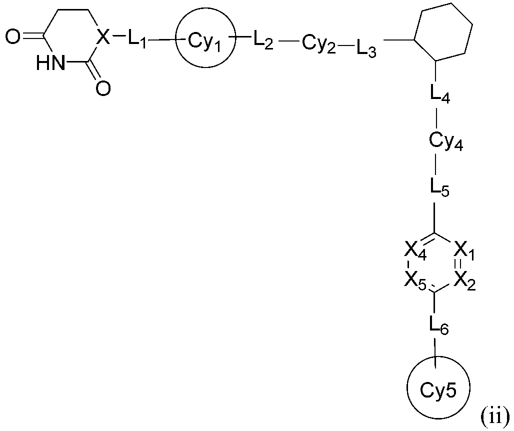
X is selected from N and CH;
the ring Cy1 is selected from 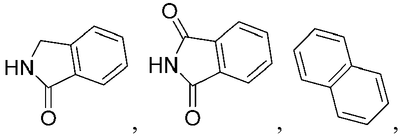 and phenyl, wherein the 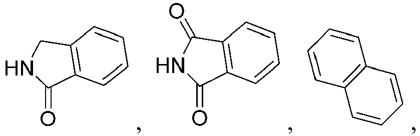 and phenyl are unsubstituted or each independently substituted with 1-3 Rₐ₁;
each Rₐ₁ is independently selected from hydrogen, C₁₋₆ alkyl, and C₁₋₆ alkoxy;
L1, L2, L3, L4, L5, and L6 are independently selected from a bond, -(CH₂)ₙ-(NH)ₘ-, -O-, and -C(O)-;
n and m are independently 0, 1 or 2;
Cy2 is selected from a 4-6 membered nitrogen-containing heterocyclyl group and a 4-6 membered nitrogen-containing heterocyclyl group substituted with 1-3 Rₐ₂;
Cy4 is selected from a 3-8 membered heterocyclyl group, a 3-8 membered heterocyclyl group substituted with 1-3 Rₐ₄, a 6-13 membered bicyclic spiro heterocyclyl group and a 6-13 membered bicyclic spiro heterocyclyl group substituted with 1-3 Rₐ₄, wherein the heteroatom of the 3-8 membered heterocyclyl group and the 6-13 membered bicyclic spiro heterocyclyl group is selected from O, N and S;
each Rₐ₂ and Rₐ₄ at each occurrence are independently selected from hydrogen, C₁₋₆ alkyl, and a 3-8 membered heterocyclyl group, wherein the C₁₋₆ alkyl and 3-8 membered heterocyclyl group are unsubstituted or substituted with one or more groups selected from C₁₋₆ alkyl and a 3-8 membered heterocyclyl group;
X₁ is N or CR_{b1};
X₂ is N or CR_{b2};
X₄ is N or CR_{b4};
X₅ is N or CR_{b5};
R_{b1}, R_{b2}, R_{b3}, R_{b4} and R_{b5} are independently selected from hydrogen and halogen;
Cy5 is selected from a 6-13 membered bicyclic fused cycloalkenyl group, a 6-13 membered bicyclic fused heteroaryl group, and an 8-21 membered polycyclic fused heterocyclyl group, wherein the 6-13 membered bicyclic fused cycloalkenyl group, 6-13 membered bicyclic fused heteroaryl group, and 8-21 membered polycyclic fused heterocyclyl group are unsubstituted or optionally substituted with 1-3 Rₐ₅; and
each Rₐ₅ is independently selected from hydrogen, hydroxy, C₁₋₆ alkyl, C₁₋₆ alkoxy, halogenated C₁₋₆ alkyl, and phenyl, wherein the phenyl is unsubstituted or substituted with one or more groups selected from halogen, halogenated C₁₋₆ alkyl, and C₁₋₆ alkoxy.

**Another embodiment of the present invention relates to a compound represented by formula (ii), or an isomer or pharmaceutically acceptable salt thereof,**
X is selected from N and CH;
Cy1 is selected from 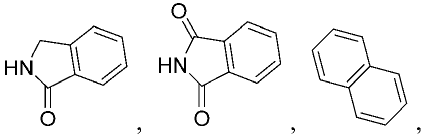 and phenyl, wherein the 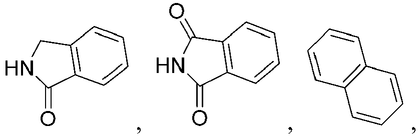 and phenyl are unsubstituted or each independently substituted with 1-3 Rₐ₁;
each Rₐ₁ is independently selected from hydrogen, methoxy, methyl, and ethyl;
L1 is selected from a bond and -NH-;
L2 is selected from a bond and -NH-;
Cy2 is selected from 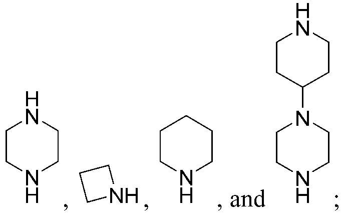
L3 is selected from -CH₂-, -C(O)-, and -NH-CH₂-;
Cy3 is selected from 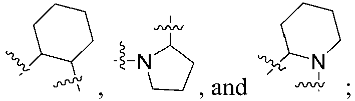
L4 is selected from -CH₂-, -NH-CH₂-, and -C(O)-;
Cy4 is selected from 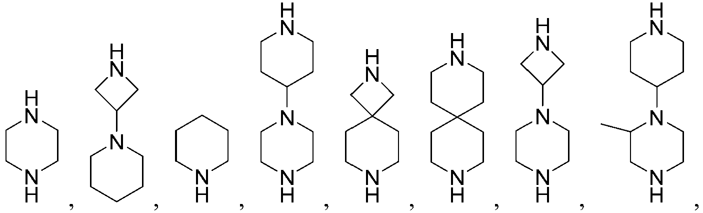 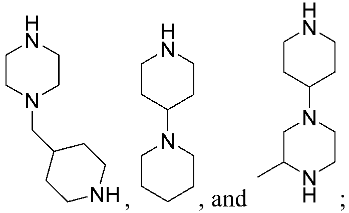
L5 is selected from a bond; 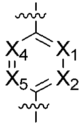 is selected from 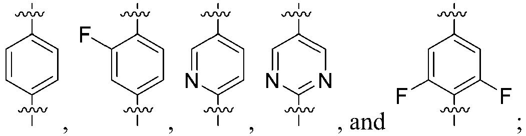
L6 is selected from a bond and -O-;
Cy5 is selected from 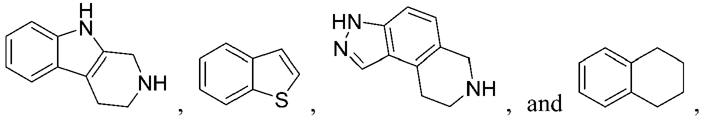 wherein the 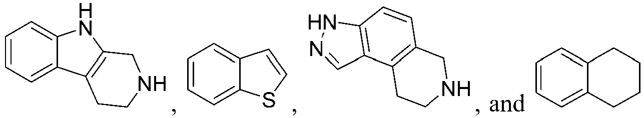 are unsubstituted or optionally substituted with 1-3 Rₐ₅; and
each Rₐ₅ is independently selected from hydroxy, methyl, methoxy, phenyl, 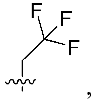 and 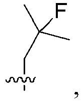 wherein the phenyl is unsubstituted or substituted with one or more groups selected from fluorine, methoxy, trifluoromethyl, and

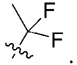

In another embodiment of the present invention, Cy1 is selected from 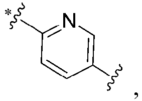 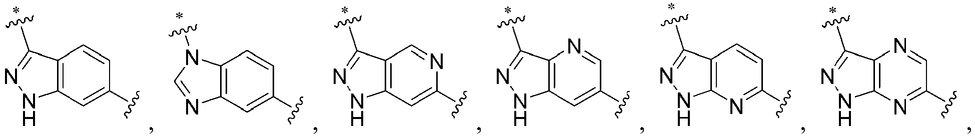 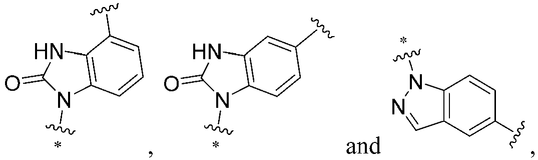 wherein the * end is connected to L1.

In another embodiment of the present invention, the 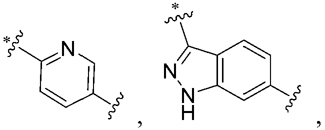 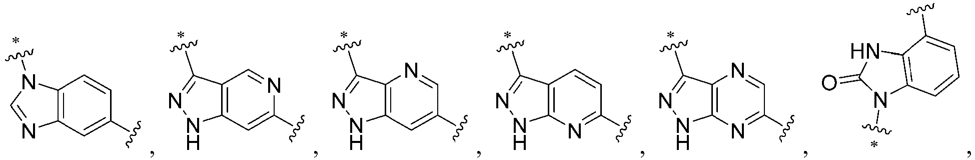 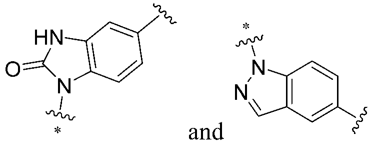 are unsubstituted or each independently substituted with 1-3 Rₐ₁; more preferably, each Rₐ₁ is independently selected from hydrogen, halogen, hydroxyl, carboxyl, amino, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₁₋₆ alkylcarbonyl, aminocarbonyl, C₁₋₆ alkylaminocarbonyl, and (C₁₋₆ alkyl)₂ aminocarbonyl; still more preferably, each Rₐ₁ is independently substituted with 1-3 halogen atoms (such as F) or C₁₋₆ alkyl groups (such as methyl).

In another embodiment of the present invention, Cy1 is selected from 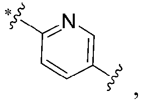 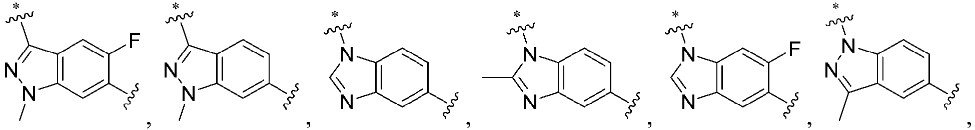 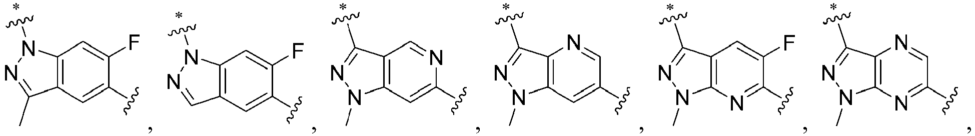 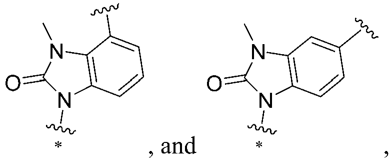 wherein the * end is connected to L1.

In another embodiment of the present invention, L1, L2, L3, L4, L5, and L6 are the same or different and are independently selected from a bond, -O-, -NH-, C₁₋₆ alkylene, -NHC(O)-, NH(C₁₋₆ alkylene), and -C(O)-.

In another embodiment of the present invention, L1 is selected from a bond and -NHC(O)-; preferably, when L1 is -NHC(O)-, the C(O) end is connected to Cy1.

In another embodiment of the present invention, L2 is selected from a bond, NH, and NH(C₁₋₃ alkylene).

In another embodiment of the present invention, L2 is selected from a bond, -NH-, and - NHCH₂-; preferably a bond.

In another embodiment of the present invention, L3 is selected from C₁₋₃ alkylene, -C(O)-, - CH₂NR₄-, -NR₅-CH₂-, and NHC₁₋₃ alkylene, wherein R₄ and R₅ are independently selected from C3-6 cycloalkyl.

In another embodiment of the present invention, L3 is selected from methylene, -C(O)-, and - NHCH₂-; preferably methylene.

In another embodiment of the present invention, L4 is selected from a bond, C₁₋₃ alkylene, and -C(O)-.

In another embodiment of the present invention, L4 is selected from a bond, methylene, and - C(O)-; preferably methylene.

In another embodiment of the present invention, L5 is selected from a bond and C₁₋₃ alkylene.

In another embodiment of the present invention, L5 is selected from a bond and methylene; preferably a bond.

In another embodiment of the present invention, L6 is selected from a bond and -O-; preferably a bond.

In another embodiment of the present invention, Cy2 is selected from a 4-10 membered cycloalkyl group, a 4-10 membered heterocyclyl group, a 4-10 membered cycloalkenyl group, a 4-10 membered cycloalkyl group substituted with 1-3 Ra2, a 4-10 membered heterocyclyl group substituted with 1-3 Ra2, a 4-10 membered cycloalkenyl group substituted with 1-3 Ra2, and Cy21-Cy22, wherein the heteroatom of the 4-10 membered heterocyclyl group is selected from O, N and S; Cy21 and Cy22 are the same or different, and are independently selected from a 4-6 membered cycloalkyl group, a 4-6 membered heterocyclyl group, and a 4-6 membered cycloalkenyl group.

In another embodiment of the present invention, Cy2 is selected from a 4-6 membered cycloalkyl group, a 4-6 membered heterocyclyl group, a 4-6 membered cycloalkenyl group, a 4-6 membered cycloalkyl group substituted with 1-3 Rₐ₂, a 4-6 membered heterocyclyl group substituted with 1-3 Rₐ₂, and a 4-6 membered cycloalkenyl group substituted with 1-3 Rₐ₂, wherein the heteroatom of the 4-6 membered heterocyclyl group is selected from O, N and S.

In another embodiment of the present invention, Cy2 is selected from a 6-9 membered heterocyclyl group that is unsubstituted or substituted with 1-3 Rₐ₂, and Cy21-Cy22; wherein Cy21 and Cy22 are the same or different, and are independently selected from cyclohexyl, piperazinyl, and piperidinyl.

In another embodiment of the present invention, Cy2 is selected from piperazinyl and piperidinyl that are unsubstituted or substituted with 1-3 H or F atoms.

In another embodiment of the present invention, Cy2 is selected from piperazinyl, piperidinyl, 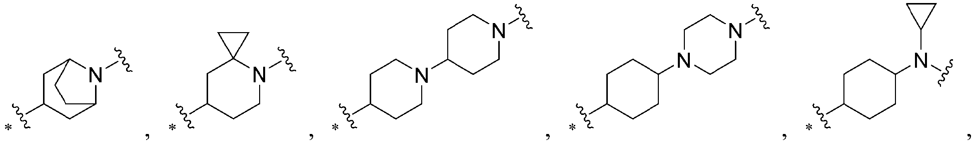 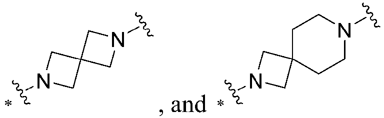 that are unsubstituted or substituted with 1-3 Rₐ₂.

In another embodiment of the present invention, Cy2 is selected from 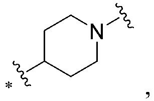 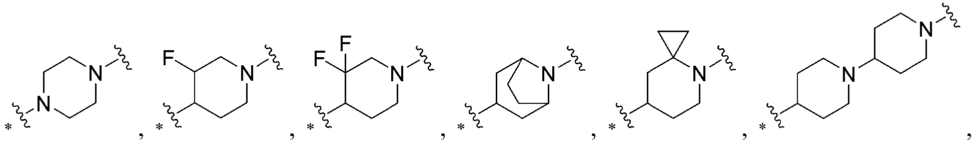 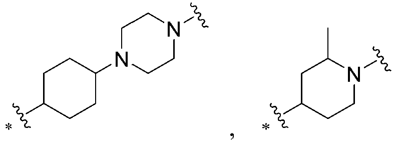 (for example, 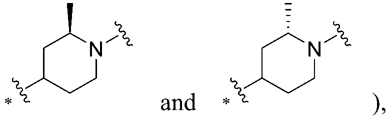 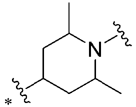 (for example, 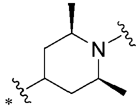 ), 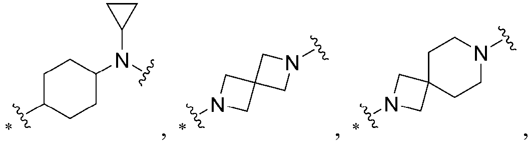 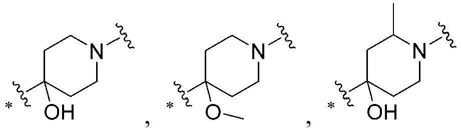 (for example, 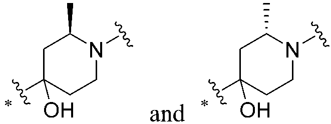 ), 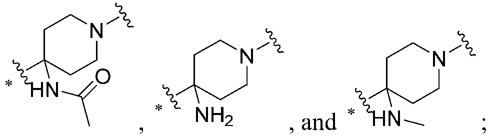 wherein the * end is connected to Cy1.

In another embodiment of the present invention, Cy3 is selected from a 5-10 membered cycloalkyl group, a 5-10 membered heterocyclyl group, a 6-13 membered bicyclic condensed cycloalkyl group, a 6-13 membered bicyclic condensed cycloalkenyl group, a 6-13 membered bicyclic condensed heterocyclyl group, an 8-13 membered bicyclic condensed heteroaryl group, an 8-13 membered bicyclic condensed aryl group, an 8-21 membered polycyclic condensed cycloalkyl group, an 8-21 membered polycyclic condensed cycloalkenyl group, an 8-21 membered polycyclic condensed heterocyclyl group, an 8-21 membered polycyclic condensed heteroaryl group, and an 8-21 membered polycyclic condensed aryl group.

In another embodiment of the present invention, Cy3 is selected from a 5-6 membered cycloalkyl group and a 5-6 membered heterocyclyl group, wherein the 5-6 membered cycloalkyl group and 5-6 membered heterocyclyl group are unsubstituted or each independently substituted with 1-3 Ra3, wherein the heteroatom of the 5-6 membered heterocyclyl group is N, O, S, or Si.

In another embodiment of the present invention, Cy3 is selected from a 5-6 membered cycloalkyl group and a 5-6 membered heterocyclyl group; wherein the 5-6 membered cycloalkyl group and 5-6 membered heterocyclyl group are unsubstituted or each independently substituted with 1-3 Rₐ₃.

In another embodiment of the present invention, Cy3 is selected from cyclohexyl, piperidinyl, tetrahydropyranyl, dioxanyl, and pyrrolidinyl; wherein the cyclohexyl, piperidinyl, tetrahydropyranyl, dioxanyl, and pyrrolidinyl are unsubstituted or each independently substituted with 1-3 Rₐ₃; preferably cyclohexyl.

In another embodiment of the present invention, Cy3 is selected from 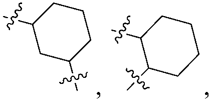 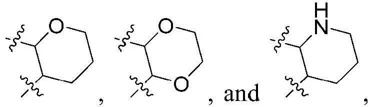 wherein the 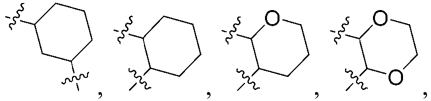 and 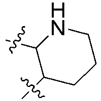 are unsubstituted or independently substituted with 1-3 Rₐ₃.

In another embodiment of the present invention, Cy4 is selected from a 5-6 membered heterocyclyl group that is unsubstituted or optionally substituted with one, two or more groups selected from C₁₋₆ alkyl and C₁₋₆ alkoxy.

In another embodiment of the present invention, Cy4 is selected from piperazinyl and 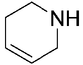 , for example, 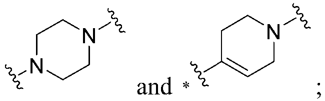 wherein the * end is connected to L5.

In another embodiment of the present invention, Cy5 is selected from the following groups that are unsubstituted or substituted with 1-3 Rₐ₅: a 6-13 membered bicyclic fused cycloalkenyl group, a 6-13 membered bicyclic fused heteroaryl group, and an 8-21 membered polycyclic fused heterocyclyl group.

In another embodiment of the present invention, Cy5 is selected from 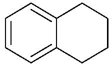 that is unsubstituted or substituted with 1-3 OH or phenyl groups, for example,

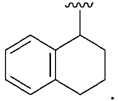

In another embodiment of the present invention, Cy5 is 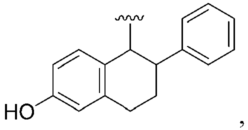 for example,

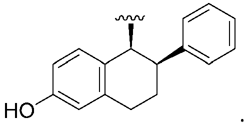

In another embodiment of the present invention, Rₐ₁ is selected from halogen, C₁₋₆ alkyl, and C₁₋₆ alkoxy.

In another embodiment of the present invention, Rₐ₁ is selected from F, methyl, ethyl, and methoxy.

In another embodiment of the present invention, Rₐ₂, Rₐ₃ and Rₐ₄ are the same or different, and are each independently selected from H, OH, amino, thiol, silanol, selenol, halogen, C₁₋₆ alkyl, C₁₋₆ alkoxy, C₁₋₆ alkyl-NH-, C₃₋₆ cycloalkyl, C₁₋₆ alkyl-C(O)NH-, halogenated C₁₋₆ alkyl, halogenated C₁₋₆ alkoxy, and hydroxy C₁₋₆ alkyl.

In another embodiment of the present invention, Rₐ₂ is selected from H, OH, amino, halogen, C₁₋₆ alkyl, C₁₋₆ alkoxy, C₁₋₆ alkyl-NH-, C₃₋₆ cycloalkyl, and C₁₋₆ alkyl-C(O)NH-.

In another embodiment of the present invention, Rₐ₂ is selected from H, F, OH, amino, methylamino, acetamido, methyl, methoxy, and cyclopropyl.

In another embodiment of the present invention, Rₐ₃ and Rₐ₄ are the same or different and are independently selected from H and halogen.

In another embodiment of the present invention, Rₐ₃ and Rₐ₄ are the same or different and are independently selected from H and F.

In another embodiment of the present invention, Rₐ₅ is selected from hydroxy and C₆₋₁₀ aryl.

In another embodiment of the present invention, Rₐ₅ is selected from hydroxy and phenyl.

In another embodiment of the present invention, Ra5 is selected from hydroxy, C6-10 aryl, and C6-10 aryl substituted with halogen.

In another embodiment of the present invention, 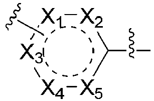 is selected from the following groups that are unsubstituted or optionally substituted with 1 to 4 substituents selected from R_{b1}, R_{b2}, R_{b3}, R_{b4}, and R_{b5}: phenyl and a 6-membered heteroaryl group containing 1 or 2 heteroatoms, for example,

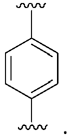

Another embodiment of the present invention relates to a compound represented by formula (i), or an isomer or pharmaceutically acceptable salt thereof, having a structure represented by formula (iii): 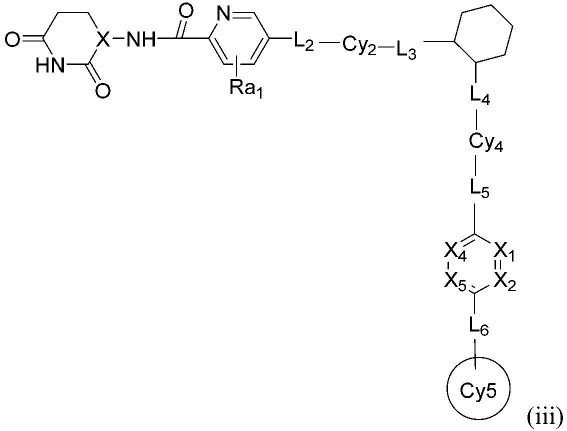
X is selected from N and CH;
Rₐ₁ is selected from hydrogen, C₁₋₆ alkyl, and C₁₋₆ alkoxy;
L2, L3, L4, L5, and L6 are independently selected from a bond, -(CH₂)ₙ-(NH)ₘ-, -O-, and - C(O)-;
n and m are independently 0, 1 or 2;
Cy2 is selected from a 4-6 membered nitrogen-containing heterocyclyl group and a 4-6 membered nitrogen-containing heterocyclyl group substituted with 1-3 Rₐ₂;
Cy4 is selected from a 3-8 membered heterocyclyl group, a 3-8 membered heterocyclyl group substituted with 1-3 Rₐ₄, a 6-13 membered bicyclic spiro heterocyclyl group and a 6-13 membered bicyclic spiro heterocyclyl group substituted with 1-3 Rₐ₄, wherein the heteroatom of the 3-8 membered heterocyclyl group and the 6-13 membered bicyclic spiro heterocyclyl group is selected from O, N and S;
each Rₐ₂ and Rₐ₄ at each occurrence are independently selected from hydrogen, C₁₋₆ alkyl, and a 3-8 membered heterocyclyl group, wherein the C₁₋₆ alkyl and 3-8 membered heterocyclyl group are unsubstituted or substituted with one or more groups selected from C₁₋₆ alkyl and a 3-8 membered heterocyclyl group;
X₁ is N or CR_{b1};
X₂ is N or CR_{b2};
X₄ is N or CR_{b4};
X₅ is N or CR_{b5};
R_{b1}, R_{b2}, R_{b3}, R_{b4} and R_{b5} are independently selected from hydrogen and halogen;
Cy5 is selected from a 6-13 membered bicyclic fused cycloalkenyl group, a 6-13 membered bicyclic fused heteroaryl group, and an 8-21 membered polycyclic fused heterocyclyl group, wherein the 6-13 membered bicyclic fused cycloalkenyl group, 6-13 membered bicyclic fused heteroaryl group, and 8-21 membered polycyclic fused heterocyclyl group are unsubstituted or optionally substituted with 1-3 Rₐ₅; and
each Rₐ₅ is independently selected from hydrogen, hydroxy, C₁₋₆ alkyl, C₁₋₆ alkoxy, halogenated C₁₋₆ alkyl, and phenyl, wherein the phenyl is unsubstituted or substituted with one or more groups selected from halogen, halogenated C₁₋₆ alkyl, and C₁₋₆ alkoxy.

**Another embodiment of the present invention relates to a compound represented by formula (i), or an isomer or pharmaceutically acceptable salt thereof, having a structure represented by formula (iv):** 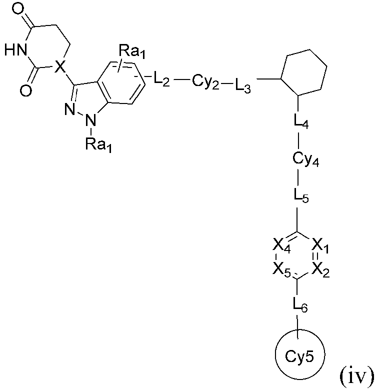
X is selected from N and CH;
each Rₐ₁ is independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
L2, L3, L4, L5, and L6 are independently selected from a bond, -(CH₂)ₙ-(NH)ₘ-, -O-, and - C(O)-;
n and m are independently 0, 1 or 2;
Cy2 is selected from a 4-6 membered nitrogen-containing heterocyclyl group and a 4-6 membered nitrogen-containing heterocyclyl group substituted with 1-3 Rₐ₂;
Cy4 is selected from a 3-8 membered heterocyclyl group, a 3-8 membered heterocyclyl group substituted with 1-3 Rₐ₄, a 6-13 membered bicyclic spiro heterocyclyl group and a 6-13 membered bicyclic spiro heterocyclyl group substituted with 1-3 Rₐ₄, wherein the heteroatom of the 3-8 membered heterocyclyl group and the 6-13 membered bicyclic spiro heterocyclyl group is selected from O, N and S;
each Rₐ₂ and Rₐ₄ at each occurrence are independently selected from hydrogen, C₁₋₆ alkyl, and a 3-8 membered heterocyclyl group, wherein the C₁₋₆ alkyl and 3-8 membered heterocyclyl group are unsubstituted or substituted with one or more groups selected from C₁₋₆ alkyl and a 3-8 membered heterocyclyl group;
X₁ is N or CR_{b1};
X₂ is N or CR_{b2};
X₄ is N or CR_{b4};
X₅ is N or CR_{b5};
R_{b1}, R_{b2}, R_{b3}, R_{b4} and R_{b5} are independently selected from hydrogen and halogen;
Cy5 is selected from a 6-13 membered bicyclic fused cycloalkenyl group, a 6-13 membered bicyclic fused heteroaryl group, and an 8-21 membered polycyclic fused heterocyclyl group, wherein the 6-13 membered bicyclic fused cycloalkenyl group, 6-13 membered bicyclic fused heteroaryl group, and 8-21 membered polycyclic fused heterocyclyl group are unsubstituted or optionally substituted with 1-3 Rₐ₅; and
each Rₐ₅ is independently selected from hydrogen, hydroxy, C₁₋₆ alkyl, C₁₋₆ alkoxy, halogenated C₁₋₆ alkyl, and phenyl, wherein the phenyl is unsubstituted or substituted with one or more groups selected from halogen, halogenated C₁₋₆ alkyl, and C₁₋₆ alkoxy.

**Another embodiment of the present invention relates to a compound represented by formula (i), or an isomer or pharmaceutically acceptable salt thereof, having a structure represented by formula (v):** 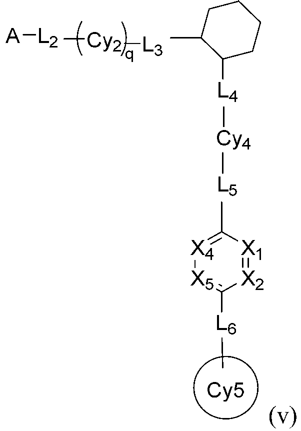
A is 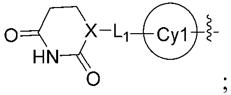
X is selected from N and CH;
Cy1 is selected from 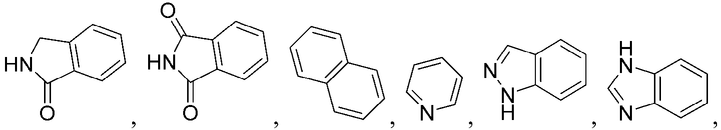 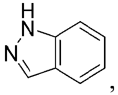 and phenyl, wherein the 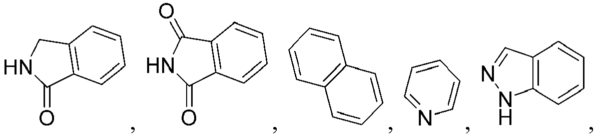 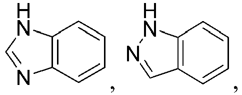 and phenyl are unsubstituted or each independently substituted with 1-3 Rₐ₁;
each Rₐ₁ is independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
L1, L2, L3, L4, L5 and L6 are independently selected from a bond, -(CH₂)ₙ-(NH)ₘ-, -O-, -S-, -NR₂, -CR₃₁R₃₂-, -C(O)-, -C(O)O-, -C(O)NR₄-, -NR₅-C(O)-, C₂₋₈ alkenyl and C₂₋₈ alkynyl;
n and m are independently any integer from zero to six;
R₂, R₃₁, R₃₂, R₄ and R₅ are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
Cy2 is selected from a 4-6 membered cycloalkyl group, a 4-6 membered heterocyclyl group, a 4-6 membered cycloalkenyl group, a 4-6 membered cycloalkyl group substituted with 1-3 Rₐ₂, a 4-6 membered heterocyclyl group substituted with 1-3 Rₐ₂, and a 4-6 membered cycloalkenyl group substituted with 1-3 Rₐ₂, wherein the heteroatom of the 4-6 membered heterocyclyl group is selected from O, N and S;
Cy4 is selected from a 3-8 membered cycloalkyl group, a 3-8 membered heterocyclyl group, a 3-8 membered cycloalkenyl group, a 6-13 membered bicyclic condensed cycloalkyl group, a 6-13 membered bicyclic condensed cycloalkenyl group, a 6-13 membered bicyclic condensed heterocyclyl group, a 3-8 membered cycloalkyl group substituted with 1-3 Rₐ₄, a 3-8 membered heterocyclyl group substituted with 1-3 Rₐ₄, a 3-8 membered cycloalkenyl group substituted with 1-3 Rₐ₄, a 6-13 bicyclic condensed cycloalkyl group substituted with 1-3 Rₐ₄, a 6-13 membered bicyclic condensed cycloalkenyl group substituted with 1-3 Rₐ₄, and a 6-13 membered bicyclic condensed heterocyclyl group substituted with 1-3 Rₐ₄, wherein the heteroatom of the 3-8 membered heterocyclyl group or the 6-13 membered bicyclic condensed heterocyclyl group is selected from O, N and S;
each Rₐ₂ and Rₐ₄ at each occurrence are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, a 3-8 membered cycloalkyl group, a 3-8 membered heterocyclyl group, and a 3-8 membered cycloalkenyl group, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, 3-8 membered cycloalkyl group, 3-8 membered heterocyclyl group, and 3-8 membered cycloalkenyl group are unsubstituted or optionally substituted with one or more groups selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, halogenated C₁₋₆ alkoxy, a 3-8 membered cycloalkyl group, a 3-8 membered heterocyclyl group, and a 3-8 membered cycloalkenyl group;
X₁ is N or CR_{b1};
X₂ is N or CR_{b2};
X₄ is N or CR_{b4};
X₅ is N or CR_{b5};
R_{b1}, R_{b2}, R_{b3}, R_{b4} and R_{b5} are independently selected from hydrogen, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
Cy5 is selected from a 6-13 membered bicyclic condensed group and an 8-21 membered polycyclic condensed group; wherein the 6-13 membered bicyclic condensed group and the 8-21 membered polycyclic condensed group are unsubstituted or substituted with 1-3 Rₐ₅; and
each Rₐ₅ is independently selected from hydroxy, amino, carboxyl, cyano, nitro, halogen, C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, a 4-6 membered heterocyclyl group, a 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, a 4-6 membered heterocyclylcarbonyl group and a 5-6 membered heteroaryl-oxy group, wherein the C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, 4-6 membered heterocyclyl group, 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, 4-6 membered heterocyclylcarbonyl group and 5-6 membered heteroaryl-oxy group are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy.

**The compound of any embodiment of the present invention, or the isomer or pharmaceutically acceptable salt thereof,**
Cy1 is selected from 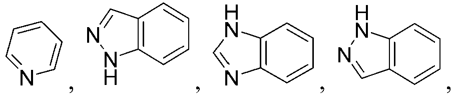 and phenyl, wherein the 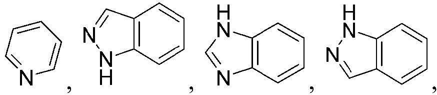 and phenyl are unsubstituted or each independently substituted with 1-3 Rₐ₁.

**The compound of any embodiment of the present invention, or the isomer or pharmaceutically acceptable salt thereof,**
Cy1 is 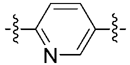 that is unsubstituted or substituted with 1-3 Rₐ₁.

**The compound of any embodiment of the present invention, or the isomer or pharmaceutically acceptable salt thereof,**
Cy1 is 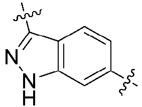 that is unsubstituted or substituted with 1-3 Rₐ₁.

**The compound of any embodiment of the present invention, or the isomer or pharmaceutically acceptable salt thereof,**
Cy1 is 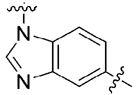 that is unsubstituted or substituted with 1-3 Rₐ₁.

**The compound of any embodiment of the present invention, or the isomer or pharmaceutically acceptable salt thereof,**
Cy1 is 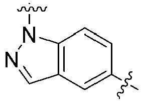 that is unsubstituted or substituted with 1-3 Rₐ₁.

**The compound of any embodiment of the present invention, or the isomer or pharmaceutically acceptable salt thereof,**
Cy1 is 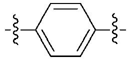 that is unsubstituted or substituted with 1-3 Rₐ₁.

In another embodiment of the present invention, the compound represented by formula (i) has the structure shown below: 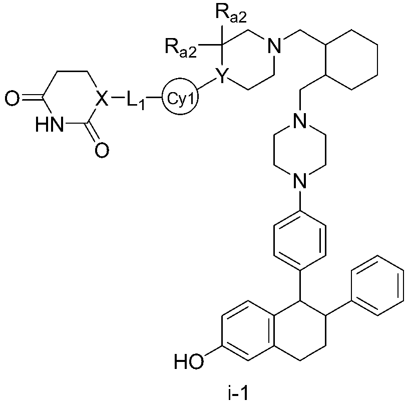 wherein Y is selected from N and CRₐ₂; X, L₁, Cy1, and Rₐ₂ each have the definition described above.

In another embodiment of the present invention, the compound represented by formula (i-1) has the structure shown below: 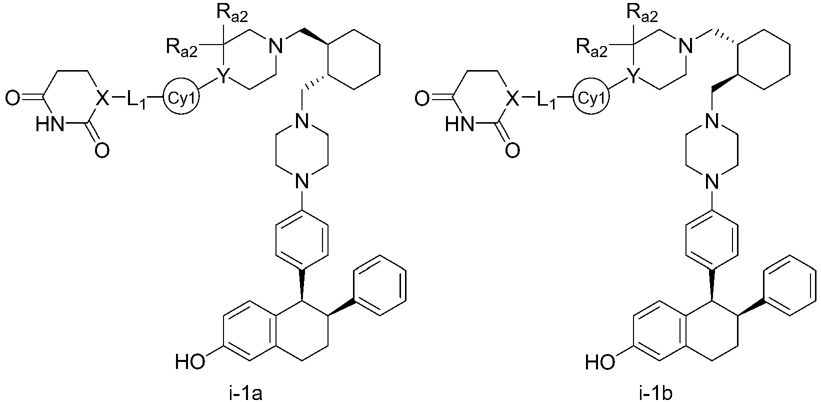 wherein Y is selected from N and CRₐ₂; X, L₁, Cy1, and Rₐ₂ each have the definition described above.

In another embodiment of the present invention, the compound represented by formula (i) has the structure shown below: 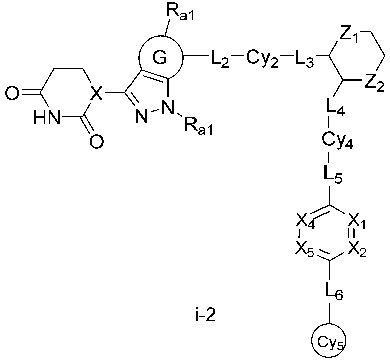 wherein Z₁ and Z₂ are the same or different and are independently selected from C(Rₐ₃)(Rₐ₃), O, N(Rₐ₃), S, and Si(Rₐ₃)(Rₐ₃); wherein X, G, L2, L₃, L4, L₅, Cy2, Cy4, Cy5, Rₐ₁, Rₐ₃₃, X₁, X2, X4, and X₅ each have the definition described above.

In another embodiment of the present invention, the compound represented by formula (i-2) has the structure shown below: 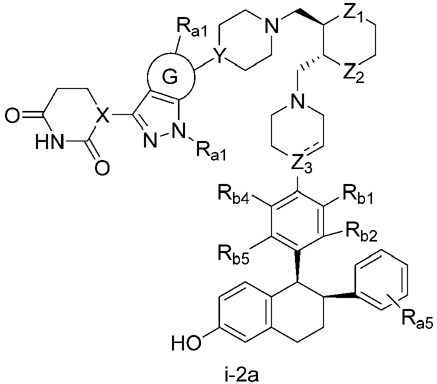 wherein Z₃ is selected from N, C, and CRₐ₄; 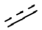 indicates a single or double bond; G, Y, Z₁, Z₂, R_{b1}, R_{b2}, R_{b4}, R_{b5}, Rₐ₁, and Rₐ₅ each have the definition described above.

In some embodiments of the present invention, the compounds of formula (i), or the isomers or pharmaceutically acceptable salts thereof as described above are shown in the following tables:

**Table 1**

| **Serial No.** | **Structure** | **Serial No.** | **Structure** |
|---|---|---|---|
| I-1 | 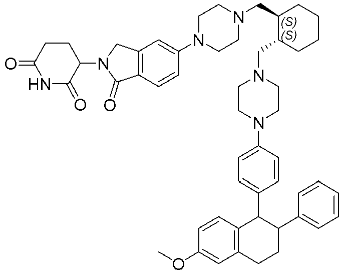 | I-2 | 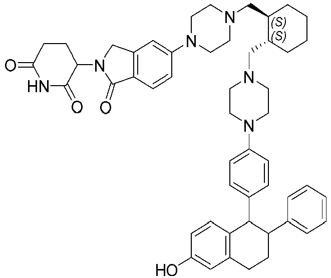 |
| I-3 | 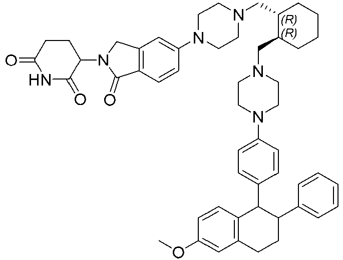 | I-4 | 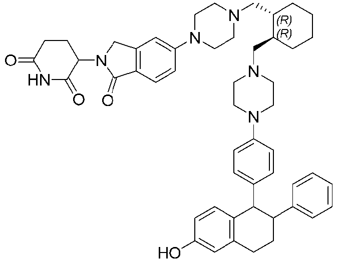 |
| I-5 | 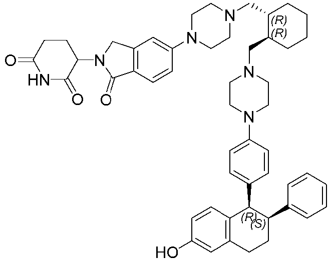 | I-6 | 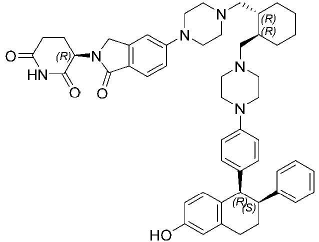 |
| | | I-7 | 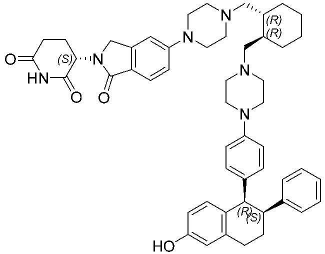 |
| I-8 | 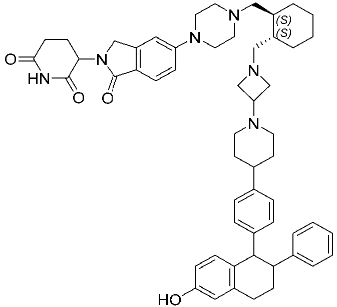 | I-9 | 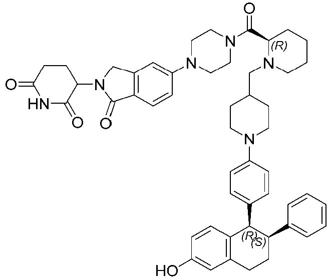 |
| I-11 | 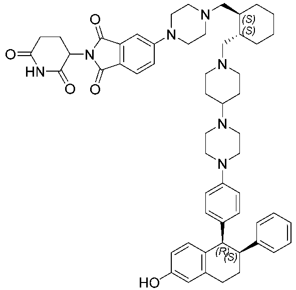 | I-12 | 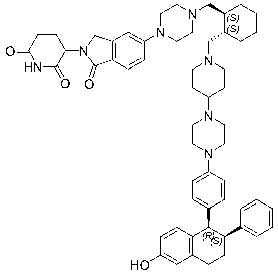 |
| I-13 | 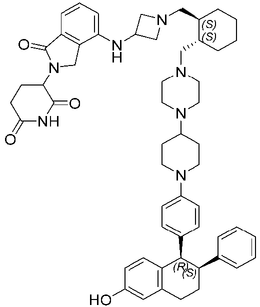 | I-14 | 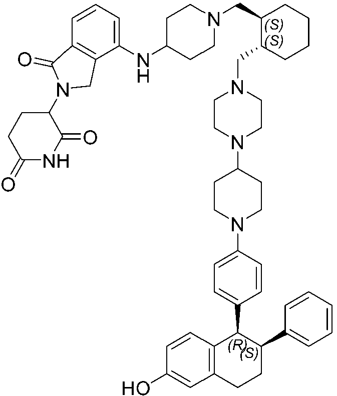 |
| I-15 | 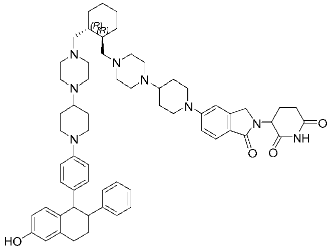 | I-16 | 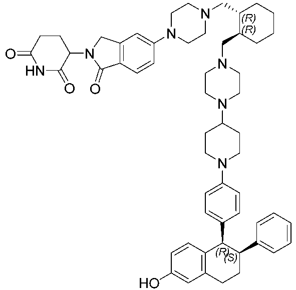 |
| I-17 | 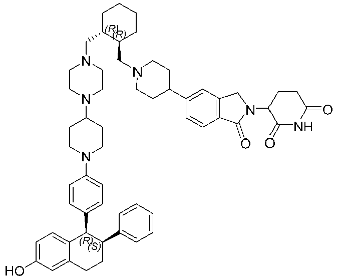 | I-18 | 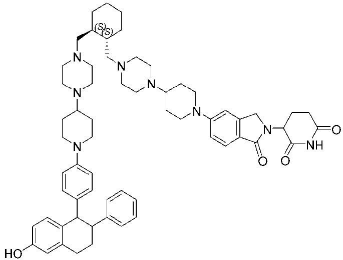 |
| I-19 | 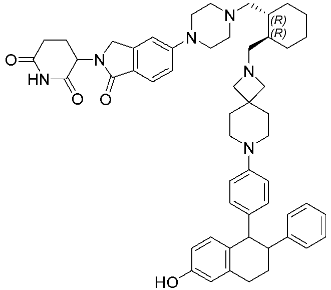 | I-20 | 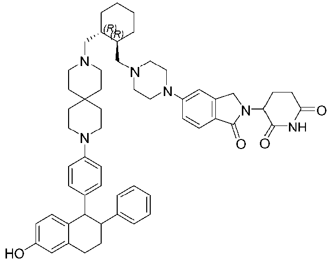 |
| I-21 | 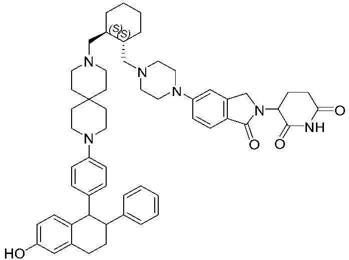 | I-22 | 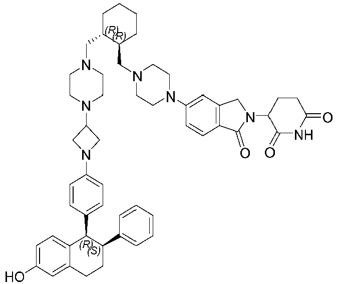 |
| I-23 | 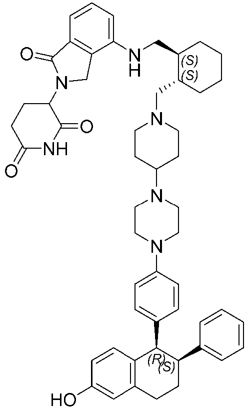 | I-24 | 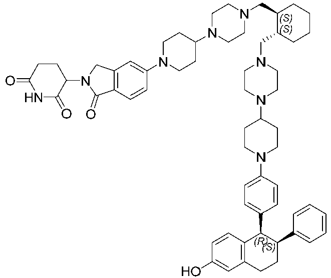 |
| I-25 | 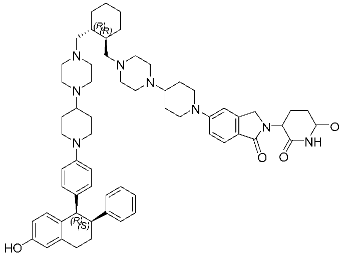 | I-26 | 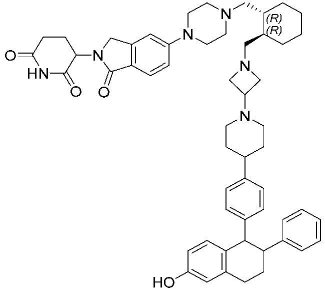 |
| I-27 | 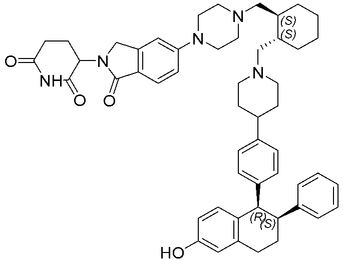 | I-28 | 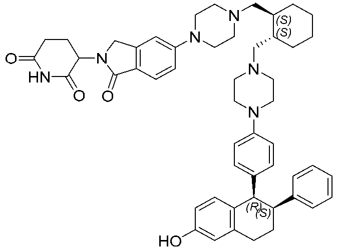 |
| I-29 | 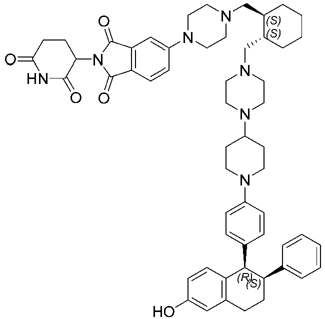 | I-30 | 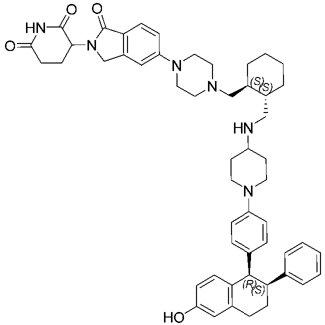 |
| I-31 | 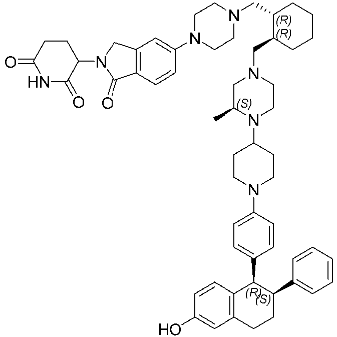 | I-32 | 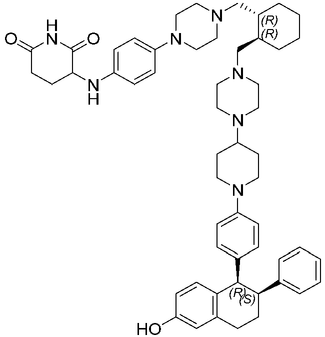 |
| I-33 | 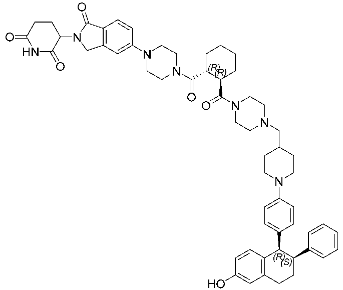 | I-34 | 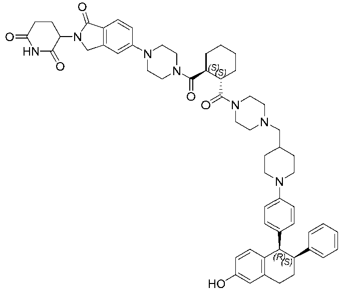 |
| I-35 | 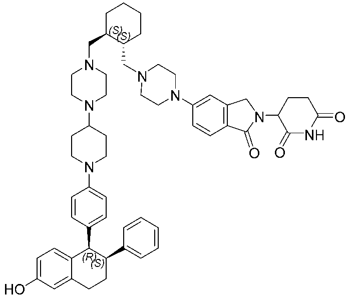 | I-36 | 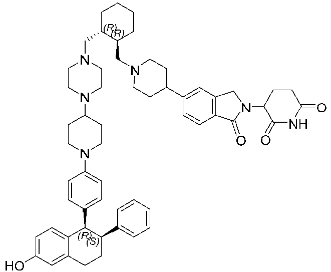 |
| I-37 | 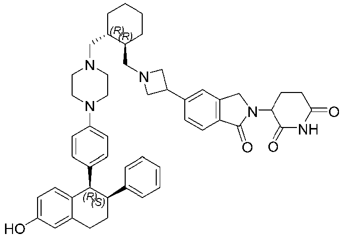 | I-38 | 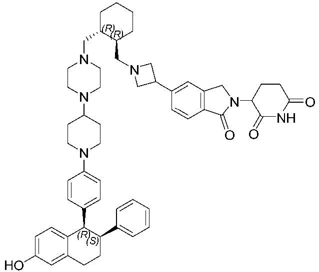 |
| I-39 | 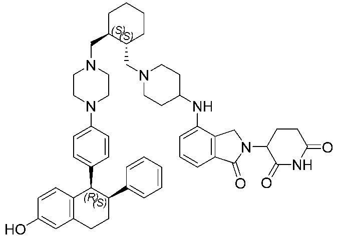 | I-40 | 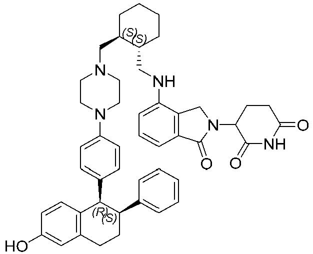 |
| I-41 | 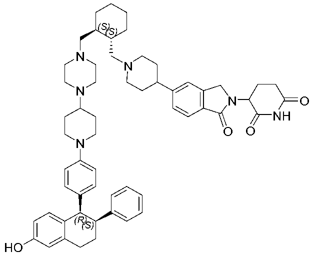 | I-42 | 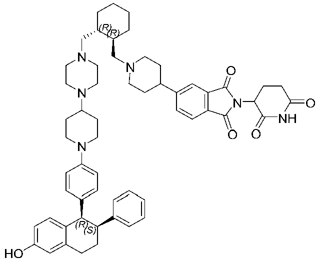 |
| I-43 | 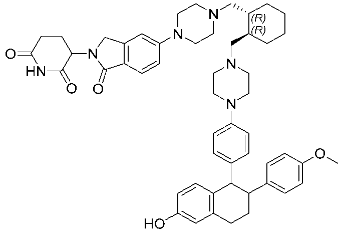 | I-44 | 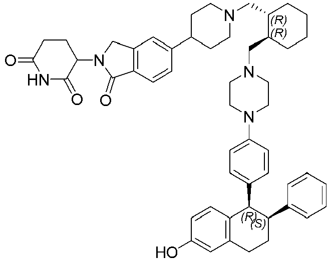 |
| I-45 | 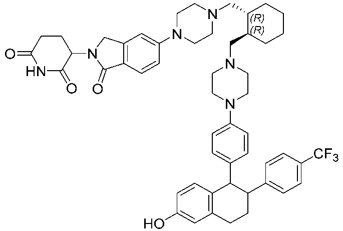 | I-46 | 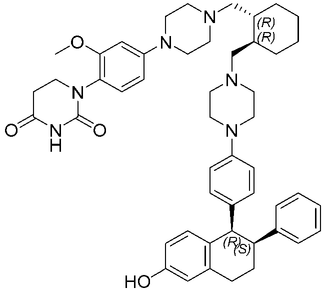 |
| I-47 | 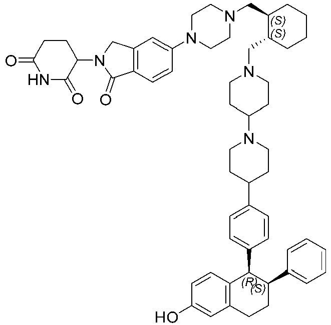 | I-48 | 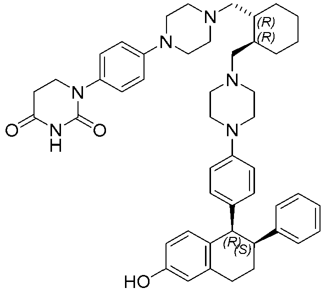 |
| I-49 | 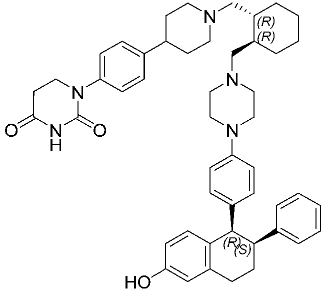 | I-50 | 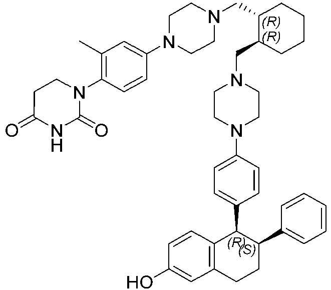 |
| I-51 | 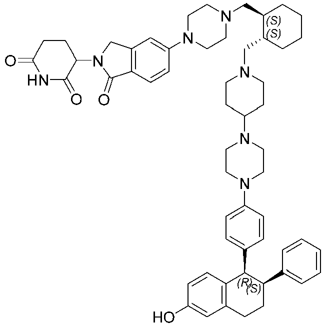 | I-52 | 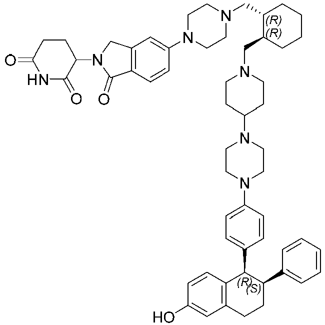 |
| I-53 | 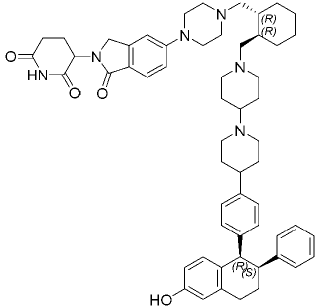 | I-54 | 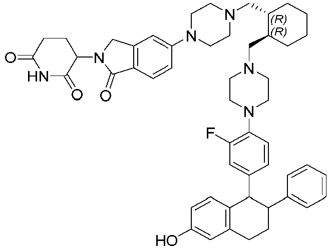 |
| I-55 | 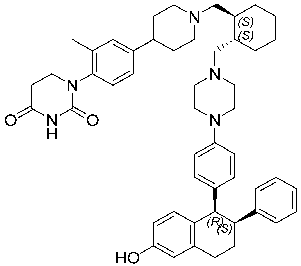 | I-56 | 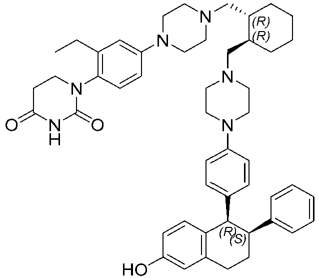 |
| I-57 | 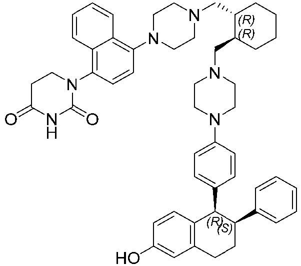 | I-58 | 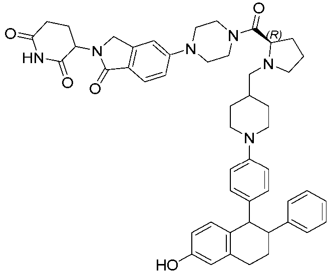 |
| I-59 | 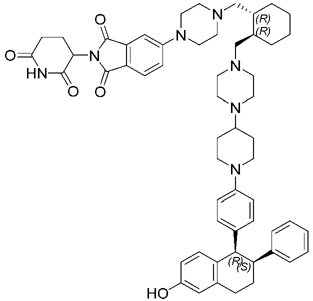 | I-60 | 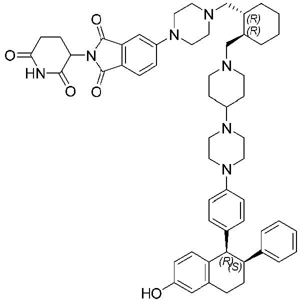 |
| I-61 | 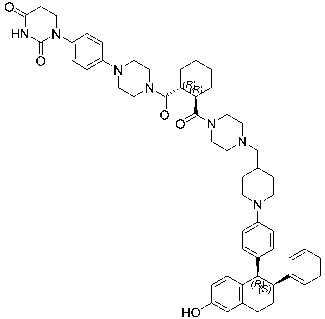 | I-62 | 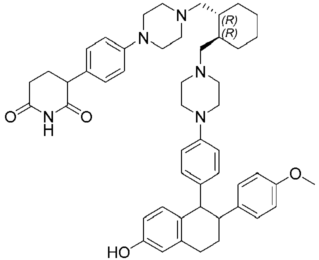 |
| I-63 | 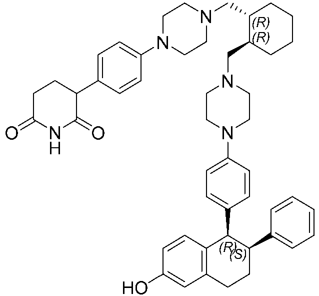 | I-64 | 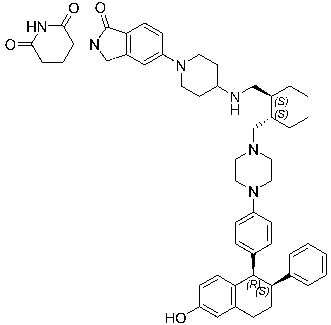 |
| I-65 | 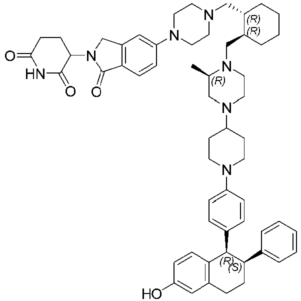 | I-66 | 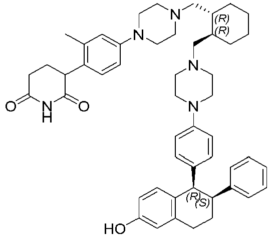 |
| I-67 | 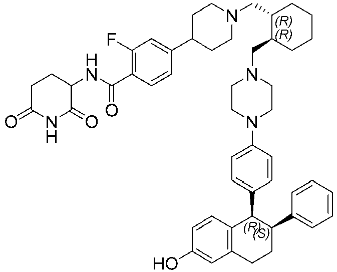 | I-68 | 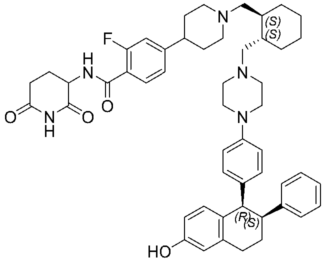 |
| I-69 | 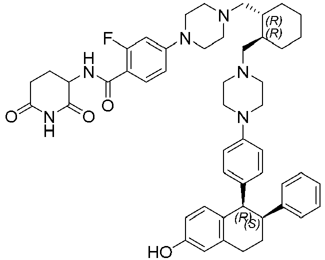 | | |

**Table 2**

| Serial No. | Structure | Serial No. | Structure |
|---|---|---|---|
| II-1 | 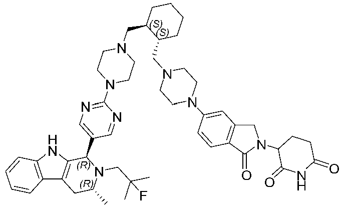 | II-2 | 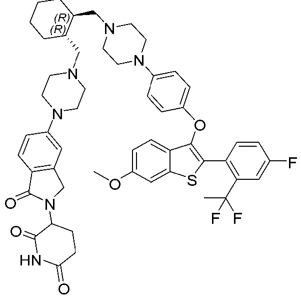 |
| II-3 | 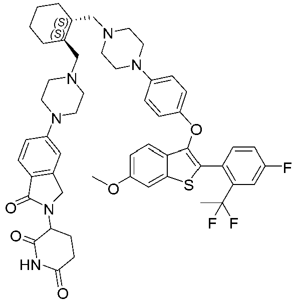 | II-4 | 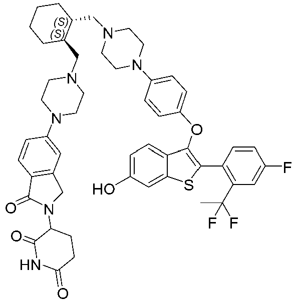 |
| II-5 | 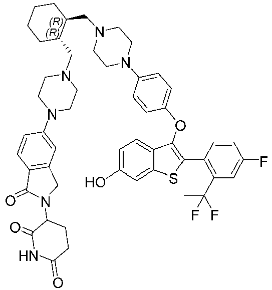 | II-6 | 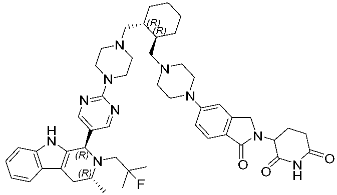 |
| II-7 | 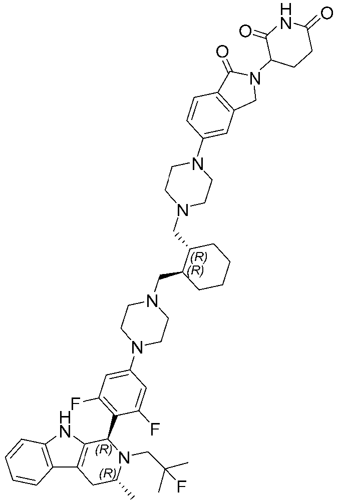 | II-8 | 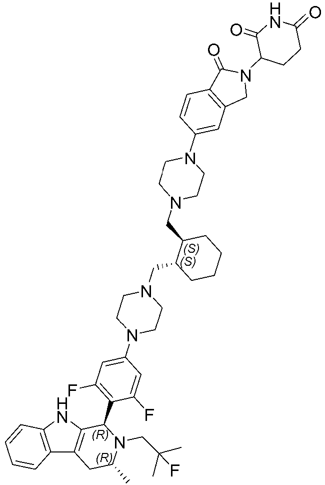 |
| II-9 | 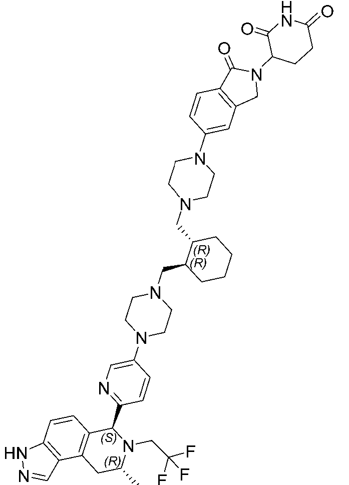 | II-10 | 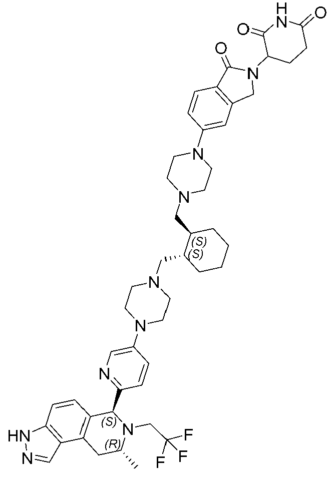 |
| II-11 | 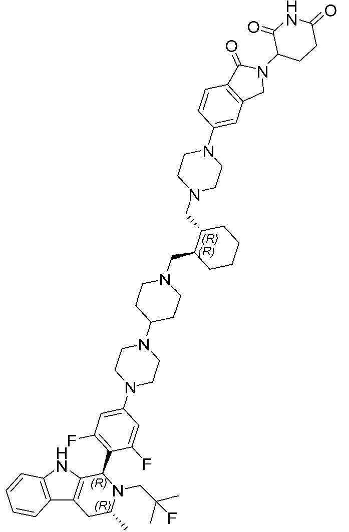 | II-12 | 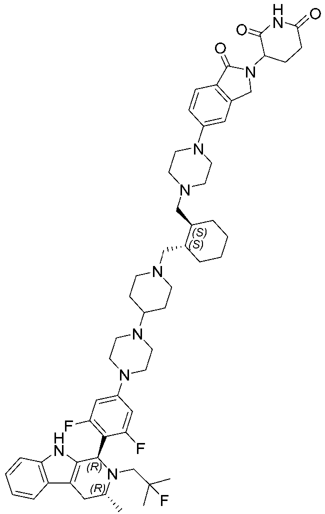 |
| II-13 | 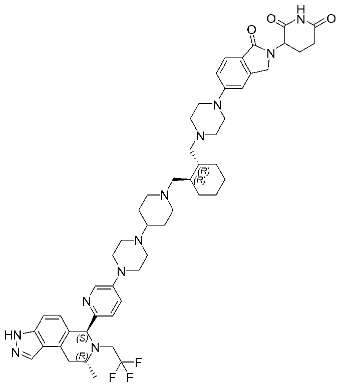 | II-14 | 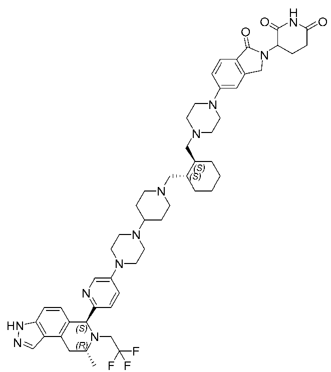 |

**Table 3**

| **Serial No.** | **Structure** | **Serial No.** | **Structure** |
|---|---|---|---|
| III-1 | 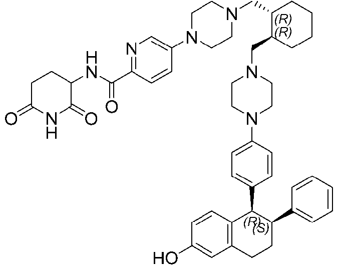 | III-2 | 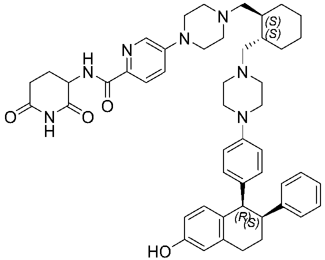 |
| III-3 | 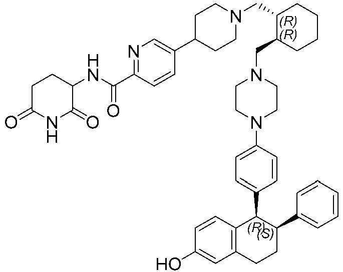 | III-4 | 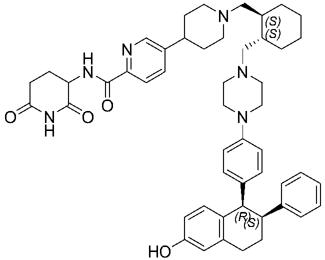 |

**Table 4**

| **Serial No.** | **Structure** | **Serial No.** | **Structure** |
|---|---|---|---|
| IV-1 | 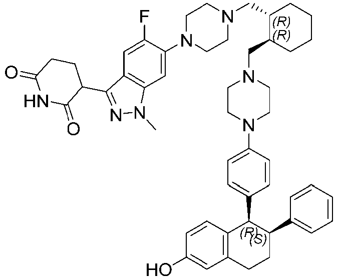 | IV-2 | 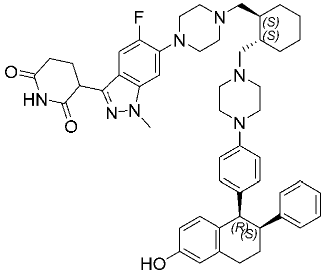 |
| IV-3 | 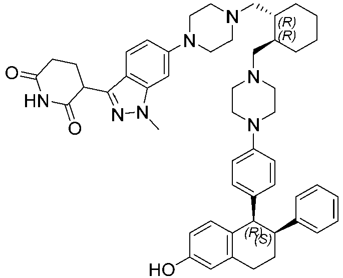 | IV-4 | 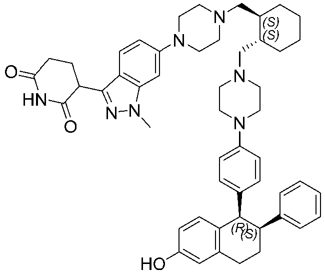 |
| IV-5 | 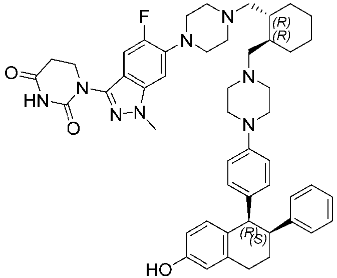 | IV-6 | 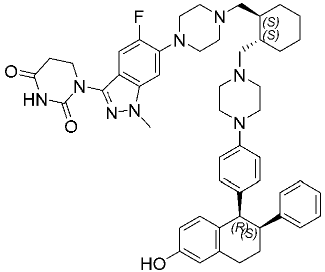 |
| IV-7 | 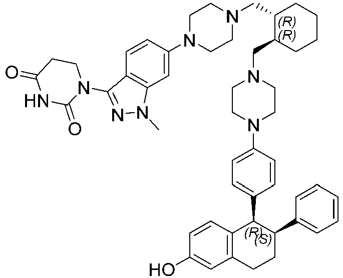 | IV-8 | 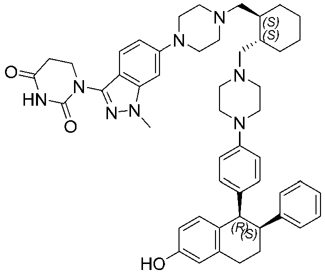 |
| IV-9 | 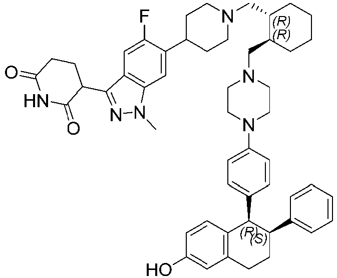 | IV-10 | 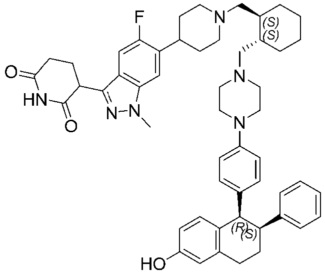 |
| IV-11 | 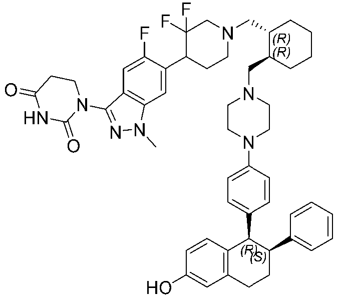 | IV-12 | 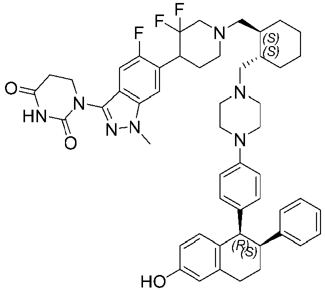 |
| IV-13 | 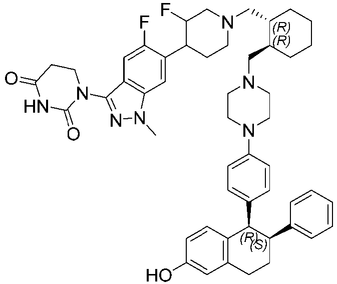 | IV-14 | 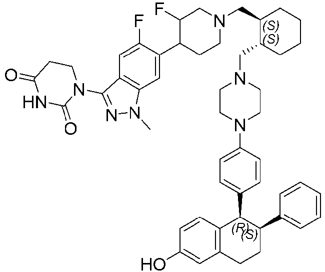 |
| IV-15 | 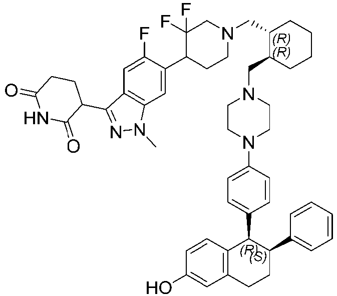 | IV-16 | 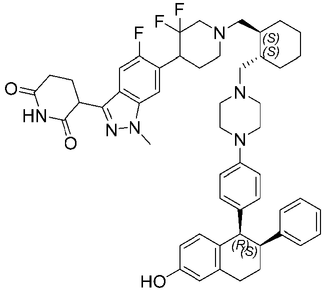 |
| IV-17 | 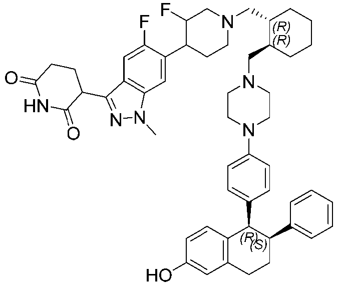 | IV-18 | 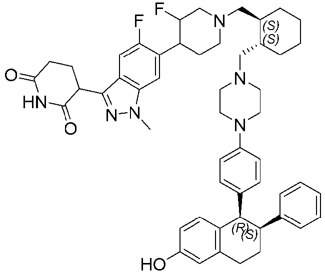 |

**Table 5**

| **Serial No.** | **Structure** | **Serial No.** | **Structure** |
|---|---|---|---|
| V-1 | 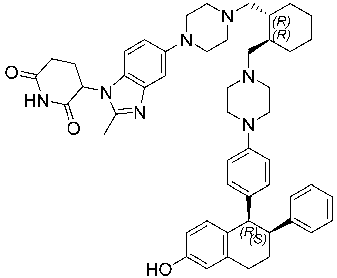 | V-2 | 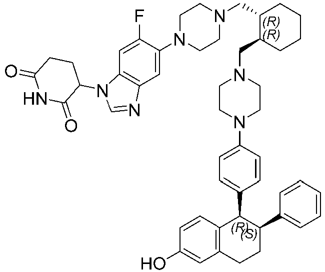 |
| V-3 | 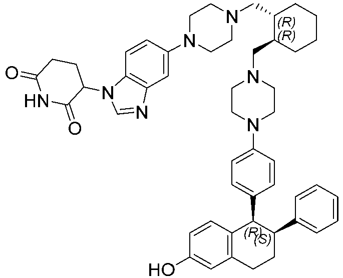 | V-4 | 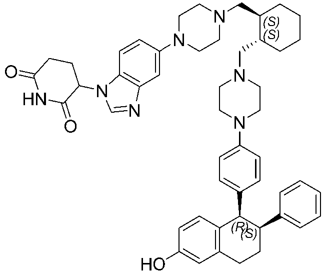 |
| V-5 | 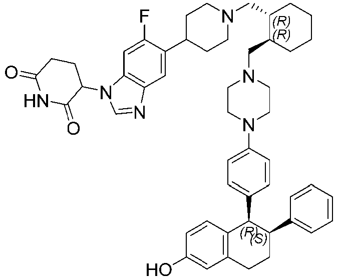 | V-6 | 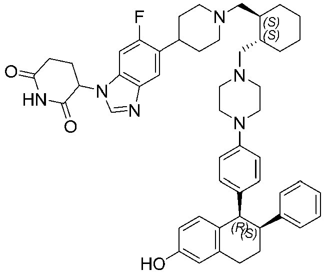 |
| V-7 | 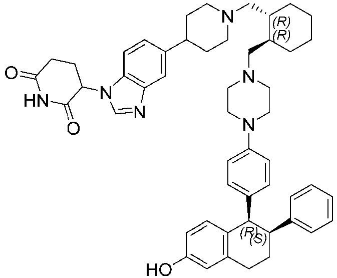 | V-8 | 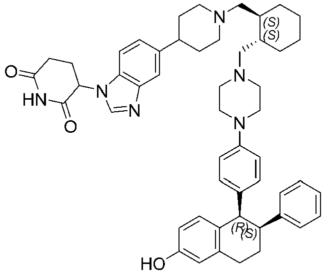 |
| V-9 | 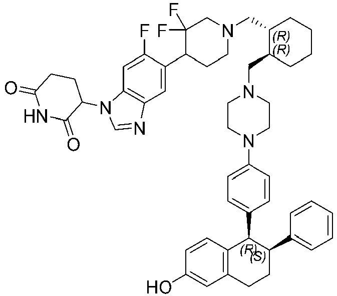 | V-10 | 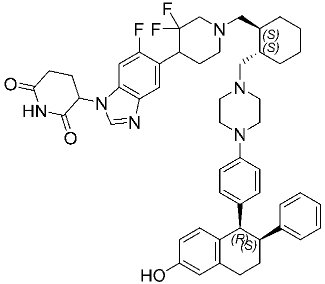 |
| V-11 | 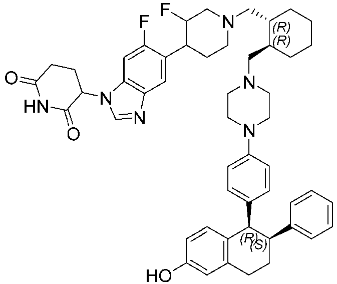 | V-12 | 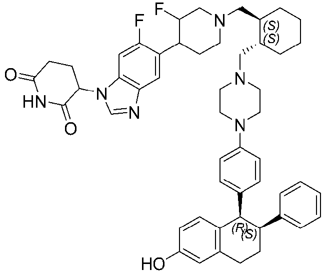 |

**Table 6**

| **Serial No.** | **Structure** | **Serial No.** | **Structure** |
|---|---|---|---|
| VI-1 | 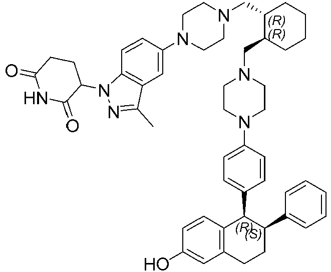 | VI-2 | 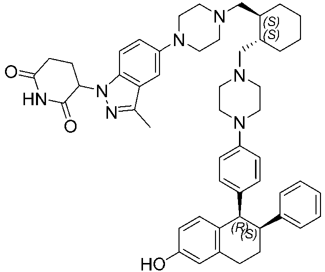 |
| VI-3 | 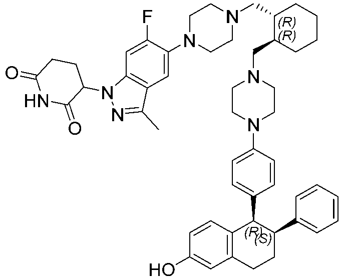 | VI-4 | 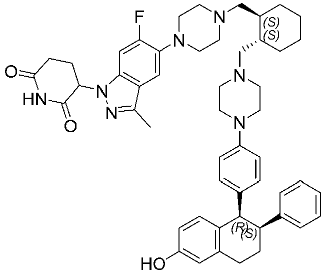 |
| VI-5 | 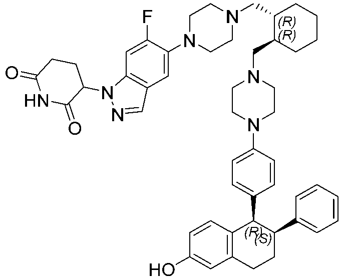 | VI-6 | 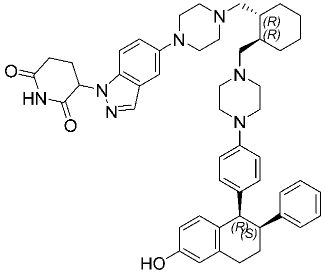 |
| VI-7 | 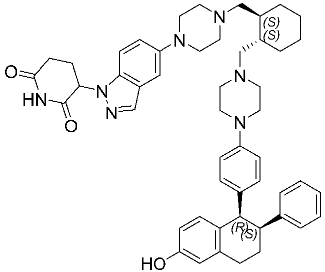 | VI-8 | 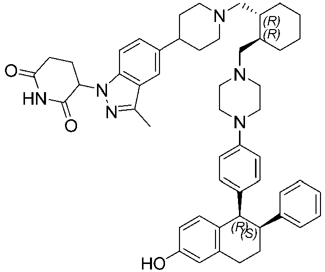 |
| VI-9 | 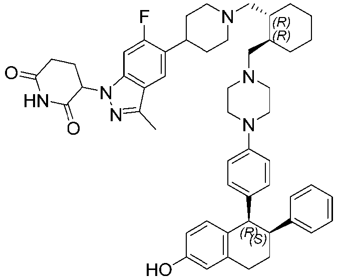 | VI-10 | 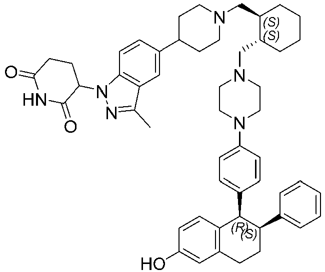 |
| VI-11 | 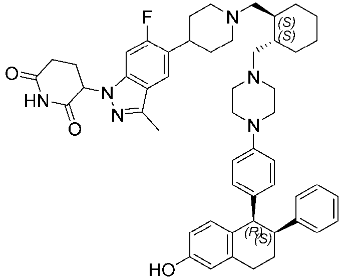 | VI-12 | 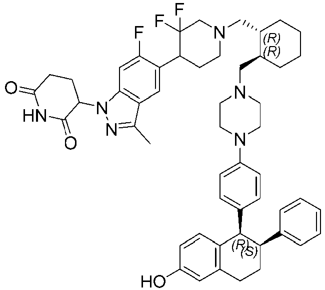 |
| VI-13 | 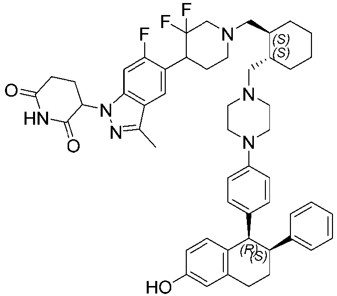 | VI-14 | 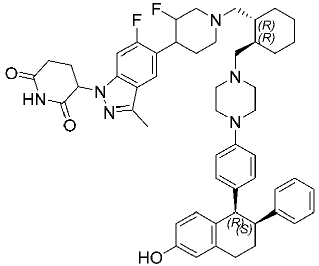 |
| VI-15 | 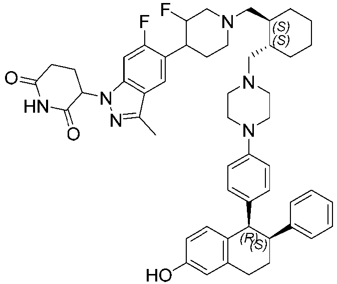 | VI-16 | 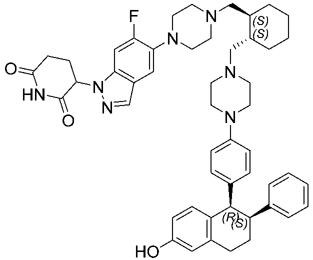 |
| VI-17 | 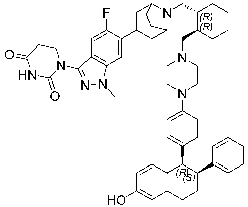 | VI-18 | 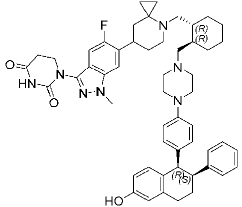 |
| VI-19 | 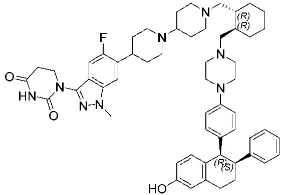 | VI-20 | 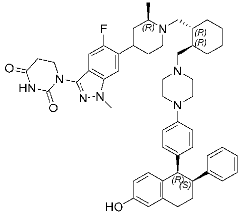 |
| VI-21 | 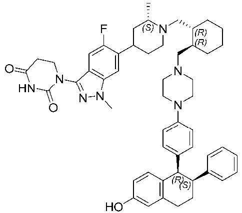 | VI-22 | 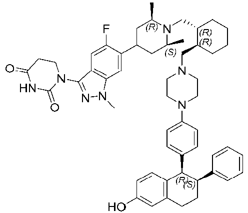 |
| VI-23 | 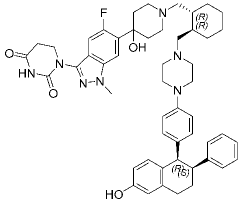 | VI-24 | 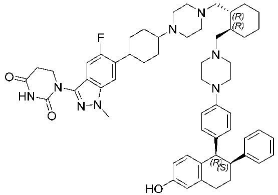 |
| VI-25 | 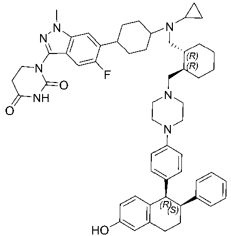 | VI-26 | 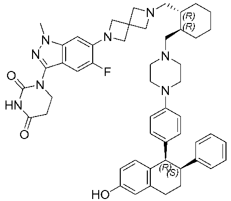 |
| VI-27 | 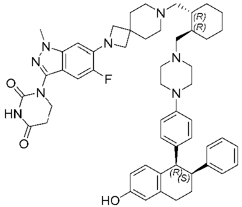 | VI-28 | 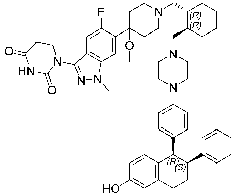 |
| VI-29 | 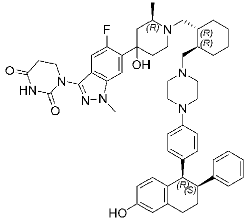 | VI-30 | 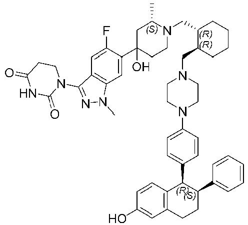 |
| VI-31 | 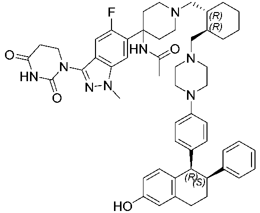 | VI-32 | 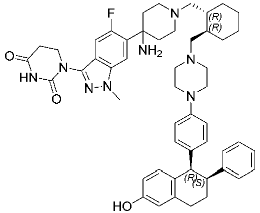 |
| VI-33 | 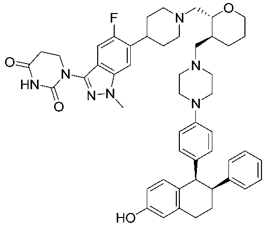 | VI-34 | 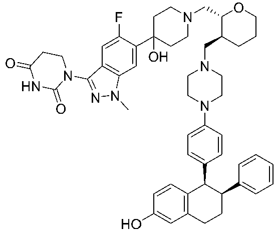 |
| VI-35 | 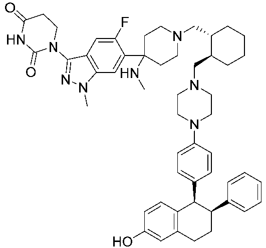 | VI-36 | 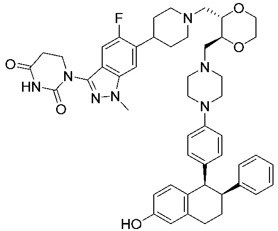 |
| VI-37 | 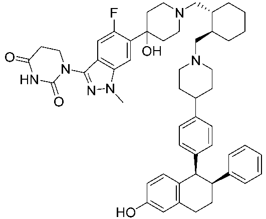 | VI-38 | 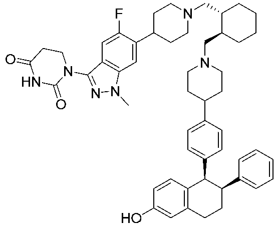 |
| VI-39 | 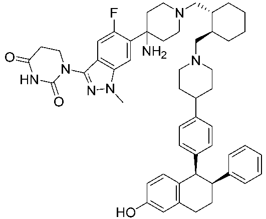 | VI-40 | 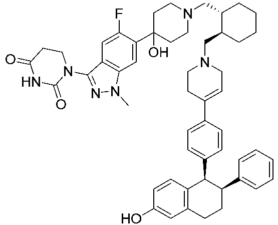 |
| VI-41 | 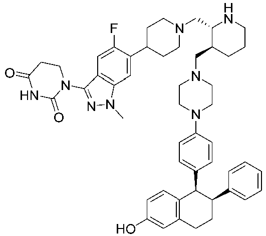 | VI-42 | 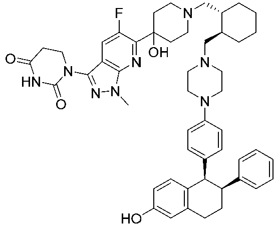 |
| VI-43 | 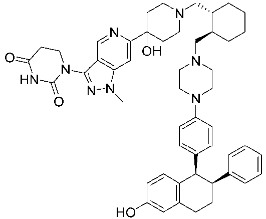 | VI-44 | 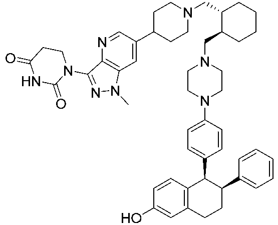 |
| VI-45 | 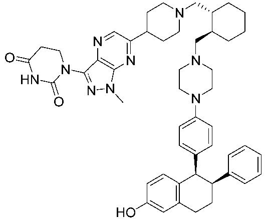 | | |

**Table 7**

| **Serial No.** | **Structure** | **Serial No.** | **Structure** |
|---|---|---|---|
| VII-1 | 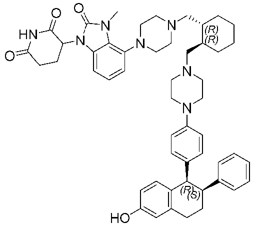 | VII-2 | 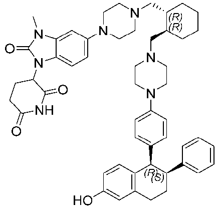 |
| VII-3 | 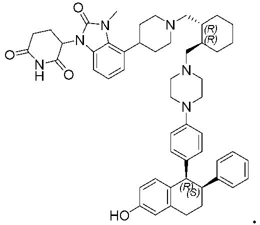 | | |

The present invention also provides a pharmaceutical composition, comprising the compound of formula (i), or the isomer or pharmaceutically acceptable salt thereof, and one or more pharmaceutically acceptable carriers, diluents or excipients.

The present invention also provides use of the compound of formula (i), or the isomer or pharmaceutically acceptable salt thereof, or the pharmaceutical composition in the preparation of a medicament for treating and/or preventing a disease or condition by degrading a target protein.

According to an embodiment of the present invention, the disease or condition is selected from abnormal cell proliferation, tumors, immune diseases, diabetes, cardiovascular diseases, infectious diseases and inflammatory diseases; and preferably tumors and infectious diseases.

According to an embodiment of the present invention, the tumor is cancer; preferably selected from breast cancer, endometrial cancer, testicular cancer, cervical cancer, prostate cancer, ovarian cancer, fallopian tube tumors, leukemia, skin cancer, squamous cell carcinoma, basal cell carcinoma, bladder cancer, colorectal cancer, esophageal cancer, head and neck cancer, kidney cancer, liver cancer, lung cancer, pancreatic cancer, gastric cancer, lymphoma, melanoma, sarcoma, peripheral neuroepithelioma, glioma, astrocytoma, ependymoma, glioblastoma, neuroblastoma, gangliocytoma, medulloblastoma, pineal cell tumor, meningioma, neurofibroma, neurilemoma, thyroid cancer, Wilms' tumor and teratoma; and more preferably selected from breast cancer, endometrial cancer, testicular cancer, cervical cancer, prostate cancer, ovarian cancer and fallopian tube tumors.

According to an embodiment of the present invention, the infectious disease is selected from viral pneumonia, influenza, avian influenza, meningitis, gonorrhea, or a disease from infection with HIV, HBV, HCV, HSV, HPV, RSV, CMV, Ebola virus, flavivirus, trace virus, rotavirus, coronavirus, EBV, drug-resistant virus, RNA virus, DNA virus, adenovirus, poxvirus, picornavirus, togavirus, orthomyxovirus, retrovirus, hepadnavirus, Gram-negative bacteria, Gram-positive bacteria, atypical bacteria, Staphylococcus, Streptococcus, Escherichia coli, Salmonella, Helicobacter pylori, Chlamydiaceae, Mycoplasmaceae, fungi, protozoa, intestinal worms, worms, prions or parasites.

The present invention also provides use of the compound of formula (i), or the isomer or pharmaceutically acceptable salt thereof, or the pharmaceutical composition in the preparation of a medicament for treating and/or preventing an estrogen-receptor mediated or dependent disease or condition, wherein the estrogen-receptor mediated or dependent disease or condition is a tumor, preferably cancer, more preferably selected from breast cancer, endometrial cancer, testicular cancer, cervical cancer, prostate cancer, ovarian cancer and fallopian tube tumor, and most preferably breast cancer.

The present invention also provides a method for treating and/or preventing a disease or condition by degrading a target protein, comprising administering to a patient a therapeutically effective amount of a compound of formula (i), or an isomer or pharmaceutically acceptable salt thereof.

The present invention also provides a method for treating and/or preventing an estrogen-receptor mediated or dependent disease or condition, comprising administering to a patient a therapeutically effective amount of a compound of formula (i), or an isomer or pharmaceutically acceptable salt thereof.

### Beneficial effects:

The present invention provides a compound represented by formula (i), or an isomer or pharmaceutically acceptable salt thereof, which can be used as an estrogen-receptor degrading agent for preparing a medicament for treating and/or preventing an estrogen-receptor mediated or dependent disease or condition.

### Brief Description of the Drawings

FIG. 1 depicts the ER protein degradation of compound IV-9 for MCF-7 cells;
FIG. 2 depicts the ER protein degradation of compound VI-20 for MCF-7 cells;
FIG. 3 depicts the ER protein degradation of compound VI-23 for MCF-7 cells;
FIG. 4 depicts the ER protein degradation of compound VI-24 for MCF-7 cells;
FIG. 5 depicts the ER protein degradation of compound VI-29 for MCF-7 cells; and
FIG. 6 depicts the ER protein degradation of compound VI-28 for MCF-7 cells.

### Definitions and explanation of terms

Unless otherwise indicated, the definitions of groups and terms recited in the specification and claims of this application, including definitions provided as examples, exemplary definitions, preferred definitions, definitions recited in tables, definitions of specific compounds in examples, etc., may be arbitrarily combined and coupled with each other. Such combined and coupled group definitions and compound structures should be understood to be within the scope of the specification and/or claims of this application.

The "halogen" described in the present invention refers to fluorine, chlorine, bromine, iodine, etc., and preferably fluorine and chlorine.

The "halogenated" described in the present invention means that any hydrogen atom in a substituent may be substituted with one or more halogen atoms which may be the same or different. "Halogen" is as defined above.

The "C₁₋₆ alkyl" described in the present invention refers to a straight or branched alkyl derived from a hydrocarbon moiety containing 1-6 carbon atoms by removing a hydrogen atom, such as methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, and 1-methyl-2-methylpropyl. The "C₁₋₆ alkyl" is preferably C₁₋₄ alkyl or C₁₋₃ alkyl.

The "C₂₋₈ alkenyl" described in the present invention refers to a straight or branched or cyclic olefin group derived from an olefin moiety of 2-8 carbon atoms containing a carbon-carbon double bond by removing a hydrogen atom, such as ethenyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, and 1,4-hexadienyl.

The "C₂₋₈ alkynyl" described in the present invention refers to a straight or branched alkyne group derived from an alkyne moiety of 2-8 carbon atoms containing a carbon-carbon triple bond by removing a hydrogen atom, such as ethynyl, propynyl, 2-butynyl, 2-pentynyl, 3-pentynyl, 4-methyl-2-pentynyl, 2-hexynyl, and 3-hexynyl. The "C₂₋₈ alkynyl" is preferably C₂₋₄ alkynyl or C₂₋₃ alkynyl.

The "Ci-₆ alkoxy" described in the present invention refers to a group in which the "C₁₋₆ alkyl" defined above is attached to the parent molecule through an oxygen atom., i.e., a "C₁₋₆ alkyl-O-" group, such as methoxy, ethoxy, n-propoxy, isopropoxy, n-butoxy, tert-butoxy, n-pentoxy, neopentoxy and n-hexoxy. The "C₁₋₆ alkoxy" is preferably C₁₋₄ alkoxy or C₁₋₃ alkoxy.

The "C₁₋₆ alkylamino", "(C₁₋₆ alkyl)₂ amino", "C₁₋₆ alkylcarbonylamino", "C₁₋₆ alkylsulfonylamino", "C₁₋₆ alkylaminocarbonyl, "(C₁₋₆ alkyl)₂ amino-carbonyl", "C₁₋₆ alkoxycarbonyl", "C₁₋₆ alkylsulfonyl", "C₁₋₆ alkylthio, "C₁₋₆ alkylcarbonyl", "aminocarbonyl", and "hydroxy C₁₋₆ alkyl" described in the present invention refer to C₁₋₆ alkyl-NH-, (C₁₋₆ alkyl)(C₁₋₆ alkyl)N-, C₁₋₆ alkyl-C(O)-NH-, C₁₋₆ alkyl-S(O)₂-NH₂-, C₁₋₆ alkyl-NH-C(O)-, (C₁₋₆ alkyl)(C₁₋₆ alkyl)N-C(O)-, C₁₋₆ alkyl-O-C(O)-, C₁₋₆ alkyl-S(O)₂-, C₁₋₆ alkyl-S-, C₁₋₆ alkyl-C(O)-, NH₂-C(O)-, and OH-C₁₋₆ alkyl-, respectively; and the "C₁₋₆ alkyl" is as defined above, preferably "C₁₋₄ alkyl or C₁₋₃ alkyl".

The "condensed ring" described in the present invention refers to a polycyclic structure formed by two or more cyclic structures attached in a fused, spiro or bridged manner. Each of the cyclic structures refers to any monocycloalkyl, monoheterocyclyl, monocycloalkenyl, phenyl, or monoheteroaryl. The fused ring refers to a condensed ring structure formed by two or more cyclic structures sharing two adjacent ring atoms (i.e., sharing one bond). The bridged ring refers to a condensed ring structure formed by two or more cyclic structures sharing two non-adjacent ring atoms. The spiro ring refers to a condensed ring structure formed by two or more cyclic structures sharing one ring atom. Multiple cyclic structures are optionally connected by a fused, spiro or bridged manner.

Two cyclic structures connected by a fused, spiro or bridged connection are bicyclic condensed groups, such as a 6-13 membered bicyclic condensed group. The 6-13 membered bicyclic condensed group further includes a 6-13 membered bicyclic condensed cycloalkyl group, a 6-13 membered bicyclic condensed cycloalkenyl group, a 6-13 membered bicyclic condensed heterocyclyl group, an 8-13 membered bicyclic condensed heteroaryl group, and an 8-13 membered bicyclic condensed aryl group.

More than two ring structures connected by a fused, spiro or bridged connection are polycyclic condensed groups, such as a monocyclic ring structure connected with a 6-13 membered bicyclic condensed group by a fused, spiro or bridged connection, such as an 8-21 membered polycyclic condensed group. The 8-21 membered polycyclic condensed group further includes an 8-21 membered polycyclic condensed cycloalkyl group, an 8-21 membered polycyclic condensed cycloalkenyl group, an 8-21 membered polycyclic condensed heterocyclyl group, an 8-21 membered polycyclic condensed heteroaryl group, and an 8-21 membered polycyclic condensed aryl group.

The 8-21 membered polycyclic condensed heterocyclyl group includes an 8-21 membered polycyclic bridged heterocyclyl group, an 8-21 membered polycyclic fused heterocyclyl group and an 8-21 membered polycyclic spiro heterocyclyl group.

Examples of the 8-21 membered polycyclic fused heterocyclyl group include, but are not limited to

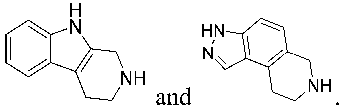

The 3-8 membered monocyclic group described in the present invention includes monocycloalkyl, monoheterocyclyl, monocycloalkenyl, monoheteroaryl and phenyl.

The monocycloalkyl is a cyclic hydrocarbyl group containing 3-13 carbon atoms, including a 3-13 membered cycloalkyl group, a 3-8 membered cycloalkyl group, a 4-7 membered cycloalkyl group, a 4-6 membered cycloalkyl group, and a 5-6 membered cycloalkyl group. Examples of the 3-8 membered cycloalkyl group include, but are not limited to: cyclopropyl, cyclobutyl, cyclopentyl, cyclohexyl, cycloheptyl, cyclooctyl, and the like.

The monoheterocyclyl refers to a non-aromatic cyclic group in which at least one ring carbon atom is replaced by a heteroatom, preferably 1-3 heteroatoms, selected from O, S, and N. The monoheterocyclyl may be saturated or partially saturated, including a 3-13 membered heterocyclyl group, a 3-8 membered heterocyclyl group, a 3-6 membered heterocyclyl group, a 4-6 membered heterocyclyl group, a 4-7 membered heterocyclyl group, a 5-7 membered heterocyclyl group, a 5-6 membered heterocyclyl group, a 4-6 membered nitrogen-containing heterocyclyl group, a 5-6 membered oxygen-containing heterocyclyl group, a 3-8 membered nitrogen-containing heterocyclyl group, a 5-6 membered nitrogen-containing heterocyclyl group, a 5-6 membered saturated heterocyclyl group, and the like. Examples of the "3-8 membered saturated heterocyclyl group" include, but are not limited to, aziridine, oxirane, thiirane, azetidinyl, oxetanyl, thietanyl, tetrahydrofuranyl, pyrrolidinyl, tetrahydrothiophenyl, imidazolidinyl, pyrazolidinyl, 1,2-oxazolidinyl, 1,3-oxazolidinyl, 1,2-thiazolidinyl, 1,3-thiazolidinyl, tetrahydro-2H-pyranyl, tetrahydro-2H-thiopyranyl, piperidinyl, piperazinyl, morpholinyl, 1,4-dioxanyl, and 1,4-oxathianyl; and examples of the "3-8 membered partially saturated heterocyclyl group" include but are not limited to 4,5-dihydroisoxazolyl, 4,5-dihydrooxazolyl, 2,5-dihydrooxazolyl, 2,3-dihydrooxazolyl, 3,4-dihydro-2H-pyrrolyl, 2,3-dihydro-1H-pyrrolyl, 2,5-dihydro-1H-imidazolyl, 4,5-dihydro-1H-imidazolyl, 4,5-dihydro-1H-pyrazolyl, 4,5-dihydro-3H-pyrazolyl,oxazolyl, 4,5-dihydrothiazolyl, 2,5-dihydrothiazolyl, 2H-pyranyl, 4H-pyranyl, 2H-thiopyranyl, 4H-thiopyranyl, 2,3,4,5-tetrahydropyridinyl, 1,2-isoxazinyl, 1,4-isoxazinyl, or 6H-1,3-oxazinyl, and the like.

The monocycloalkenyl refers to a partially saturated carbocyclic group, including a 3-13 membered cycloalkenyl group, a 3-8 membered cycloalkenyl group, a 4-7 membered cycloalkenyl group, and a 5-6 membered cycloalkenyl group.

The heteroaryl described in the present invention refers to an aromatic cyclic group in which at least one ring carbon atom is replaced by a heteroatom selected from O, S, and N. The monoheteroaryl can be a 5-7 membered heteroaryl group or a 5-6 membered heteroaryl group, and examples thereof include, but are not limited to, furanyl, imidazolyl, isoxazolyl, thiazolyl, isothiazolyl, oxadiazolyl, oxazolyl, pyridinyl, pyridazinyl, pyrimidinyl, pyrazinyl, pyrazolyl, pyrrolyl, tetrazolyl, thiadiazolyl, thienyl, triazolyl and triazinyl.

The 6-13 membered bicyclic condensed cycloalkyl group includes a fused cycloalkyl group, a bridged cycloalkyl group, and a spiro cycloalkyl group. It may be saturated, partially saturated, or unsaturated, but is not aromatic. The fused cycloalkyl group may be a 6-11 membered fused cycloalkyl group or a 7-10 membered fused cycloalkyl group, and representative examples thereof include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. The spiro cycloalkyl group may be a 7-12 membered spiro cycloalkyl group, a 7-11 membered spiro cycloalkyl group, or a 10-11 membered spiro cycloalkyl group, and examples thereof include, but are not limited to: 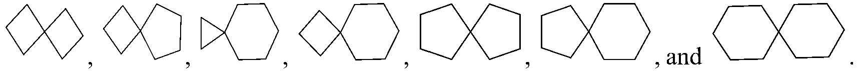 The bridged cycloalkyl group may be a 6-11 membered bridged cycloalkyl group or a 7-10 membered bridged cycloalkyl group, and examples thereof include, but are not limited to:

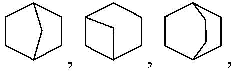

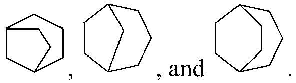

The 6-13 membered bicyclic condensed cycloalkenyl group includes a fused cycloalkenyl group, a bridged cycloalkenyl group, and a spiro cycloalkenyl group. Representative examples of the 6-13 membered bicyclic fused cycloalkenyl group include, but are not limited to

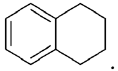

The 6-13 membered bicyclic condensed heterocyclyl group includes a fused heterocyclyl group, a spiro heterocyclyl group, and a bridged heterocyclyl group, and may be saturated, partially saturated, or unsaturated, but is not aromatic. The condensed heterocyclyl group is a 5-6 membered monocyclic heterocyclyl group condensed to a benzene ring, a 5-6 membered monocyclic cycloalkyl group, a 5-6 membered monocyclic cycloalkenyl group, a 5-6 membered monocyclic heterocyclyl group, or a 5-6 membered monocyclic heteroaryl group. The fused heterocyclyl group may be a 6-12 membered fused group, a 7-10 membered fused group, a 6-10 membered fused group, an 8-10 membered bicyclic fused heterocyclyl group, or a 6-12 membered saturated fused group, and representative examples thereof include, but are not limited to: 3-azabicyclo[3.1.0]hexyl, 3,6-diazabicyclo[3.2.0]heptyl, 3,8-diazabicyclo[4.2.0]octyl, 3,7-diazabicyclo[4.2.0]octyl, octahydropyrrolo[3,4-c]pyrrolyl, octahydropyrrolo[3,4-b]pyrrolyl, octahydropyrrolo[3,4-b][1,4]oxazinyl, octahydro-1H-pyrrolo[3,4-c]pyridinyl, 2,3-dihydrobenzofuran-2-yl, 2,3-dihydrobenzofuran-3-yl, indolin-1-yl, indolin-2-yl, indolin-3-yl, 2,3-dihydrobenzothiophen-2-yl, octahydro-1H-indolyl, and octahydrobenzofuranyl. The spiro heterocyclyl group may be a 6-12 membered spiro heterocyclyl group, a 7-11 membered spiro heterocyclyl group, a 6-12 membered saturated spirocyclic group, or a 10-11 membered bicyclic spiro heterocyclyl group, and examples thereof include, but are not limited to:

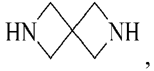

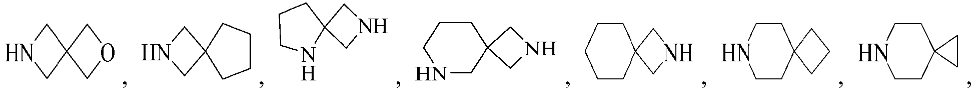

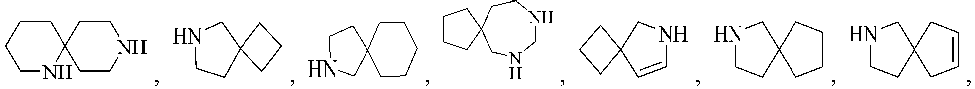

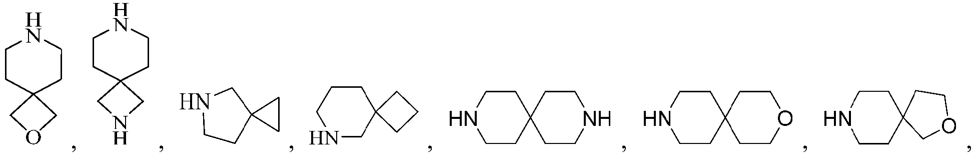

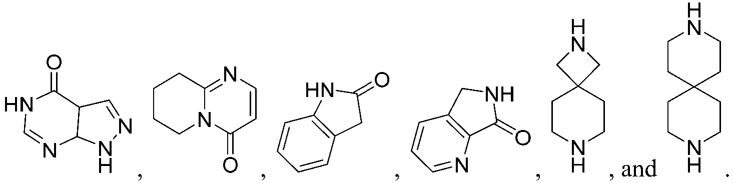

The bridged heterocyclyl group may be a 6-12 membered bridged heterocyclyl group, a 7-11 membered bridged heterocyclyl group, or a 6-12 membered saturated bridged group, and examples thereof include, but are not limited to:

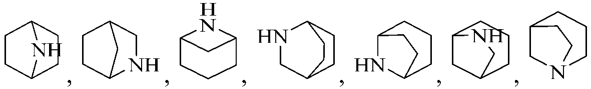

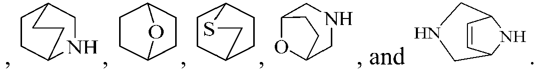

The 8-13 membered bicyclic condensed heteroaryl group refers to a group formed by a monocyclic heteroaryl ring condensed to phenyl or a monoheteroaryl group. The condensed heteroaryl group may be an 8-13 membered fused heteroaryl group, a 9-10 membered fused heteroaryl group, or an 8-10 membered bicyclic fused heteroaryl group, and examples thereof include, but are not limited to,

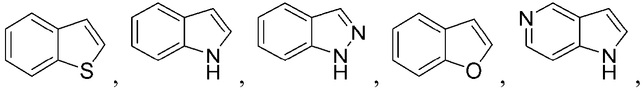

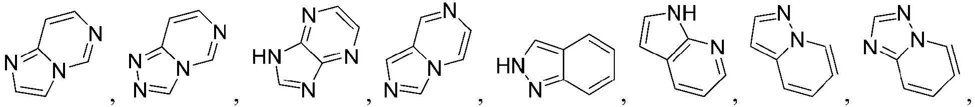

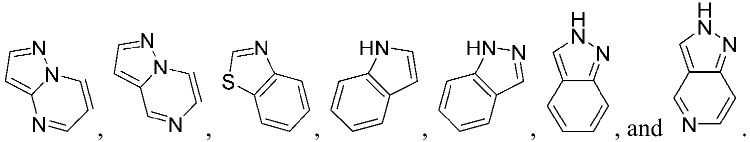

The 8-13 membered bicyclic condensed aryl group refers to a group formed by a monoaromatic ring condensed to a monoaryl group, including naphthalene, phenanthrene and the like.

The "pharmaceutically acceptable salt" described in the present invention refers to pharmaceutically acceptable acid and base addition salts or solvates thereof. Such pharmaceutically acceptable salts include salts of acids such as hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acids (such as acetic acid, HOOC-(CH₂)n-COOH (wherein n is 0 to 4)), and the like. Salts of bases include sodium salt, potassium salt, calcium salt, ammonium salt, and the like. A wide variety of non-toxic pharmaceutically acceptable addition salts are known to those skilled in the art.

The "isomer" described in the present invention refers to a stereoisomer and a tautomer.

The "stereoisomer" refers to an enantiomer that arise when a compound has an asymmetric atom or a cis-trans isomer that arise when a compound has a double bond or a ring structure. All enantiomers, diastereomers, racemates, cis-trans isomers, geometric isomers, epimers and mixtures thereof of the compound of formula (I) are included in the scope of the present invention.

The "tautomer" refers to a functional group isomer that arise due to the rapid movement of an atom between two positions in a molecule, and is a special type of functional group isomer. For example, tautomerism occurs in carbonyl compounds containing α-H, such as 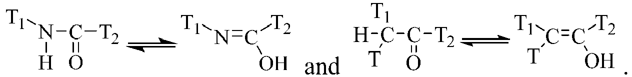 For example, other tautomerism phenomena occur by proton migration, such as phenol-keto tautomerism, nitroso-oxime tautomerism, and imine-enamine tautomerism.

T, T1, and T2 are independently any group that conforms to the bonding rules of the compound.

Rₐ₂ and Rₐ₄ described in the present invention can be optionally connected to L2, L3, L4 or L5 when complying with the bonding rules.

### Detailed Description of the Invention

The technical solution of the present disclosure will be further described in detail below with reference to specific examples. It should be understood that the following examples are merely for exemplary illustration and explanation of the present disclosure, and should not be construed as limiting the scope of protection of the present disclosure. Any technology implemented based on the above content of the present disclosure shall be encompassed within the intended scope of protection of the present disclosure.

Unless otherwise indicated, the starting materials and reagents used in the following examples are commercially available or can be prepared by known methods.

### Abbreviations of some reagents

BnBr, benzyl bromide
TIPT, tetraisopropyl titanate
STAB, sodium triacetoxyborohydride
TBAF, tetrabutylammonium fluoride
NMO, N-methylmorpholine N-oxide
TPAP, tetra-n-propylammonium perruthenate
Pd-PEPPSI IHept-Cl, (SP-4-1)-[1,3-bis[2,6-bis(1-propylbutyl)phenyl]-4,5-dichloro-1,3-dihydro-2H-imidazol-2-ylidene]dichloro(3-chloropyridine-κN)palladium
Xphos-Pd-G2, chloro(2-dicyclohexylphosphino-2',4',6'-triisopropyl-1,1'-biphenyl)[2-(2'-amino-1,1'-biphenyl)]palladium(II)
Pd(dppf)Cl2, 1,1'-bis(diphenylphosphino)ferrocene dichloropalladium(II)
Mn(TMHD)3, tris(2,2,6,6-tetramethyl-3,5-heptanedionato)manganese(III)
PhSiH3, phenylsilane
PMBCl, 4-methoxybenzyl chloride

The individual specific compounds contained in the compound of formula (i) of the present invention have similar structures with similar synthesis methods or mechanisms. To facilitate understanding by those skilled in the art, a general synthesis method is given as an example, as follows:

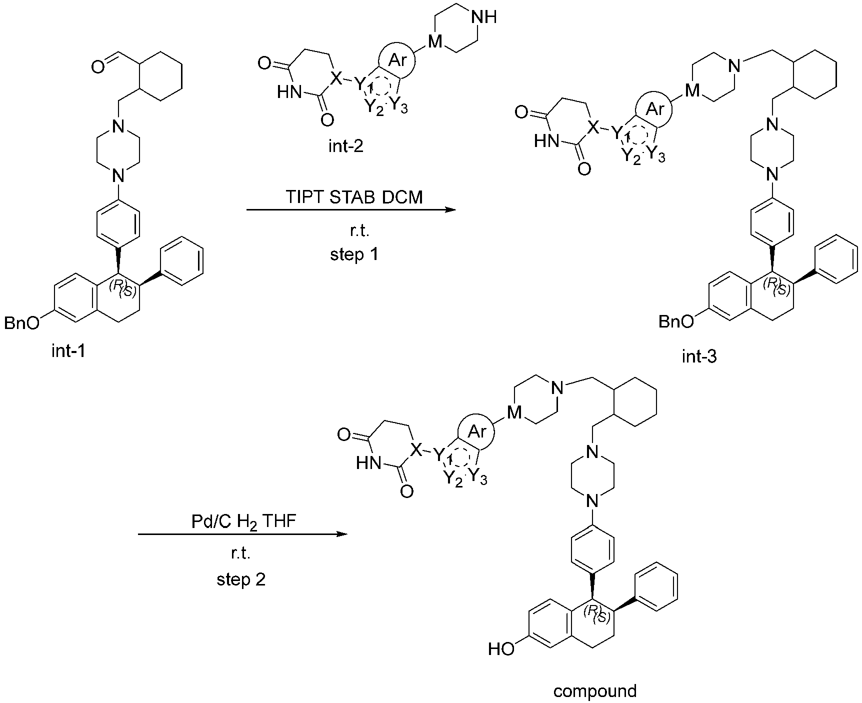

Step 1: Using an aldehyde compound int-1 and an amino compound int-2 as starting materials, intermediate int-3 is obtained via reductive amination.

Step 2: Intermediate int-3 is then subjected to one-step hydrogenation reduction to obtain the final product compound.

In the above general formulas, X is selected from N and CH;
Y₁, Y₂ and Y₃ are each selected from N, NRₐ₁, CRₐ₁, C(Rₐ₁)₂, and C(O);
M is selected from N and CRₐ₂; and 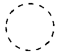
refers to a single or double bond that conforms to the rules of bonding.

The above general formulas int-1, int-2, int-3, and compound can be further substituted with substituents, and the range of the substituents is the same as that of any embodiment described in the specification.

Example 1: Synthesis of 1-(5-fluoro-6-(4-((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)dihydropyrimidine-2,4(1H,3H)-dione (Compound IV-5)

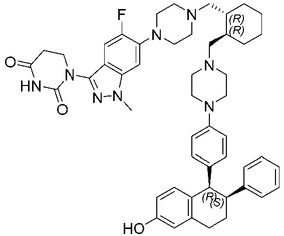

### Step 1: Synthesis of 6-methoxy-2-phenyl-3,4-dihydronaphthalen-1(2H)-one

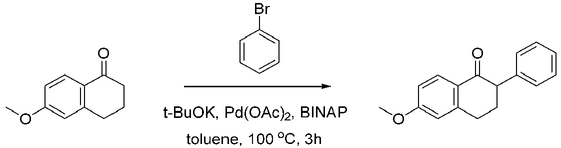

At room temperature, toluene (750 ml), potassium tert-butoxide (75.60 g, 673.73 mmol), palladium acetate (7.8 g, 34.74 mmol), and 1.1'-binaphthyl-2.2'-diphenyl phosphine (25.8 g, 41.43 mmol) were added to a single-necked flask in sequence and stirred evenly. 6-Methoxy-1-tetralone (60 g, 340.5 mmol) and bromobenzene (53.4 g, 340.5 mmol) were then added. The mixture was heated to 100°C and the reaction was carried out for 3 h. The reaction mixture was cooled to room temperature, quenched with a saturated ammonium chloride solution (500 mL), and extracted with methyl tert-butyl ether (500 mL x 3). The organic phases were combined and washed with saturated brine and water in sequence. The combined organic phases were dried over anhydrous sodium sulfate, concentrated, and purified by column chromatography (eluent: ethyl acetate/petroleum ether = 0-15%) to afford 51 g of a white solid with a yield of 59.3%. ¹H NMR (400 MHz, DMSO-d₆) δ 7.92 - 7.85 (m, 1H), 7.32 (t, *J* = 7.6 Hz, 2H), 7.28 - 7.22 (m, 1H), 7.21 - 7.15 (m, 2H), 6.93 (d, *J =* 7.2 Hz, 2H), 3.85 (s, 4H), 3.11 (ddd, J = 16.0, 10.8, 4.6 Hz, 1H), 2.96 (dt, J = 16.8, 4.4 Hz, 1H), 2.31 (dddt, J = 35.2, 17.7, 9.3, 4.4 Hz, 2H). MS (ESI) m/z: 253.4 [M+H]⁺.

### Step 2: Synthesis of 4-bromo-7-methoxy-3-phenyl-1,2-dihydronaphthalene

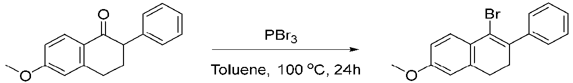

6-methoxy-2-phenyl-3,4-dihydronaphthalen-1(2H)-one (51 g, 202.2 mmol) and toluene (800 mL) were added to a 500 mL flask and stirred until completely dissolved. A solution of phosphorus tribromide (108 g, 398.9 mmol) in toluene (400 mL) was slowly added and the mixture was heated to reflux. After reacting for 24 h, most of the toluene was evaporated. After cooling, 1000 mL of dichloromethane was added, and the mixture was washed once with 500 ml of saturated sodium bisulfite and once with water. The reaction mixture was dried with anhydrous sodium sulfate, concentrated, and purified by column chromatography (eluent: dichloromethane/petroleum ether = 0-30%) to afford 42 g of a white solid with a yield of 66%.

### Step 3: Synthesis of 4-(6-methoxy-2-phenyl-3,4-dihydronaphthalen-1-yl)phenol

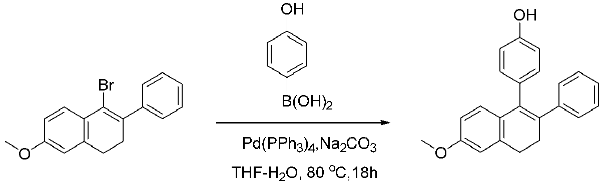

The compound 4-bromo-7-methoxy-3-phenyl-1,2-dihydronaphthalene (42 g, 133.2 mmol), 4-hydroxyphenylboronic acid (23.85 g, 172.9 mmol), tetrakistriphenylphosphine palladium (5.13 g, 4.4 mmol) and tetrahydrofuran (600 ml) were added to a 2L three-necked flask, and a solution of sodium carbonate (42.34 g, 399.4 mmol) in water (200 mL) was added, and the mixture was stirred and warmed to reflux for 18 h. The reaction mixture was cooled and adjusted with dilute hydrochloric acid until distinct layers form. The layers were separated, and the aqueous layer was extracted with ethyl acetate (500 mL x 2). The organic phases were combined and washed with a saturated sodium carbonate solution and saturated brine in sequence, dried over anhydrous sodium sulfate, filtered, concentrated, and purified by column chromatography (eluent: dichloromethane/petroleum ether = 0-30%) to afford 30.4 g of a white solid with a yield of 69%. ¹H NMR (400 MHz, DMSO-d₆) δ 9.31 (s, 1H), 7.12 (t, J = 7.5 Hz, 2H), 7.06 - 6.99 (m, 3H), 6.84 (d, J = 2.6 Hz, 1H), 6.80 - 6.75 (m, 2H), 6.67 - 6.57 (m, 4H), 3.75 (s, 3H), 2.89 (dd, J = 9.2, 6.5 Hz, 2H), 2.68 (dd, J = 9.2, 6.4 Hz, 2H).

### Step 4: Synthesis of 4-(6-methoxy-2-phenyl-1,2,3,4-tetrahydronaphthalen-1-yl)phenol

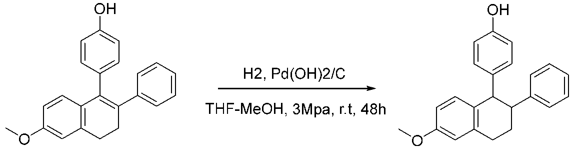

At room temperature, methanol (250 ml), tetrahydrofuran (250 ml), 20% palladium hydroxide/carbon (13 g), and 4-(6-methoxy-2-phenyl-3,4-dihydronaphthalen-1-yl)phenol (26.94 g, 82.03 mmol) were added to a 1L autoclave in sequence, and the autoclave was sealed. Nitrogen replacement was performed once and hydrogen replacement was performed three times. Finally, the hydrogen pressure was controlled at 3 MPa and the mixture was stirred at room temperature for 48 h. The reaction solution was filtered through celite, and the filtrate was concentrated to afford 18.9 g of an off-white solid with a yield of 100%. ¹H NMR (400 MHz, DMSO-d₆) δ 9.00 (s, 1H), 7.19 - 7.11 (m, 3H), 6.86 - 6.81 (m, 2H), 6.81 - 6.75 (m, 2H), 6.66 (dd, J = 8.5, 2.6 Hz, 1H), 6.37 (d, J = 8.1 Hz, 2H), 6.17 (d, J = 8.3 Hz, 2H), 4.18 (d, J = 5.0 Hz, 1H), 3.74 (s, 3H), 3.30 (ddd, J = 13.1, 5.2, 2.1 Hz, 1H), 3.12 - 2.91 (m, 2H), 2.12 (qd, J = 12.6, 6.1 Hz, 1H), 1.73 (dd, J = 13.0, 6.1 Hz, 1H).

### Step 5: Preparation of 4-((1R,2S)-6-methoxy-2-phenyl-1,2,3,4-tetrahydronaphthalen-1-yl)phenol

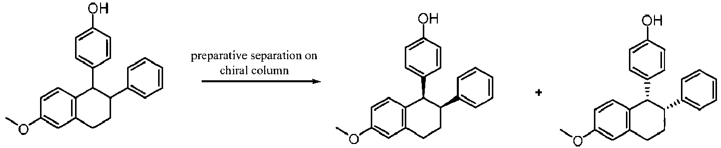

4-(6-methoxy-2-phenyl-1,2,3,4-tetrahydronaphthalen-1-yl)phenol (0.14 g, 0.25 mmol) was subjected to preparative purification via a chiral resolution column to afford an absolute configuration of a product (Peak B) as 0.05 g of a white solid with a yield of 35%.

The separation and preparation method is as follows:
Instrument: WATERS SFC-350
Column: CHIRALPAK IA 5cm × 25cm, 5um
Mobile phase: A is CO2, and B is IPA
Wavelength: 220nm
Flow rate: 200ml/min
Solvent: MeOH, DCM
Gradient conditions: B: 50%
Cycle time: 11min
Column temperature: 35°C
Separation detection method:
   Instrument: WATERS ACQUITY UPC²
   Column: Lux 3u-Cellulose-3 4.6 × 50mm, 3um
   Mobile phase: A is CO2, and B is MeOH (0.1% DEA)
   Wavelength: 220nm
   Column temperature: 35°C
   Flow rate: 4ml/min
   Injection volume: 1uL
   Injection concentration: 1mg/ml
   Solvent: MeOH
   Gradient conditions: B: 10% to 50% in 2 min, hold 1 min at 50%
   Run time: 3 min
   Retention time: 0.972min, 1.095min

### Step 6: Synthesis of 4-((1R,2S)-6-methoxy-2-phenyl-1,2,3,4-tetrahydronaphthalen-1-yl)phenyl 1,1,2,2,3,3,4,4,4-perfluorobutyl-1-sulfonate

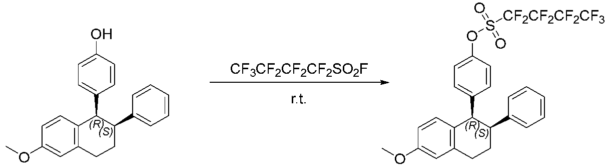

At room temperature, 4-((1*R*,2*S*)-6-methoxy-2-phenyl-1,2,3,4-tetrahydronaphthalen-1-yl)phenol (22.00 g, 66.58 mmol), perfluorobutylsulfonyl fluoride (26.14 g, 86.55 mmol), K₂CO₃(18.40 g, 133.16 mmol), CH₃CN (120 mL) and THF (120 mL) were added to a single-necked flask in sequence. The reaction was carried out at room temperature for 20 h under nitrogen protection before termination. The reaction solution was filtered, the filter cake was washed, and the mother liquor was collected, concentrated, and purified by column chromatography (an EA/PE system) to afford 27.14 g of a white solid with a yield of 67%.

### Step 7: Synthesis of tert-butyl 4-(4-((1R,2S)-6-methoxy-2-phenyl-1,2,3,4-tetrahydronaphthalen-1-yl)phenyl)piperazine-1-carboxylate

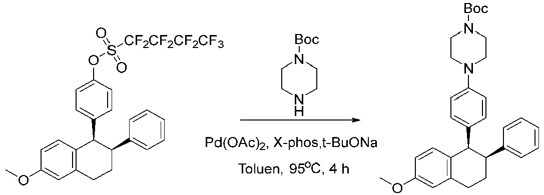

At room temperature, 4-((1R, 2S)-6-methoxy-2-phenyl-1,2,3,4-tetrahydronaphthalen-1-yl)phenyl 1,1,2,2,3,3,4,4,4-nonafluorobutane-1-sulfonate (7.4 g, 12.1 mmol), N-Boc piperazine (6.7 g, 36.2 mmol), palladium acetate (542 mg, 2.42 mmol), 2-dicyclohexylphosphino-2',4',6'-triisopropylbiphenyl (1.7 g, 3.63 mmol) and sodium tert-butoxide (4.6 g, 48.4 mmol) were placed in toluene (200 mL). The reaction was carried out at 95°C under nitrogen protection for 4 h. The reaction completion was determined by TLC. The reaction mixture was cooled to room temperature, poured into ice water, and extracted with EA. The organic phase was concentrated, sanded, and subjected to column chromatography with PE/EA (0-8%) to afford 4.7 g of a brown oil with a yield of 78%.

### Step 8: Synthesis of (5R,6S)-6-phenyl-5-(4-(piperazin-1-yl)phenyl)-5,6,7,8-tetrahydronaphthalen-2-ol

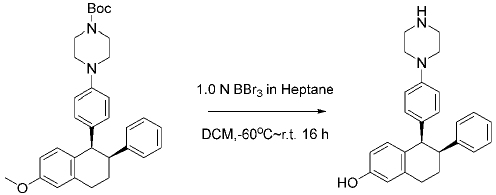

At room temperature, tert-butyl 4-(4-((1R, 2S)-6-methoxy-2-phenyl-1,2,3,4-tetrahydronaphthalen-1-yl)phenyl)piperazine-1-carboxylate (3.0 g, 6.0 mmol) was dissolved in 100 mL of dichloromethane, and 30 mL of 1.0 N boron tribromide in n-heptane was added dropwise at -60°C. After the addition, the mixture was naturally warmed to room temperature overnight. The mixture was cooled and methanol was added dropwise to quench the reaction. The reaction mixture was concentrated and subjected to silica gel column chromatography to afford 1.7 g of a yellow solid with a yield of 73%. MS (ESI) *m*/*z:* 385.2[M+H]⁺.

### Step 9: Synthesis of tert-butyl 4-(4-((1R,2S)-6-hydroxy-2-phenyl-1,2,3,4-tetrahydronaphthalen-1-yl)phenyl)piperazine-1-carboxylate

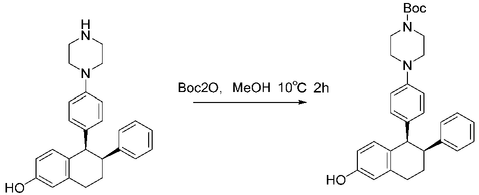

At room temperature, (SR,6S)-6-phenyl-5-(4-(piperazin-1-yl)phenyl)-5,6,7,8-tetrahydronaphthalene-2-ol (0.38 g, 1.0 mmol) was placed in methanol (10 mL), and Boc anhydride (0.26 g, 1.2 mmol) was added at 10°C. The mixture was stirred for 2 h, and the reaction solution was dried by rotary evaporation. The pH was adjusted to 8 by adding an aqueous EA/NaHCO₃ solution. The layers were separated, and the organic phase was concentrated to give 0.36 g of a white solid with a yield of 75%. MS (ESI) m/z: 485.2[M+H]⁺.

### Step 10: Synthesis of tert-butyl 4-(4-((1R,2S)-6-(benzyloxy)-2-phenyl-1,2,3,4-tetrahydronaphthalen-1-yl)phenyl)piperazine-1-carboxylate

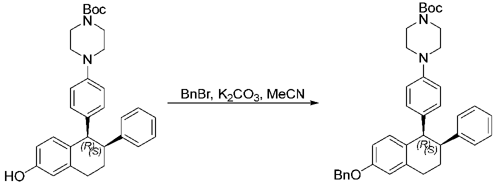

At room temperature, tert-butyl 4-(4-((1R,2S)-6-hydroxy-2-phenyl-1,2,3,4-tetrahydronaphthalen-1-yl)phenyl)piperazine-1-carboxylate (220.00 g, 0.45 mol), potassium carbonate (94.11 g, 0.68 mol), 18-crown-6 (2.2 g, 1% w/w), and acetonitrile (2 L) were added to a reaction flask in sequence, and then BnBr (89.28 g, 0.52 mol) was added dropwise. The reaction was carried out at 60°C for 18 h. The reaction solution was cooled to room temperature, added to water, crystallized, and filtered. The filter cake was slurried with methanol and filtered to afford 247.90 g of a product with a yield of 95%.

### Step 11: Synthesis of 1-(4-((1R,2S)-6-(benzyloxy)-2-phenyl-1,2,3,4-tetrahydronaphthalen-1-yl)phenyl)piperazine hydrochloride

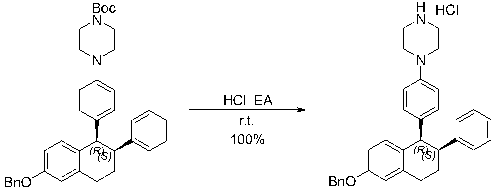

At room temperature, HCl in ethyl acetate (1 L) and tert-butyl 4-(4-((1R,2S)-6-(benzyloxy)-2-phenyl-1,2,3,4-tetrahydronaphthalen-1-yl)phenyl)piperazine-1-carboxylate (265.00 g, 0.46 mol) were added to a reaction flask in sequence, and the reaction was carried out at room temperature for 2 h. After the reaction was complete, the reaction mixture was filtered to afford 235.65 g of a product with a yield of 100%.

### Step 12: Synthesis of 1-(4-((1R,2S)-6-(benzyloxy)-2-phenyl-1,2,3,4-tetrahydronaphthalen-1-yl)phenyl)-4-(((1R,2R)-2-(((tert-butyldiphenylsilyl)oxy)methyl)cyclohexyl)methyl)piperazine

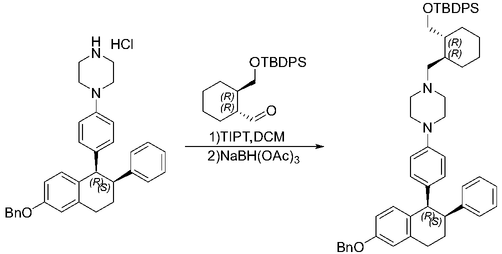

At room temperature, 1-(4-((1R,2S)-6-(benzyloxy)-2-phenyl-1,2,3,4-tetrahydronaphthalen-1-yl)phenyl)piperazine hydrochloride (50.00 g, 97.83 mmol), (1R,2R)-2-(((tert-butyldiphenylsilyl)oxy)methyl)cyclohexane-1-carboxaldehyde (48.40 g, 127.17 mmol) and dichloromethane (500 mL) were added to a reaction flask in sequence, followed by TIPT (50 mL). The reaction mixture was stirred at room temperature for 16 h, and then STAB (31.00 g, 146.74 mmol) was added. The reaction mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and slurried with methanol to afford 74.00 g of a product with a yield of 90%.

### Step 13: Synthesis of ((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methanol

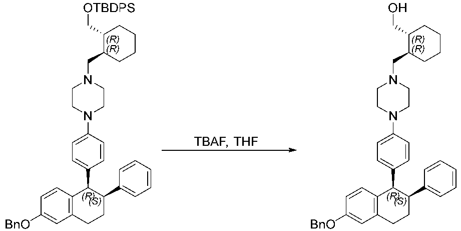

At room temperature, 1-(4-((1R,2S)-6-(benzyloxy)-2-phenyl-1,2,3,4-tetrahydronaphthalen-1-yl)phenyl)-4-(((1R,2R)-2-(((tert-butyldiphenylsilyl)oxy)methyl)cyclohexyl)methyl)piperazine (74.00 g, 88.17 mmol), TBAF (23.00 g, 88.17 mmol) and tetrahydrofuran (300 mL) were added to a reaction flask in sequence, and the mixture was stirred at 50°C for 4 h. After the reaction was complete, the reaction mixture was dried by rotary evaporation, and dichloromethane and water were added. The layers were separated. The organic phase was dried by rotary evaporation, and slurried with methanol to afford 45.00 g of a product with a yield of 85%.

### Step 14: Synthesis of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde

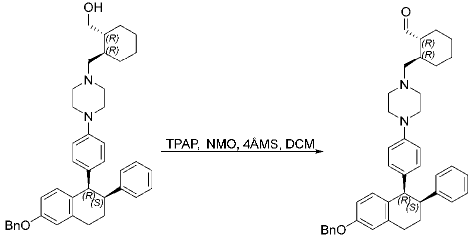

At room temperature, ((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methanol (45.00 g, 74.89 mmol), 4Å molecular sieve (45 g, 100% w/w), NMO (13.16 g, 112.34 mmol) and DCM (450 mL) were added to a reaction flask in sequence, followed by TPAP (2.63 g, 7.49 mmol), and the reaction was carried out at room temperature for 12 h. After the reaction was complete, the reaction mixture was filtered, and water was added. The layers were separated. The organic phase was dried by rotary evaporation, and subjected to column chromatography (MeOH/DCM system) to afford 40.00 g of a product with a yield of 89%.

### Step 15: Synthesis of tert-butyl 4-(3-(2,4-dioxotetrahydropyrimidin-1(2H)-yl)-5-fluoro-1-methyl-1H-indazol-6-yl)piperazine-1-carboxylate

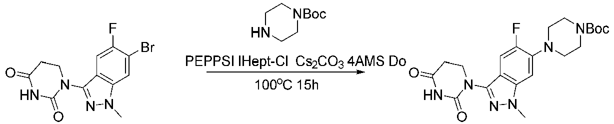

At room temperature, 1-(6-bromo-5-fluoro-1-methyl-1H-indazol-3-yl)dihydropyrimidine-2,4(1H,3H)-dione (0.25 g, 0.73 mmol), 1-Boc-piperazine (0.14 g, 0.73 mmol), Pd-PEPPSI IHept-Cl (0.07 g, 0.07 mmol), cesium carbonate (0.72 g, 2.20 mmol), 4A molecular sieve (0.70 g), and 1,4-dioxane (15 ml) were added to a reaction flask in sequence. Nitrogen replacement was performed 3-4 times. The mixture was heated to 100°C and the reaction was carried out for 15 h. After the reaction was complete, the reaction mixture was cooled to room temperature, and ethyl acetate and water were added. The layers were separated. The organic phase was dried, and then subjected to column chromatography using a PE/EA system to afford 0.14 g of a product with a yield of 44%.

### Step 16: Synthesis of 1-(5-fluoro-1-methyl-6-(piperazin-1-yl)-1H-indazol-3-yl)dihydropyrimidine-2,4(1H,3H)-dione

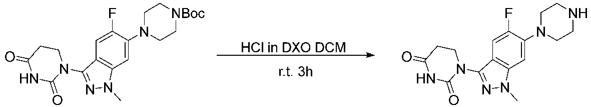

At room temperature, tert-butyl 4-(3-(2,4-dioxotetrahydropyrimidin-1(2H)-yl)-5-fluoro-1-methyl-1H-indazol-6-yl)piperazine-1-carboxylate (0.14 g, 0.32 mmol) and dichloromethane (10 ml) were added to a reaction flask in sequence. After complete dissolution, hydrochloric acid/dioxane (4 ml) was added, and the mixture was stirred at room temperature for 3 h. After the reaction was complete, the reaction mixture was stripped of solvent to afford 0.11 g of a crude product, which was directly taken to the next step.

### Step 17: Synthesis of 1-(6-(4-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-5-fluoro-1-methyl-1H-indazol-3-yl)dihydropyrimidine-2,4(1H,3H)-dione

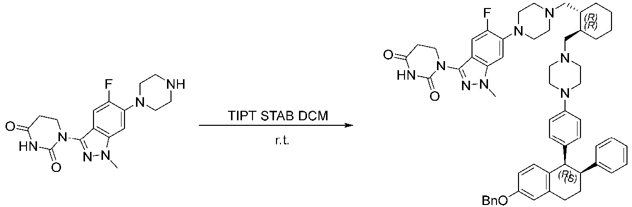

At room temperature, 1-(5-fluoro-1-methyl-6-(piperazin-1-yl)-1H-indazol-3-yl)dihydropyrimidine-2,4(1H,3H)-dione (0.11 g, 0.34 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.19 g, 0.31 mmol) and dichloromethane (10 ml) were added to a reaction flask in sequence, followed by TIPT (0.5 ml). The mixture was stirred at room temperature for 16 h, and then STAB (0.13 g, 0.62 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.20 g of a product with a yield of 63%.

### Step 18: 1-(5-fluoro-6-(4-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)dihydropyrimidine-2,4(1H,3H)-dione

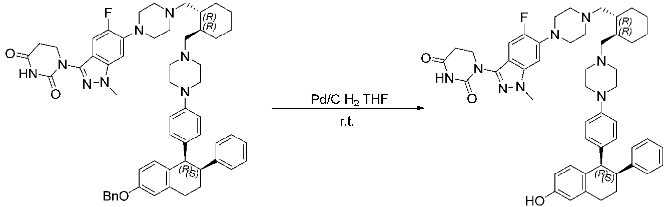

At room temperature, 1-(6-(4-(((1R,2R)-2-((4-(((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-5-fluoro-1-methyl-1H-indazol-3-yl)dihydropyrimidine-2,4(1H,3H)-dione (0.20 g, 0.22 mmol), 10% palladium on carbon (0.02 g), and tetrahydrofuran (10 ml) were added to a reaction flask in sequence. Hydrogen replacement was then performed 3-4 times, and the mixture was stirred at room temperature for 24 h. After the reaction was complete, the reaction mixture was filtered through celite. The filter cake was rinsed with tetrahydrofuran. The filtrate was stripped of solvent, and then subjected to column chromatography using a DCM/MeOH system to afford 0.07 g of a product with a yield of 39%.

¹H NMR (400 MHz, DMSO-*d*₆) δ 10.55 (s, 1H), 9.13 (s, 1H), 7.36 (d, *J* = 12.8 Hz, 1H), 7.19 - 7.04 (m, 4H), 6.83 (d, *J* = 7.2 Hz, 2H), 6.67 - 6.59 (m, 2H), 6.54 (d, *J* = 8.3 Hz, 2H), 6.48 (dd, *J* = 8.3, 2.5 Hz, 1H), 6.21 (d, *J* = 8.2 Hz, 2H), 4.14 (d, *J* = 4.9 Hz, 1H), 3.95 - 3.90 (m, 3H), 3.90 - 3.86 (m, 2H), 3.31 - 3.21 (m, 2H), 3.12 - 3.04 (m, 4H), 3.02 - 2.88 (m, 5H), 2.74 (t, *J* = 6.7 Hz, 2H), 2.68 - 2.51 (m, 4H), 2.29 - 2.00 (m, 8H), 1.88 - 1.81 (m, 2H), 1.74 - 1.66 (m, 2H), 1.62 - 1.55 (m, 2H), 1.52 - 1.39 (m, 2H), 1.30 - 1.12 (m, 2H), 1.07 - 0.89 (m, 2H). MS (ESI) m/z: 839.5 [M+H]⁺.

### Example 2 Synthesis of 3-(4-(4-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-3-methyl-2-oxo-2,3-dihydro-1H-benzo[d]imidazol-1-yl)piperidine-2,6-dione

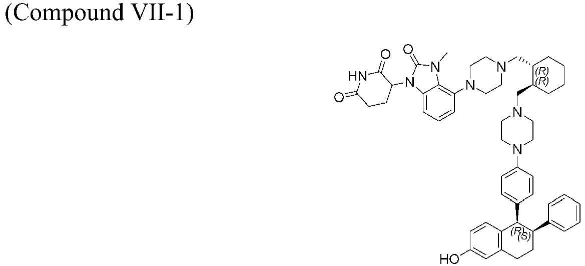

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 1-(4-methoxybenzyl)-2,6-dioxopiperidine-3-yltrifluoromethanesulfonate

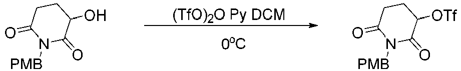

At room temperature, 3-hydroxy-1-(4-methoxybenzyl)piperidine-2,6-dione (4.00 g, 16.05 mmol) and dichloromethane (50 ml) were added to a reaction flask in sequence. After complete dissolution, the temperature was lowered to -10°C, and trifluoromethanesulfonic anhydride (6.80 g, 24.07 mmol) was added slowly under controlled temperature. After the addition was complete, the reaction was carried out while controlling the temperature at 0°C for 2 h. After the reaction was complete, the reaction mixture was stripped of solvent directly, and subjected to column chromatography using a PE/EA system to afford 4.41 g of a product with a yield of 72%.

### Step 2: Synthesis of 3-(4-bromo-3-methyl-2-oxo-2,3-dihydro-1H-benzimidazol-1-yl)-1-(4-methoxybenzyl)piperidine-2,6-dione

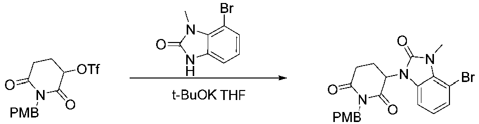

At room temperature, 7-bromo-1-methyl-1,3-dihydro-2H-benzimidazole-2-one (1.00 g, 3.61 mmol), potassium tert-butoxide (0.49 g, 4.33 mmol), and tetrahydrofuran (20 ml) were added to a reaction flask in sequence. The mixture was cooled to 0°C and stirred for 30 min. Then, 1-(4-methoxybenzyl)-2,6-dioxopiperidin-3-yltrifluoromethanesulfonate (1.38 g, 3.61 mmol) was added. After the addition, the mixture was allowed to return to room temperature and the reaction was carried out for 3 h. After the reaction was complete, ethyl acetate and water were added. The layers were separated. The organic phase was dried, and then subjected to column chromatography using a PE/EA system to afford 1.48 g of a product with a yield of 90%.

### Step 3: Synthesis of tert-butyl 4-(1-(1-(4-methoxybenzyl)-2,6-dioxopiperidin-3-yl)-3-methyl-2-oxo-2,3-dihydro-1H-benzo[d]imidazol-4-yl)piperazine-1-carboxylate

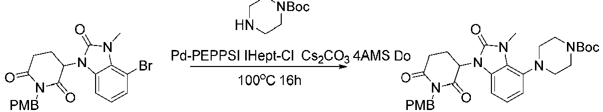

At room temperature, 3-(4-bromo-3-methyl-2-oxo-2,3-dihydro-1H-benzimidazol-1-yl)-1-(4-methoxybenzyl)piperidine-2,6-dione (0.74 g, 1.62 mmol), 1-Boc-piperazine (0.30 g, 1.62 mmol), Pd-PEPPSI IHept-Cl (0.16 g, 0.16 mmol), cesium carbonate (1.58 g, 4.85 mmol), 4A molecular sieve (0.16 g), and 1,4-dioxane (9 ml) were added to a reaction flask in sequence. Nitrogen replacement was performed 3-4 times. The mixture was heated to 100°C and the reaction was carried out for 15 h. After the reaction was complete, the reaction mixture was cooled to room temperature, and ethyl acetate and water were added. The layers were separated. The organic phase was dried, and then subjected to column chromatography using a PE/EA system to afford 0.41 g of a product with a yield of 45%.

### Step 4: Synthesis of 3-(3-methyl-2-oxo-4-(piperazin-1-yl)-2,3-dihydro-1H-benzimidazol-1-yl)piperidine-2,6-dione

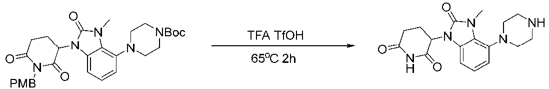

At room temperature, tert-butyl 4-(1-(1-(4-methoxybenzyl)-2,6-dioxopiperidin-3-yl)-3-methyl-2-oxo-2,3-dihydro-1H-benzo[d]imidazol-4-yl)piperazine-1-carboxylate (0.41 g, 0.73 mmol), trifluoroacetic acid (3 ml), and TfOH (0.5 ml) were added to a reaction flask in sequence, and the mixture was heated to 65°C and stirred for 2 h. After the reaction was complete, the reaction mixture was stripped of solvent and subjected to reversed-phase column chromatography using an (acetonitrile/water) system to afford 0.10 g of a crude product, which was directly taken to the next step.

### Step 5: Synthesis of 3-(4-(4-(((1R,2R)-2-((4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-3-methyl-2-oxo-2,3-dihydro-1H-benzo[d]imidazol-1-yl)piperidine-2,6-dione

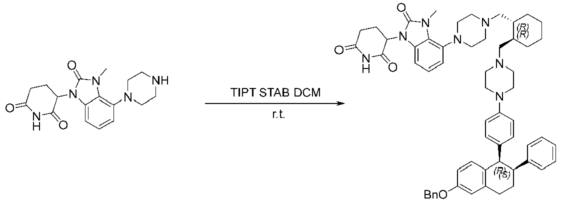

At room temperature, 3-(3-methyl-2-oxo-4-(piperazin-1-yl)-2,3-dihydro-1H-benzimidazol-1-yl)piperidine-2,6-dione (0.10 g, 0.21 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.13 g, 0.21 mmol) and dichloromethane (10 ml) were added to a reaction flask in sequence, followed by TIPT (0.5 ml). The mixture was stirred at room temperature for 16 h, and then STAB (0.09 g, 0.42 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.11 g of a product with a yield of 58%.

### Step 6: Synthesis of 3-(4-(4-(((1R,2R)-2-((4-(4-(1R,2S)-6-hydroxy-2-phenyl-1,2,3,4-tetrahydronaphthalen-1-yl)phenyl)piperazin-1-yl)methyl)cyclohexyl)methyl)piperazin-1-yl)-3-methyl-2-oxo-2,3-dihydro-1H-benzo[d]imidazol-1-yl)piperidine-2,6-dione

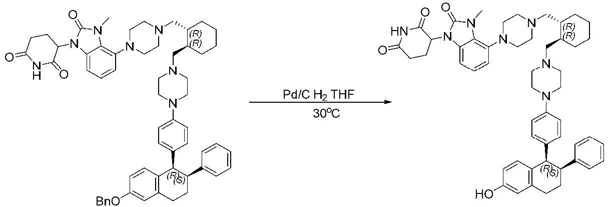

At room temperature, 3-(4-(4-(((1R,2R)-2-((4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-3-methyl-2-oxo-2,3-dihydro-1H-benzo[d]imidazol-1-yl)piperidine-2,6-dione (0.11 g, 0.12 mmol), 10% palladium on carbon (0.01 g), and tetrahydrofuran (10 ml) were added to a reaction flask in sequence. Hydrogen replacement was then performed 3-4 times, and the mixture was stirred at 30°C for 16 h. After the reaction was complete, the reaction mixture was filtered through celite. The filter cake was rinsed with tetrahydrofuran. The filtrate was stripped of solvent, and subjected to column chromatography using a DCM/MeOH system to afford 0.030 g of a product (0.026 g stored) with a yield of 29%.

¹H NMR (400 MHz, DMSO-*d*₆) δ 11.10 (s, 1H), 9.12 (s, 1H), 7.12 (d, *J* = 8.9 Hz, 3H), 7.05 - 6.87 (m, 3H), 6.83 (d, *J* = 7.1 Hz, 2H), 6.69 - 6.54 (m, 3H), 6.48 (d, *J* = 8.3 Hz, 1H), 6.24 (s, 2H), 5.60 - 4.99 (m, 1H), 4.15 (d, *J* = 4.7 Hz, 1H), 3.61 (s, 3H), 3.43 - 3.20 (m, 10H), 3.12 - 2.82 (m, 10H), 2.81 - 2.54 (m, 4H), 2.20 - 1.98 (m, 4H), 1.83 - 1.59 (m, 5H), 1.49 - 1.39 (m, 2H), 1.29 - 1.17 (m, 3H), 1.07 - 0.92 (m, 2H). MS (ESI) m/z: 836.5 [M+H]⁺.

### Example 3 Synthesis of 1-(5-fluoro-6-(1-(((1R,2R)-2-((4-(4-(((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)dihydropyrimidine-2,4(1H,3H)-dione (Compound IV-9)

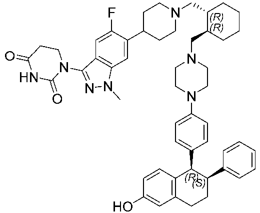

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 4-(3-(2,4-dioxotetrahydropyrimidin-1(2H)-yl)-5-fluoro-1-methyl-1H-indazol-6-yl)-3,6-dihydropyridine-1(2H)-carboxylate

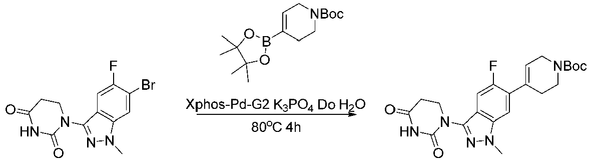

At room temperature, tert-butyl 1-(6-bromo-5-fluoro-1-methyl-1H-indazol-3-yl)dihydropyrimidine-2,4(1H,3H)-dione (0.25 g, 0.73 mmol), 4-(4,4,5,5-tetramethyl-1,3,2-dioxaborolan-2-yl)-3,6-dihydropyridine-1(2H)-carboxylate (0.34 g, 1.10 mmol), Xphos-Pd-G2 (0.06 g, 0.07 mmol), potassium phosphate (0.31 g, 1.47 mmol), water (3 ml), and 1,4-dioxane (10 ml) were added to a reaction flask in sequence. Nitrogen replacement was performed 3-4 times. The mixture was heated to 80°C and the reaction was carried out for 4 h. After the reaction was complete, the reaction mixture was cooled to room temperature, stripped of solvent, diluted with ethyl acetate, and filtered through celite. The filtrate was dried, stripped of solvent, and subjected to column chromatography using a PE/EA system to afford 0.25 g of a product with a yield of 78%.

### Step 2: Synthesis of tert-butyl 4-(3-(2,4-dioxotetrahydropyrimidin-1(2H)-yl)-5-fluoro-1-methyl-1H-indazol-6-yl)piperidine-1-carboxylate

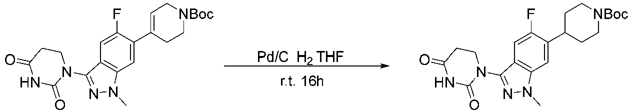

At room temperature, tert-butyl 4-(3-(2,4-dioxotetrahydropyrimidin-1(2H)-yl)-5-fluoro-1-methyl-1H-indazol-6-yl)-3,6-dihydropyridine-1(2H)-carboxylate (0.25 g, 0.57 mmol) and tetrahydrofuran (15 ml) were added to a reaction flask in sequence, followed by 10% palladium on carbon (0.03 g). Hydrogen replacement was performed 3-4 times, and the mixture was stirred at room temperature for 16 h. After the reaction was complete, the reaction mixture was filtered through celite, and stripped of solvent to afford 0.25g of a product, which was directly taken to the next step.

### Step 3: Synthesis of 1-(5-fluoro-1-methyl-6-(piperidin-4-yl)-1H-indazol-3-yl)dihydropyrimidine-2,4(1H,3H)-dione

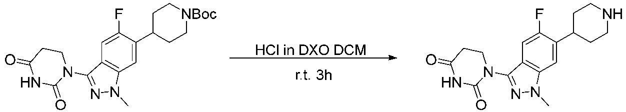

At room temperature, tert-butyl 4-(3-(2,4-dioxotetrahydropyrimidin-1(2H)-yl)-5-fluoro-1-methyl-1H-indazol-6-yl)piperidine-1-carboxylate (0.25 g, 0.56 mmol) and dichloromethane (10 ml) were added to a reaction flask in sequence. After complete dissolution, hydrochloric acid/dioxane (9 ml) was added, and the mixture was stirred at room temperature for 3 h. After the reaction was complete, the reaction mixture was stripped of solvent to afford 0.19 g of a crude product, which was directly taken to the next step.

### Step 4: Synthesis of 1-(6-(1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-5-fluoro-1-methyl-1H-indazol-3-yl)dihydropyrimidine-2,4(1H,3H)-dione

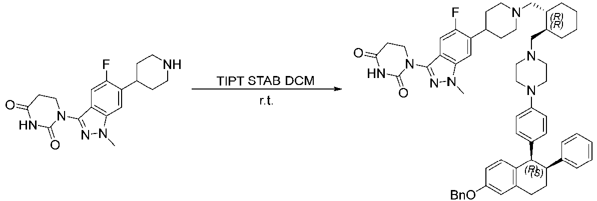

At room temperature, 1-(5-fluoro-1-methyl-6-(piperidin-4-yl)-1H-indazol-3-yl)dihydropyrimidine-2,4(1H,3H)-dione (0.19 g, 0.50 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.27 g, 0.45 mmol) and dichloromethane (10 ml) were added to a reaction flask in sequence, followed by TIPT (0.5 ml). The mixture was stirred at room temperature for 16 h, and then STAB (0.19 g, 0.91 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.27 g of a product with a yield of 58%.

### Step 5: Synthesis of 1-(5-fluoro-6-(1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)dihydropyrimidine-2,4(1H,3H)-dione

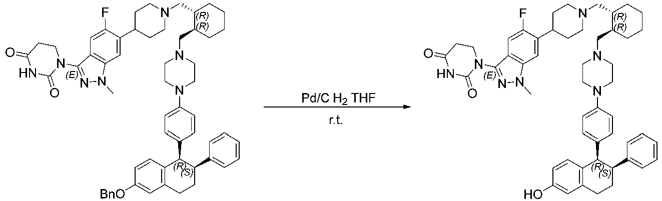

At room temperature, 1-(6-(1-(((1R,2R)-2-((4-((1R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-5-fluoro-1-methyl-1H-indazol-3-yl)dihydropyrimidine-2,4(1H,3H)-dione (0.27 g, 0.29 mmol), 10% palladium on carbon (0.03 g), and tetrahydrofuran (10 ml) were added to a reaction flask in sequence. Hydrogen replacement was then performed 3-4 times, and the mixture was stirred at room temperature for 24 h. After the reaction was complete, the reaction mixture was filtered through celite. The filter cake was rinsed with tetrahydrofuran. The filtrate was stripped of solvent, and subjected to column chromatography using a DCM/MeOH system to afford 0.135 g of a product (0.130 g stored) with a yield of 54%.

¹H NMR (400 MHz, DMSO-*d*₆) δ 10.57 (s, 1H), 9.15 (s, 1H), 7.58 (d, *J* = 6.0 Hz, 1H), 7.36 (d, *J* = 10.8 Hz, 1H), 7.13 (dd, *J* = 10.3, 6.9 Hz, 3H), 6.83 (d, *J* = 7.2 Hz, 2H), 6.69 - 6.31 (m, 5H), 6.21 (d, *J* = 8.2 Hz, 2H), 4.13 (d, *J* = 4.9 Hz, 1H), 3.99 (s, 3H), 3.90 (t, *J* = 6.7 Hz, 2H), 3.10 - 2.81 (m, 9H), 2.75 (t, *J* = 6.7 Hz, 2H), 2.48 - 2.24 (m, 6H), 2.19 - 2.03 (m, 3H), 1.96 - 1.86 (m, 4H), 1.83 - 1.75 (m, 4H), 1.73 - 1.64 (m, 2H), 1.60 - 1.50 (m, 2H), 1.46 - 1.31 (m, 2H), 1.27 - 1.12 (m, 2H), 1.03 - 0.85 (m, 2H). MS (ESI) m/z: 838.5 [M+H]⁺.

### Example 4 Synthesis of N-(2,6-dioxopiperidin-3-yl)-2-fluoro-4-(4-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperazin-1-yl)benzamide (Compound I-69)

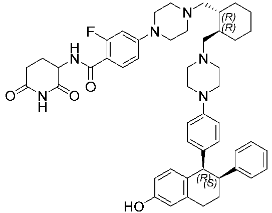

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 4-(3-fluoro-4-(methoxycarbonyl)phenyl)piperazine-1-carboxylate

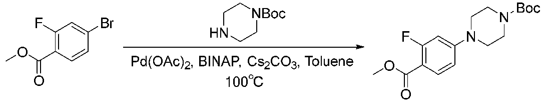

At room temperature, methyl 4-bromo-2-fluorobenzoate (2.00 g, 9.26 mmol), N-Boc-piperazine (3.45 g, 18.52 mmol), and Pd(OAc)₂ (0.10 g, 0.46 mmol), BINAP (0.58 g, 0.93 mmol), Cs₂CO₃(9.05 g, 27.77 mmol) and Toluene (20 ml) were added to a reaction flask in sequence. Nitrogen replacement was performed 3-4 times. The mixture was heated to 100°C and the reaction was carried out for 16 h. After the reaction was complete, the reaction mixture was cooled to room temperature, and filtered. The filtrate was concentrated, and subjected to column chromatography using a PE/EA system to afford 2.50 g of a product as a brown oil with a yield of 83%.

### Step 2: Synthesis of 4-(4-(tert-butoxycarbonyl)piperazin-1-yl)-2-fluorobenzoic acid

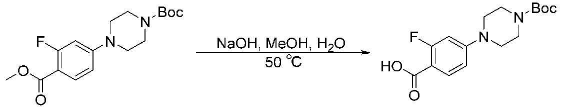

At room temperature, tert-butyl 4-(3-fluoro-4-(methoxycarbonyl)phenyl)piperazine-1-carboxylate (2.50 g, 7.78 mmol), NaOH (1.24 g, 31.11 mmol), MeOH (30 ml) and H₂O (10 ml) were added to a reaction flask in sequence. The mixture was heated to 50°C and the reaction was carried out for 4 h. After the reaction was complete, the reaction mixture was stripped of solvent, and adjusted to acidity with an aqueous HCl solution. The product precipitated out, and after filtration, 2.20 g of a white solid was obtained, with a yield of 92%.

### Step 3: Synthesis of tert-butyl 4-(4-((2,6-dioxopiperidin-3-yl)carbamoyl)-3-fluorophenyl)piperazine-1-carboxylate

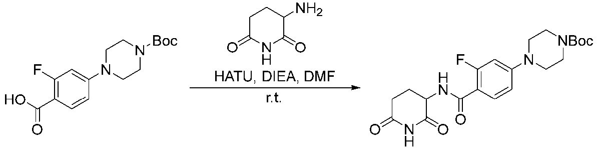

At room temperature, 4-(4-(tert-butyloxycarbonyl)piperazin-1-yl)-2-fluorobenzoic acid (0.20 g, 0.65 mmol), 3-aminopiperidine-2,6-dione hydrochloride (0.13 g, 0.78 mmol), HATU (0.37 g, 0.98 mmol), DIEA (0.25 g, 1.95 mmol) and DMF (3 ml) were added to a reaction flask in sequence, and the mixture was stirred at room temperature for 2 h. After the reaction was complete, water (3 mL) and saturated brine (3 mL) were added to the reaction solution. A large amount of solid precipitated out. After filtration, the filter cake was dissolved in DCM, dried over anhydrous magnesium sulfate, and then directly taken to the next step.

### Step 4: Synthesis of N-(2,6-dioxopiperidin-3-yl)-2-fluoro-4-(piperazin-1-yl)benzamide

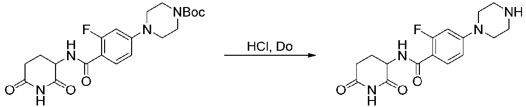

At room temperature, tert-butyl 4-(4-((2,6-dioxopiperidin-3-yl)carbamoyl)-3-fluorophenyl)piperazine-1-carboxylate (0.27 g, 0.65 mmol) and dichloromethane (2 mL) were added to a reaction flask in sequence. After complete dissolution, hydrochloric acid/dioxane (2 mL) was added, and the mixture was stirred at room temperature for 3 h. After the reaction was complete, the reaction mixture was stripped of solvent to afford 0.26 g of a crude product, which was directly taken to the next step.

### Step 5: Synthesis of 4-(4-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-N-(2,6-dioxopiperidin-3-yl)-2-fluorobenzamide

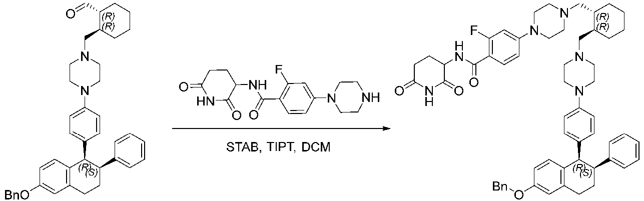

At room temperature, N-(2,6-dioxopiperidin-3-yl)-2-fluoro-4-(piperazin-1-yl)benzamide (0.15 g, 0.47 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.28 g, 0.47 mmol) and dichloromethane (5 mL) were added to a reaction flask in sequence, followed by TIPT (0.5 mL). The mixture was stirred at room temperature for 16 h, and then STAB (0.15 g, 0.71 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.21 g of a product with a three-step yield of 62%.

### Step 6: Synthesis of N-(2,6-dioxopiperidin-3-yl)-2-fluoro-4-(4-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperazin-1-yl)benzamide

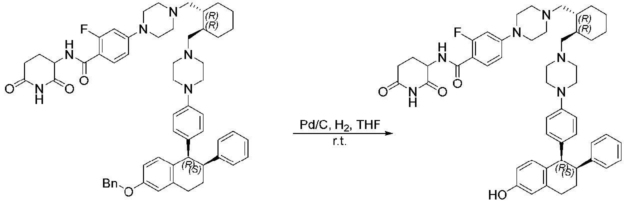

At room temperature, 4-(4-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-N-(2,6-dioxopiperidin-3-yl)-2-fluorobenzamide (0.21 g, 0.23 mmol), 10% palladium on carbon (0.10 g), and THF (3 mL) were added to a reaction flask in sequence. Hydrogen replacement was performed 3-4 times, and the mixture was stirred at room temperature for 24 h. After the reaction was complete, the reaction mixture was filtered through celite. The filter cake was rinsed with THF. The filtrate was stripped of solvent, and then subjected to column chromatography using a DCM/MeOH system to afford 0.07 g of a product with a yield of 37%.

¹H NMR (400 MHz, DMSO-*d*₆) δ 10.86 (s, 1H), 9.12 (s, 1H), 8.05 (t, *J* = 7.2 Hz, 1H), 7.62 (t, *J* = 9.0 Hz, 1H), 7.17 - 7.10 (m, 3H), 6.85 - 6.74 (m, 4H), 6.66 - 6.59 (m, 2H), 6.55 - 6.46 (m, 3H), 6.21 (d, *J* = 8.3 Hz, 2H), 4.73 (dt, *J* = 12.9, 6.8 Hz, 1H), 4.14 (d, *J* = 4.9 Hz, 1H), 3.29 - 3.20 (m, 6H), 3.00 - 2.93 (m, 5H), 2.83 - 2.68 (m, 1H), 2.48 - 2.29 (m, 10H), 2.16 - 1.99 (m, 5H), 1.90 - 1.81 (m, 2H), 1.73 - 1.68 (m, 1H), 1.60 - 1.52 (m, 2H), 1.45 - 1.40 (m, 2H), 1.23 - 1.15 (m, 2H), 1.02 - 0.93 (m, 2H), 0.87 - 0.81 (m, 1H). MS (ESI) m/z: 827.4 [M+H]⁺.

### Example 5 Synthesis of 3-(6-(4-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperidine-2,6-dione (Compound IV-3)

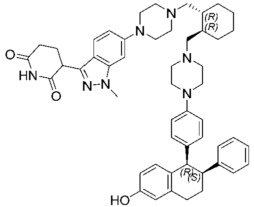

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 3-(2,6-bis(benzyloxy)pyridin-3-yl)-6-bromo-1-methyl-1H-indazole

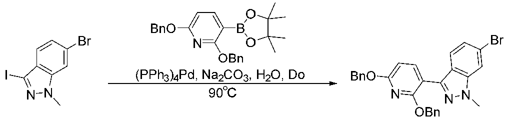

At room temperature, 6-bromo-3-iodo-1-methyl-1H-indazole (1.00 g, 2.97 mmol), 2,6-bis(benzyloxy)-3-(4,4,5,5-tetramethyl-1,3,2-dioxaborolan-2-yl)pyridine (1.24 g, 2.97 mmol), sodium carbonate (0.94 g, 8.90 mmol), tetrakis(triphenylphosphine)palladium (0.34 g, 0.30 mmol), water (1 mL), and dioxane (10 ml) were added to a reaction flask in sequence. The reaction was carried out at 90°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 1.20 g of a product with a yield of 81%.

### Step 2: Synthesis of tert-butyl 4-(3-(2,6-bis(benzyloxy)pyridin-3-yl)-1-methyl-1H-indazol-6-yl)piperazine-1-carboxylate

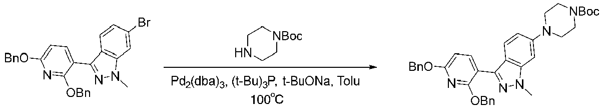

At room temperature, 3-(2,6-bis(benzyloxy)pyridin-3-yl)-6-bromo-1-methyl-1H-indazole (0.25 g, 0.50 mmol), 1-Boc-piperazine (0.28 g, 1.50 mmol), Pd₂(dba)₃ (0.05 g, 0.05 mmol), 10% tri-tert-butylphosphine in toluene (200 mg, 0.10 mmol), sodium tert-butoxide (0.10 g, 1.00 mmol) and toluene (10 ml) were added to a reaction flask in sequence. The reaction was carried out at 100°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 0.20 g of a product with a yield of 66%.

### Step 3: Synthesis of tert-butyl 4-(3-(2,6-dioxopiperidin-3-yl)-1-methyl-1H-indazol-6-yl)piperazine-1-carboxylate

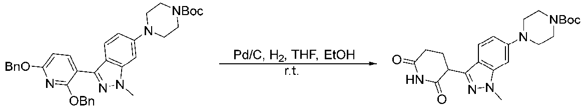

At room temperature, tert-butyl 4-(3-(2,6-bis(benzyloxy)pyridin-3-yl)-1-methyl-1H-indazol-6-yl)piperazine-1-carboxylate (0.20 g, 0.33 mmol), 10% Pd/C (0.02 g), tetrahydrofuran (5 mL) and ethanol (5 ml) were added to a reaction flask in sequence, and the reaction was carried out at room temperature for 24 h. After the reaction was complete, the reaction mixture was filtered and dried by rotary evaporation to afford 0.13 g of a crude product, which was directly taken to the next step.

### Step 4: Synthesis of 3-(1-methyl-6-(piperazin-1-yl)-1H-indazol-3-yl)piperazin-2,6-dione

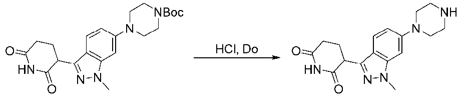

At room temperature, tert-butyl 4-(3-(2,6-dioxopiperidin-3-yl)-1-methyl-1H-indazol-6-yl)piperazine-1-carboxylate (0.13 g, 0.30 mmol) and HCl in dioxane (5 ml) were added to a reaction flask in sequence, and the reaction was carried out at room temperature for 2 h. After the reaction was complete, the reaction mixture was dried by rotary evaporation to afford 0.11 g of a crude product, which was directly taken to the next step.

### Step 5: Synthesis of 3-(6-(4-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperazin-2,6-dione

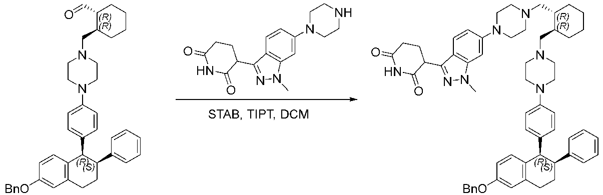

At room temperature, 3-(1-methyl-6-(piperazin-1-yl)-1H-indazol-3-yl)piperazin-2,6-dione (0.06 g, 0.18 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.11 g, 0.18 mmol) and dichloromethane (10 ml) were added to a reaction flask in sequence, followed by TIPT (0.5 ml). The mixture was stirred at room temperature for 16 h, and then STAB (0.08 g, 0.36 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.03 g of a product with a yield of 27%.

### Step 6: Synthesis of 3-(6-(4-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperidine-2,6-dione

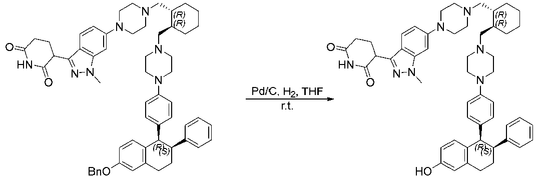

At room temperature, 3-(6-(4-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperazin-2,6-dione (0.03 g, 0.03 mmol), 10% palladium on carbon (0.01 g), and tetrahydrofuran (5 ml) were added to a reaction flask in sequence. Hydrogen replacement was then performed 3-4 times, and the mixture was stirred at 30°C for 16 h. After the reaction was complete, the reaction mixture was filtered through celite. The filter cake was rinsed with tetrahydrofuran. The filtrate was stripped of solvent, and then subjected to column chromatography using a DCM/MeOH system to afford 0.01 g of a product with a yield of 37%.

¹H NMR (400 MHz, DMSO-*d*₆) δ 10.87 (s, 1H), 9.15 (s, 1H), 7.51 (d, *J* = 9.0 Hz, 1H), 7.11 (dt, *J* = 11.5, 7.1 Hz, 3H), 6.93 (d, *J* = 9.1 Hz, 1H), 6.82 (d, *J* = 8.5 Hz, 3H), 6.65 - 6.60 (m, 2H), 6.55 (d, *J* = 8.3 Hz, 2H), 6.48 (dd, *J* = 8.2, 2.6 Hz, 1H), 6.22 (d, *J* = 8.2 Hz, 2H), 4.27 (dd, *J* = 9.3, 5.1 Hz, 1H), 4.14 (d, *J* = 5.0 Hz, 1H), 3.85 (s, 3H), 3.14 - 2.86 (m, 10H), 2.72 - 2.55 (m, 6H), 2.35 - 1.97 (m, 7H), 1.83 - 1.46 (m, 9H), 1.03 - 0.96 (m, 2H), 0.87 - 0.81 (m, 1H). MS (ESI) m/z: 820.8 [M+H]⁺.

### Example 6 Synthesis of 3-(5-(4-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperazin-1-yl)-3-methyl-2-oxo-2,3-dihydro-1H-benzo[d]imidazol-1-yl)piperidine-2,6-dione (Compound VII-2)

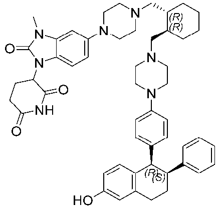

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 4-(1-(2,6-dioxopiperidin-3-yl)-3-methyl-2-oxo-2,3-dihydro-1H-benzo[d]imidazol-5-yl)piperazine-1-carboxylate

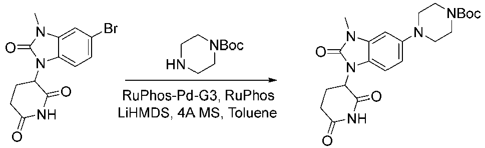

At room temperature, 3-(5-bromo-3-methyl-2-oxo-2,3-dihydro-1H-benzo[D]imidazol-1-yl)piperidine-2,6-dione (0.50 g, 1.48 mmol), N-Boc-piperazine (0.55 g, 2.96 mmol), RuPhos-Pd-G3 (0.25 g, 0.30 mmol), RuPhos (0.14 g, 0.30 mmol), 1M LiHMDS (6 mL), 4A MS and Toluene (5 ml) were added to a reaction flask in sequence. Nitrogen replacement was performed 3-4 times. The mixture was heated to 100°C and the reaction was carried out for 5 h. After the reaction was complete, the reaction mixture was cooled to room temperature, concentrated, and subjected to column chromatography using a PE/EA system to afford 0.45 g of a brown oil with a yield of 68%.

### Step 2: Synthesis of 3-(3-methyl-2-oxo-5-piperazin-1-yl-2,3-dihydro-1H-benzo[d]imidazol-1-yl)piperazin-2,6-dione

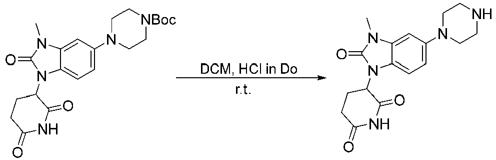

At room temperature, tert-butyl 4-(1-(2,6-dioxopiperidin-3-yl)-3-methyl-2-oxo-2,3-dihydro-1H-benzo[d]imidazol-5-yl)piperazine-1-carboxylate (0.45 mg, 1.01 mmol) and dichloromethane (2 ml) were added to a reaction flask in sequence. After complete dissolution, hydrochloric acid/dioxane (2 ml) was added, and the mixture was stirred at room temperature for 3 h. After the reaction was complete, the reaction mixture was stripped of solvent to afford 0.30 g of a crude product, which was directly taken to the next step.

### Step 3: Synthesis of 3-(5-(4-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperazin-1-yl)-3-methyl-2-oxo-2,3-dihydro-1H-benzo[d]imidazol-1-yl)piperazin-2,6-dione

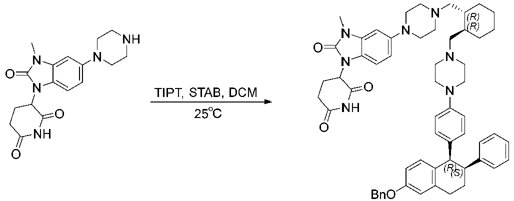

At room temperature, 3-(3-methyl-2-oxo-5-piperazin-1-yl-2,3-dihydro-1H-benzo[d]imidazol-1-yl)piperazin-2,6-dione (0.15 g, 0.44 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.26 g, 0.44 mmol) and dichloromethane (5 ml) were added to a reaction flask in sequence, followed by TIPT (0.45 ml). The mixture was stirred at room temperature for 16 h, and then STAB (0.14 g, 0.66 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.11 g of a product with a two-step yield of 23%.

### Step 4: Synthesis of 3-(5-(4-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperazin-1-yl)-3-methyl-2-oxo-2,3-dihydro-1H-benzo[d]imidazol-1-yl)piperidine-2,6-dione

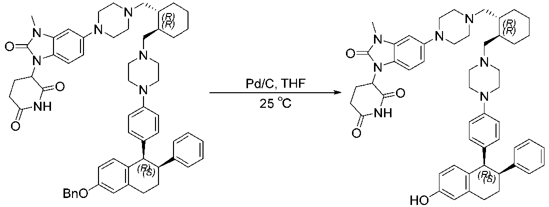

At room temperature, 3-(5-(4-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperazin-1-yl)-3-methyl-2-oxo-2,3-dihydro-1H-benzo[d]imidazol-1-yl)piperidine-2,6-dione (0.11 g, 0.12 mmol), 10% palladium on carbon (0.10 g), and THF (3 ml) were added to a reaction flask in sequence. Hydrogen replacement was performed 3-4 times, and the mixture was stirred at 25°C for 24 h. After the reaction was complete, the reaction mixture was filtered through celite. The filter cake was rinsed with THF. The filtrate was stripped of solvent, and then subjected to column chromatography using a DCM/MeOH system to afford 0.01 g of a product with a yield of 11%.

1H NMR (400 MHz, DMSO-d6) δ 11.08 (s, 1H), 9.16 (s, 1H), 7.11 (dd, J = 11.4, 6.9 Hz, 3H), 6.95 (d, J = 8.6 Hz, 1H), 6.82 (d, J = 8.6 Hz, 3H), 6.63 - 6.48 (m, 6H), 6.22 (d, J = 8.0 Hz, 2H), 5.76 (s, 3H), 5.30 (dd, J = 12.7, 5.5 Hz, 1H), 4.14 (d, J = 5.0 Hz, 1H), 3.14 (s, 2H), 3.04 (s, 2H), 2.94 (dd, J = 17.2, 9.0 Hz, 5H), 2.76 - 2.56 (m, 10H), 2.30 (d, J = 26.0 Hz, 2H), 2.09 (dd, J = 12.9, 6.7 Hz, 2H), 1.98 (d, J = 10.8 Hz, 2H), 1.87 - 1.40 (m, 10H), 1.21 (s, 2H), 0.99 (d, J = 13.1 Hz, 2H). MS (ESI) m/z: 837.0 [M+H]⁺.

### Example 7 Synthesis of N-(2,6-dioxopiperidin-3-yl)-5-(4-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)pyridinecarboxamide (Compound III-1)

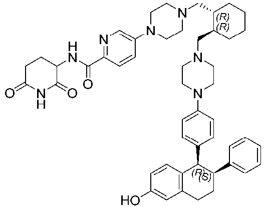

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 4-(6-(methoxycarbonyl)pyridin-3-yl)piperazine-1-carboxylate

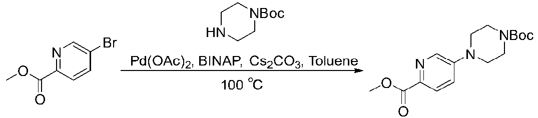

At room temperature, methyl 5-bromopyridine-2-carboxylate (2.00 g, 9.26 mmol), N-Boc-piperazine (3.45 g, 18.52 mmol), and Pd(OAc)₂ (0.10 g, 0.46 mmol), BINAP (0.58 g, 0.93 mmol), Cs₂CO₃(9.05 g, 27.77 mmol) and Toluene (20 ml) were added to a reaction flask in sequence. Nitrogen replacement was performed 3-4 times. The mixture was heated to 100°C and the reaction was carried out for 16 h. After the reaction was complete, the reaction mixture was cooled to room temperature, and filtered. The filtrate was concentrated, and subjected to column chromatography using a PE/EA system to afford 2.50 g of a product as a brown oil with a yield of 83%.

### Step 2: Synthesis of 5-(4-(tert-butoxycarbonyl)piperazin-1-yl)pyridinecarboxylic acid

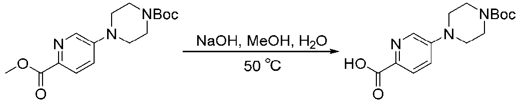

At room temperature, tert-butyl 4-(6-(methoxycarbonyl)pyridin-3-yl)piperazine-1-carboxylate (2.50 g, 7.78 mmol), NaOH (1.24 g, 31.11 mmol), MeOH (30 ml) and H₂O (10 ml) were added to a reaction flask in sequence. The mixture was heated to 50°C and the reaction was carried out for 4 h. After the reaction was complete, the reaction mixture was stripped of solvent, adjusted to acidity with an aqueous HCl solution, and extracted three times with DCM to afford 2.20 g of a yellow solid with a yield of 92%.

### Step 3: Synthesis of tert-butyl 4-(6-((2,6-dioxopiperidin-3-yl)carbamoyl)pyridin-3-yl)piperazine-1-carboxylate

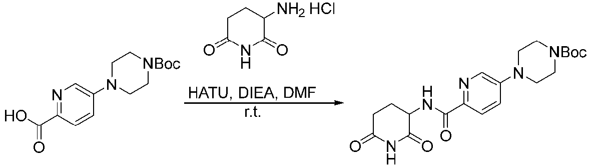

At room temperature, 5-(4-(tert-butyloxycarbonyl)piperazin-1-yl)pyridinecarboxylic acid (0.20 g, 0.65 mmol), 3-aminopiperidine-2,6-dione hydrochloride (0.13 g, 0.78 mmol), HATU (0.37 g, 0.98 mmol), DIEA (0.25 g, 1.95 mmol) and DMF (3 ml) were added to a reaction flask in sequence, and the mixture was stirred at room temperature for 2 h. After the reaction was complete, water (3 mL) and saturated brine (3 mL) were added to the reaction solution. A large amount of solid precipitated out. After filtration, the filter cake was dissolved in DCM, dried over anhydrous magnesium sulfate, and then directly taken to the next step.

### Step 4: Synthesis of N-(2,6-dioxopiperidin-3-yl)-5-(piperazin-1-yl)pyridine-3-carboxamide

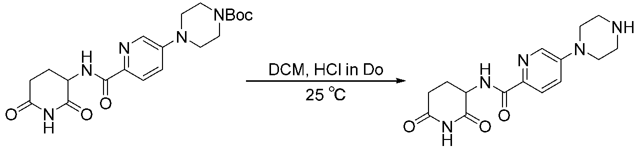

At room temperature, tert-butyl 4-(6-((2,6-dioxopiperidin-3-yl)carbamoyl)pyridin-3-yl)piperazine-1-carboxylate (0.27 mg, 0.65 mmol) and dichloromethane (2 ml) were added to a reaction flask in sequence. After complete dissolution, hydrochloric acid/dioxane (2 ml) was added, and the mixture was stirred at room temperature for 3 h. After the reaction was complete, the reaction mixture was stripped of solvent to afford 0.26 g of a crude product, which was directly taken to the next step.

### Step 5: Synthesis of 5-(4-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperazin-1-yl)-N-(2,6-dioxopiperidin-3-yl)pyridine-3-carboxamide

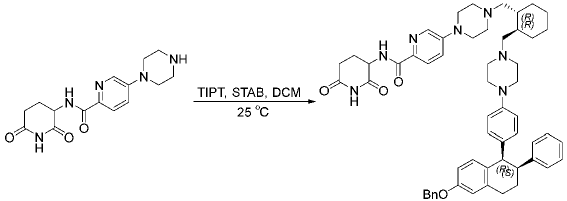

At room temperature, N-(2,6-dioxopiperidin-3-yl)-5-(piperazin-1-yl)pyridine-3-carboxamide (0.15 g, 0.47 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.28 g, 0.47 mmol), and dichloromethane (5 ml) were added to a reaction flask in sequence, followed by TIPT (0.45 ml). The mixture was stirred at room temperature for 16 h, and then STAB (0.15 g, 0.71 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.21 g of a product with a three-step yield of 62%.

### Step 6: Synthesis of N-(2,6-dioxopiperidin-3-yl)-5-(4-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperazin-1-yl)pyridinecarboxamide

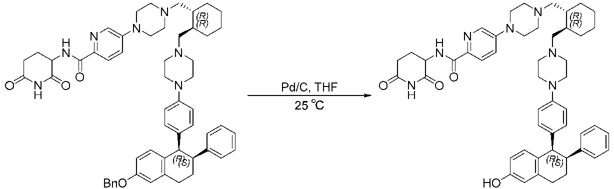

At room temperature, 5-(4-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperazin-1-yl)-N-(2,6-dioxopiperidin-3-yl)pyridine-3-carboxamide (0.21 g, 0.23 mmol), 10% palladium on carbon (0.10 g), and THF (3 ml) were added to a reaction flask in sequence. Hydrogen replacement was performed 3-4 times, and the mixture was stirred at room temperature for 24 h. After the reaction was complete, the reaction mixture was filtered through celite. The filter cake was rinsed with THF. The filtrate was stripped of solvent, and then subjected to column chromatography using a DCM/MeOH system to afford 0.04 g of a product with a yield of 21%.

¹H NMR (400 MHz, DMSO-*d*₆) δ 10.86 (s, 1H), 9.12 (s, 1H), 8.73 (d, *J* = 8.3 Hz, 1H), 8.31 (d, *J* = 2.8 Hz, 1H), 7.86 (d, *J* = 8.8 Hz, 1H), 7.41 (dd, *J* = 8.9, 2.8 Hz, 1H), 7.13 (dd, *J* = 10.3, 6.8 Hz, 3H), 6.86 - 6.82 (m, 2H), 6.66 - 6.60 (m, 2H), 6.54 - 6.46 (m, 3H), 6.21 (d, *J* = 8.3 Hz, 2H), 4.75 (ddd, *J* = 13.0, 8.3, 5.4 Hz, 1H), 4.14 (d, *J* = 4.9 Hz, 1H), 3.26 (s, 4H), 3.02 - 2.72 (m, 8H), 2.60 - 2.51 (m, 4H), 2.42 (d, *J* = 32.5 Hz, 8H), 2.24 - 1.96 (m, 6H), 1.86 (s, 1H), 1.70 (dd, *J* = 13.2, 5.8 Hz, 1H), 1.57 (d, *J* = 8.9 Hz, 2H), 1.44 (s, 2H), 1.22 (d, *J* = 12.7 Hz, 2H), 0.98 (s, 2H). MS (ESI) m/z: 810.4 [M+H]⁺.

### Example 8 Synthesis of 3-(4-(1-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperidin-4-yl)-3-methyl-2-oxo-2,3-dihydro-1H-benzo[d]imidazol-1-yl)piperidin-2,6-dione (Compound VII-3)

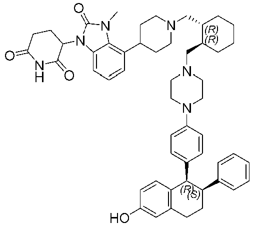

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 4-(1-(2,6-dioxopiperidin-3-yl)-3-methyl-2-oxo-2,3-dihydro-1H-benzo[d]imidazol-4-yl)-3,6-dihydropyridine-1(2H)-carboxylate

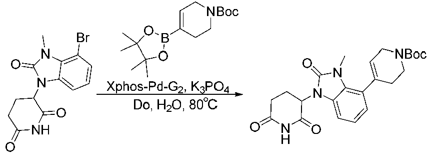

At room temperature, 3-(4-bromo-3-methyl-2-oxo-2,3-dihydro-1H-benzo[D]imidazol-1-yl)piperidin-2,6-dione (0.25 g, 0.74 mmol), N-Boc-1,2,5,6-tetrahydropyridine-4-boronic acid pinacol ester (0.34 g, 1.11 mmol), Xphos-Pd-G2 (0.06 g, 0.07 mmol), K₃PO₄ (0.31 g, 1.48 mmol), Do (5 mL) and H₂O (1 ml) were added to a reaction flask in sequence. Nitrogen replacement was performed 3-4 times. The mixture was heated to 80°C and the reaction was carried out for 3 h. After the reaction was complete, the reaction mixture was cooled to room temperature, concentrated, and subjected to column chromatography using a PE/EA system to afford 0.22 g of a white solid with a yield of 67%.

### Step 2: Synthesis of tert-butyl 4-(1-(2,6-dioxopiperidin-3-yl)-3-methyl-2-oxo-2,3-dihydro-1H-benzo[d]imidazol-4-yl)piperidine- 1-carboxylate

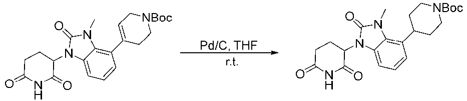

At room temperature, tert-butyl 4-(1-(2,6-dioxopiperidin-3-yl)-3-methyl-2-oxo-2,3-dihydro-1H-benzo[d]imidazol-4-yl)-3,6-dihydropyridine-1(2H)-carboxylate (0.22 g, 0.50 mmol), 10% palladium on carbon (0.10 g) and THF (3 ml) were added to a reaction flask in sequence. Hydrogen replacement was performed 3-4 times, and the mixture was stirred at room temperature for 8 h. After the reaction was complete, the reaction mixture was filtered through celite. The filter cake was rinsed with THF, and the filtrate was stripped of solvent to afford 0.20 g of a crude product, which was then directly taken to the next step.

### Step 3: Synthesis of 3-(3-methyl-2-oxo-4-(piperidin-4-yl)-2,3-dihydro-1H-benzo[d]imidazol-1-yl)piperidin-2,6-dione

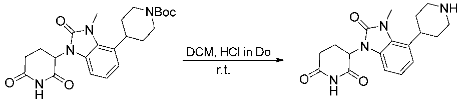

At room temperature, tert-butyl 4-(1-(2,6-dioxopiperidin-3-yl)-3-methyl-2-oxo-2,3-dihydro-1H-benzo[d]imidazol-4-yl)piperidin-1-carboxylate (0.20 mg, 0.45 mmol) and dichloromethane (2 ml) were added to a reaction flask in sequence. After complete dissolution, hydrochloric acid/dioxane (2 ml) was added, and the mixture was stirred at room temperature for 3 h. After the reaction was complete, the reaction mixture was stripped of solvent to afford 0.11 g of a crude product, which was directly taken to the next step.

### Step 4: Synthesis of 3-(4-(1-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperidin-4-yl)-3-methyl-2-oxo-2,3-dihydro-1H-benzo[d]imidazol-1-yl)piperidin-2,6-dione

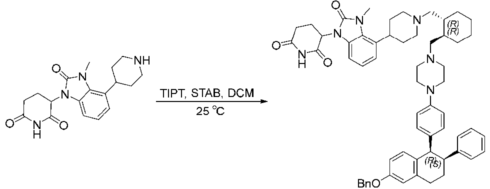

At room temperature, 3-(3-methyl-2-oxo-4-(piperidin-4-yl)-2,3-dihydro-1H-benzo[d]imidazol-1-yl)piperidin-2,6-dione (0.11 g, 0.32 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.19 g, 0.32 mmol) and dichloromethane (5 ml) were added to a reaction flask in sequence, followed by TIPT (0.3 ml). The mixture was stirred at room temperature for 16 h, and then STAB (0.10 g, 0.48 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.06 g of a product with a three-step yield of 13%.

### Step 5: Synthesis of 3-(4-(1-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperidin-4-yl)-3-methyl-2-oxo-2,3-dihydro-1H-benzo[d]imidazol-1-yl)piperidin-2,6-dione

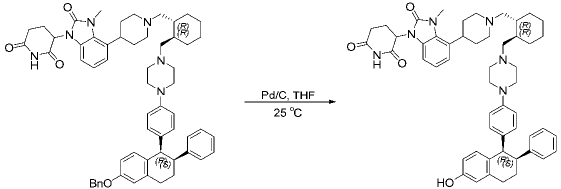

At room temperature, 3-(4-(1-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperidin-4-yl)-3-methyl-2-oxo-2,3-dihydro-1H-benzo[d]imidazol-1-yl)piperidin-2,6-dione (0.06 g, 0.06 mmol), 10% palladium on carbon (0.05 g), and THF (3 ml) were added to a reaction flask in sequence. Hydrogen replacement was performed 3-4 times, and the mixture was stirred at 25°C for 24 h. After the reaction was complete, the reaction mixture was filtered through celite. The filter cake was rinsed with THF. The filtrate was stripped of solvent, and then subjected to column chromatography using a DCM/MeOH system to afford 0.008 g of a product with a yield of 14%.

¹H NMR (400 MHz, DMSO-d6) δ 11.11 (s, 1H), 9.15 (s, 1H), 7.12 (q, J = 5.9, 4.5 Hz, 3H), 7.02 - 6.95 (m, 3H), 6.83 (d, J = 7.3 Hz, 2H), 6.59 (dd, J = 15.9, 6.0 Hz, 4H), 6.48 (d, J = 8.5 Hz, 1H), 6.23 (d, J = 8.1 Hz, 2H), 5.40 - 5.33 (m, 1H), 4.14 (d, J = 5.0 Hz, 1H), 3.59 (s, 3H), 3.07 (s, 3H), 2.97 - 2.86 (m, 4H), 2.64 (dd, J = 17.3, 12.2 Hz, 8H), 2.20 - 2.08 (m, 2H), 1.98 - 1.90 (m, 4H), 1.68 (d, J = 30.7 Hz, 4H), 1.53 - 1.44 (m, 2H), 1.24 (s, 10H), 1.05 - 0.97 (m, 2H), 0.86 (t, J = 6.5 Hz, 1H). MS (ESI) m/z: 836.0 [M+H]⁺.

### Example 9 Synthesis of 3-(5-(4-(((1R,2R)-2-((4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1H-indazol-1-yl)piperidine-2,6-dione (compound VI-6)

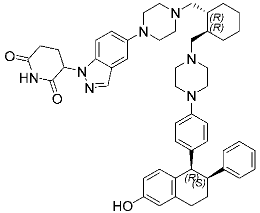

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 4-(3-(2,4-dioxotetrahydropyrimidin-1(2H)-yl)-5-fluoro-1-methyl-1H-indazol-6-yl)piperazine-1-carboxylate

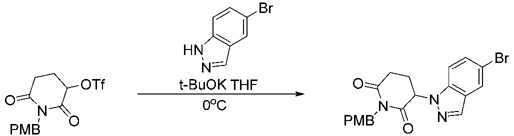

At room temperature, 5-bromoindazole (0.50 g, 2.54 mmol), potassium tert-butoxide (0.34 g, 3.05 mmol), and tetrahydrofuran (15 ml) were added to a reaction flask in sequence. The mixture was cooled to 0°C and stirred for 30 min. Then, 1-(4-methoxybenzyl)-2,6-dioxopiperidin-3-yltrifluoromethanesulfonate (0.97 g, 2.54 mmol) was added. After the addition, the mixture was allowed to return to room temperature and the reaction was carried out for 3 h. After the reaction was complete, ethyl acetate and water were added. The layers were separated. The organic phase was dried, and then subjected to column chromatography using a PE/EA system to afford 0.47 g of a product with a yield of 43%.

### Step 2: Synthesis of tert-butyl 4-(1-(1-(4-methoxybenzyl)-2,6-dioxopiperidin-3-yl)-1H-indazol-5-yl)piperazine-1-carboxylate

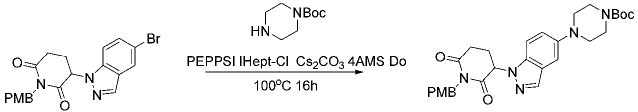

At room temperature, tert-butyl 4-(3-(2,4-dioxotetrahydropyrimidin-1(2H)-yl)-5-fluoro-1-methyl-1H-indazol-6-yl)piperazine-1-carboxylate (0.47 g, 1.09 mmol), 1-Boc-piperazine (0.20 g, 1.09 mmol), PEPPSI IHept-Cl (0.11 g, 0.11 mmol), cesium carbonate (1.07 g, 3.28 mmol), 4A molecular sieve (0.11 g), and 1,4-dioxane (15 ml) were added to a reaction flask in sequence. Nitrogen replacement was performed 3-4 times. The mixture was heated to 100°C and the reaction was carried out for 15 h. After the reaction was complete, the reaction mixture was cooled to room temperature, and ethyl acetate and water were added. The layers were separated. The organic phase was dried, and then subjected to column chromatography using a PE/EA system to afford 0.13 g of a product with a yield of 21%.

### Step 3: Synthesis of 3-(5-(piperazin-1-yl)-1H-indazol-1-yl)piperidine-2,6-dione

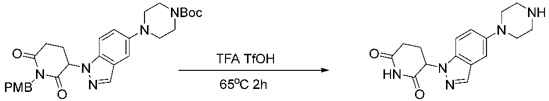

At room temperature, tert-butyl 4-(1-(1-(4-methoxybenzyl)-2,6-dioxopiperidin-3-yl)-1H-indazol-5-yl)piperazine-1-carboxylate (0.13 g, 0.23 mmol), trifluoroacetic acid (3 ml) and TfOH (0.5 ml) were added to a reaction flask in sequence, and the mixture was heated to 65°C and stirred for 2 h. After the reaction was complete, the reaction mixture was stripped of solvent and subjected to reversed-phase column chromatography using an (acetonitrile/water) system to afford 0.06 g of a crude product, which was directly taken to the next step.

### Step 4: Synthesis of 3-(5-(4-(((1R,2R)-2-((4-(4-(1R,2S)-6-(benzyloxy)-2-phenyl-1,2,3,4-tetrahydronaphthalen-1-yl)phenyl)piperazin-1-yl)yl)cyclohexyl)methyl)piperazin-1-yl)-1H-indazol-1-yl)piperidine-2,6-dione

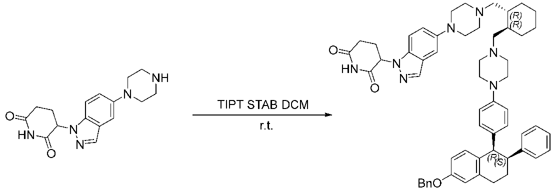

At room temperature, 3-(5-(piperazin-1-yl)-1H-indazol-1-yl)piperidine-2,6-dione (0.06 g, 0.18 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.11 g, 0.18 mmol), and dichloromethane (10 ml) were added to a reaction flask in sequence, followed by TIPT (0.5 ml). The mixture was stirred at room temperature for 16 h, and then STAB (0.08 g, 0.36 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.03 g of a product with a yield of 27%.

### Step 5: Synthesis of 3-(5-(4-(((1R,2R)-2-((4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1H-indazol-1-yl)piperidine-2,6-dione

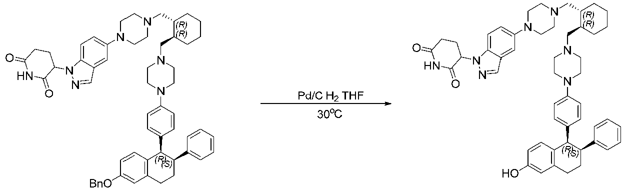

At room temperature, 3-(5-(4-(((1R,2R)-2-((4-(4-(2R,2S)-6-(benzyloxy)-2-phenyl-1,2,3,4-tetrahydronaphthalen-1-yl)phenyl)piperazin-1-yl)yl)cyclohexyl)methyl)piperazin-1-yl)-1H-indazol-1-yl)piperidine-2,6-dione (0.03 g, 0.04 mmol), 10% palladium on carbon (0.01 g), and tetrahydrofuran (10 ml) were added to a reaction flask in sequence. Hydrogen replacement was then performed 3-4 times, and the mixture was stirred at 30°C for 16 h. After the reaction was complete, the reaction mixture was filtered through celite. The filter cake was rinsed with tetrahydrofuran. The filtrate was stripped of solvent, and subjected to column chromatography using a DCM/MeOH system to afford 0.008 g of a product (0.004 g stored) with a yield of 29%.

¹H NMR (400 MHz, DMSO-*d*₆) δ 11.08 (s, 1H), 9.25 (s, 1H), 7.93 (s, 1H), 7.53 (d, *J* = 9.0 Hz, 1H), 7.26 (d, *J* = 9.4 Hz, 1H), 7.12 (d, *J* = 10.0 Hz, 3H), 6.82 (d, *J* = 7.2 Hz, 2H), 6.63 (d, *J* = 5.9 Hz, 2H), 6.52 (dd, *J* = 21.0, 8.2 Hz, 3H), 6.22 (d, *J* = 8.1 Hz, 2H), 5.89 - 5.77 (m, 1H), 4.66 - 4.39 (m, 1H), 4.13 (d, *J* = 5.0 Hz, 1H), 3.24 - 3.02 (m, 8H), 2.98 - 2.66 (m, 9H), 2.40 - 2.31 (m, 2H), 2.29 - 2.16 (m, 2H), 2.13 - 2.01 (m, 2H), 1.87 - 1.76 (m, 2H), 1.72 - 1.63 (m, 2H), 1.65 - 1.56 (m, 2H), 1.46 - 1.37 (m, 2H), 1.31 - 1.13 (m, 4H), 1.12 - 0.95 (m, 2H), 0.87 - 0.79 (m, 2H). MS (ESI) m/z: 806.6 [M+H]⁺.

### Example 10 Synthesis of N-(2,6-dioxopiperidin-3-yl)-2-fluoro-4-(1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)benzamide (Compound I-67)

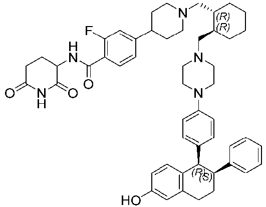

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 4-(3-fluoro-4-(methoxycarbonyl)phenyl)-3,6-dihydropyridine-1 (2H)-carboxylate

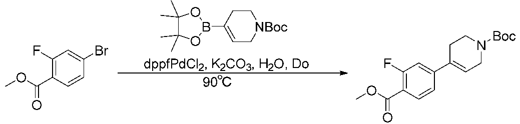

At room temperature, methyl 4-bromo-2-fluorobenzoate (2.00 g, 8.58 mmol), tert-butyl 4-(4,4,5,5-tetramethyl-1,3,2-dioxaborolan-2-yl)-3,6-dihydropyridine-1(2H)-carboxylate (2.78 g, 9.01 mmol), dppfPdCl₂ (0.31 g, 0.43 mmol), cesium carbonate (7.00 g, 21.46 mmol), water (5 mL) and dioxane (20 ml) were added to a reaction flask in sequence. The reaction was carried out at 90°C under a nitrogen atmosphere for 5 h. After the reaction was complete, the mixture was cooled to room temperature, and ethyl acetate and water were added. The layers were separated. The organic phase was dried, and subjected to column chromatography using a PE/EA system to afford 2.50 g of a brown oil with a yield of 83%.

### Step 2: Synthesis of 4-(1-(tert-butoxycarbonyl)-1,2,3,6-tetrahydropyridin-4-yl)-2-fluorobenzoic acid

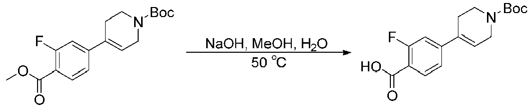

At room temperature, tert-butyl 4-(3-fluoro-4-(methoxycarbonyl)phenyl)-3,6-dihydropyridine-1(2H)-carboxylate (2.50 g, 7.78 mmol), NaOH (1.24 g, 31.11 mmol), MeOH (30 ml) and H₂O (10 ml) were added to a reaction flask in sequence. The mixture was heated to 50°C and the reaction was carried out for 4 h. After the reaction was complete, the reaction mixture was stripped of solvent, and adjusted to acidity with an aqueous HCl solution. The product precipitated out, and after filtration, 2.20 g of a white solid was obtained, with a yield of 92%.

### Step 3: Synthesis of tert-butyl 4-(4-((2,6-dioxopiperidin-3-yl)carbamoyl)-3-fluorophenyl)-3,6-dihydropyridine-1(2H)-carboxylate

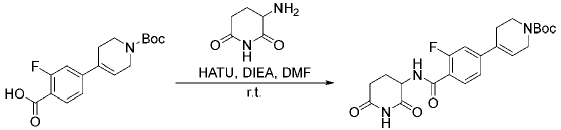

At room temperature, 4-(1-(tert-butoxycarbonyl)-1,2,3,6-tetrahydropyridin-4-yl)-2-fluorobenzoic acid (0.20 g, 0.65 mmol), 3-aminopiperidine-2,6-dione hydrochloride (0.13 g, 0.78 mmol), HATU (0.37 g, 0.98 mmol), DIEA (0.25 g, 1.95 mmol) and DMF (3 ml) were added to a reaction flask in sequence, and the mixture was stirred at room temperature for 2 h. After the reaction was complete, water (3 mL) and saturated brine (3 mL) were added to the reaction solution. A large amount of solid precipitated out. After filtration, the filter cake was dissolved in DCM, dried over anhydrous magnesium sulfate, and then directly taken to the next step.

### Step 4: Synthesis of N-(2,6-dioxopiperidin-3-yl)-2-fluoro-4-(1,2,3,6-tetrahydropyridin-4-yl)benzamide

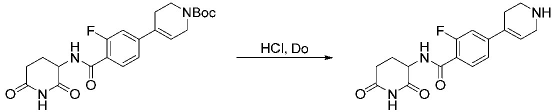

At room temperature, tert-butyl 4-(4-((2,6-dioxopiperidin-3-yl)carbamoyl)-3-fluorophenyl)-3,6-dihydropyridine-1(2H)-carboxylate (0.27 g, 0.65 mmol) and dichloromethane (2 mL) were added to a reaction flask in sequence. After complete dissolution, hydrochloric acid/dioxane (2 mL) was added, and the mixture was stirred at room temperature for 3 h. After the reaction was complete, the reaction mixture was stripped of solvent to afford 0.26 g of a crude product, which was directly taken to the next step.

### Step 5: Synthesis of 4-(1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-1,2,3,6-tetrahydropyridin-4-yl)-N-(2,6-dioxopiperidin-3-yl)-2-fluorobenzamide

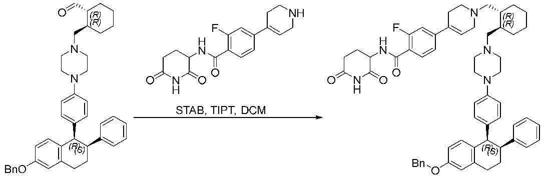

At room temperature, N-(2,6-dioxopiperidin-3-yl)-2-fluoro-4-(1,2,3,6-tetrahydropyridin-4-yl)benzamide (0.15 g, 0.47 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.28 g, 0.47 mmol) and dichloromethane (5 mL) were added to a reaction flask in sequence, followed by TIPT (0.5 mL). The mixture was stirred at room temperature for 16 h, and then STAB (0.15 g, 0.71 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.21 g of a product with a three-step yield of 62%.

### Step 6: Synthesis of N-(2,6-dioxopiperidin-3-yl)-2-fluoro-4-(1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)benzamide

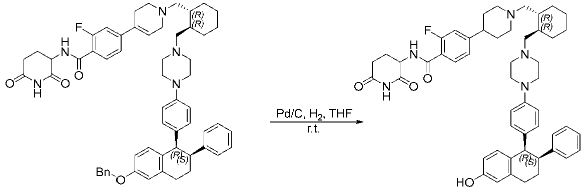

At room temperature, 4-(1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-1,2,3,6-tetrahydropyridin-4-yl)-N-(2,6-dioxopiperidin-3-yl)-2-fluorobenzamide (0.21 g, 0.23 mmol), 10% palladium on carbon (0.10 g), and THF (3 mL) were added to a reaction flask in sequence. Hydrogen replacement was performed 3-4 times, and the mixture was stirred at room temperature for 24 h. After the reaction was complete, the reaction mixture was filtered through celite. The filter cake was rinsed with THF. The filtrate was stripped of solvent, and then subjected to column chromatography using a DCM/MeOH system to afford 0.01 g of a product with a yield of 5%.

¹H NMR (400 MHz, DMSO-*d*₆) δ 10.88 (s, 1H), 9.13 (s, 1H), 8.49 (dd, *J* = 8.3, 3.8 Hz, 1H), 7.64 (dd, *J* = 11.1, 4.9 Hz, 1H), 7.23 - 7.09 (m, 5H), 6.83 (d, *J* = 7.1 Hz, 2H), 6.65 - 6.46 (m, 5H), 6.23 (d, *J* = 8.2 Hz, 2H), 4.85 - 4.66 (m, 1H), 4.15 (d, *J* = 4.9 Hz, 1H), 3.09 - 2.62 (m, 15H), 2.28 - 2.07 (m, 5H), 1.86 - 1.43 (m, 15H), 1.03 - 0.94 (m, 3H), 0.87 - 0.82 (m, 2H). MS (ESI) m/z: 826.6 [M+H]⁺.

### Example 11 Synthesis of 3-(5-fluoro-6-(4-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperazin-2,6-dione (Compound IV-1)

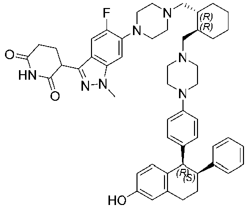

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 3-(2,6-bis(benzyloxy)pyridin-3-yl)-6-bromo-5-fluoro-1-methyl-1H-indazole

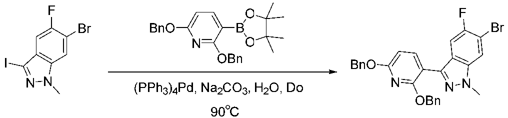

At room temperature, 6-bromo-5-fluoro-3-iodo-1-methyl-1H-indazole (1.00 g, 2.97 mmol), 2,6-bis(benzyloxy)-3-(4,4,5,5-tetramethyl-1,3,2-dioxaborolan-2-yl)pyridine (1.24 g, 2.97 mmol), sodium carbonate (0.94 g, 8.90 mmol), tetrakis(triphenylphosphine)palladium (0.34 g, 0.30 mmol), water (1 mL), and dioxane (10 ml) were added to a reaction flask in sequence. The reaction was carried out at 90°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 1.20 g of a product with a yield of 81%.

### Step 2: Synthesis of tert-butyl 4-(3-(2,6-bis(benzyloxy)pyridin-3-yl)-5-fluoro-1-methyl-1H-indazol-6-yl)piperazine-1-carboxylate

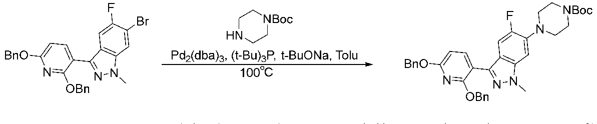

At room temperature, 3-(2,6-bis(benzyloxy)pyridin-3-yl)-6-bromo-5-fluoro-1-methyl-1H-indazole (0.25 g, 0.50 mmol), 1-Boc-piperazine (0.28 g, 1.50 mmol), Pd₂(dba)₃ (0.05 g, 0.05 mmol), 10% tri-tert-butylphosphine in toluene (200 mg, 0.10 mmol), sodium tert-butoxide (0.10 g, 1.00 mmol) and toluene (10 ml) were added to a reaction flask in sequence. The reaction was carried out at 100°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 0.20 g of a product with a yield of 66%.

### Step 3: Synthesis of tert-butyl 4-(3-(2,6-dioxopiperidin-3-yl)-5-fluoro-1-methyl-1H-indazol-6-yl)piperazine-1-carboxylate

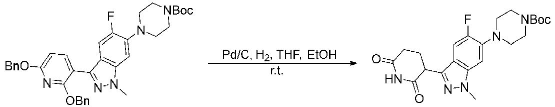

At room temperature, tert-butyl 4-(3-(2,6-bis(benzyloxy)pyridin-3-yl)-5-fluoro-1-methyl-1H-indazol-6-yl)piperazine-1-carboxylate (0.20 g, 0.33 mmol), 10% Pd/C (0.02 g), tetrahydrofuran (5 mL) and ethanol (5 ml) were added to a reaction flask in sequence, and the reaction was carried out at room temperature for 24 h. After the reaction was complete, the reaction mixture was filtered and dried by rotary evaporation to afford 0.13 g of a crude product, which was directly taken to the next step.

### Step 4: Synthesis of 3-(5-fluoro-1-methyl-6-(piperazin-1-yl)-1H-indazol-3-yl)piperazin-2,6-dione

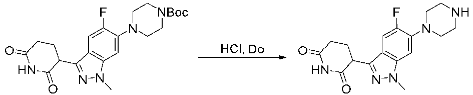

At room temperature, tert-butyl 4-(3-(2,6-dioxopiperidin-3-yl)-5-fluoro-1-methyl-1H-indazol-6-yl)piperazine-1-carboxylate (0.13 g, 0.30 mmol) and HCl in dioxane (5 ml) were added to a reaction flask in sequence, and the reaction was carried out at room temperature for 2 h. After the reaction was complete, the reaction mixture was dried by rotary evaporation to afford 0.11 g of a crude product, which was directly taken to the next step.

### Step 5: Synthesis of 3-(6-(4-(((1R,2R)-2-((4-(4-(1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-5-fluoro-1-methyl-1H-indazol-3-yl)piperidine-2,6-dione

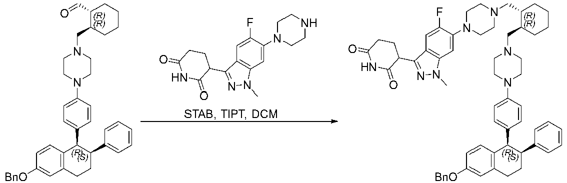

At room temperature, 3-(5-fluoro-1-methyl-6-(piperazin-1-yl)-1H-indazol-3-yl)piperazin-2,6-dione (0.15 g, 0.47 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.28 g, 0.47 mmol) and dichloromethane (5 mL) were added to a reaction flask in sequence, followed by TIPT (0.5 mL). The mixture was stirred at room temperature for 16 h, and then STAB (0.15 g, 0.71 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.21 g of a product with a three-step yield of 62%.

### Step 6: Synthesis of 3-(5-fluoro-6-(4-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperazin-2,6-dione

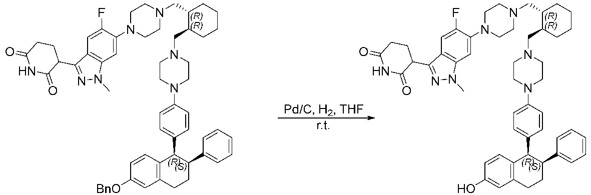

At room temperature, 3-(6-(4-(((1R,2R)-2-((4-(4-(1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-5-fluoro-1-methyl-1H-indazol-3-yl)piperidine-2,6-dione (0.21 g, 0.23 mmol), 10% palladium on carbon (0.10 g), and THF (3 mL) were added to a reaction flask in sequence. Hydrogen replacement was performed 3-4 times, and the mixture was stirred at room temperature for 24 h. After the reaction was complete, the reaction mixture was filtered through celite. The filter cake was rinsed with THF. The filtrate was stripped of solvent, and then subjected to column chromatography using a DCM/MeOH system to afford 0.07 g of a product with a yield of 37%.

¹H NMR (400 MHz, DMSO-*d*₆) *δ* 10.88 (s, 1H), 9.15 (d, *J* = 4.4 Hz, 1H), 7.48 (d, *J* = 12.6 Hz, 1H), 7.11 (dt, *J* = 15.7, 7.3 Hz, 4H), 6.83 (d, *J* = 7.2 Hz, 2H), 6.67 - 6.54 (m, 4H), 6.48 (dd, *J* = 8.3, 2.5 Hz, 1H), 6.23 (d, *J* = 8.1 Hz, 2H), 4.28 (dd, *J* = 9.8, 5.1 Hz, 1H), 4.14 (d, *J* = 5.0 Hz, 1H), 3.91 (d, *J* = 17.0 Hz, 3H), 3.24 - 2.84 (m, 15H), 2.77 - 2.53 (m, 7H), 2.41 - 2.29 (m, 2H), 2.26 - 1.98 (m, 3H), 1.88 - 1.40 (m, 7H), 1.28 - 1.15 (m, 3H), 1.07 - 0.93 (m, 2H). MS (ESI) m/z: 838.6 [M+H]⁺.

### Example 12 Synthesis of 3-(6-fluoro-5-(4-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperazin-2,6-dione (Compound VI-5)

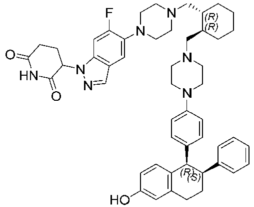

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 3-(5-bromo-6-fluoro-1H-indazol-1-yl)-1-(4-methoxyphenylmethyl)piperazine-2,6-dione

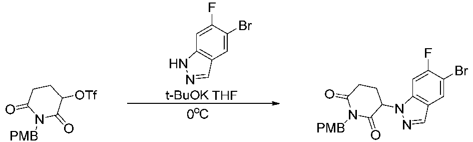

At room temperature, 5-bromo-6-fluoro-1H-indazole (0.50 g, 2.54 mmol), potassium tert-butoxide (0.34 g, 3.05 mmol), and tetrahydrofuran (15 ml) were added to a reaction flask in sequence. The mixture was cooled to 0°C and stirred for 30 min. Then, 1-(4-methoxybenzyl)-2,6-dioxopiperidin-3-yltrifluoromethanesulfonate (0.97 g, 2.54 mmol) was added. After the addition, the mixture was allowed to return to room temperature and the reaction was carried out for 3 h. After the reaction was complete, ethyl acetate and water were added. The layers were separated. The organic phase was dried, and then subjected to column chromatography using a PE/EA system to afford 0.47 g of a product with a yield of 43%.

### Step 2: Synthesis of tert-butyl 4-(6-fluoro-1-(1-(4-methoxybenzyl)-2,6-dioxopiperidin-3-yl)-1H-indazol-5-yl)piperazine-1-carboxylate

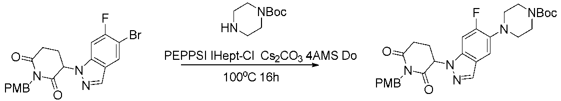

At room temperature, 3-(5-bromo-6-fluoro-1H-indazol-1-yl)-1-(4-methoxyphenylmethyl)piperazine-2,6-dione (0.47 g, 1.09 mmol), 1-Boc-piperazine (0.20 g, 1.09 mmol), PEPPSI IHept-Cl (0.11 g, 0.11 mmol), cesium carbonate (1.07 g, 3.28 mmol), 4A molecular sieve (0.11 g), and 1,4-dioxane (15 ml) were added to a reaction flask in sequence. Nitrogen replacement was performed 3-4 times. The mixture was heated to 100°C and the reaction was carried out for 16 h. After the reaction was complete, the reaction mixture was cooled to room temperature, and ethyl acetate and water were added. The layers were separated. The organic phase was dried, and then subjected to column chromatography using a PE/EA system to afford 0.13 g of a product with a yield of 21%.

### Step 3: Synthesis of 3-(6-fluoro-5-(piperazin-1-yl)-1H-indazol-1-yl)piperazin-2,6-dione

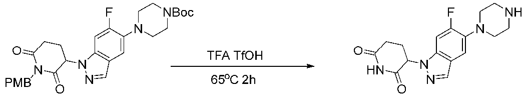

At room temperature, tert-butyl 4-(6-fluoro-1-(1-(4-methoxybenzyl)-2,6-dioxopiperidin-3-yl)-1H-indazol-5-yl)piperazine-1-carboxylate (0.13 g, 0.23 mmol), trifluoroacetic acid (3 ml), and TfOH (0.5 ml) were added to a reaction flask in sequence, and the mixture was heated to 65°C and stirred for 2 h. After the reaction was complete, the reaction mixture was stripped of solvent and subjected to reversed-phase column chromatography using an (acetonitrile/water) system to afford 0.07 g of a crude product, which was directly taken to the next step.

### Step 5: Synthesis of 3-(5-(4-(((1R,2R)-2-((4-(4-(1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-6-fluoro-1H-indazol-1-yl)piperidine-2,6-dione

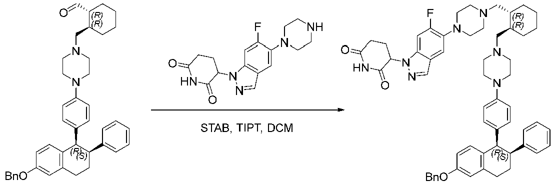

At room temperature, 3-(6-fluoro-5-(piperazin-1-yl)-1H-indazol-1-yl)piperazin-2,6-dione (0.15 g, 0.47 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.28 g, 0.47 mmol), and dichloromethane (5 mL) were added to a reaction flask in sequence, followed by TIPT (0.5 mL). The mixture was stirred at room temperature for 16 h, and then STAB (0.15 g, 0.71 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.21 g of a product with a three-step yield of 62%.

### Step 6: Synthesis of 3-(6-fluoro-5-(4-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperazin-2,6-dione

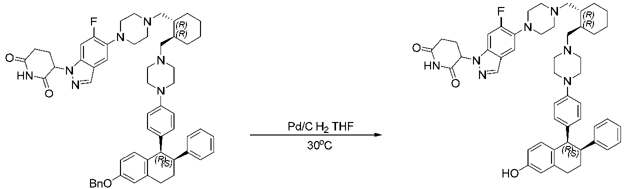

At room temperature, 3-(5-(4-(((1R,2R)-2-((4-(4-(1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-6-fluoro-1H-indazol-1-yl)piperidine-2,6-dione (0.21 g, 0.23 mmol), 10% palladium on carbon (0.10 g), and THF (3 mL) were added to a reaction flask in sequence. Hydrogen replacement was performed 3-4 times, and the mixture was stirred at room temperature for 24 h. After the reaction was complete, the reaction mixture was filtered through celite. The filter cake was rinsed with THF. The filtrate was stripped of solvent, and then subjected to column chromatography using a DCM/MeOH system to afford 0.07 g of a product with a yield of 37%.

¹H NMR (400 MHz, DMSO-*d*₆) δ 11.11 (s, 1H), 9.14 (s, 1H), 8.00 (s, 1H), 7.55 (d, *J* = 12.4 Hz, 1H), 7.37 (d, *J* = 8.2 Hz, 1H), 7.10 (d, *J* = 9.5 Hz, 2H), 6.81 (dd, *J* = 7.5, 3.7 Hz, 2H), 6.65 - 6.56 (m, 4H), 6.48 (dd, *J* = 8.3, 2.5 Hz, 1H), 6.26 - 6.21 (m, 2H), 5.76 (dd, *J* = 11.5, 5.1 Hz, 1H), 4.15 (d, *J* = 5.0 Hz, 1H), 3.17 - 2.89 (m, 17H), 2.85 - 2.55 (m, 8H), 2.26 - 2.21 (m, 1H), 2.11 - 2.05 (m, 1H), 1.81 - 1.43 (m, 8H), 1.26 - 1.16 (m, 3H), 1.06 - 0.95 (m, 2H). MS (ESI) m/z: 824.5 [M+H]⁺.

### Example 13 Synthesis of N-(2,6-dioxopiperidin-3-yl)-5-(1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)pyridinecarboxamide (Compound III-3)

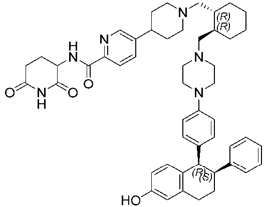

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 1'-(tert-butyl) 6-methyl 3',6'-dihydro-[3,4'-bipyridine]-1',6(2'H)-dicarboxylate

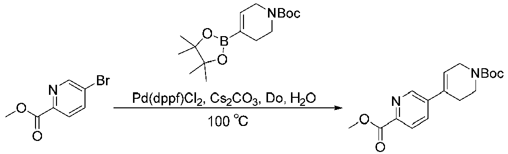

At room temperature, methyl 5-bromopyridine-2-carboxylate (1.00 g, 4.63 mmol), N-Boc-1,2,5,6-tetrahydropyridine-4-boronic acid pinacol ester (2.15 g, 6.94 mmol), Pd(dppf)Cl₂ (0.34 g, 0.46 mmol), Cs₂CO₃ (3.00 g, 9.26 mmol), Do (10 ml) and H₂O (2 ml) were added to a reaction flask in sequence. Nitrogen replacement was performed 3-4 times. The mixture was heated to 100°C and the reaction was carried out for 3 h. After the reaction was complete, the reaction mixture was cooled to room temperature, and filtered. The filtrate was concentrated, and subjected to column chromatography using a PE/EA system to afford 1.25 g of a product as a brown oil with a yield of 83%.

### Step 2: Synthesis of 1'-(tert-butoxycarbonyl)-1',2',3',6'-tetrahydro-[3,4'-bipyridine]-6-carboxylic acid

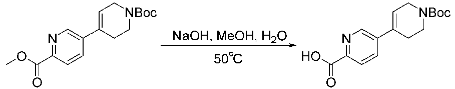

At room temperature, tert-butyl 4-(6-(methoxycarbonyl)pyridin-3-yl)piperazine-1-carboxylate (1.25 g, 3.93 mmol), NaOH (0.63 g, 15.72 mmol), MeOH (15 ml) and H₂O (5 ml) were added to a reaction flask in sequence. The mixture was heated to 50°C and the reaction was carried out for 4 h. After the reaction was complete, the reaction mixture was stripped of solvent, adjusted to acidity with an aqueous HCl solution, and extracted three times with DCM to afford 1.10 g of a yellow solid with a yield of 92%.

### Step 3: Synthesis of tert-butyl 6-((2,6-dioxopiperidin-3-yl)carbamoyl)-3',6'-dihydro-[3,4'-bipyridine]-1'(2'H)-carboxylate

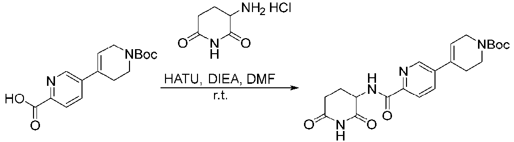

At room temperature, 1'-(tert-butyloxycarbonyl)-1',2',3',6'-tetrahydro-[3,4'-bipyridine]-6-carboxylic acid (0.20 g, 0.65 mmol), 3-aminopiperidine-2,6-dione hydrochloride (0.13 g, 0.78 mmol), HATU (0.37 g, 0.98 mmol), DIEA (0.25 g, 1.95 mmol), and DMF (3 ml) were added to a reaction flask in sequence, and the mixture was stirred at room temperature for 2 h. After the reaction was complete, water (3 mL) and saturated brine (3 mL) were added to the reaction solution. A large amount of solid precipitated out. After filtration, the filter cake was dissolved in DCM, dried over anhydrous magnesium sulfate, and then directly taken to the next step.

### Step 4: Synthesis of N-(2,6-dioxopiperidin-3-yl)-1',2',3',6'-tetrahydro-[3,4'-bipyridine]-6-carboxamide

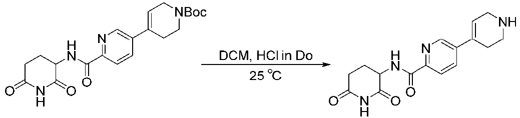

At room temperature, tert-butyl 6-((2,6-dioxopiperidin-3-yl)carbamoyl)-3',6'-dihydro-[3,4'-bipyridine]-1'(2'H)-carboxylate (0.27 mg, 0.65 mmol) and dichloromethane (2 ml) were added to a reaction flask in sequence. After complete dissolution, hydrochloric acid/dioxane (2 ml) was added, and the mixture was stirred at room temperature for 3 h. After the reaction was complete, the reaction mixture was stripped of solvent to afford 0.26 g of a crude product, which was directly taken to the next step.

### Step 5: Synthesis of 1'-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-N-(2,6-dioxopiperidin-3-yl)-1',2',3',6'-tetrahydro-[3,4'-bipyridine]-6-carboxamide

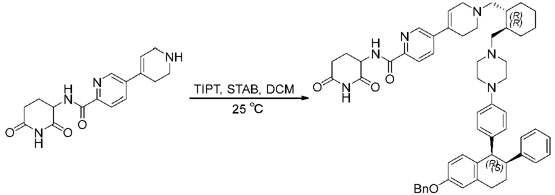

At room temperature, N-(2,6-dioxopiperidin-3-yl)-1',2',3',6'-tetrahydro-[3,4'-bipyridine]-6-carboxamide (0.15 g, 0.47 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.28 g, 0.47 mmol), and dichloromethane (5 ml) were added to a reaction flask in sequence, followed by TIPT (0.45 ml). The mixture was stirred at room temperature for 16 h, and then STAB (0.15 g, 0.71 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.21 g of a product with a three-step yield of 62%.

### Step 6: Synthesis of N-(2,6-dioxopiperidin-3-yl)-5-(1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)pyridine-2-carboxamide

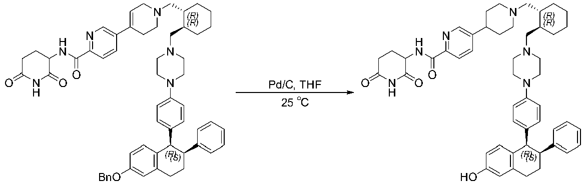

At room temperature, 1'-(((1R,2R)-2-((4-(4-((1R,2S)-6-(benzyloxy)-2-phenyl-1,2,3,4-tetrahy dronaphthalen-1-yl)phenyl)piperazin-1-yl)methyl)cyclohexyl)methyl)-N-(2,6-dioxopiperidin-3-y l)-1',2',3',6'-tetrahydro-[3,4'-bipyridine]-6-carboxamide (0.16 g, 0.18 mmol), 10% palladium on c arbon (0.05 g), and THF (3 ml) were added to a reaction flask in sequence. Hydrogen replacemen t was performed 3-4 times, and the mixture was stirred at room temperature for 24 h. After the rea ction was complete, the reaction mixture was filtered through celite. The filter cake was rinsed wi th THF. The filtrate was stripped of solvent, and then subjected to column chromatography using a DCM/MeOH system to afford 0.033 g (0.028 g stored) of a product with a yield of 22.9%.

¹H NMR (400 MHz, DMSO-d6) δ 10.88 (s, 1H), 9.15 (s, 1H), 9.03 (d, J = 8.5 Hz, 1H), 8.58 ( s, 1H), 8.01 (d, J = 8.2 Hz, 1H), 7.88 (d, J = 8.4 Hz, 1H), 7.11 (q, J = 10.0, 8.6 Hz, 3H), 6.82 (d, J = 7.3 Hz, 2H), 6.64 - 6.60 (m, 2H), 6.55 (d, J = 8.5 Hz, 2H), 6.48 (d, J = 8.5 Hz, 1H), 6.22 (d, J = 8.2 Hz, 2H), 4.79 (t, J = 6.8 Hz, 1H), 4.14 (d, J = 5.1 Hz, 1H), 3.17 (s, 1H), 3.03 - 2.96 (m, 4H), 2.82 - 2.75 (m, 2H), 2.54 (s, 2H), 2.22 (dd, J = 12.6, 4.2 Hz, 3H), 2.12 - 1.96 (m, 4H), 1.87 (d, J = 37.5 Hz, 8H), 1.72 - 1.66 (m, 2H), 1.62 (s, 2H), 1.50 (dd, J = 12.5, 7.1 Hz, 2H), 1.40 (s, 1H), 1. 22 (d, J = 11.7 Hz, 4H), 1.00 (p, J = 9.6, 8.5 Hz, 3H), 0.85 (q, J = 8.7, 7.9 Hz, 2H). MS (ESI) m/z: 809.6 [M+H]+.

### Example 14 Synthesis of 3-(5-(1-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperidin-4-yl)-3-methyl-1H-indazol-1-yl)piperidin-2,6-dione (Compound VI-8)

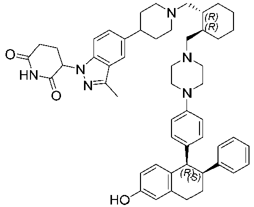

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 5-(4-(((1S,2S)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperazin-1-yl)-N-(2,6-dioxopiperidin-3-yl)pyridine-3-carboxamide

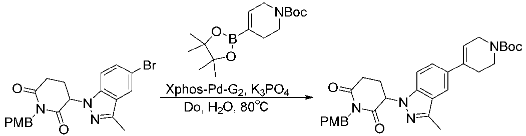

At room temperature, 3-(5-bromo-3-methyl-1H-indazol-1-yl)-1-(4-methoxybenzyl)piperidine-2,6-dione (0.40 g, 0.90 mmol), N-Boc-1,2,5,6-tetrahydropyridine-4-boronic acid pinacol ester (0.28 g, 0.90 mmol), Xphos-Pd-G2 (0.07 g, 0.09 mmol), K₃PO₄ (0.38 g, 1.81 mmol), Do (5 mL) and H₂O (1 ml) were added to a reaction flask in sequence. Nitrogen replacement was performed 3-4 times. The mixture was heated to 80°C and the reaction was carried out for 2 h. After the reaction was complete, the reaction mixture was cooled to room temperature, concentrated, and subjected to column chromatography using a PE/EA system to afford 0.30 g of a white solid with a yield of 61%.

### Step 2: Synthesis of 3-(3-methyl-5-(1,2,3,6-tetrahydropyridin-4-yl)-1H-indazol-1-yl)piperidine-2,6-dione

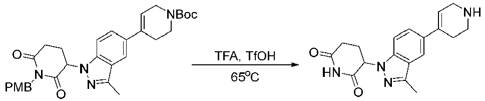

At room temperature, 5-(4-(((1S,2S)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperazin-1-yl)-N-(2,6-dioxopiperidin-3-yl)pyridine-3-carboxamide (0.27 g, 0.50 mmol) was dissolved in TFA (6 mL), and TfOH (1 mL) was added. The reaction was carried out at 65°C for 5 h. After the reaction was complete, the reaction solution was concentrated and purified by reversed-phase column chromatography (H₂O/ACN) to afford 0.12 g of a yellow solid with a yield of 74.6%.

### Step 3: Synthesis of 3-(5-(1-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)-1,2,3,6-tetrahydropyridin-4-yl)-3-methyl-1H-indazol-1-yl)piperidine-2,6-dione

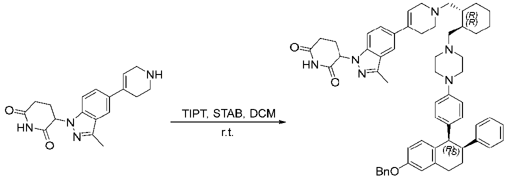

At room temperature, 3-(3-methyl-5-(1,2,3,6-tetrahydropyridin-4-yl)-1H-indazol-1-yl)piperidin-2,6-dione (0.12 g, 0.37 mmol), (1S,2S)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.22 g, 0.37 mmol) and dichloromethane (5 ml) were added to a reaction flask in sequence, followed by TIPT (0.4 ml). The mixture was stirred at room temperature for 16 h, and then STAB (0.12 g, 0.55 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.06 g of a white solid with a yield of 16.4%.

### Step 4: Synthesis of 3-(5-(1-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperidin-4-yl)-3-methyl-1H-indazol-1-yl)piperidin-2,6-dione

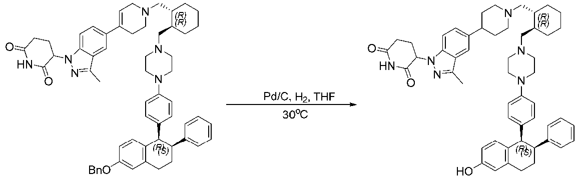

At room temperature, 3-(5-(1-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)-1,2,3,6-tetrahydropyridin-4-yl)-3-methyl-1H-indazol-1-yl)piperidin-2,6-dione (0.10 g, 0.11 mmol), 10% palladium on carbon (0.05 g), and THF (3 ml) were added to a reaction flask in sequence. Hydrogen replacement was then performed 3-4 times, and the mixture was stirred at 30°C for 24 h. After the reaction was complete, the reaction mixture was filtered through celite. The filter cake was rinsed with THF. The filtrate was stripped of solvent, and then subjected to column chromatography using a DCM/MeOH system to afford 0.006 g (0.002 g stored) of a product with a yield of 6.7%.

¹H NMR (400 MHz, DMSO-d6) δ 11.07 (s, 1H), 9.15 (d, J = 7.1 Hz, 1H), 7.51 - 7.43 (m, 2H), 7.31 (d, J = 8.6 Hz, 1H), 7.08 (d, J = 7.3 Hz, 2H), 6.81 (d, J = 7.8 Hz, 2H), 6.64 - 6.57 (m, 4H), 6.48 (dd, J = 8.2, 2.6 Hz, 1H), 6.24 (d, J = 8.2 Hz, 2H), 5.73 (dd, J = 11.8, 5.2 Hz, 1H), 4.39 (t, J = 5.2 Hz, 1H), 4.15 (d, J = 5.1 Hz, 1H), 2.98 - 2.85 (m, 7H), 2.72 (qd, J = 13.9, 11.0, 4.5 Hz, 5H), 2.37 - 2.29 (m, 3H), 2.21 (q, J = 8.3, 7.4 Hz, 2H), 2.14 - 1.88 (m, 6H), 1.78 - 1.65 (m, 6H), 1.43 (h, J = 3.1 Hz, 2H), 1.30 (s, 8H), 1.10 - 0.77 (m, 4H). MS (ESI) m/z: 819.7 [M+H]⁺.

### Example 15 Synthesis of 3-(6-fluoro-5-(1-(((1R,2R)-2-((4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-3-methyl-1H-indazol-1-yl)piperidin-2,6-dione (Compound VI-9)

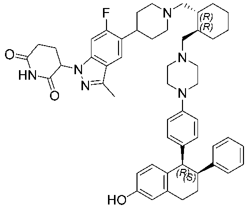

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 3-(5-bromo-6-fluoro-3-methyl-1h-indazol-1-yl)-1-(4-methoxybenzyl)piperidine-2,6-dione

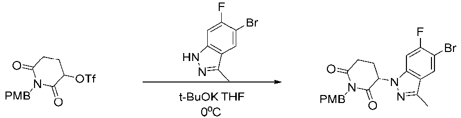

At room temperature, 5-bromo-6-fluoro-3-methylindazole (0.50 g, 2.18 mmol), potassium tert-butoxide (0.29 g, 2.62 mmol), and tetrahydrofuran (15 ml) were added to a reaction flask in sequence. The mixture was cooled to 0°C and stirred for 30 min. Then, 1-(4-methoxybenzyl)-2,6-dioxopiperidin-3-yltrifluoromethanesulfonate (0.83 g, 2.18 mmol) was added. After the addition, the mixture was allowed to return to room temperature and the reaction was carried out for 3 h. After the reaction was complete, ethyl acetate and water were added. The layers were separated. The organic phase was dried, and then subjected to column chromatography using a PE/EA system to afford 0.88 g of a product with a yield of 44%.

### Step 2: Synthesis of tert-butyl 4-(6-fluoro-1-(1-(4-methoxybenzyl)-2,6-dioxopiperidin-3-yl)-3-methyl-1H-indazol-5-yl)-3,6-dihydropyridine-1(2H)-carboxylate

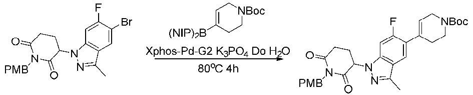

At room temperature, 3-(5-bromo-6-fluoro-3-methyl-1h-indazol-1-yl)-1-(4-methoxybenzyl)piperidin-2,6-dione (0.88 g, 1.91 mmol), N-Boc-1,2,5,6-tetrahydropyridine-4-boronic acid pinacol ester (0.59 g, 1.91 mmol), Xphos-Pd-G2 (0.16 g, 0.20 mmol), potassium phosphate (0.81 g, 3.82 mmol), water (3 ml) and 1,4-dioxane (10 ml) were added to a reaction flask in sequence. Nitrogen replacement was performed 3-4 times. The mixture was heated to 80°C and the reaction was carried out for 4 h. After the reaction was complete, the reaction mixture was cooled to room temperature, stripped of solvent, diluted with ethyl acetate, and filtered through celite. The filtrate was dried, stripped of solvent, and subjected to column chromatography using a PE/EA system to afford 0.80 g of a product with a yield of 80%.

### Step 3: Synthesis of tert-butyl 4-(6-fluoro-1-(1-(4-methoxybenzyl)-2,6-dioxopiperidin-3-yl)-3-methyl-1H-indazol-5-yl)piperidin-1-carboxylate

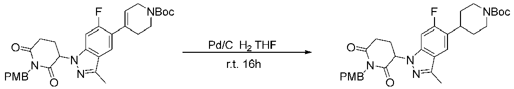

At room temperature, tert-butyl 4-(6-fluoro-1-(1-(4-methoxybenzyl)-2,6-dioxopiperidin-3-yl)-3-methyl-1H-indazol-5-yl)-3,6-dihydropyridine-1(2H)-carboxylate (0.80 g, 1.42 mmol) and tetrahydrofuran (15 ml) were added to a reaction flask in sequence, followed by 10% palladium on carbon (0.08 g). Hydrogen replacement was performed 3-4 times, and the mixture was stirred at room temperature for 16 h. After the reaction was complete, the reaction mixture was filtered through celite, and stripped of solvent to afford 0.50g of a product, which was directly taken to the next step.

### Step 4: Synthesis of 3-(6-fluoro-3-methyl-5-(piperidin-4-yl)-1h-indazol-1-yl)piperidin-2,6-dione

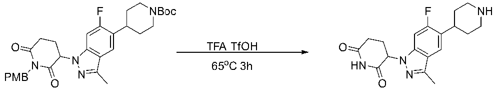

At room temperature, tert-butyl 4-(6-fluoro-1-(1-(4-methoxybenzyl)-2,6-dioxopiperidin-3-yl)-3-methyl-1H-indazol-5-yl)piperidin-1-carboxylate (0.50 g, 0.89 mmol), trifluoroacetic acid (3 ml), and TfOH (0.5 ml) were added to a reaction flask in sequence, and the mixture was heated to 65°C and stirred for 2 h. After the reaction was complete, the reaction mixture was stripped of solvent and subjected to reversed-phase column chromatography using an (acetonitrile/water) system to afford 0.20 g of a crude product, which was directly taken to the next step.

### Step 5: Synthesis of 3-(5-(1-(((1R,2R)-2-((4-((1R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-6-fluoro-3-methyl-1H-indazol-1-yl)piperidin-2,6-dione

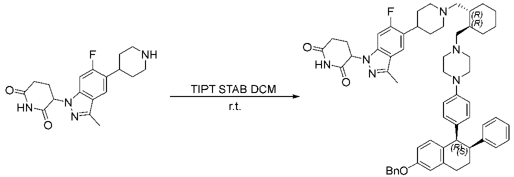

At room temperature, 3-(6-fluoro-3-methyl-5-(piperidin-4-yl)-1h-indazol-1-yl)piperidin-2,6-dione (0.20 g, 0.58 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.35 g, 0.58 mmol), and dichloromethane (10 ml) were added to a reaction flask in sequence, followed by TIPT (0.5 ml). The mixture was stirred at room temperature for 16 h, and then STAB (0.25 g, 0.1.16 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.15 g of a product with a yield of 28%.

### Step 6: Synthesis of 3-(6-fluoro-5-(1-(((1R,2R)-2-((4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-3-methyl-1H-indazol-1-yl)piperidin-2,6-dione

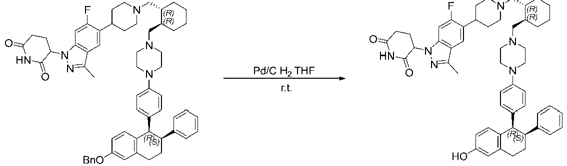

At room temperature, 3-(5-(1-(((1R,2R)-2-((4-((1R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-6-fluoro-3-methyl-1H-indazol-1-yl)piperidin-2,6-dione (0.15 g, 0.16 mmol), 10% palladium on carbon (0.02 g), and tetrahydrofuran (10 ml) were added to a reaction flask in sequence. Hydrogen gas was then added to replace the flask 3-4 times, and the mixture was stirred at room temperature for 24 h. After the reaction was complete, the reaction mixture was filtered through celite. The filter cake was rinsed with tetrahydrofuran. The filtrate was stripped of solvent, and then subjected to column chromatography using a DCM/MeOH system to afford 0.05 g of a product with a yield of 37%.

¹H NMR (400 MHz, DMSO-*d*₆) δ 11.09 (s, 1H), 9.15 (s, 1H), 7.55 - 7.38 (m, 2H), 7.09 (q, *J* = 6.7 Hz, 2H), 7.01 (d, *J* = 7.2 Hz, 1H), 6.81 (d, *J* = 7.5 Hz, 2H), 6.67 - 6.58 (m, 4H), 6.48 (d, *J* = 8.2 Hz, 1H), 6.25 (d, *J* = 8.2 Hz, 2H), 5.74 - 5.61 (m, 1H), 4.16 (d, *J* = 4.9 Hz, 1H), 3.79 - 3.47 (m, 6H), 3.25 - 3.11 (m, 5H), 3.04 - 2.85 (m, 6H), 2.81 - 2.65 (m, 6H), 2.29 (s, 3H), 2.25 - 2.19 (m, 1H), 2.12 - 1.95 (m, 4H), 1.76 - 1.51 (m, 7H), 1.35 - 1.13 (m, 3H), 1.09 - 0.94 (m, 2H). MS (ESI) m/z: 837.5 [M+H]⁺.

### Example 16 Synthesis of 3-(5-fluoro-6-(1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)piperidin-2,6-dione (Compound IV-9)

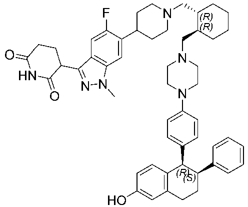

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 3-(2,6-bis(benzyloxy)pyridin-3-yl)-6-bromo-5-fluoro-1-methyl-1H-indazole

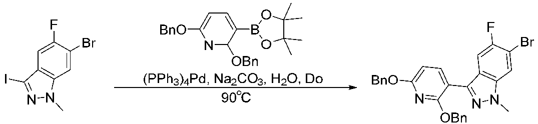

At room temperature, 6-bromo-5-fluoro-3-iodo-1-methyl-1H-indazole (1.00 g, 2.97 mmol), 2,6-bis(benzyloxy)-3-(4,4,5,5-tetramethyl-1,3,2-dioxaborolan-2-yl)pyridine (1.24 g, 2.97 mmol), sodium carbonate (0.94 g, 8.90 mmol), tetrakis(triphenylphosphine)palladium (0.34 g, 0.30 mmol), water (1 mL), and dioxane (10 ml) were added to a reaction flask in sequence. The reaction was carried out at 90°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 1.20 g of a product with a yield of 81%.

### Step 2: Synthesis of tert-butyl 4-(3-(2,6-bis(benzyloxy)pyridin-3-yl)-5-fluoro-1-methyl-1H-indazol-6-yl)-3,6-dihydropyridine-1(2H)-carboxylate

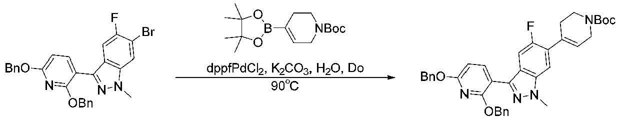

At room temperature, tert-butyl 3-(2,6-bis(benzyloxy)pyridin-3-yl)-6-bromo-5-fluoro-1-methyl-1H-indazole (0.60 g, 1.16 mmol), 4-(4,4,5,5-tetramethyl-1,3,2-dioxaborolan-2-yl)-3,6-dihydropyridine-1(2H)-carboxylate (0.40 g, 1.27 mmol), dppfPdCl₂ (0.09 g, 0.12 mmol), cesium carbonate (0.48 g, 3.47 mmol), water (1 mL) and dioxane (10 ml) were added to a reaction flask in sequence. The reaction was carried out at 90°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 0.47 g of a product with a yield of 66%.

### Step 3: Synthesis of tert-butyl 4-(3-(2,6-dioxopiperidin-3-yl)-5-fluoro-1-methyl-1H-indol-6-yl)piperidine-1-carboxylate

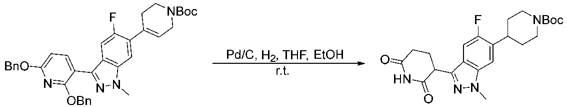

At room temperature, tert-butyl 4-(3-(2,6-bis(benzyloxy)pyridin-3-yl)-5-fluoro-1-methyl-1H-indazol-6-yl)-3,6-dihydropyridine-1(2H)-carboxylate (0.40 g, 0.66 mmol), 10% Pd/C (0.04 g), tetrahydrofuran (5 mL) and ethanol (5 ml) were added to a reaction flask in sequence, and the reaction was carried out at room temperature for 24 h. After the reaction was complete, the reaction mixture was filtered and dried by rotary evaporation to afford 0.26 g of a crude product, which was directly taken to the next step.

### Step 4: Synthesis of 3-(5-fluoro-1-methyl-6-(piperidin-4-yl)-1H-indol-3-yl)piperidin-2,6-dione

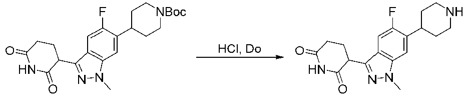

At room temperature, tert-butyl 4-(3-(2,6-dioxopiperidin-3-yl)-5-fluoro-1-methyl-1H-indol-6-yl)piperidine-1-carboxylate (0.26 g, 0.60 mmol) and HCl in dioxane (5 ml) were added to a reaction flask in sequence, and the reaction was carried out at room temperature for 2 h. After the reaction was complete, the reaction mixture was dried by rotary evaporation to afford 0.22 g of a crude product, which was directly taken to the next step.

### Step 5: Synthesis of 3-(6-(1-(((1R,2R)-2-((4-(4-(((2R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-5-fluoro-1-methyl-1H-indazol-3-yl)piperidin-2,6-dione

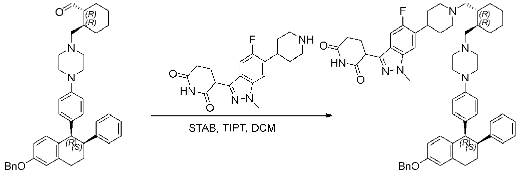

At room temperature, 3-(5-fluoro-1-methyl-6-(piperidin-4-yl)-1H-indol-3-yl)piperidin-2,6-dione (0.15 g, 0.47 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.28 g, 0.47 mmol) and dichloromethane (5 mL) were added to a reaction flask in sequence, followed by TIPT (0.5 mL). The mixture was stirred at room temperature for 16 h, and then STAB (0.15 g, 0.71 mmol) was added. The reaction was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.21 g of a product with a three-step yield of 62%.

### Step 6: Synthesis of 3-(5-fluoro-6-(1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)piperidin-2,6-dione

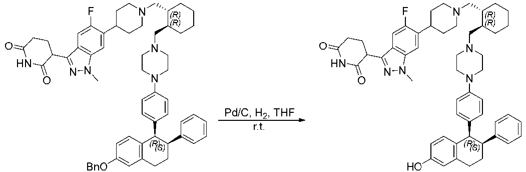

At room temperature, 3-(6-(1-(((1R,2R)-2-((4-(4-(((2R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-5-fluoro-1-methyl-1H-indazol-3-yl)piperidin-2,6-dione (0.21 g, 0.23 mmol), 10% palladium on carbon (0.10 g), and THF (3 mL) were added to a reaction flask in sequence. Hydrogen replacement was performed 3-4 times, and the mixture was stirred at room temperature for 24 h. After the reaction was complete, the reaction mixture was filtered through celite. The filter cake was rinsed with THF. The filtrate was stripped of solvent, and then subjected to column chromatography using a DCM/MeOH system to afford 0.11 g of a product with a yield of 58%.

¹H NMR (400 MHz, DMSO-*d*₆) *δ* 10.90 (s, 1H), 9.15 (s, 1H), 7.46 (d, *J* = 53.3 Hz, 2H), 7.04 (d, *J* = 39.6 Hz, 3H), 6.82 (d, *J* = 7.5 Hz, 2H), 6.63 (t, *J* = 6.3 Hz, 4H), 6.49 (dd, *J* = 8.2, 2.7 Hz, 1H), 6.23 (d, *J* = 8.1 Hz, 2H), 4.34 (dd, *J* = 10.5, 5.0 Hz, 1H), 4.15 (d, *J* = 5.0 Hz, 1H), 3.90 (d, *J* = 46.4 Hz, 3H), 3.63 (s, 1H), 3.22 - 2.86 (m, 12H), 2.75 - 2.57 (m, 4H), 2.42 - 2.26 (m, 3H), 2.17 - 1.41 (m, 17H), 1.08 - 0.78 (m, 3H). MS (ESI) m/z: 837.7 [M+H]⁺.

### Example 17 Synthesis of N-(2,6-dioxopiperidin-3-yl)-5-(4-(((1S,2S)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)pyridinecarboxamide (Compound III-2)

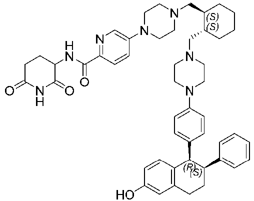

The synthesis method of (1S, 2S)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 5-(4-(((1S,2S)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperazin-1-yl)-N-(2,6-dioxopiperidin-3-yl)pyridine-3-carboxamide

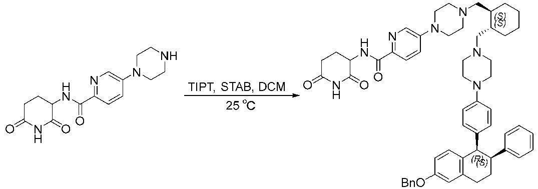

At room temperature, N-(2,6-dioxopiperidin-3-yl)-5-(piperazin-1-yl)pyridine-3-carboxamide (0.09 g, 0.28 mmol), (1S,2S)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.17 g, 0.28 mmol), and dichloromethane (5 ml) were added to a reaction flask in sequence, followed by TIPT (0.3 ml). The mixture was stirred at room temperature for 16 h, and then STAB (0.09 g, 0.42 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.09 g of a product with a yield of 35%.

### Step 2: Synthesis of N-(2,6-dioxopiperidin-3-yl)-5-(4-(((1S,2S)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperazin-1-yl)pyridinecarboxamide

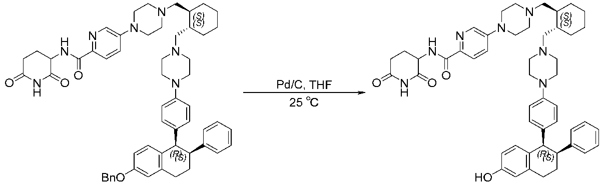

At room temperature, 5-(4-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperazin-1-yl)-N-(2,6-dioxopiperidin-3-yl)pyridine-3-carboxamide (0.09 g, 0.10 mmol), 10% palladium on carbon (0.05 g), and THF (3 ml) were added to a reaction flask in sequence. Hydrogen replacement was performed 3-4 times, and the mixture was stirred at room temperature for 24 h. After the reaction was complete, the reaction mixture was filtered through celite. The filter cake was rinsed with THF. The filtrate was stripped of solvent, and then subjected to column chromatography using a DCM/MeOH system to afford 0.03 g of a product with a yield of 37%.

¹H NMR (400 MHz, DMSO-d6) δ 10.87 (s, 1H), 9.13 (s, 1H), 8.74 (d, J = 8.3 Hz, 1H), 8.31 (d, J = 2.8 Hz, 1H), 7.86 (d, J = 8.8 Hz, 1H), 7.41 (dd, J = 9.0, 2.9 Hz, 1H), 7.13 (dd, J = 9.7, 6.7 Hz, 3H), 6.84 (d, J = 7.1 Hz, 2H), 6.65 - 6.59 (m, 2H), 6.53 (d, J = 8.3 Hz, 2H), 6.48 (dd, J = 8.3, 2.5 Hz, 1H), 6.21 (d, J = 8.3 Hz, 2H), 4.74 (td, J = 8.0, 4.1 Hz, 1H), 4.14 (d, J = 5.0 Hz, 1H), 3.22 (d, J = 42.3 Hz, 4H), 3.08 - 2.62 (m, 10H), 2.53 (s, 5H), 2.31 (t, J = 7.4 Hz, 2H), 2.23 - 1.95 (m, 6H), 1.93 - 1.64 (m, 4H), 1.57 (s, 2H), 1.44 (d, J = 8.7 Hz, 2H), 1.22 (d, J = 12.2 Hz, 2H), 0.98 (h, J = 7.2 Hz, 2H). MS (ESI) m/z: 810.7 [M+H]⁺.

### Example 18 Synthesis of 3-(6-(4-(((1S,2S)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperidine-2,6-dione (Compound IV-4)

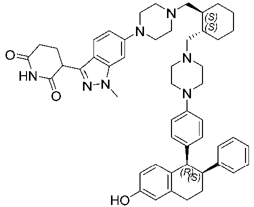

The synthesis method of (1S, 2S)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 3-(2,6-bis(benzyloxy)pyridin-3-yl)-6-bromo-1-methyl-1H-indazole

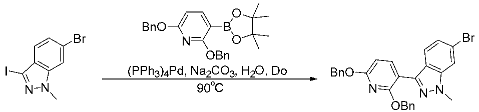

At room temperature, 6-bromo-3-iodo-1-methyl-1H-indazole (1.00 g, 2.97 mmol), 2,6-bis(benzyloxy)-3-(4,4,5,5-tetramethyl-1,3,2-dioxaborolan-2-yl)pyridine (1.24 g, 2.97 mmol), sodium carbonate (0.94 g, 8.90 mmol), tetrakis(triphenylphosphine)palladium (0.34 g, 0.30 mmol), water (1 mL), and dioxane (10 ml) were added to a reaction flask in sequence. The reaction was carried out at 90°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 1.20 g of a product with a yield of 81%.

### Step 2: Synthesis of tert-butyl 4-(3-(2,6-bis(benzyloxy)pyridin-3-yl)-1-methyl-1H-indazol-6-yl)piperazine-1-carboxylate

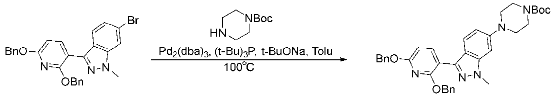

At room temperature, 3-(2,6-bis(benzyloxy)pyridin-3-yl)-6-bromo-1-methyl-1H-indazole (0.25 g, 0.50 mmol), 1-Boc-piperazine (0.28 g, 1.50 mmol), Pd₂(dba)₃ (0.05 g, 0.05 mmol), 10% tri-tert-butylphosphine in toluene (200 mg, 0.10 mmol), sodium tert-butoxide (0.10 g, 1.00 mmol) and toluene (10 ml) were added to a reaction flask in sequence. The reaction was carried out at 100°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 0.20 g of a product with a yield of 66%.

### Step 3: Synthesis of tert-butyl 4-(3-(2,6-dioxopiperidin-3-yl)-1-methyl-1H-indazol-6-yl)piperazine-1-carboxylate

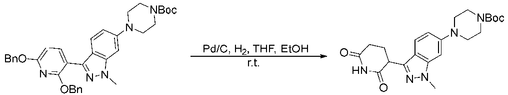

At room temperature, tert-butyl 4-(3-(2,6-bis(benzyloxy)pyridin-3-yl)-1-methyl-1H-indazol-6-yl)piperazine-1-carboxylate (0.20 g, 0.33 mmol), 10% Pd/C (0.02 g), tetrahydrofuran (5 mL) and ethanol (5 ml) were added to a reaction flask in sequence, and the reaction was carried out at room temperature for 24 h. After the reaction was complete, the reaction mixture was filtered and dried by rotary evaporation to afford 0.13 g of a crude product, which was directly taken to the next step.

### Step 4: Synthesis of 3-(1-methyl-6-(piperazin-1-yl)-1H-indazol-3-yl)piperazin-2,6-dione

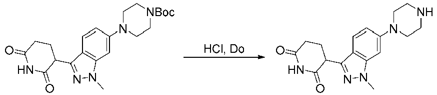

At room temperature, tert-butyl 4-(3-(2,6-dioxopiperidin-3-yl)-1-methyl-1H-indazol-6-yl)piperazine-1-carboxylate (0.13 g, 0.30 mmol) and HCl in dioxane (5 ml) were added to a reaction flask in sequence, and the reaction was carried out at room temperature for 2 h. After the reaction was complete, the reaction mixture was dried by rotary evaporation to afford 0.11 g of a crude product, which was directly taken to the next step.

### Step 5: Synthesis of 3-(6-(4-(((1S,2S)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperazin-2,6-dione

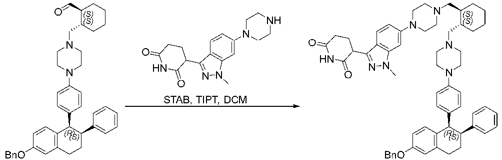

At room temperature, 3-(1-methyl-6-(piperazin-1-yl)-1H-indazol-3-yl)piperazin-2,6-dione (0.06 g, 0.18 mmol), (1S,2S)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.11 g, 0.18 mmol) and dichloromethane (10 ml) were added to a reaction flask in sequence, followed by TIPT (0.5 ml). The mixture was stirred at room temperature for 16 h, and then STAB (0.08 g, 0.36 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.03 g of a product with a yield of 27%.

### Step 6: Synthesis of 3-(6-(4-(((1S,2S)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperidine-2,6-dione

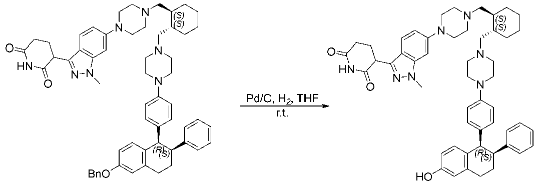

At room temperature, 3-(6-(4-(((1S,2S)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-1-methyl-1H-indazol-3-yl)piperazin-2,6-dione (0.03 g, 0.03 mmol), 10% palladium on carbon (0.01 g), and tetrahydrofuran (5 ml) were added to a reaction flask in sequence. Hydrogen replacement was then performed 3-4 times, and the mixture was stirred at 30°C for 16 h. After the reaction was complete, the reaction mixture was filtered through celite. The filter cake was rinsed with tetrahydrofuran. The filtrate was stripped of solvent, and then subjected to column chromatography using a DCM/MeOH system to afford 0.01 g of a product with a yield of 37%.

¹H NMR (400 MHz, DMSO-*d*₆) *δ* 10.87 (s, 1H), 9.12 (s, 1H), 7.50 (d, *J* = 8.9 Hz, 1H), 7.17 - 7.10 (m, 3H), 6.91 (dd, *J* = 9.0, 1.9 Hz, 1H), 6.86 - 6.81 (m, 3H), 6.66 - 6.60 (m, 2H), 6.55 - 6.46 (m, 3H), 6.21 (d, *J* = 8.3 Hz, 2H), 4.26 (dd, *J* = 9.2, 5.1 Hz, 1H), 4.14 (d, *J* = 5.0 Hz, 1H), 3.88 (s, 3H), 3.30 - 3.15 (m, 6H), 3.01 - 2.88 (m, 6H), 2.69 - 2.56 (m, 3H), 2.47 - 2.24 (m, 8H), 2.19 - 2.04 (m, 4H), 1.92 - 1.81 (m, 2H), 1.73 - 1.68 (m, 1H), 1.61 - 1.53 (m, 2H), 1.49 - 1.39 (m, 2H), 1.23 - 1.15 (m, 2H), 1.08 - 0.68 (m, 3H). MS (ESI) m/z: 821.0[M+H]⁺.

### Example 19 Synthesis of 3-(6-fluoro-5-(4-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperazin-1-yl)-1H-benzimidazol-1-yl)piperidine-2,6-dione (Compound V-2)

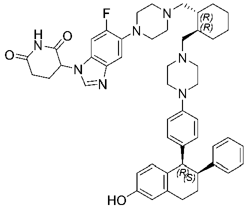

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 2,6-bis(benzyloxy)-3-nitropyridine

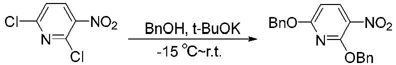

At room temperature, BnOH (7.57 g, 69.95 mmol) was dissolved in THF (25 mL). The solution was cooled to -15°C. Potassium tert-butoxide (7.00 g, 62.18 mmol) was added. The mixture was stirred at room temperature for 30 min. At -15°C, the above reaction solution was added dropwise to 2,6-dichloro-3-nitropyridine (5.00 g, 25.91 mmol) in THF (15 mL). After the addition, the reaction was carried out at room temperature for 16 h. After the reaction was complete, the reaction solution was poured into ice water, stirred for 30 min, and filtered. The filter cake was slurried with methyl tert-butyl ether to afford 6.50 g of a white solid with a yield of 74.6%.

### Step 2: Synthesis of 2,6-bis(benzyloxy)pyridine-3-amine

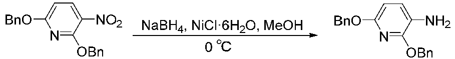

At room temperature, 2,6-bis(benzyloxy)-3-nitropyridine (6.50 g, 19.32 mmol) was dissolved in THF (10 mL) and MeOH (10 mL), and NiCl₂·6H₂O (0.45 g, 1.93 mmol) was added. The mixture was cooled to 0°C, and NaBH₄ (1.82 g, 48.31 mmol) was slowly added. The reaction was carried out in an ice bath for 1 h. After the reaction was complete, the reaction was quenched by adding ice water (1 mL). The reaction solution was concentrated and subjected to column chromatography (EA/PE) to afford 4.41 g of a colorless oil with a yield of 74.5%.

### Step 3: Synthesis of 2,6-bis(benzyloxy)-N-(4-bromo-5-fluoro-2-nitrophenyl)pyridine-3-amine

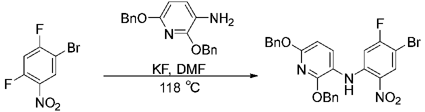

At room temperature, 2,4-difluoro-5-bromonitrobenzene (4.41 g, 13.06 mmol), 2,6-bis(benzyloxy)pyridine-3-amine (4.00 g, 13.06 mmol), KF (0.90 g, 15.67 mmol) and DMF (20 ml) were added to a reaction flask in sequence. The mixture was heated to 118 °C and the reaction was carried out for 8 h. After the reaction was complete, the reaction solution was cooled to room temperature, and water (20 mL) was added to the reaction solution to precipitate a solid. The solid was filtered, and the filter cake was slurried with methyl tert-butyl ether to afford 3.5 g of a yellow solid with a yield of 35.9%.

### Step 4: Synthesis of N¹-2,6-bis(benzyloxy)pyridin-3-yl)-4-bromo-5-fluorobenzene-1,2-diamine

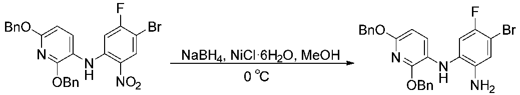

At room temperature, 2,6-bis(benzyloxy)-N-(4-bromo-5-fluoro-2-nitrophenyl)pyridine-3-amine (3.50 g, 6.67 mmol) was dissolved in THF (20 mL) and MeOH (20 mL), and NiCl₂·6H₂O (0.16 g, 1.67 mmol) was added. The mixture was cooled to 0 °C, and NaBH₄ (0.63 g, 16.69 mmol) was slowly added. The reaction was carried out in an ice bath for 1 h. After the reaction was complete, the reaction was quenched by adding ice water (1 mL). The reaction solution was concentrated and subjected to column chromatography (EA/PE) to afford 3.25 g of a brown oil with a yield of 98.8%.

### Step 5: Synthesis of 1-(2,6-bisbenzyloxy)pyridin-3-yl)-5-bromo-6-fluoro-1H-benzimidazole

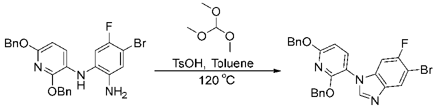

At room temperature, N¹-(2,6-bis(benzyloxy)pyridin-3-yl)-4-bromo-5-fluorobenzene-1,2-diamine (3.25 g, 6.57 mmol) was dissolved in toluene (30 mL), and trimethyl orthoformate (1.74 g, 16.44 mmol) and TsOH (0.23 g, 1.31 mmol) were added. The reaction was carried out at 120 °C for 1 h. After the reaction was complete, the reaction solution was cooled to room temperature, concentrated, and subjected to column chromatography (EA/PE) to give 3.00 g of a brown oil with a yield of 90.5%.

### Step 6: Synthesis of tert-butyl 4-(1-(2,6-bisbenzyloxy)pyridin-3-yl)-6-fluoro-1H-benzimidazol-5-yl)piperazine-1-carboxylate

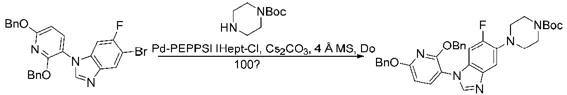

At room temperature, 1-(2,6-bisbenzyloxy)pyridin-3-yl)-5-bromo-6-fluoro-1H-benzimidazole (0.60 g, 1.19 mmol), N-Boc-piperazine (0.44 g, 2.38 mmol), Pd-PEPPSI IHept-Cl (0.12 g, 0.12 mmol), Cs2CO3 (1.16 g, 3.57 mmol), 4 Å MS (0.60 g), and Do (5 ml) were added to a reaction flask in sequence. Nitrogen replacement was performed 3-4 times. The mixture was heated to 100°C and the reaction was carried out for 8 h. After the reaction was complete, the reaction mixture was cooled to room temperature, and filtered. The filtrate was concentrated, subjected to column chromatography using a DCM/MeOH system, and then slurried with EA to afford 0.60 g of a white solid with a yield of 82.8%.

### Step 7: Synthesis of tert-butyl 4-(1-(2,6-dioxopiperidin-3-yl)-6-fluoro-1H-benzimidazol-5-yl)piperazine-1-carboxylate

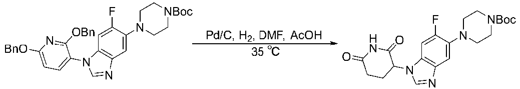

At room temperature, tert-butyl 4-(1-(2,6-bisbenzyloxy)pyridin-3-yl)-6-fluoro-1H-benzimidazol-5-yl)piperazine-1-carboxylate (0.60 g, 0.98 mmol), DMF (15 mL), AcOH (2 mL) and Pd/C (200 mg) were added to a reaction flask in sequence. Hydrogen replacement was performed 3-4 times. The mixture was heated to 35 °C and the reaction was carried out for 8 h. After the reaction was complete, the reaction mixture was filtered, and water was added to the filtrate to precipitate a solid. After filtration, 0.60 g of a crude product was obtained, which was then directly taken to the next step.

### Step 8: Synthesis of 3-(6-fluoro-5-(piperazin-1-yl)-1H-benzimidazol-1-yl)piperidine-2,6-dione

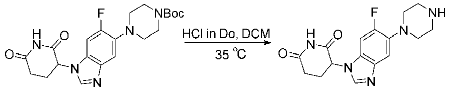

At room temperature, tert-butyl 4-(1-(2,6-dioxopiperidin-3-yl)-6-fluoro-1H-benzimidazol-5-yl)piperazine-1-carboxylate (0.30 g, 0.70 mmol), DCM (3 mL), and HCl in Do (5 mL) were added to a reaction flask in sequence. The mixture was heated to 35 °C and the reaction was carried out for 30 min. After the reaction was complete, the reaction mixture was concentrated to afford 0.25 g of a crude product, which was then directly taken to the next step.

### Step 9: Synthesis of 3-(5-(4-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperazin-1-yl)-6-fluoro-1H-benzimidazol-1-yl)piperidine-2,6-dione

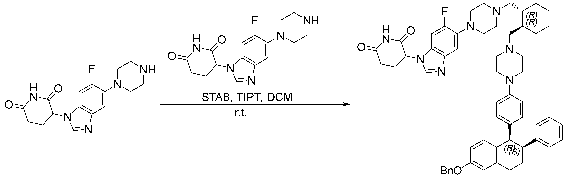

At room temperature, 3-(6-fluoro-5-(piperazin-1-yl)-1H-benzimidazol-1-yl)piperidine-2,6-dione (0.10 g, 0.30 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.15 g, 0.24 mmol) and dichloromethane (5 ml) were added to a reaction flask in sequence, followed by TIPT (0.3 mL). The mixture was stirred at room temperature for 8 h, and then STAB (0.10 g, 0.45 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.125 g of a brown solid with a yield of 45.3%.

### Step 4: Synthesis of 3-(6-fluoro-5-(4-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperazin-1-yl)-1H-benzimidazol-1-yl)piperidine-2,6-dione

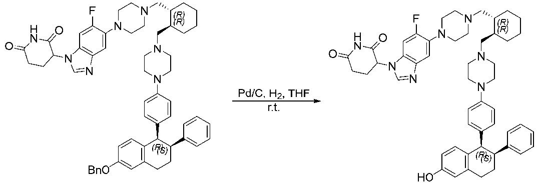

At room temperature, 3-(5-(4-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)piperazin-1-yl)-6-fluoro-1H-benzimidazol-1-yl)piperidine-2,6-dione (0.13 g, 0.14 mmol), 10% palladium on carbon (0.05 g), and THF (5 ml) were added to a reaction flask in sequence. Hydrogen replacement was performed 3-4 times, and the mixture was stirred at room temperature for 8 h. After the reaction was complete, the reaction mixture was filtered through celite. The filter cake was rinsed with THF. The filtrate was stripped of solvent, and then subjected to column chromatography using a DCM/MeOH system to afford 0.017 g (0.012 g stored) of a product.

¹H NMR (400 MHz, DMSO-d6) δ 11.19 (s, 1H), 9.14 (s, 1H), 8.20 (s, 1H), 7.47 (d, J = 13.1 Hz, 1H), 7.32 (d, J = 8.1 Hz, 1H), 7.13 - 7.08 (m, 2H), 6.83 (d, J = 7.3 Hz, 2H), 6.62 - 6.46 (m, 5H), 6.22 (d, J = 8.1 Hz, 2H), 5.63 (dd, J = 13.0, 5.1 Hz, 1H), 4.14 (d, J = 5.1 Hz, 2H), 3.07 - 2.68 (m, 19H), 2.23 - 2.05 (m, 4H), 1.65 (dd, *J* = 91.4, 44.9 Hz, 10H), 1.27 - 1.15 (m, 4H), 0.99 (s, 2H). MS (ESI) m/z: 824.4 [M+H]⁺.

### Example 20 Synthesis of 3-(6-fluoro-5-(1-(((1R,2R)-2-((4-(4-(((2R,2S)-6-hydroxy-2-phenyl-1,2,3,4-tetrahydronaphthalen-1-yl)phenyl)piperazin-1-yl)methyl)cyclohexyl)methyl)piperidin-4-yl)-1H-benzo[d]imidazol-1-yl)piperidin-2,6-dione (Compound V-5)

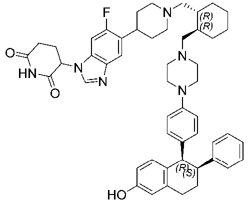

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 2,6-bis(benzyloxy)-3-nitropyridine

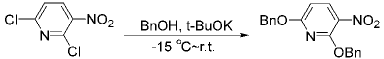

At room temperature, BnOH (7.57 g, 69.95 mmol) was dissolved in THF (25 mL). The solution was cooled to -15°C. Potassium tert-butoxide (7.00 g, 62.18 mmol) was added. The mixture was stirred at room temperature for 30 min. At -15°C, the above reaction solution was added dropwise to 2,6-dichloro-3-nitropyridine (5.00 g, 25.91 mmol) in THF (15 mL). After the addition, the reaction was carried out at room temperature for 16 h. After the reaction was complete, the reaction solution was poured into ice water, stirred for 30 min, and filtered. The filter cake was slurried with methyl tert-butyl ether to afford 6.50 g of a white solid with a yield of 74.6%.

### Step 2: Synthesis of 2,6-bis(benzyloxy)pyridine-3-amine

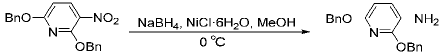

At room temperature, 2,6-bis(benzyloxy)-3-nitropyridine (6.50 g, 19.32 mmol) was dissolved in THF (10 mL) and MeOH (10 mL), and NiCl₂·6H₂O (0.45 g, 1.93 mmol) was added. The mixture was cooled to 0°C, and NaBH₄ (1.82 g, 48.31 mmol) was slowly added. The reaction was carried out in an ice bath for 1 h. After the reaction was complete, the reaction was quenched by adding ice water (1 mL). The reaction solution was concentrated and subjected to column chromatography (EA/PE) to afford 4.41 g of a colorless oil with a yield of 74.5%.

### Step 3: Synthesis of 2,6-bis(benzyloxy)-N-(4-bromo-5-fluoro-2-nitrophenyl)pyridine-3-amine

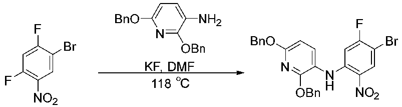

At room temperature, 2,4-difluoro-5-bromonitrobenzene (4.41 g, 13.06 mmol), 2,6-bis(benzyloxy)pyridine-3-amine (4.00 g, 13.06 mmol), KF (0.90 g, 15.67 mmol) and DMF (20 ml) were added to a reaction flask in sequence. The mixture was heated to 118 °C and the reaction was carried out for 8 h. After the reaction was complete, the reaction solution was cooled to room temperature, and water (20 mL) was added to the reaction solution to precipitate a solid. The solid was filtered, and the filter cake was slurried with methyl tert-butyl ether to afford 3.5 g of a yellow solid with a yield of 35.9%.

### Step 4: Synthesis of N¹-2,6-bis(benzyloxy)pyridin-3-yl)-4-bromo-5-fluorobenzene-1,2-diamine

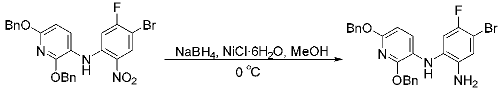

At room temperature, 2,6-bis(benzyloxy)-N-(4-bromo-5-fluoro-2-nitrophenyl)pyridine-3-amine (3.50 g, 6.67 mmol) was dissolved in THF (20 mL) and MeOH (20 mL), and NiCl₂·6H₂O (0.16 g, 1.67 mmol) was added. The mixture was cooled to 0 °C, and NaBH₄ (0.63 g, 16.69 mmol) was slowly added. The reaction was carried out in an ice bath for 1 h. After the reaction was complete, the reaction was quenched by adding ice water (1 mL). The reaction solution was concentrated and subjected to column chromatography (EA/PE) to afford 3.25 g of a brown oil with a yield of 98.8%.

### Step 5: Synthesis of 1-(2,6-bisbenzyloxy)pyridin-3-yl)-5-bromo-6-fluoro-1H-benzimidazole

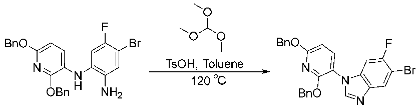

At room temperature, N¹-(2,6-bis(benzyloxy)pyridin-3-yl)-4-bromo-5-fluorobenzene-1,2-diamine (3.25 g, 6.57 mmol) was dissolved in toluene (30 mL), and trimethyl orthoformate (1.74 g, 16.44 mmol) and TsOH (0.23 g, 1.31 mmol) were added. The reaction was carried out at 120 °C for 1 h. After the reaction was complete, the reaction solution was cooled to room temperature, concentrated, and subjected to column chromatography (EA/PE) to give 3.00 g of a brown oil with a yield of 90.5%.

### Step 6: Synthesis of tert-butyl 4-(1-(2,6-bis(benzyloxy)pyridin-3-yl)-6-fluoro-1H-benzo[d]imidazol-5-yl)-3,6-dihydropyridine-1(2H)-carboxylate

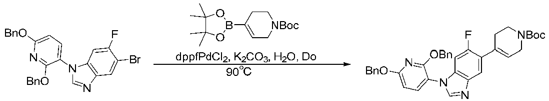

At room temperature, 1-(2,6-bisbenzyloxy)pyridin-3-yl)-5-bromo-6-fluoro-1H-benzimidazole (1.20 g, 2.38 mmol), 2,6-bis(benzyloxy)-3-(4,4,5,5-tetramethyl-1,3,2-dioxaborolan-2-yl)pyridine (1.24 g, 2.97 mmol), potassium carbonate (1.23 g, 8.90 mmol), and dppfPdCl₂ (0.22 g, 0.30 mmol), water (1 mL) and dioxane (10 ml) were added to a reaction flask in sequence. The reaction was carried out at 90°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 1.20 g of a product with a yield of 81%.

### Step 7: Synthesis of tert-butyl 4-(1-(2,6-dioxopiperidin-3-yl)-6-fluoro-1H-benzo[d]imidazol-5-yl)piperidine-1-carboxylate

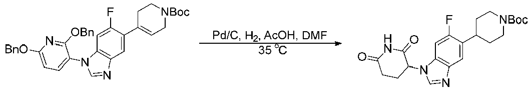

At room temperature, tert-butyl 4-(1-(2,6-bis(benzyloxy)pyridin-3-yl)-6-fluoro-1H-benzo[d]imidazol-5-yl)-3,6-dihydropyridine-1(2H)-carboxylate (0.60 g, 0.98 mmol), DMF (15 mL), AcOH (2 mL) and Pd/C (200 mg) were added to a reaction flask in sequence. Hydrogen replacement was performed 3-4 times. The mixture was heated to 35 °C and the reaction was carried out for 8 h. After the reaction was complete, the reaction mixture was filtered, and water was added to the filtrate to precipitate a solid. After filtration, 0.60 g of a crude product was obtained, which was then directly taken to the next step.

### Step 8: Synthesis of 3-(6-fluoro-5-(piperidin-4-yl)-1H-benzo[d]imidazol-1-yl)piperidin-2,6-dione

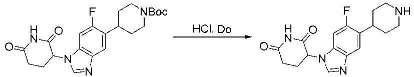

At room temperature, tert-butyl 4-(1-(2,6-dioxopiperidin-3-yl)-6-fluoro-1H-benzo[d]imidazol-5-yl)piperidine-1-carboxylate (0.30 g, 0.70 mmol), DCM (3 mL), and HCl in Do (5 mL) were added to a reaction flask in sequence. The mixture was heated to 35 °C and the reaction was carried out for 30 min. After the reaction was complete, the reaction mixture was concentrated to afford 0.25 g of a crude product, which was then directly taken to the next step.

### Step 9: Synthesis of 3-(5-(1-(((1R,2R)-2-((4-(4-(((2R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-6-fluoro-1H-benzo[d]imidazol-1-yl)piperidin-2,6-dione

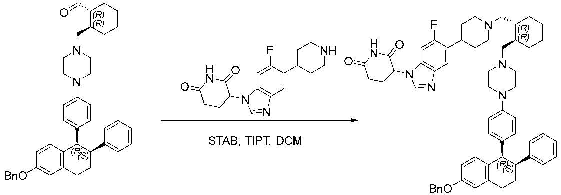

At room temperature, 3-(6-fluoro-5-(piperidin-4-yl)-1H-benzo[d]imidazol-1-yl)piperidin-2,6-dione (0.10 g, 0.30 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.15 g, 0.24 mmol) and dichloromethane (5 ml) were added to a reaction flask in sequence, followed by TIPT (0.3 mL). The mixture was stirred at room temperature for 8 h, and then STAB (0.10 g, 0.45 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.13 g of a brown solid with a yield of 46%.

### Step 10: 3-(6-fluoro-5-(1-(((1R,2R)-2-((4-(4-(((2R,2S)-6-hydroxy-2-phenyl-1,2,3,4-tetrahydronaphthalen-1-yl)phenyl)piperazin-1-yl)methyl)cyclohexyl)methyl)piperidin-4-yl)-1H-benzo[d]imidazol-1-yl)piperidin-2,6-dione

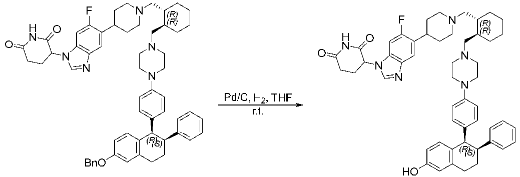

At room temperature, 3-(5-(1-(((1R,2R)-2-((4-(4-(((2R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-6-fluoro-1H-benzo[d]imidazol-1-yl)piperidin-2,6-dione (0.08 g, 0.14 mmol), 10% palladium on carbon (0.05 g), and THF (5 ml) were added to a reaction flask in sequence. Hydrogen replacement was performed 3-4 times, and the mixture was stirred at room temperature for 8 h. After the reaction was complete, the reaction mixture was filtered through celite. The filter cake was rinsed with THF. The filtrate was stripped of solvent, and then subjected to column chromatography using a DCM/MeOH system to afford 0.02 g of a product with a yield of 27%.

¹H NMR (400 MHz, DMSO-*d*₆) *δ* 11.21 (s, 1H), 9.15 (s, 1H), 8.26 (s, 1H), 7.47 (d, *J* = 10.3 Hz, 1H), 7.11 (d, *J* = 7.5 Hz, 3H), 6.82 (d, *J* = 7.4 Hz, 2H), 6.65 - 6.54 (m, 4H), 6.48 (dd, *J* = 8.2, 2.5 Hz, 1H), 6.22 (d, *J* = 8.3 Hz, 2H), 5.73 - 5.60 (m, 1H), 4.15 - 4.11 (m, 1H), 3.54 - 3.50 (m, 1H), 3.15 - 2.66 (m, 18H), 2.28 - 1.94 (m, 8H), 1.80 - 1.52 (m, 9H), 1.09 - 0.94 (m, 3H), 0.92 - 0.77 (m, 2H). MS (ESI) m/z: 821.4 [M-H]⁻.

### Example 21 Synthesis of 3-(5-(1-(((1R,2R)-2-((4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1H-benzo[d]imidazol-1-yl)piperidin-2,6-dione (Compound V-7)

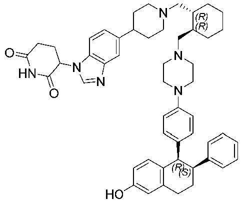

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 2,6-bis(benzyloxy)pyridine-3-amine

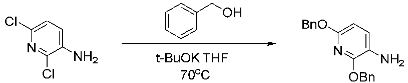

At room temperature, 2,6-dichloro-3-aminopyridine (9.00 g, 55.21 mmol), potassium tert-butoxide (18.58 g, 165.64 mmol), benzyl alcohol (17.92 g, 165.64 mmol) and tetrahydrofuran (200 ml) were added to a reaction flask in sequence, and the mixture was heated to 70 °C and stirred for 20 h. After the reaction was complete, the reaction mixture was cooled, and ethyl acetate and water were added. The layers were separated. The organic phase was dried, and then subjected to column chromatography using a PE/EA system to afford 4.08 g of a product with a yield of 48%.

### Step 2: Synthesis of 2,6-bis(benzyloxy)-N-(4-bromo-2-nitrophenyl)pyridine-3-amine

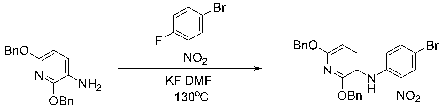

2,6-bis(benzyloxy)pyridine-3-amine (3.58 g, 11.70 mmol), 4-bromo-1-fluoro-2-nitrobenzene (3.09 g, 14.03 mmol), potassium fluoride (0.82 g, 14.03 mmol) and DMF (20 ml) were added to a reaction flask in sequence, and the mixture was heated to 130 °C and stirred for 18 h. After the reaction was complete, the reaction mixture was cooled, and ethyl acetate and water were added. The layers were separated. The organic phase was dried, and then subjected to column chromatography using a PE/EA system to afford 5.90 g of a product with a yield of 99%.

### Step 3: Synthesis of N1-(2,6-bis(benzyloxy)pyridin-3-yl)-4-bromophenyl-1,2-diamine

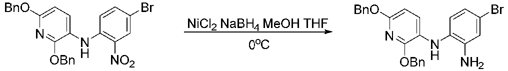

Methanol (80 ml), tetrahydrofuran (80 ml), 2,6-bis(benzyloxy)-N-(4-bromo-2-nitrophenyl)pyridine-3-amine (5.90 g, 11.65 mmol) and nickel chloride hexahydrate (0.28 g, 1.17 mmol) were added to a reaction flask in sequence. The mixture was cooled to 0 °C, and sodium borohydride (1.10 g, 29.01 mmol) was added in portions under controlled temperature. After the addition, the reaction was carried out in an ice bath for 1 h. After the reaction was complete, water was added to quench the reaction, and the reaction mixture was stirred for 10 min and then stripped of solvent. The crude product was subjected to column chromatography using a PE/EA system to afford 3.30 g of a product with a yield of 59%.

### Step 4: Synthesis of 1-(2,6-bis(benzyloxy)pyridin-3-yl)-5-bromo-1H-benzo[d]imidazole

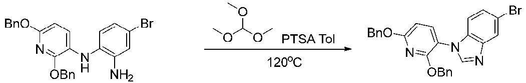

Toluene (15 ml), N1-(2,6-bis(benzyloxy)pyridin-3-yl)-4-bromophenyl-1,2-diamine (2.00 g, 4.20 mmol), trimethyl orthoformate (1.11 g, 10.50 mmol) and PTSA (0.14 g, 0.84 mmol) were added to a reaction flask in sequence. The mixture was heated to 120 °C and the reaction was carried out for 2 h. After the reaction was complete, the reaction mixture was cooled to room temperature, stripped of solvent toluene, and co-distilled once with dichloromethane. The crude product was subjected to column chromatography using a PE/EA system to afford 1.21 g of a product with a yield of 59%.

### Step 5: Synthesis of tert-butyl 4-(1-(2,6-bis(benzyloxy)pyridin-3-yl)-1H-benzo[d]imidazol-5-yl)-3,6-dihydropyridine-1(2H)-carboxylate

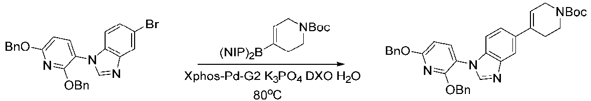

At room temperature, 1-(2,6-bis(benzyloxy)pyridin-3-yl)-5-bromo-1H-benzo[d]imidazol (0.60 g, 1.23 mmol), N-Boc-1,2,5,6-tetrahydropyridine-4-boronic acid pinacol ester (0.57 g, 1.85 mmol), Xphos-Pd-G2 (0.10 g, 0.12 mmol), potassium phosphate (0.52 g, 2.47 mmol), water (3 ml) and 1,4-dioxane (10 ml) were added to a reaction flask in sequence. Nitrogen replacement was performed 3-4 times. The mixture was heated to 80°C and the reaction was carried out for 4 h. After the reaction was complete, the reaction mixture was cooled to room temperature, stripped of solvent, diluted with ethyl acetate, and filtered through celite. The filtrate was dried, stripped of solvent, and subjected to column chromatography using a PE/EA system to afford 0.51 g of a product with a yield of 71%.

### Step 6: Synthesis of tert-butyl 4-(1-(2,6-dioxopiperidin-3-yl)-1H-benzo[d]imidazol-5-yl)piperidin-1-carboxylate

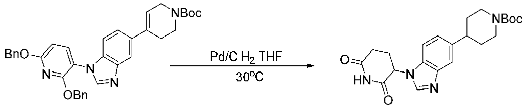

At room temperature, tert-butyl 4-(1-(2,6-bis(benzyloxy)pyridin-3-yl)-1H-benzo[d]imidazol-5-yl)-3,6-dihydropyridine-1(2H)-carboxylate (0.51 g, 0.87 mmol) and tetrahydrofuran (15 ml) were added to a reaction flask in sequence, followed by 10% palladium on carbon (0.08 g). Hydrogen replacement was performed 3-4 times, and the mixture was stirred at room temperature for 16 h. After the reaction was complete, the reaction mixture was filtered through celite and stripped of solvent to afford 0.15 g of a product with a yield of 41%.

### Step 7: Synthesis of 3-(5-(piperidin-4-yl)-1H-benzo[d]imidazol-1-yl)piperidin-2,6-dione

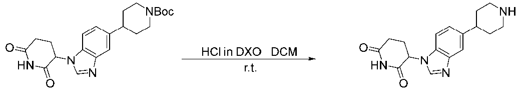

At room temperature, 3-(5-(piperidin-4-yl)-1H-benzo[d]imidazol-1-yl)piperidin-2,6-dione (0.15 g, 0.35 mmol), hydrochloric acid/dioxane (3 ml), and dichloromethane (3 ml) were added to a reaction flask in sequence, and the mixture was stirred at room temperature for 2 h. After the reaction was complete, the reaction mixture was stripped of solvent to afford 0.16 g of a crude product, which was directly taken to the next step (the charged amount in the next step was based on the theoretical yield of 0.11 g).

### Step 8: Synthesis of 3-(5-(1-(((1R,2R)-2-((4-(((1R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-1H-benzo[d]imidazol-1-yl)piperidin-2,6-dione

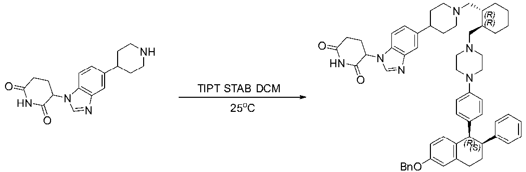

At room temperature, 3-(5-(piperidin-4-yl)-1H-benzo[d]imidazol-1-yl)piperidin-2,6-dione (0.11 g, 0.35 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.21 g, 0.35 mmol) and dichloromethane (10 ml) were added to a reaction flask in sequence, followed by TIPT (0.5 ml). The mixture was stirred at room temperature for 16 h, and then STAB (0.15 g, 0.70 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.26 g of a product with a yield of 83%.

### Step 9: Synthesis of 3-(5-(1-(((1R,2R)-2-((4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1H-benzo[d]imidazol-1-yl)piperidin-2,6-dione

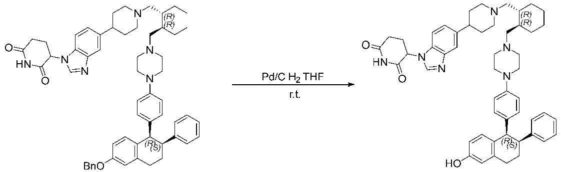

At room temperature, 3-(5-(1-(((1R,2R)-2-((4-(((1R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-1H-benzo[d]imidazol-1-yl)piperidin-2,6-dione (0.26 g, 0.29 mmol), 10% palladium on carbon (0.02 g), and tetrahydrofuran (10 ml) were added to a reaction flask in sequence. Hydrogen replacement was then performed 3-4 times, and the mixture was stirred at room temperature for 90 h. After the reaction was complete, the reaction mixture was filtered through celite. The filter cake was rinsed with tetrahydrofuran. The filtrate was stripped of solvent, and then subjected to column chromatography using a DCM/MeOH system to afford 0.006 g (0.003 g stored) of a product with a yield of 5%.

¹H NMR (400 MHz, DMSO-*d*₆) δ 11.21 (s, 1H), 9.15 (s, 1H), 8.25 (s, 1H), 7.55 (s, 1H), 7.47 (d, *J* = 8.4 Hz, 1H), 7.21 - 7.07 (m, 4H), 6.82 (d, *J* = 7.7 Hz, 2H), 6.67 - 6.54 (m, 3H), 6.47 (dd, *J* = 8.4, 2.5 Hz, 1H), 6.23 (d, *J* = 7.9 Hz, 2H), 5.69 (dd, *J* = 12.8, 5.0 Hz, 1H), 4.47 (s, 1H), 4.14 (d, *J* = 5.0 Hz, 1H), 3.18 - 3.05 (m, 4H), 3.00 - 2.85 (m, 6H), 2.82 - 2.67 (m, 4H), 2.30 - 2.14 (m, 2H), 2.12 - 1.91 (m, 4H), 1.77 - 1.60 (m, 6H), 1.39 - 1.18 (m, 10H), 1.08 - 0.97 (m, 2H), 0.90 - 0.79 (m, 2H). MS (ESI) m/z: 805.2 [M+H]⁺.

### Example 22 Synthesis of 3-(5-(4-(((1R,2R)-2-((4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1H-benzo[d]imidazol-1-yl)piperidine-2,6-dione (Compound V-3)

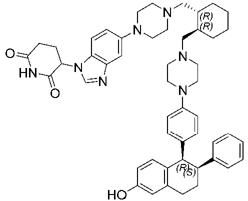

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 4-(1-(2,6-bis(benzyloxy)pyridin-3-yl)-1H-benzo[d]imidazol-5-yl)piperazine-1-carboxylate

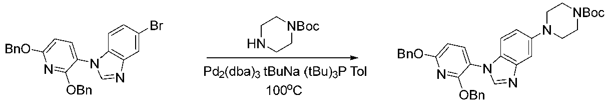

At room temperature, 1-(2,6-bis(benzyloxy)pyridin-3-yl)-5-bromo-1H-benzo[d]imidazolium (0.42 g, 0.84 mmol), 1-Boc-piperazine (0.63 g, 3.37 mmol), and Pd₂(dba)₃ (0.08 g, 0.08 mmol), sodium tert-butoxide (0.16 g, 1.69 mmol), 10% tri-tert-butylphosphine solution (0.30 g, 0.17 mmol) and toluene (10 ml) were added to a reaction flask in sequence. Nitrogen replacement was performed 3-4 times. The mixture was heated to 100°C and the reaction was carried out for 16 h. After the reaction was complete, the reaction mixture was cooled to room temperature, stripped of solvent, diluted with ethyl acetate, and filtered through celite. The filtrate was dried, stripped of solvent, and subjected to column chromatography using a PE/EA system to afford 0.30 g of a product with a yield of 59%.

### Step 2: Synthesis of tert-butyl 4-(1-(2,6-dioxopiperidin-3-yl)-1H-benzo[d]imidazol-5-yl)piperazine-1-carboxylate

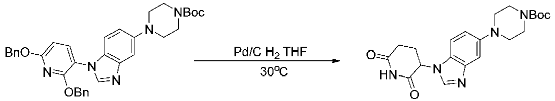

At room temperature, tert-butyl 4-(1-(2,6-bis(benzyloxy)pyridin-3-yl)-1H-benzo[d]imidazol-5-yl)piperazine-1-carboxylate (0.30 g, 0.50 mmol) and tetrahydrofuran (15 ml) were added to a reaction flask in sequence, followed by 10% palladium on carbon (0.03 g). Hydrogen replacement was performed 3-4 times, and the mixture was stirred at room temperature for 16 h. After the reaction was complete, the reaction mixture was filtered through celite and stripped of solvent to afford 0.08 g of a product with a yield of 35%.

### Step 3: Synthesis of 3-(5-(piperazin-1-yl)-1H-benzo[d]imidazol-1-yl)piperidine-2,6-dione

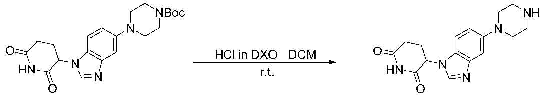

At room temperature, tert-butyl 4-(1-(2,6-dioxopiperidin-3-yl)-1H-benzo[d]imidazol-5-yl)piperazine-1-carboxylate (0.08 g, 0.18 mmol), hydrochloric acid/dioxane (3 ml), and dichloromethane (3 ml) were added to a reaction flask in sequence, and the mixture was stirred at room temperature for 2 h. After the reaction was complete, the reaction mixture was stripped of solvent to afford 0.09 g of a crude product, which was directly taken to the next step.

### Step 4: Synthesis of 3-(5-(4-(((1R,2R)-2-((4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-1H-benzo[d]imidazol-1-yl)piperidine-2,6-dione

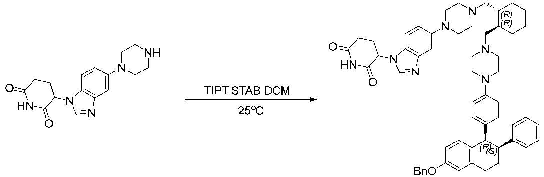

At room temperature, 3-(5-(piperazin-1-yl)-1H-benzo[d]imidazol-1-yl)piperidine-2,6-dione (0.07 g, 0.23 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.11 g, 0.19 mmol) and dichloromethane (10 ml) were added to a reaction flask in sequence, followed by TIPT (0.5 ml). The mixture was stirred at room temperature for 16 h, and then STAB (0.10 g, 0.47 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.10 g of a product with a yield of 62%.

### Step 5: 3-(5-(4-(((1R,2R)-2-((4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)-1H-benzo[d]imidazol-1-yl)piperidine-2,6-dione

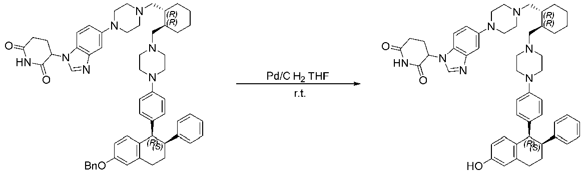

At room temperature, 3-(5-(4-(((1R,2R)-2-((4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-1H-benzo[d]imidazol-1-yl)piperidine-2,6-dione (0.10 g, 0.12 mmol), 10% palladium on carbon (0.01 g), and tetrahydrofuran (10 ml) were added to a reaction flask in sequence. Hydrogen replacement was then performed 3-4 times, and the mixture was stirred at room temperature for 48 h. After the reaction was complete, the reaction mixture was filtered through celite. The filter cake was rinsed with tetrahydrofuran. The filtrate was stripped of solvent, and then subjected to column chromatography using a DCM/MeOH system to afford 0.006 g (0.002 g stored) of a product with a yield of 5%.

¹H NMR (400 MHz, DMSO-*d*₆) δ 11.18 (s, 1H), 8.22 (s, 1H), 8.14 (s, 1H), 7.37 (d, *J =* 9.0 Hz, 1H), 7.14 (d, *J* = 9.1 Hz, 4H), 7.00 (d, *J* = 8.9 Hz, 1H), 6.83 (d, *J* = 7.3 Hz, 2H), 6.69 - 6.58 (m, 2H), 6.50 (dd, *J* = 19.9, 8.6 Hz, 3H), 6.21 (d, *J* = 8.0 Hz, 1H), 5.61 (dd, *J* = 13.0, 4.9 Hz, 1H), 4.13 (d, *J =* 4.8 Hz, 1H), 3.11 - 3.02 (m, 5H), 3.02 - 2.84 (m, 6H), 2.81 - 2.60 (m, 3H), 2.42 - 2.31 (m, 4H), 2.29 - 2.16 (m, 2H), 2.14 - 2.03 (m, 4H), 1.95 - 1.78 (m, 3H), 1.75 - 1.36 (m, 7H), 1.25 - 0.92 (m, 6H). MS (ESI) m/z: 806.4 [M+H]⁺.

### Example 23 Synthesis of 3-(5-(4-(((1R,2R)-2-((4-(4-(1R,2S)-6-hydroxy-2-phenyl-1,2,3,4-tetrahydronaphthalen-1-yl)phenyl)piperazin-1-yl)methyl)cyclohexyl)methyl)piperazin-1-yl)-2-methyl-1H-benzimidazol-1-yl)piperidine-2,6-dione (Compound V-1)

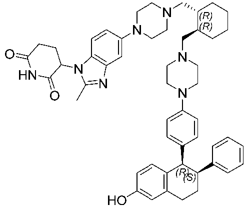

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 1-(2,6-bis(benzyloxy)pyridin-3-yl)-5-bromo-2-methyl-1H-benzo[d]imidazole

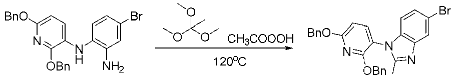

Acetic acid (10 ml), N1-(2,6-bis(benzyloxy)pyridin-3-yl)-4-bromophenyl-1,2-diamine (0.84 g, 1.77 mmol), and triethyl orthoformate (0.32 g, 2.65 mmol) were added to a reaction flask in sequence. The mixture was heated to 120 °C and the reaction was carried out for 3 h. After the reaction was complete, the reaction mixture was cooled to room temperature and stripped of solvent until no obvious droplets remained. The mixture was then sanded and subjected to column chromatography using a PE/EA system to afford 0.92 g of a product (with a small amount of acetic acid residue, which did not affect the next step).

### Step 2: Synthesis of tert-butyl 4-(1-(2,6-bis(benzyloxy)pyridin-3-yl)-2-methyl-1H-benzo[d]imidazol-5-yl)piperazine-1-carboxylate

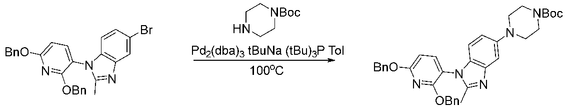

At room temperature, 1-(2,6-bis(benzyloxy)pyridin-3-yl)-5-bromo-2-methyl-1H-benzo[d]imidazole (0.92 g, 1.84 mmol), 1-Boc-piperazine (1.37 g, 7.36 mmol), and Pd₂(dba)₃ (0.17 g, 0.18 mmol), sodium tert-butoxide (0.35 g, 3.68 mmol), 10% tri-tert-butylphosphine solution (0.74 g, 0.37 mmol) and toluene (10 ml) were added to a reaction flask in sequence. Nitrogen replacement was performed 3-4 times. The mixture was heated to 100°C and the reaction was carried out for 16 h. After the reaction was complete, the reaction mixture was cooled to room temperature, stripped of solvent, diluted with ethyl acetate, and filtered through celite. The filtrate was dried, stripped of solvent, and subjected to column chromatography using a PE/EA system to afford 0.46 g of a product with a yield of 42%.

### Step 3: Synthesis of tert-butyl 4-(1-(2,6-dioxopiperidin-3-yl)-2-methyl-1H-benzo[d]imidazol-5-yl)piperazine-1-carboxylate

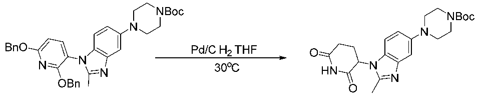

At room temperature, tert-butyl 4-(1-(2,6-bis(benzyloxy)pyridin-3-yl)-2-methyl-1H-benzo[d]imidazol-5-yl)piperazine-1-carboxylate (0.46 g, 0.76 mmol) and tetrahydrofuran (15 ml) were added to a reaction flask in sequence, followed by 10% palladium on carbon (0.03 g). Hydrogen replacement was performed 3-4 times, and the mixture was stirred at room temperature for 16 h. After the reaction was complete, the reaction mixture was filtered through celite and stripped of solvent to afford 0.18 g of a product with a yield of 57%.

### Step 4: Synthesis of 3-(2-methyl-5-(piperazin-1-yl)-1H-benzo[d]imidazol-1-yl)piperidine-2,6-dione

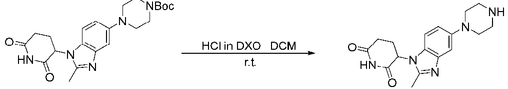

At room temperature, tert-butyl 4-(1-(2,6-dioxopiperidin-3-yl)-2-methyl-1H-benzo[d]imidazol-5-yl)piperazine-1-carboxylate (0.18 g, 0.41 mmol), hydrochloric acid/dioxane (3 ml), and dichloromethane (3 ml) were added to a reaction flask in sequence, and the mixture was stirred at room temperature for 2 h. After the reaction was complete, the reaction mixture was stripped of solvent to afford a crude product, which was directly taken to the next step (the charged amount in the next step was based on the theoretical yield of 0.13 g).

### Step 5: Synthesis of 3-(5-(4-(((1R,2R)-2-((4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-2-methyl-1H-benzo[d]imidazol-1-yl)piperidine-2,6-dione

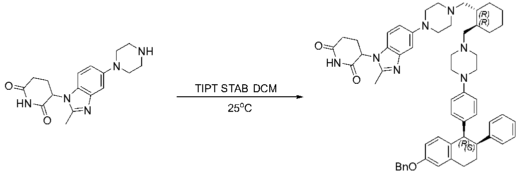

At room temperature, 3-(2-methyl-5-(piperazin-1-yl)-1H-benzo[d]imidazol-1-yl)piperidine-2,6-dione (0.13 g, 0.23 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.20 g, 0.33 mmol) and dichloromethane (10 ml) were added to a reaction flask in sequence, followed by TIPT (0.5 ml). The mixture was stirred at room temperature for 16 h, and then STAB (0.13 g, 0.61 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.04 g of a product with a two-step yield of 15%.

### Step 6: Synthesis of 3-(5-(4-(((1R,2R)-2-((4-(4-(1R,2S)-6-hydroxy-2-phenyl-1,2,3,4-tetrahydronaphthalen-1-yl)phenyl)piperazin-1-yl)methyl)cyclohexyl)methyl)piperazin-1-yl)-2-methyl-1H-benzimidazol-1-yl)piperidine-2,6-dione

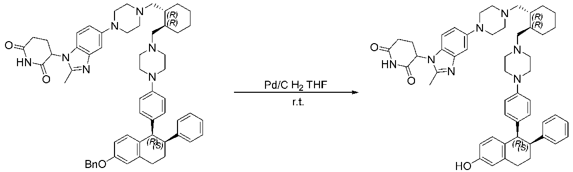

At room temperature, 3-(5-(4-(((1R,2R)-2-((4-((1R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)-2-methyl-1H-benzo[d]imidazol-1-yl)piperidine-2,6-dione (0.04 g, 0.04 mmol), 10% palladium on carbon (0.01 g), and tetrahydrofuran (10 ml) were added to a reaction flask in sequence. Hydrogen replacement was then performed 3-4 times, and the mixture was stirred at room temperature for 6 h. After the reaction was complete, the reaction mixture was filtered through celite. The filter cake was rinsed with tetrahydrofuran. The filtrate was stripped of solvent, and then subjected to column chromatography using a DCM/MeOH system to afford 0.002 g of a product with a yield of 6%. MS (ESI) m/z: 821.09[M+H]⁺.

### Example 24 Synthesis of 3-(5-fluoro-6-(1-(((1S,2S)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)piperidin-2,6-dione (Compound IV-10)

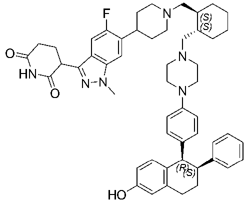

The synthesis method of (1S, 2S)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 3-(2,6-bis(benzyloxy)pyridin-3-yl)-6-bromo-5-fluoro-1-methyl-1H-indazole

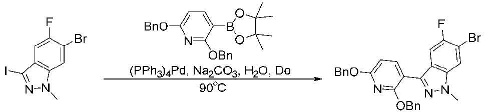

At room temperature, 6-bromo-5-fluoro-3-iodo-1-methyl-1H-indazole (1.00 g, 2.97 mmol), 2,6-bis(benzyloxy)-3-(4,4,5,5-tetramethyl-1,3,2-dioxaborolan-2-yl)pyridine (1.24 g, 2.97 mmol), sodium carbonate (0.94 g, 8.90 mmol), tetrakis(triphenylphosphine)palladium (0.34 g, 0.30 mmol), water (1 mL), and dioxane (10 ml) were added to a reaction flask in sequence. The reaction was carried out at 90°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 1.20 g of a product with a yield of 81%.

### Step 2: Synthesis of tert-butyl 4-(3-(2,6-bis(benzyloxy)pyridin-3-yl)-5-fluoro-1-methyl-1H-indazol-6-yl)-3,6-dihydropyridine-1(2H)-carboxylate

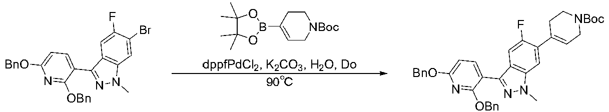

At room temperature, tert-butyl 3-(2,6-bis(benzyloxy)pyridin-3-yl)-6-bromo-5-fluoro-1-methyl-1H-indazole (0.60 g, 1.16 mmol), 4-(4,4,5,5-tetramethyl-1,3,2-dioxaborolan-2-yl)-3,6-dihydropyridine-1(2H)-carboxylate (0.40 g, 1.27 mmol), dppfPdCl₂ (0.09 g, 0.12 mmol), cesium carbonate (0.48 g, 3.47 mmol), water (1 mL) and dioxane (10 ml) were added to a reaction flask in sequence. The reaction was carried out at 90°C under a nitrogen atmosphere for 5 h. After the reaction was complete, the reaction mixture was cooled to room temperature, and ethyl acetate and water were added. The layers were separated. The organic phase was dried, and then subjected to column chromatography using a PE/EA system to afford 0.47 g of a product with a yield of 66%.

### Step 3: Synthesis of tert-butyl 4-(3-(2,6-dioxopiperidin-3-yl)-5-fluoro-1-methyl-1H-indol-6-yl)piperidine-1-carboxylate

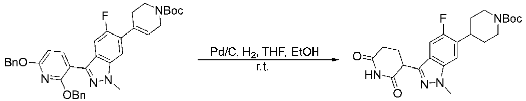

At room temperature, tert-butyl 4-(3-(2,6-bis(benzyloxy)pyridin-3-yl)-5-fluoro-1-methyl-1H-indazol-6-yl)-3,6-dihydropyridine-1(2H)-carboxylate (0.40 g, 0.66 mmol), 10% Pd/C (0.04 g), tetrahydrofuran (5 mL) and ethanol (5 ml) were added to a reaction flask in sequence, and the reaction was carried out at room temperature for 24 h. After the reaction was complete, the reaction mixture was filtered and dried by rotary evaporation to afford 0.26 g of a crude product, which was directly taken to the next step.

### Step 4: Synthesis of 3-(5-fluoro-1-methyl-6-(piperidin-4-yl)-1H-indol-3-yl)piperidin-2,6-dione

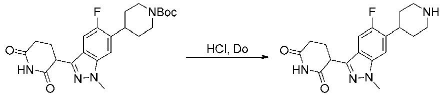

At room temperature, tert-butyl 4-(3-(2,6-dioxopiperidin-3-yl)-5-fluoro-1-methyl-1H-indol-6-yl)piperidine-1-carboxylate (0.26 g, 0.60 mmol) and HCl in dioxane (5 ml) were added to a reaction flask in sequence, and the reaction was carried out at room temperature for 2 h. After the reaction was complete, the reaction mixture was dried by rotary evaporation to afford 0.22 g of a crude product, which was directly taken to the next step.

### Step 5: Synthesis of 3-(6-(1-(((1S,2S)-2-((4-(4-(((2R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-5-fluoro-1-methyl-1H-indazol-3-yl)piperidin-2,6-dione

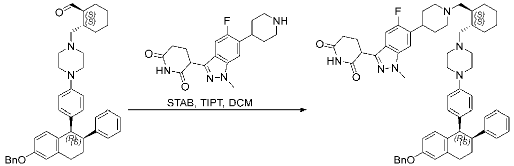

At room temperature, 3-(5-fluoro-1-methyl-6-(piperidin-4-yl)-1H-indol-3-yl)piperidin-2,6-dione (0.15 g, 0.47 mmol), (1S,2S)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.28 g, 0.47 mmol) and dichloromethane (5 mL) were added to a reaction flask in sequence, followed by TIPT (0.5 mL). The mixture was stirred at room temperature for 16 h, and then STAB (0.15 g, 0.71 mmol) was added. The reaction was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.21 g of a product with a three-step yield of 62%.

### Step 6: Synthesis of 3-(5-fluoro-6-(1-(((1S,2S)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)piperidin-2,6-dione

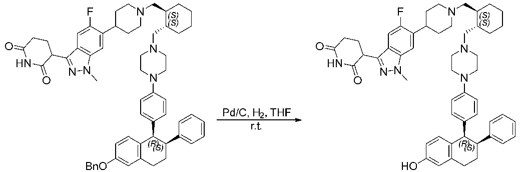

At room temperature, 3-(6-(1-(((1S,2S)-2-((4-(4-(((2R,2S)-6-(benzyloxy)-2-phenyl-1,2,3,4-tetrahydronaphthalen-1-yl)phenyl)piperazin-1-yl)methyl)cyclohexyl)methyl)piperidin-4-yl)-5-fluoro-1-methyl-1H-indazol-3-yl)piperidin-2,6-dione (0.21 g, 0.23 mmol), 10% palladium on carbon (0.10 g), and THF (3 mL) were added to a reaction flask in sequence. Hydrogen replacement was performed 3-4 times, and the mixture was stirred at room temperature for 24 h. After the reaction was complete, the reaction mixture was filtered through celite. The filter cake was rinsed with THF. The filtrate was stripped of solvent, and then subjected to column chromatography using a DCM/MeOH system to afford 0.11 g of a product with a yield of 58%.

¹H NMR (400 MHz, DMSO-*d*₆) *δ* 10.91 (s, 1H), 9.16 (s, 1H), 7.59 - 7.40 (m, 2H), 7.16 - 7.05 (m, 3H), 6.78 (s, 2H), 6.65 - 6.54 (m, 4H), 6.47 (dd, *J* = 8.2, 2.6 Hz, 1H), 6.22 (d, *J=* 8.1 Hz, 2H), 4.32 (dd, *J=* 10.1, 5.0 Hz, 1H), 4.14 (d, *J* = 5.0 Hz, 1H), 3.95 (s, 3H), 3.64 - 3.60 (m, 1H), 3.20 - 2.88 (m, 12H), 2.72 - 2.59 (m, 4H), 2.43 - 2.19 (m, 3H), 2.17 - 1.44 (m, 17H), 1.06 - 0.83 (m, 3H). MS (ESI) m/z: 837.7 [M+H]⁺.

Example 25 Synthesis of 1-(5-fluoro-6-(8-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)-8-azabicyclo[3.2.1]oct-3-yl)-1-methyl-1H-indazol-3-yl)dihydropyrimidine-2,4(1H,3H)-dione (Compound VI-17)

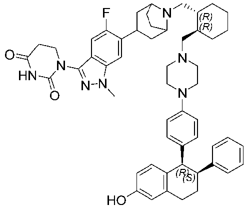

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 3-(3-(2,4-dioxotetrahydropyrimidin-1(2H)-yl)-5-fluoro-1-methyl-1H-indazol-6-yl)-8-azabicyclo-4-oct-2-en-8-carboxylate

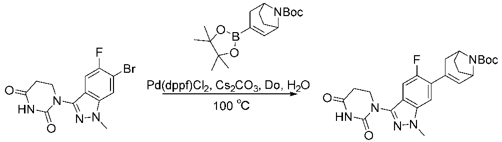

At room temperature, 1-(6-bromo-5-fluoro-1-methyl-1H-indazol-3-yl)dihydropyrimidine-2,4(1H,3H)-dione (0.10 g, 0.29 mmol), 8-tert-butoxycarbonyl-8-azabicyclo[3.2.1]oct-2-en-3-boronic acid pinacol ester (0.11 g, 0.32 mmol), Pd(dppf)Cl₂ (0.02 g, 0.03 mmol), Cs₂CO₃ (0.29 g, 0.88 mmol), Do (5 ml) and H₂O (1 ml) were added to a reaction flask in sequence. Nitrogen replacement was performed 3-4 times. The mixture was heated to 80°C and the reaction was carried out for 2 h. After the reaction was complete, the reaction solution was cooled to room temperature, and filtered. The filtrate was concentrated, subjected to column chromatography using a DCM/MeOH system, and then slurried with EA to afford 0.10 g of a white solid with a yield of 72.9%.

### Step 2: Synthesis of 1-(6-(8-azabicyclo[3.2.1]oct-2-en-3-yl)-5-fluoro-1-methyl-1H-indazol-3-yl)dihydropyrimidine-2,4(1H,3H)-dione

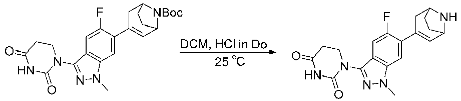

At room temperature, tert-butyl 3-(3-(2,4-dioxotetrahydropyrimidin-1(2H)-yl)-5-fluoro-1-methyl-1H-indol-6-yl)-8-azabicyclo-4-oct-2-en-8-carboxylate (0.10 mg, 0.21 mmol) and dichloromethane (2 ml) were added to a reaction flask in sequence. After complete dissolution, hydrochloric acid/dioxane (5 ml) was added, and the mixture was stirred at room temperature for 2 h. After the reaction was complete, the reaction mixture was stripped of solvent to afford 0.10 g of a crude product as a white solid, which was then directly taken to the next step.

### Step 3: Synthesis of 1-(6-(8-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)-8-azabicyclo[3.2.1]oct-2-en-3-yl)-5-fluoro-1-methyl-1H-indazol-3-yl)dihydropyrimidine-2,4(1H,3H)-dione

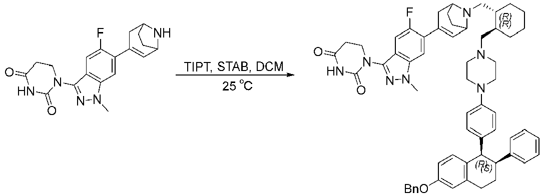

At room temperature, 1-(6-(8-azabicyclo[3.2.1]oct-2-en-3-yl)-5-fluoro-1-methyl-1H-indol-3-yl)dihydropyrimidine-2,4(1H,3H)-dione (0.10 g, 0.27 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.11 g, 0.19 mmol) and dichloromethane (5 ml) were added to a reaction flask in sequence, followed by TIPT (0.30 mL). The mixture was stirred at room temperature for 8 h, and then STAB (0.086 g, 0.41 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.15 g of a product with a three-step yield of 53.9%.

### Step 4: Synthesis of 1-(5-fluoro-6-(8-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)-8-azabicyclo[3.2.1]oct-3-yl)-1-methyl-1H-indazol-3-yl)dihydropyrimidine-2,4(1H,3H)-dione

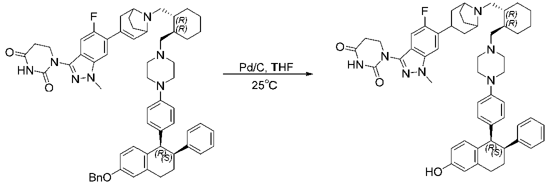

At room temperature, 1-(6-(8-(((1R,2R)-2-((4-(4-((1R,2S)-6-(benzyloxy)-2-phenyl-1,2,3,4-tet rahydronaphthalen-1-yl)phenylpiperazin-1-yl)methyl)cyclohexyl)methyl)-8-azabicyclo[3.2.1]oct-2-en-3-yl)-5-fluoro-1-methyl-1H-indol-3-yl)dihydropyrimidine-2,4(1H,3H)-dione (0.15 g, 0.16 m mol), 10% palladium on carbon (0.05 g), and THF (3 ml) were added to a reaction flask in sequen ce. Hydrogen replacement was performed 3-4 times, and the mixture was stirred at room tempera ture for 8 h. After the reaction was complete, the reaction mixture was filtered through celite. The filter cake was rinsed with THF. The filtrate was stripped of solvent, and then subjected to colum n chromatography using a DCM/MeOH system to afford 0.015 g (0.010 g stored) of a product wi th a yield of 11.0%.

¹H NMR (400 MHz, DMSO-d6) δ 10.58 (d, J = 2.5 Hz, 1H), 9.15 (s, 1H), 7.72 (s, 1H), 7.41 (d, J = 10.7 Hz, 1H), 7.13 (dd, J = 7.4, 4.6 Hz, 3H), 6.83 (d, J = 4.2 Hz, 2H), 6.62 - 6.60 (m, 2H), 6.54 (d, J = 7.4 Hz, 2H), 6.49 (d, J = 2.9 Hz, 1H), 6.21 (d, J = 8.2 Hz, 2H), 4.13 (d, J = 4.9 Hz, 1H), 4.01 (s, 3H), 3.95 (s, 2H), 3.90 (t, J = 6.7 Hz, 4H), 3.51 (s, 1H), 3.27 (s, 2H), 2.98 (d, J = 20.0 Hz, 9H), 2.75 (d, J = 2.1 Hz, 2H), 2.15 - 1.92 (m, 8H), 1.72 - 1.51 (m, 8H), 1.24 (s, 4H), 1.13 - 1.01 (m, 2H). MS (ESI) m/z: 864.7 [M+H]⁺.

Example 26 Synthesis of 1-(5-fluoro-6-(4-((1R, 2R)-2-((4-(4-(1R, 2S)-6-hydroxy-2-phenyl-1,2,3,4-tetrahydronaphthalen-1-yl)phenyl)piperazin-1-yl)methyl)cyclohexyl)methyl)-4-azaspiro[2.5]oct-7-yl)-1-methyl-1H-indazol-3-yl)dihydropyrimidine-2,4(1H,3H)-dione (Compound VI-18)

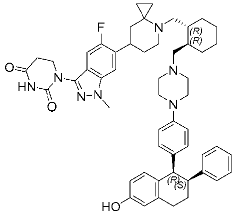

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 7-(((trifluoromethyl)sulfonyl)oxy)-4-azaspiro[2.5]oct-6-en-4-carboxylate

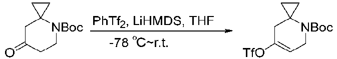

At room temperature, tert-butyl 7-oxo-4-azaspiro[2.5]octane-4-carboxylate (1 g, 4.44 mmol) and N-phenylbis(trifluoromethanesulfonyl)imide (2.38 g, 6.66 mmol) were dissolved in THF (20 mL) and cooled to -78 °C, and LiHMDS (7 mL, 7.00 mmol) was added. After the addition, the reaction was carried out at room temperature for 8 h. After the reaction was complete, water (3 mL) was added to the reaction solution, and the reaction solution was directly concentrated and subjected to column chromatography (EA/PE) to afford 1.2 g of a yellow solid with a yield of 75%.

### Step 2: Synthesis of tert-butyl 7-(3-(2,4-dioxotetrahydropyrimidin-1(2H)-yl)-5-fluoro-1-methyl-1H-indazol-6-yl)-4-azaspiro[2.5]oct-6-en-4-carboxylate

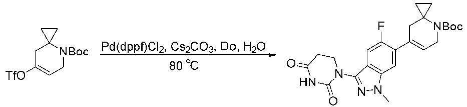

At room temperature, tert-butyl 7-(((trifluoromethyl)sulfonyl)oxy)-4-azaspiro[2.5]oct-6-en-4-carboxylate (0.18 g, 0.77 mmol), 1-(5-fluoro-1-methyl-6-(4,4,5,5-tetramethyl-1,3,2-dioxaborolan-2-yl)-1H-indol-3-yl)dihydropyrimidine-2,4(1H,3H)-dione (0.20 g, 0.52 mmol), Pd(dppf)Cl₂ (0.04 g, 0.05 mmol), Na₂CO₃ (0.11 g, 1.03 mmol), Do (10 ml) and H₂O (2 ml) were added to a reaction flask in sequence. Nitrogen replacement was performed 3-4 times. The mixture was heated to 80°C and the reaction was carried out for 2 h. After the reaction was complete, the reaction mixture was cooled to room temperature, and filtered. The filtrate was concentrated and subjected to column chromatography using a DCM/MeOH system to afford 0.20 g of a brown solid with a yield of 81.3%.

### Step 3: Synthesis of 1-(5-fluoro-1-methyl-6-(4-azaspiro[2.5]oct-6-en-7-yl)-1H-indazol-3-yl)dihydropyrimidine-2,4(1H,3H)-dione

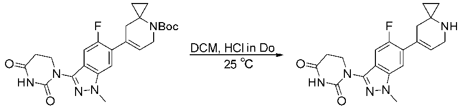

At room temperature, tert-butyl 4-(3-(2,4-dioxotetrahydropyrimidin-1(2H)-yl)-5-fluoro-1-methyl-1H-indol-6-yl)-3,3-difluoro-3,6-dihydropyridine-1(2H)-carboxylate (0.20 mg, 0.42 mmol) and dichloromethane (2 ml) were added to a reaction flask in sequence. After complete dissolution, hydrochloric acid/dioxane (3 ml) was added, and the mixture was stirred at room temperature for 1 h. After the reaction was complete, the reaction mixture was stripped of solvent to afford 0.20 g of a crude product as a white solid, which was then directly taken to the next step.

### Step 4: Synthesis of 1-(6-(4-(((1R,2R)-2-((4-(4-(2R,2S)-6-(benzyloxy)-2-phenyl-1,2,3,4-tetrahydronaphthalen-1-yl)phenyl)piperazin-1-yl)methyl)cyclohexyl)methyl)-4-azaspiro[2.5]oct-6-en-7-yl)-5-fluoro-1-methyl-1H-indazol-3-yl)dihydropyrimidine-2,4(1H,3H)-dione

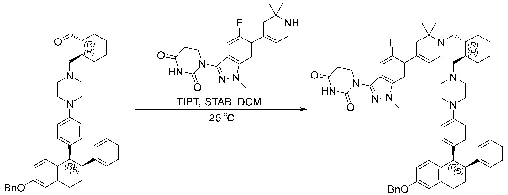

At room temperature, 1-(5-fluoro-1-methyl-6-(4-azaspiro[2.5]oct-6-en-7-yl)-1H-indazol-3-yl)dihydropyrimidine-2,4(1H,3H)-dione (0.10 g, 0.26 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.11 g, 0.18 mmol) and dichloromethane (5 ml) were added to a reaction flask in sequence, followed by TIPT (0.30 mL). The mixture was stirred at room temperature for 8 h, and then STAB (0.084 g, 0.40 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.06 g of a product with a two-step yield of 30%.

### Step 5: Synthesis of 1-(5-fluoro-6-(4-((1R,2R)-2-((4-(4-(1R,2S)-6-hydroxy-2-phenyl-1,2,3,4-tetrahydronaphthalen-1-yl)phenyl)piperazin-1-yl)methyl)cyclohexyl)methyl)-4-azaspiro[2.5]oct-7-yl)-1-methyl-1H-indazol-3-yl)dihydropyrimidine-2,4(1H,3H)-dione

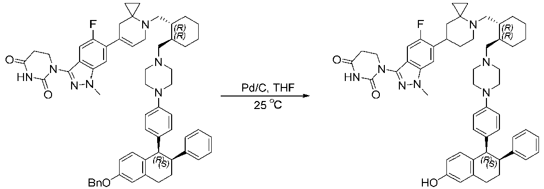

At room temperature, 1-(6-(4-(((1R,2R)-2-((4-(4-(2R,2S)-6-(benzyloxy)-2-phenyl-1,2,3,4-tetrahydronaphthalen-1-yl)phenyl)piperazin-1-yl)methyl)cyclohexyl)methyl)-4-azaspiro[2.5]oct-6-en-7-yl)-5-fluoro-1-methyl-1H-indazol-3-yl)dihydropyrimidine-2,4(1H,3H)-dione (0.060 g, 0.06 mmol), 10% palladium on carbon (0.05 g) and THF (5 ml) were added to a reaction flask in sequence. Hydrogen replacement was performed 3-4 times, and the mixture was stirred at room temperature for 8 h. After the reaction was complete, the reaction mixture was filtered through celite. The filter cake was rinsed with THF. The filtrate was stripped of solvent, and then subjected to column chromatography using a DCM/MeOH system to afford 0.012 g of a product with a yield of 22.0%.

¹H NMR (400 MHz, DMSO-*d*₆) *δ* 10.59 (s, 1H), 9.18 (s, 1H), 7.41 (d, *J=* 11.1 Hz, 1H), 7.11 (q, *J* = 6.6, 6.0 Hz, 3H), 6.83 (d, *J* = 7.0 Hz, 2H), 6.69 - 6.54 (m, 4H), 6.53 - 6.41 (m, 2H), 6.21 (t, *J=* 9.8 Hz, 2H), 4.16 - 4.12 (m, 1H), 4.01 - 3.93 (m, 3H), 3.93 - 3.78 (m, 4H), 3.06 - 2.88 (m, 7H), 2.75 (t, *J* = 6.8 Hz, 5H), 2.20 - 1.86 (m, 10H), 1.75 - 1.57 (m, 7H), 1.08 - 0.87 (m, 6H). MS (ESI) m/z: 866.4 [M+H]⁺.

### Example 27 Synthesis of 1-(6-(3,3-difluoro-1-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperidin-4-yl)-5-fluoro-1-methyl-1H-indazol-3-yl)dihydropyrimidine-2,4(1H,3H)-dione (Compound IV-12)

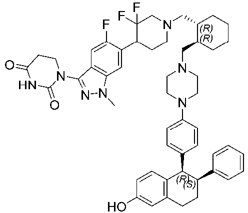

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesi of tert-butyl 3,3-difluoro-4-(((trifluoromethyl)sulfonyloxy)-3,6-dihydropyridine-1(2H)-carboxylate

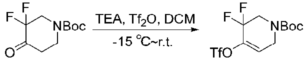

At room temperature, tert-butyl 3,3-difluoro-4-oxopiperidin-1-carboxylate (5 g, 21.26 mmol) was dissolved in DCM (50 mL). The solution was cooled to -15 °C. TEA (6.45 g, 63.77 mmol) was added. Trifluoromethanesulfonic anhydride (9.00 g, 31.88 mmol) was then slowly added dropwise. After the addition, the reaction was carried out at room temperature for 8 h. After the reaction was complete, saturated sodium bicarbonate aqueous solution (30 mL) was added to the reaction solution, and the mixture was extracted with DCM. The organic phase was concentrated and subjected to column chromatography (EA/PE) to give 3.2 g of a yellow solid with a yield of 41.0%.

### Step 2: Synthesis of tert-butyl 4-(3-(2,4-dioxotetrahydropyrimidin-1(2H)-yl)-5-fluoro-1-methyl-1H-indazol-6-yl)-3,3-difluoro-3,6-dihydropyridine-1(2H)-carboxylate

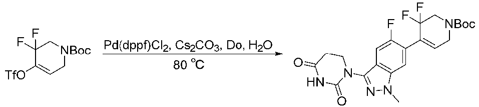

At room temperature, tert-butyl 3,3-difluoro-4-(((trifluoromethyl)sulfonyloxy)-3,6-dihydropyridine-1(2H)-carboxylate (0.18 g, 0.77 mmol), 1-(5-fluoro-1-methyl-6-(4,4,5,5-tetramethyl-1,3,2-dioxaborolan-2-yl)-1H-indazol-3-yl)dihydropyrimidine-2,4(1H,3H)-dione (0.20 g, 0.52 mmol), Pd(dppf)Cl₂ (0.04 g, 0.05 mmol), Na₂CO₃ (0.11 g, 1.03 mmol), Do (10 ml) and H₂O (2 ml) were added to a reaction flask in sequence. Nitrogen replacement was performed 3-4 times. The mixture was heated to 80°C and the reaction was carried out for 2 h. After the reaction was complete, the reaction mixture was cooled to room temperature, and filtered. The filtrate was concentrated and subjected to column chromatography using a DCM/MeOH system to afford 0.20 g of a brown solid with a yield of 81.3%.

### Step 3: Synthesis of 1-(6-(3,3-difluoro-1,2,3,6-tetrahydropyridin-4-yl)-5-fluoro-1-methyl-1H-indazol-3-yl)dihydropyrimidine-2,4(1H,3H)-dione

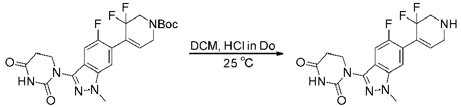

At room temperature, tert-butyl 4-(3-(2,4-dioxotetrahydropyrimidin-1(2H)-yl)-5-fluoro-1-methyl-1H-indol-6-yl)-3,3-difluoro-3,6-dihydropyridine-1(2H)-carboxylate (0.20 mg, 0.42 mmol) and dichloromethane (2 ml) were added to a reaction flask in sequence. After complete dissolution, hydrochloric acid/dioxane (3 ml) was added, and the mixture was stirred at room temperature for 1 h. After the reaction was complete, the reaction mixture was stripped of solvent to afford 0.20 g of a crude product as a white solid, which was then directly taken to the next step.

### Step 4: Synthesis of 1-(6-(1-(((1R,2R)-2-((4-(4-((1R,2S)-6-(benzyloxy)-2-phenyl-1,2,3,4-tetrahydronaphthalen-1-yl)phenylpiperazin-1-yl)methyl)cyclohexyl)methyl)-3,3-difluoro-1,2,3,6-tetrahydropyridin-4-yl)-5-fluoro-1-methyl-1H-indazol-3-yl)dihydropyrimidine-2,4(1H,3H)-dione

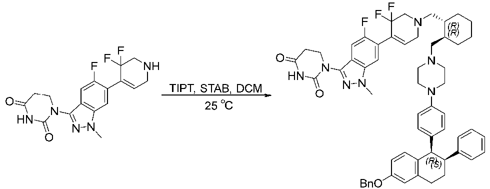

At room temperature, 1-(6-(3,3-difluoro-1,2,3,6-tetrahydropyridin-4-yl)-5-fluoro-1-methyl-1H-indol-3-yl)dihydropyrimidine-2,4(1H,3H)-dione (0.10 g, 0.26 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.11 g, 0.18 mmol) and dichloromethane (5 ml) were added to a reaction flask in sequence, followed by TIPT (0.30 mL). The mixture was stirred at room temperature for 8 h, and then STAB (0.084 g, 0.40 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.060 g of a product with a two-step yield of 29.9%.

### Step 5: Synthesis of 1-(6-(3,3-difluoro-1-(((1R,2R)-2-((4-(4-((1R,2S)-6-hydroxy-2-phenyl-1,2,3,4-tetrahydronaphthalen-1-yl)phenylpiperazin-1-yl)methyl)cyclohexyl)methyl)piperidin-4-yl)-5-fluoro-1-methyl-1H-indazol-3-yl)dihydropyrimidine-2,4(1H,3H)-dione

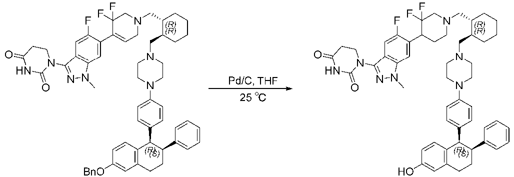

At room temperature, 1-(6-(1-(((1R,2R)-2-((4-(4-((1R,2S)-6-(benzyloxy)-2-phenyl-1,2,3,4-tet rahydronaphthalen-1-yl)phenylpiperazin-1-yl)methyl)cyclohexyl)methyl)-3,3-difluoro-1,2,3,6-tet rahydropyridin-4-yl)-5-fluoro-1-methyl-1H-indol-3-yl)dihydropyrimidine-2,4(1H,3H)-dione (0.0 60 g, 0.06 mmol), 10% palladium on carbon (0.05 g), and THF (5 ml) were added to a reaction fl ask in sequence. Hydrogen replacement was performed 3-4 times, and the mixture was stirred at r oom temperature for 8 h. After the reaction was complete, the reaction mixture was filtered throu gh celite. The filter cake was rinsed with THF. The filtrate was stripped of solvent, and then subje cted to column chromatography using a DCM/MeOH system to afford 0.012 g (0.007 g stored) of a product with a yield of 22.0%.

¹H NMR (400 MHz, DMSO-d6) δ 10.59 (s, 1H), 9.13 (d, J = 1.6 Hz, 1H), 7.74 (d, J = 5.5 Hz, 1H), 7.43 - 7.41 (m, 1H), 7.13 (dt, J = 8.8, 4.2 Hz, 3H), 6.85 - 6.81 (m, 2H), 6.65 - 6.59 (m, 2H), 6.55 - 6.46 (m, 3H), 6.21 (dd, J = 8.6, 3.7 Hz, 2H), 4.13 (t, J = 4.5 Hz, 1H), 4.02 (d, J = 3.6 Hz, 3H), 3.92 (t, J = 6.7 Hz, 3H), 3.17 (s, 1H), 3.06 - 2.91 (m, 8H), 2.76 (t, J = 6.7 Hz, 4H), 2.36 (d, J = 10.4 Hz, 4H), 2.11 (d, J = 6.5 Hz, 2H), 1.84 (s, 2H), 1.71 - 1.38 (m, 8H), 1.24 (s, 4H), 0.99 (s, 2H). MS (ESI) m/z: 874.8 [M+H]⁺.

### Example 28 Synthesis of 1-(5-fluoro-6-(1'-((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)-[1,4'-bipiperidin]-4-yl)-1-methyl-1H-indazol-3-yl)dihydropyrimidine-2,4(1H,3H)-dione (Compound VI-19)

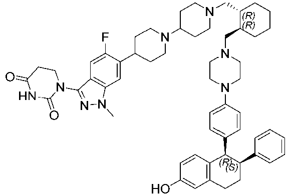

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 4-(3-(2,4-dioxotetrahydropyrimidin-1(2H)-yl)-5-fluoro-1-methyl-1H-indazol-6-yl)-[1,4'-bipiperidine]-1'-carboxylate

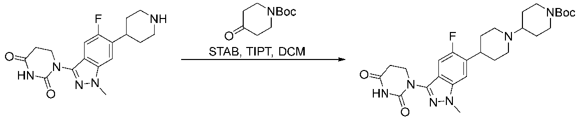

At room temperature, 1-(5-fluoro-1-methyl-6-(piperidin-4-yl)-1H-indazol-3-yl)dihydropyrimidine-2,4(1H,3H)-dione 6-bromo-3-iodo-1-methyl-1H-indazole (0.50 g, 1.45 mmol), N-tert-butoxycarbonyl-4-piperidinone (0.58 g, 2.90 mmol) and dichloromethane (10 mL) were added to a reaction flask in sequence, followed by TIPT (0.5 mL). The mixture was stirred at room temperature for 16 h, and then STAB (0.61 g, 2.90 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.50 g of a product with a yield of 65%.

### Step 2: Synthesis of 1-(6-([1,4'-bipiperidin]-4-yl)-5-fluoro-1-methyl-1H-indazol-3-yl)dihydropyrimidine-2,4(1H,3H)-dione

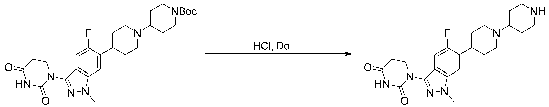

At room temperature, tert-butyl 4-(3-(2,4-dioxotetrahydropyrimidin-1(2H)-yl)-5-fluoro-1-methyl-1H-indazol-6-yl)-[1,4'-bipiperidine]-1'-carboxylate (0.50 g, 0.95 mmol) and HCl in dioxane (10 ml) were added to a reaction flask in sequence, and the reaction was carried out at room temperature for 2 h. After the reaction was complete, the reaction mixture was dried by rotary evaporation to afford 0.40 g of a crude product, which was directly taken to the next step.

### Step 3: Synthesis of 1-(6-(1'-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-[1,4'-bispiperidine]-4-yl)-5-fluoro-1-methyl-1H-indazol-3-yl)dihydropyrimidine-2,4(1H,3H)-dione

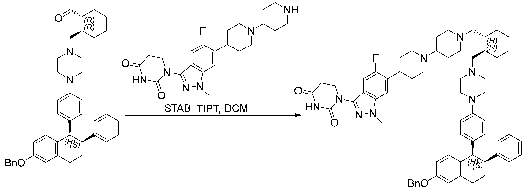

At room temperature, 1-(6-([1,4'-bipiperidin]-4-yl)-5-fluoro-1-methyl-1H-indazol-3-yl)dihydropyrimidine-2,4(1H,3H)-dione (0.08 g, 0.18 mmol), (1S,2S)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.11 g, 0.18 mmol) and dichloromethane (10 ml) were added to a reaction flask in sequence, followed by TIPT (0.5 ml). The mixture was stirred at room temperature for 16 h, and then STAB (0.08 g, 0.36 mmol) was added. The mixture was stirred at room temperature for 2 h. After the reaction was complete, dichloromethane was added for dilution, celite was added, and after stirring evenly, water was added to quench the reaction. After stirring for 20 min, the reaction mixture was filtered. The filter cake was slurried twice with dichloromethane. The organic phases were combined, stripped of solvent, and subjected to column chromatography using a DCM/MeOH system to afford 0.08 g of a product with a yield of 72%.

### Step 4: 1-(5-fluoro-6-(1'-((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)-[1,4'-bipiperidin]-4-yl)-1-methyl-1H-indazol-3-yl)dihydropyrimidine-2,4(1H,3H)-dione

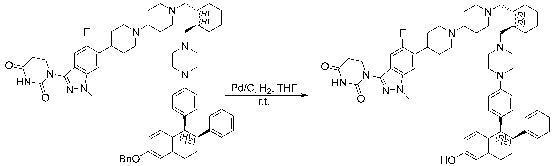

At room temperature, 1-(6-(1'-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-[1,4'-bispiperidine]-4-yl)-5-fluoro-1-methyl-1H-indazol-3-yl)dihydropyrimidine-2,4(1H,3H)-dione (0.08 g, 0.08 mmol), 10% palladium on carbon (0.01 g), and tetrahydrofuran (5 ml) were added to a reaction flask in sequence. Hydrogen replacement was then performed 3-4 times, and the mixture was stirred at 30°C for 16 h. After the reaction was complete, the reaction mixture was filtered through celite. The filter cake was rinsed with tetrahydrofuran. The filtrate was stripped of solvent, and then subjected to column chromatography using a DCM/MeOH system to afford 0.06 g of a product with a yield of 82%.

¹H NMR (400 MHz, DMSO-*d*₆) *δ* 10.58 (s, 1H), 9.17 (s, 1H), 7.40 (d, *J* = 10.8 Hz, 1H), 7.13 (q, *J* = 6.0, 5.6 Hz, 4H), 6.83 (d, *J=* 7.1 Hz, 2H), 6.64 - 6.56 (m, 5H), 6.50 - 6.47 (m, 1H), 6.23 (d, *J* = 8.1 Hz, 2H), 4.14 (d, *J* = 4.8 Hz, 1H), 4.00 (s, 3H), 3.91 (t, *J* = 6.7 Hz, 2H), 3.14 - 2.67 (m, 24H), 2.05 - 1.58 (m, 19H), 1.18 - 0.94 (m, 4H). MS (ESI) m/z: 921.8 [M+H]⁺.

Example 29 Synthesis of 1-(5-fluoro-6-((2R)-1-(((1R, 2R)-2-((4-(4-(((1R, 2S)-6-hydroxy-2-phenyl-1,2,3,4-tetrahydronaphthalen-1-yl)phenyl)piperazin-1-yl)methyl)cyclohexyl)methyl)-2-methylpiperidin-4-yl)-1-methyl-1H-indazol-3-yl)dihydropyrimidine-2,4(1H,3H)-dione (Compound VI-20)

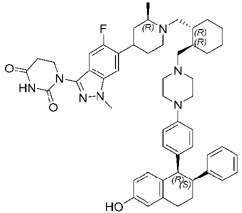

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl (R)-2-methyl-4-(((trifluoromethyl)sulfonyl)oxy)-3,6-dihydropyridine-1(2H)-carboxylate.

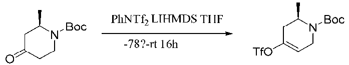

At -78°C, tert-butyl (R)-2-methyl-4-oxopiperidin-1-carboxylate (2.00 g, 9.38 mmol) and PhNTf₂ (3.68 g, 10.92 mmol), THF (20 mL) and LiHMDS (10.50 g, 10.32 mmol) were added to a single-necked flask in sequence. Under a nitrogen atmosphere, the mixture was heated to room temperature and the reaction was carried out for 16 h before termination. The reaction mixture was diluted with saturated ammonium chloride aqueous solution, and extracted with EA. The organic phase was dried over anhydrous Na₂SO₄, filtered, concentrated, and purified by column chromatography (an EA/PE system) to afford 3.00 g of a yellow oil.

### Step 2: Synthesis of tert-butyl (R)-4-(3-(2,4-dioxotetrahydropyrimidin-1(2H)-yl)-5-fluoro-1-methyl-1H-indazol-6-yl)-2-methyl-3,6-dihydropyridine-1(2H)-carboxylate.

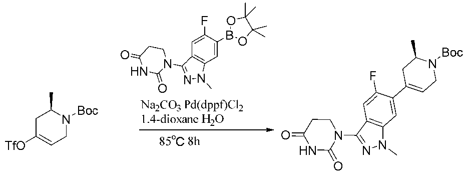

At room temperature, tert-butyl (R)-2-methyl-4-(((trifluoromethyl)sulfonyl)oxy)-3,6-dihydropyridine-1(2H)-carboxylate (0.21 g, 0.62 mmol), 1-(5-fluoro-1-methyl-6-(4,4,5,5-tetramethyl-1,3,2-dioxaborolan-2-yl)-1H-indazol-3-yl)dihydropyrimidine-2,4(1H,3H)-dione (0.20 g, 0.51 mmol), sodium carbonate (0.11 g, 1.02 mmol), Pd(dppf)Cl₂ (0.04 g, 0.05 mmol), 1,4-dioxane (20 mL), and H₂O (1 mL) were added to a single-necked flask in sequence. Under nitrogen protection, the mixture was heated to 85°C and the reaction was carried out for 8 h before termination. The reaction mixture was cooled to room temperature, diluted with water, and extracted with EA. The organic phase was dried over anhydrous Na₂SO₄, filtered, concentrated, and purified by column chromatography (an EA/PE system) to afford 0.20 g of a yellow solid.

### Step 3: Synthesis of (R)-1-(5-fluoro-1-methyl-6-(2-methyl-1,2,3,6-tetrahydropyridin-4-yl)-1H-indazol-3-yl)dihydropyrimidine-2,4(1H,3H)-dione.

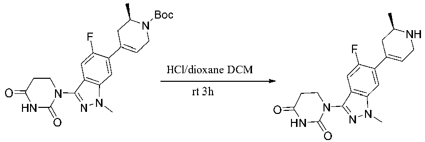

At room temperature, tert-butyl (R)-4-(3-(2,4-dioxotetrahydropyrimidin-1(2H)-yl)-5-fluoro-1-methyl-1H-indazol-6-yl)-2-methyl-3,6-dihydropyridine-1(2H)-carboxylate (0.20 g, 0.44 mmol), DCM (10 mL), and hydrochloride in dioxane (10 mL) were added to a single-necked flask in sequence. The reaction was carried out at room temperature for 3 h before termination. Concentration was performed to afford 0.20 g of a crude product as a yellow solid.

### Step 4: Synthesis of 1-(6-((R)-1-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-2-methyl-1,2,3,6-tetrahydropyridin-4-yl)-5-fluoro-1-methyl-1H-indazol-3-yl)dihydropyrimidine-2,4(1H,3H)-dione.

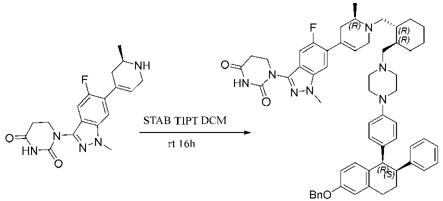

At room temperature, (R)-1-(5-fluoro-1-methyl-6-(2-methyl-1,2,3,6-tetrahydropyridin-4-yl)-1H-indazol-3-yl)dihydropyrimidine-2,4(1H,3H)-dione (0.16 g, 0.44 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.16 g, 0.44 mmol), and DCM (20 mL) were added to a single-necked flask in sequence. The reaction was carried out at room temperature for 16 h, and then sodium triacetoxyborohydride (0.18 g, 0.87 mmol) was added and the reaction was carried out for 1 h. Water (1 mL) was added. The mixture was stirred for 5 min, and filtered through celite. The filtrate was concentrated and subjected to column chromatography (a MeOH/DCM system) to afford 0.14 g of a yellow solid.

### Step 5: Synthesis of 1-(5-fluoro-6-((2R)-1-(((1R, 2R)-2-((4-(4-(((3R, 2S)-6-hydroxy-2-phenyl-1,2,3,4-tetrahydronaphthalen-1-yl)phenyl)piperazin-1-yl)methyl)cyclohexyl)methyl)-2-methylpiperidin-4-yl)-1-methyl-1H-indazol-3-yl)dihydropyrimidine-2,4(1H,3H)-dione.

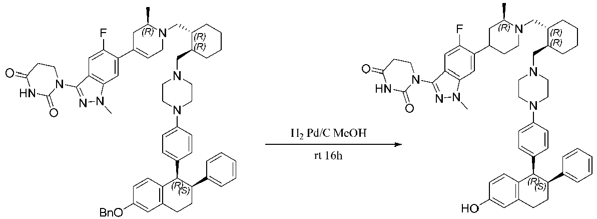

At room temperature, 1-(6-((R)-1-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-2-methyl-1,2,3,6-tetrahydropyridin-4-yl)-5-fluoro-1-methyl-1H-indazol-3-yl)dihydropyrimidine-2,4(1H,3H)-dione (0.14 g, 0.15 mmol), Pd/C (0.20 g, 10%), and MeOH (20 mL) were added to a single-necked flask in sequence. The reaction was carried out at room temperature for 16 h under a hydrogen balloon atmosphere. The mixture was filtered through celite, and the filtrate was concentrated and subjected to column chromatography (a MeOH/DCM system) to afford 0.50 g of a white solid. MS (ESI) m/z:852.9[M+H]⁺.

Example 30 Synthesis of 1-(5-fluoro-6-((2S)-1-(((1R, 2R)-2-((4-(4-(((1R, 2S)-6-hydroxy-2-phenyl-1,2,3,4-tetrahydronaphthalen-1-yl)phenyl)piperazin-1-yl)methyl)cyclohexyl)methyl)-2-methylpiperidin-4-yl)-1-methyl-1H-indazol-3-yl)dihydropyrimidine-2,4(1H,3H)-dione (Compound VI-21)

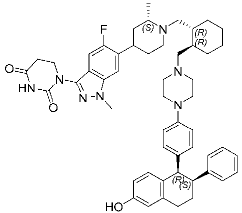

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl (S)-2-methyl-4-(((trifluoromethyl)sulfonyl)oxy)-3,6-dihydropyridine-1 (2H)-carboxylate.

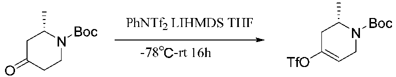

At -78°C, tert-butyl (S)-2-methyl-4-oxopiperidin-1-carboxylate (2.00 g, 9.38 mmol), PhNTf₂ (3.68 g, 10.92 mmol), THF (20 mL) and LiHMDS (10.50 g, 10.32 mmol) were added to a single-necked flask in sequence. Under a nitrogen atmosphere, the mixture was heated to room temperature and the reaction was carried out for 16 h before termination. The reaction mixture was diluted with saturated ammonium chloride aqueous solution, and extracted with EA. The organic phase was dried over anhydrous Na₂SO₄, filtered, concentrated, and purified by column chromatography (an EA/PE system) to afford 3.00 g of a yellow oil.

Step 2: Synthesis of tert-butyl (S)-4-(3-(2,4-dioxotetrahydropyrimidin-1(2H)-yl)-5-fluoro-1-methyl-1H-indazol-6-yl)-2-methyl-3,6-dihydropyridine-1(2H)-carboxylate.

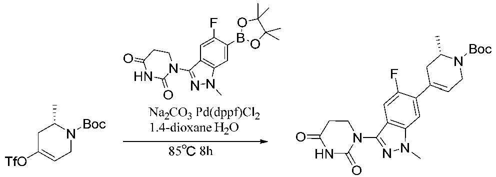

At room temperature, tert-butyl (S)-2-methyl-4-(((trifluoromethyl)sulfonyl)oxy)-3,6-dihydropyridine-1(2H)-carboxylate (0.21 g, 0.62 mmol), 1-(5-fluoro-1-methyl-6-(4,4,5,5-tetramethyl-1,3,2-dioxaborolan-2-yl)-1H-indazol-3-yl)dihydropyrimidine-2,4(1H,3H)-dione (0.20 g, 0.51 mmol), sodium carbonate (0.11 g, 1.02 mmol), Pd(dppf)Cl₂ (0.04 g, 0.05 mmol), 1,4-dioxane (20 mL), and H₂O (1 mL) were added to a single-necked flask in sequence. Under nitrogen protection, the mixture was heated to 85°C and the reaction was carried out for 8 h before termination. The reaction mixture was cooled to room temperature, diluted with water, and extracted with EA. The organic phase was dried over anhydrous Na₂SO₄, filtered, concentrated, and purified by column chromatography (an EA/PE system) to afford 0.20 g of a yellow solid.

### Step 3: Synthesis of (S)-1-(5-fluoro-1-methyl-6-(2-methyl-1,2,3,6-tetrahydropyridin-4-yl)-1H-indazol-3-yl)dihydropyrimidine-2,4(1H,3H)-dione.

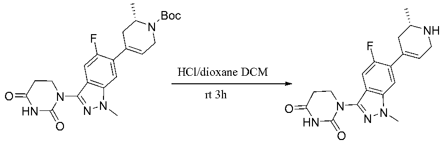

At room temperature, tert-butyl (S)-4-(3-(2,4-dioxotetrahydropyrimidin-1(2H)-yl)-5-fluoro-1-methyl-1H-indazol-6-yl)-2-methyl-3,6-dihydropyridine-1(2H)-carboxylate (0.20 g, 0.44 mmol), DCM (10 mL), and hydrochloride in dioxane (10 mL) were added to a single-necked flask in sequence. The reaction was carried out at room temperature for 3 h before termination. Concentration was performed to afford 0.20 g of a crude product as a yellow solid.

### Step 4: Synthesis of 1-(6-((S)-1-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-2-methyl-1,2,3,6-tetrahydropyridin-4-yl)-5-fluoro-1-methyl-1H-indazol-3-yl)dihydropyrimidine-2,4(1H,3H)-dione.

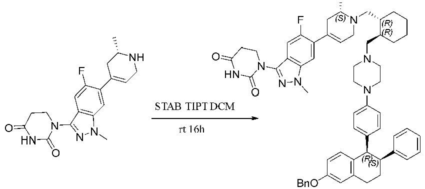

At room temperature, (S)-1-(5-fluoro-1-methyl-6-(2-methyl-1,2,3,6-tetrahydropyridin-4-yl)-1H-indazol-3-yl)dihydropyrimidine-2,4(1H,3H)-dione (0.16 g, 0.44 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.16 g, 0.44 mmol), and DCM (20 mL) were added to a single-necked flask in sequence. The reaction was carried out at room temperature for 16 h, and then sodium triacetoxyborohydride (0.18 g, 0.87 mmol) was added and the reaction was carried out for 1 h. Water (1 mL) was added. The mixture was stirred for 5 min, and filtered through celite. The filtrate was concentrated and subjected to column chromatography (a MeOH/DCM system) to afford 0.14 g of a yellow solid.

### Step 5: Synthesis of 1-(5-fluoro-6-((2S)-1-(((1R,2R)-2-((4-(4-(((3R,2S)-6-hydroxy-2-phenyl-1,2,3,4-tetrahydronaphthalen-1-yl)phenyl)piperazin-1-yl)methyl)cyclohexyl)methyl)-2-methylpiperidin-4-yl)-1-methyl-1H-indazol-3-yl)dihydropyrimidine-2,4(1H,3H)-dione.

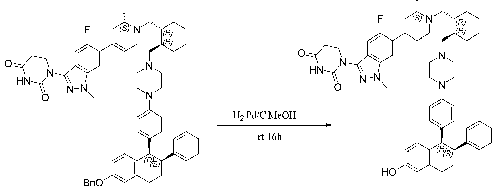

At room temperature, 1-(6-((S)-1-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-2-methyl-1,2,3,6-tetrahydropyridin-4-yl)-5-fluoro-1-methyl-1H-indazol-3-yl)dihydropyrimidine-2,4(1H,3H)-dione (0.14 g, 0.15 mmol), Pd/C (0.20 g, 10%), and MeOH (20 mL) were added to a single-necked flask in sequence. The reaction was carried out at room temperature for 16 h under a hydrogen balloon atmosphere. The mixture was filtered through celite, and the filtrate was concentrated and subjected to column chromatography (a MeOH/DCM system) to afford 0.50 g of a white solid. MS (ESI) m/z:852.9[M+H]⁺.

Example 31 Synthesis of 1-(5-fluoro-6-((2S,6R)-1-(((1R,2R)-2-((4-(4-(((1R,2S)-6-hydroxy-2-phenyl-1,2,3,4-tetrahydronaphthalen-1-yl)phenyl)piperazin-1-yl)methyl)cyclohexyl)methyl)-2,6-dimethylpiperidin-4-yl)-1-methyl-1H-indazol-3-yl)dihydropyrimidine-2,4(1H,3H)-dione (Compound VI-22)

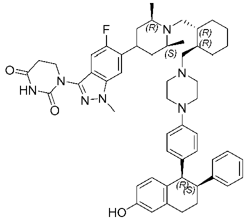

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of (2S,6R)-1-(4-methoxybenzyl)-2,6-dimethylpiperidin-4-one.

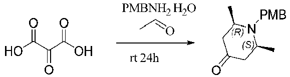

At room temperature, 40% acetaldehyde (6.00 g, 54.80 mmol) was added to a solution of acetone dicarboxylic acid (4.00 g, 27.40 mmol) in water (20 mL). Then, 4-methoxyphenylmethylamine (3.75 g, 27.40 mmol) was added in small portions over 10 min. Stirring was performed at room temperature for 24 h. The reaction mixture was extracted with dichloromethane. The combined extracts were washed with brine and dried with anhydrous sodium sulfate. After filtration, the filtrate was concentrated and subjected to column chromatography (a DCM/EA system) to afford 4.00 g of a light yellow oil.

### Step 2: Synthesis of (2S,6R)-2,6-dimethylpiperidin-4-one.

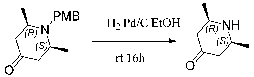

At room temperature, cis-1-(4-methoxybenzyl)-2,6-dimethylpiperidin-4-one (4.00 g, 16.20 mmol) was dissolved in ethanol (20 mL) and a catalyst (0.40 g, 10% Pd-C) was added. The mixture was stirred for 16 h under a hydrogen atmosphere. The mixture was filtered, and the filtrate was concentrated to afford 1.80 g of a crude product as a yellow oil.

### Step 3: Synthesis of tert-butyl (2S,6R)-2,6-dimethyl-4-oxopiperidin-1-carboxylate.

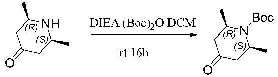

At room temperature, di-tert-butyl dicarbonate (3.14 g, 14.40 mmol) and DIEA (3.10 g, 24.0 mmol) were added to a solution of cis-1-(4-methoxybenzyl)-2,6-dimethylpiperidin-4-one (1.54 g, 12.0 mmol) in dichloromethane (30 mL), and the mixture was stirred at room temperature for 16 h. The reaction solution was concentrated and subjected to column chromatography (an EA/PE system) to afford 2.0 g of a white solid.

### Step 4: Synthesis of tert-butyl (2S,6R)-2,6-dimethyl-4-(((trifluoromethyl)sulfonyl)oxy)-3,6-dihydropyridine-1 (2H)-carboxylate.

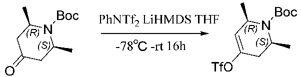

At -78°C, tert-butyl (2S,6R)-2,6-dimethyl-4-oxopiperidin-1-carboxylate (2.00 g, 8.80 mmol), PhNTf₂ (3.68 g, 10.92 mmol), THF (20 mL) and LiHMDS (10.50 mL, 10.50 mmol) were added to a single-necked flask in sequence. Under a nitrogen atmosphere, the mixture was heated to room temperature and the reaction was carried out for 16 h before termination. The reaction mixture was diluted with saturated ammonium chloride aqueous solution, and extracted with EA. The organic phase was dried over anhydrous Na₂SO₄, filtered, concentrated, and purified by column chromatography (an EA/PE system) to afford 3.00 g of a yellow oil.

### Step 5: Synthesis of tert-butyl (2S,6R)-4-(3-(2,4-dioxotetrahydropyrimidin-1(2H)-yl)-5-fluoro-1-methyl-1H-indazol-6-yl)-2,6-dimethyl-3,6-dihydropyridine-1(2H)-carboxylate.

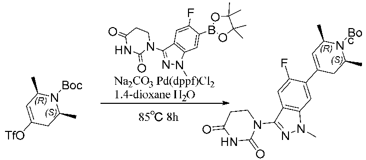

At room temperature, tert-butyl (2S,6R)-2,6-dimethyl-4-(((trifluoromethyl)sulfonyl)oxy)-3,6-dihydropyridine-1(2H)-carboxylate (0.21 g, 0.58 mmol), 1-(5-fluoro-1-methyl-6-(4,4,5,5-tetramethyl-1,3,2-dioxaborolan-2-yl)-1H-indazol-3-yl)dihydropyrimidine-2,4(1H,3H)-dione (0.20 g, 0.51 mmol), sodium carbonate (0.11 g, 1.02 mmol), Pd(dppf)Cl₂ (0.04 g, 0.05 mmol), 1,4-dioxane (20 mL), and H₂O (1 mL) were added to a single-necked flask in sequence. Under nitrogen protection, the mixture was heated to 85°C and the reaction was carried out for 8 h before termination. The reaction mixture was cooled to room temperature, diluted with water, and extracted with EA. The organic phase was dried over anhydrous Na₂SO₄, filtered, concentrated, and purified by column chromatography (an EA/PE system) to afford 0.20 g of a yellow solid.

### Step 6: Synthesis of 1-(6-((2S,6R)-2,6-dimethyl-1,2,3,6-tetrahydropyridin-4-yl)-5-fluoro-1-methyl-1H-indazol-3-yl)dihydropyrimidine-2,4(1H,3H)-dione.

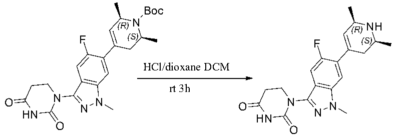

At room temperature, tert-butyl (2S,6R)-4-(3-(2,4-dioxotetrahydropyrimidin-1(2H)-yl)-5-fluoro-1-methyl-1H-indazol-6-yl)-2,6-dimethyl-3,6-dihydropyridine-1(2H)-carboxylate (0.20 g, 0.42 mmol), DCM (10 mL), and hydrochloride in dioxane (10 mL) were added to a single-necked flask in sequence. The reaction was carried out at room temperature for 3 h before termination. Concentration was performed to afford 0.20 g of a crude product as a yellow solid.

### Step 7: Synthesis of 1-(6-((2S,6R)-1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-2,6-dimethyl-1,2,3,6-tetrahydropyridin-4-yl)-5-fluoro-1-methyl-1H-indazol-3-yl)dihydropyrimidine-2,4(1H,3H)-dione.

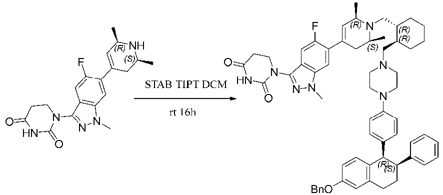

At room temperature, 1-(6-((2S,6R)-2,6-dimethyl-1,2,3,6-tetrahydropyridin-4-yl)-5-fluoro-1-methyl-1H-indazol-3-yl)dihydropyrimidine-2,4(1H,3H)-dione (0.16 g, 0.40 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.16 g, 0.44 mmol), and DCM (20 mL) were added to a single-necked flask in sequence. The reaction was carried out at room temperature for 16 h, and then sodium triacetoxyborohydride (0.18 g, 0.87 mmol) was added and the reaction was carried out for 1 h. Water (1 mL) was added. The mixture was stirred for 5 min, and filtered through celite. The filtrate was concentrated and subjected to column chromatography (a MeOH/DCM system) to afford 0.14 g of a yellow solid.

### Step 8: Synthesis of 1-(5-fluoro-6-((2S,6R)-1-(((1R,2R)-2-((4-(4-(((3R,2S)-6-hydroxy-2-phenyl-1,2,3,4-tetrahydronaphthalen-1-yl)phenyl)piperazin-1-yl)methyl)cyclohexyl)methyl)-2,6-dimethylpiperidin-4-yl)-1-methyl-1H-indazol-3-yl)dihydropyrimidine-2,4(1H,3H)-dione.

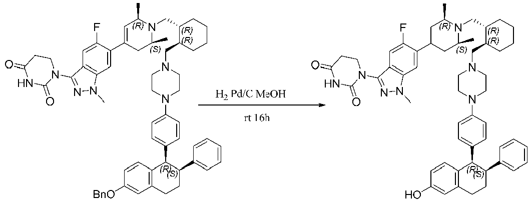

At room temperature, 1-(6-((2S,6R)-1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-2,6-dimethyl-1,2,3,6-tetrahydropyridin-4-yl)-5-fluoro-1-methyl-1H-indazol-3-yl)dihydropyrimidine-2,4(1H,3H)-dione (0.14 g, 0.14 mmol), Pd/C (0.20 g, 10%), and MeOH (20 mL) were added to a single-necked flask in sequence. The reaction was carried out at room temperature for 16 h under a hydrogen balloon atmosphere. The mixture was filtered through celite, and the filtrate was concentrated and subjected to column chromatography (a MeOH/DCM system) to afford 0.01 g of a white solid. MS (ESI) m/z:866.9[M+H]⁺.

### Example 32 Synthesis of 1-(5-fluoro-6-(4-hydroxy-1-(((1R, 2R)-2-((4-(4-((1R, 2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)dihydropyrimidine-2,4(1H, 3H)-dione (Compound VI-23)

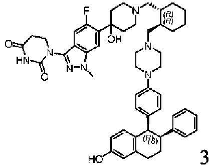

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: tert-butyl 4-(3-(2,4-dioxotetrahydropyrimidin-1(2H)-yl)-5-fluoro-1-methyl-1H-indazol-6-yl)-3,6-dihydropyridine-1(2H)-carboxylate

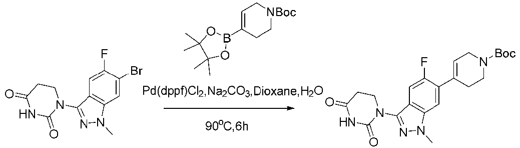

tert-butyl 1-(6-bromo-5-fluoro-1-methyl-1H-indazol-3-yl)dihydropyrimidin-2,4(1H,3H)-dione (10 g, 29.3 mmol), 4-(4,4,5,5-tetramethyl-1,3,2-dioxaborolan-2-yl)-3,6-dihydropyridine-1(2H)-carboxylate (10.9 g, 35.2 mmol) and Pd(dppf)Cl₂ (1.1 g, 1.5 mmol) were placed in 200 mL of 1,4-dioxane, and 80 mL of an aqueous solution of Na₂CO₃ (6.2 g, 58.6 mmol) was added. Nitrogen replacement was performed and the reaction was carried out at 90 °C for 6 h. The reaction completion was determined by TLC and LC-MS. The reaction solution was concentrated, water was added, and extraction was performed with EA. The organic phase was dried, concentrated, sanded, and subjected to column chromatography (DCM/MeOH) to give a crude product. The crude product was then slurried with ethyl acetate to afford 9.5 g of an off-white solid product.

### Step 2: tert-butyl 4-(3-(2,4-dioxotetrahydropyrimidin-1(2H)-yl)-5-fluoro-1-methyl-1H-indazol-6-yl)-4-hydroxypiperidine-1-carboxylate

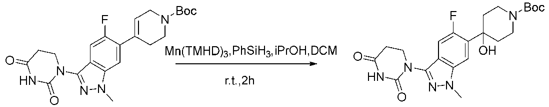

Tert-butyl 4-(3-(2,4-dioxotetrahydropyrimidin-1(2H)-yl)-5-fluoro-1-methyl-1H-indazol-6-yl)-3,6-dihydropyridine-1(2H)-carboxylate (9.5 g, 21.4 mmol) was dissolved in 300 mL of dichloromethane and 100 mL of isopropanol. Mn(TMHD)₃ (1.3 g, 2.1 mmol) was added at room temperature, and PhSiH₃ (3.5 g, 32.1 mmol) was added under an ice-water bath. After the addition, the reaction was carried out for 2 h under an oxygen balloon atmosphere. The reaction completion was determined by TLC and LC-MS. The reaction solution was concentrated, water was added, and extraction was performed with ethyl acetate. The organic phase was concentrated, sanded, and subjected to column chromatography (PE/EA/DCM/MeOH) to afford a crude product. The crude product was then slurried with ethyl acetate to afford 3.6 g of an off-white solid product.

### Step 3: 1-(5-fluoro-6-(4-hydroxypiperidin-4-yl)-1-methyl-1H-indazol-3-yl)dihydropyrimidine-2,4(1H,3H)-dione hydrochloride

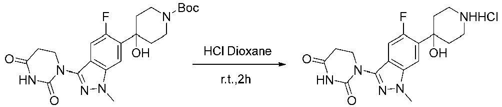

Tert-butyl 4-(3-(2,4-dioxotetrahydropyrimidin-1(2H)-yl)-5-fluoro-1-methyl-1H-indazol-6-yl)-4-hydroxypiperidine-1-carboxylate (3.6 g, 7.8 mmol) was placed in 40 mL of hydrochloride in 1,4-dioxane. The reaction was carried out at room temperature for 2 h. The reaction completion was determined by LC-MS. The reaction solution was filtered, and the filter cake was air-dried to afford 3.1 g of an off-white solid product.

### Step 4: 1-(6-(1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-4-hydroxypiperidin-4-yl)-5-fluoro-1-methyl-1H-indazol-3-yl)dihydropyrimidine-2,4(1H,3H)-dione

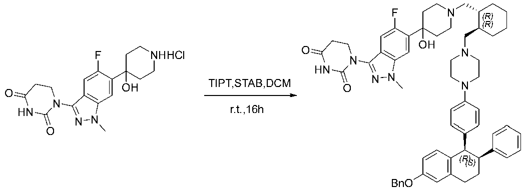

1-(5-fluoro-6-(4-hydroxypiperidin-4-yl)-1-methyl-1H-indazol-3-yl)dihydropyrimidine-2,4(1H,3H)-dione hydrochloride (2.6 g, 7.2 mmol) and (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (4.3 g, 7.2 mmol) were placed in 150 mL of dichloromethane, and 10 mL of tetraisopropyl titanate was added. The reaction was carried out at 20 °C for 16 h. Sodium triacetoxyborohydride (2.3 g, 10.8 mmol) was added. The reaction was continued for 1 h. The reaction completion was determined by TLC and LC-MS. The reaction was quenched by adding 3 mL of water, followed by 3 mL of ammonia. The mixture was stirred for 20 min, and filtered. The filtrate was concentrated, sanded and subjected to column chromatography (DCM/MeOH) to afford 2.6 g of an off-white solid product.

### Step 5: 1-(5-fluoro-6-(4-hydroxy-1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)dihydropyrimidine-2,4(1H,3H)-dione

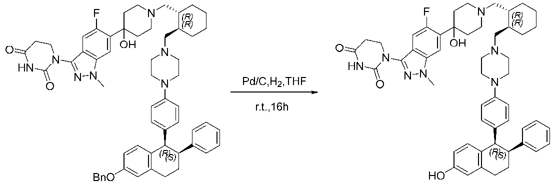

1-(6-(1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-4-hydroxypiperidin-4-yl)-5-fluoro-1-methyl-1H-indazol-3-yl)dihydropyrimidine-2,4(1H,3H)-dione (2.6 g, 2.75 mmol) was placed in 50 mL of tetrahydrofuran, and 3.0 g of 5% Pd/C was added. Hydrogen replacement was performed and the reaction was carried out at 20 °C for 16 h. The reaction completion was determined by TLC and LC-MS. The reaction solution was filtered, and the filtrate was concentrated, sanded, and subjected to column chromatography (DCM/MeOH) to afford 1.3 g of an off-white solid product. ¹H NMR (400 MHz, DMSO-*d*₆) δ 10.58 (s, 1H), 9.15 (s, 1H), 7.77 (d, *J =* 6.3 Hz, 1H), 7.38 (d, *J* = 12.7 Hz, 1H), 7.13 (dt, *J =* 11.2, 6.7 Hz, 3H), 6.83 (d, *J* = 7.3 Hz, 2H), 6.73 - 6.42 (m, 5H), 6.22 (d, *J =* 8.2 Hz, 2H), 5.25 (br, 1H), 4.14 (d, *J* = 5.1 Hz, 1H), 4.08 - 3.78 (m, 5H), 3.34 - 3.24 (m, 6H), 3.07 - 2.90 (m, 6H), 2.78 - 2.73 (m, 2H), 2.58 - 2.50 (m, 6H), 2.37 - 2.28 (m, 2H), 2.17 - 2.05 (m, 2H), 1.84 - 1.44 (m, 8H), 1.28 - 1.14 (m, 3H), 0.99 (s, 2H). MS (ESI) m/z: 854.8 [M+H]⁺.

### Example 33 Synthesis of 1-(5-fluoro-6-(4-(4-(((1R, 2R)-2-((4-(-4-((1R, 2S)-6-hydroxy-2-phenyl-1, 2, 3,4-tetrahydronaphthalen-1-yl)phenyl)piperazin-1-yl)methyl)cyclohexyl)methyl)piperazin-1-yl)cyclohexyl-1-methyl-1H-indazol-3-yl)dihydropyrimidine-2,4(1H,3H)-dione (Compound VI-24)

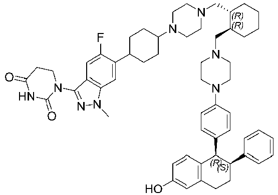

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 1-(5-fluoro-1-methyl-6-(1,4-dioxy-aspirin[4.5]dec-7-en-8-yl)-1H-indazol-3-yl)dihydropyrimidine-2,4(1H,3H)-dione.

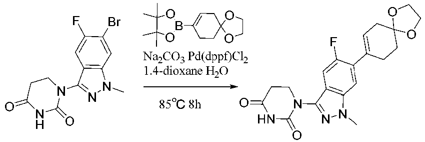

At room temperature, 4,4,5,5-tetramethyl-2-(1,4-dioxy-aspirin[4.5]dec-7-en-8-yl)-1,3,2-dioxaborolane (0.96 g, 1.23 mmol), 1-(6-bromo-5-fluoro-1-methyl-1H-indazol-3-yl)dihydropyrimidine-2,4(1H,3H)-dione (1.02 g, 3.00 mmol), sodium carbonate (0.64 g, 6.00 mmol), Pd(dppf)Cl₂ (0.22 g, 0.30 mmol), 1,4-dioxane (20 mL), H₂O (1 mL) were added to a single-necked flask in sequence. Under nitrogen protection, the mixture was heated to 85°C and the reaction was carried out for 8 h before termination. The reaction mixture was cooled to room temperature, diluted with water, and extracted with EA. The organic phase was dried over anhydrous Na₂SO₄, filtered, concentrated, and purified by column chromatography (an EA/PE system) to afford 1.0 g of a yellow solid.

### Step 2: Synthesis of 1-(5-fluoro-1-methyl-6-(1,4-dioxy-aspirin[4.5]dec-8-yl)-1H-indazol-3-yl)dihydropyrimidine-2,4(1H,3H)-dione.

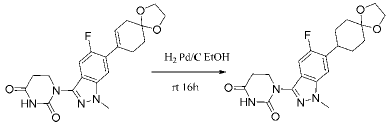

At room temperature, 1-(5-fluoro-1-methyl-6-(1,4-dioxy-aspirin[4.5]dec-7-en-8-yl)-1H-indazol-3-yl)dihydropyrimidine-2,4(1H,3H)-dione (1.00 g, 2.70 mmol) was dissolved in ethanol (20 mL) and a catalyst (0.40 g, 10% Pd-C) was added. The mixture was stirred for 16 h under a hydrogen atmosphere. The mixture was filtered, and the filtrate was concentrated to afford 1.0 g of a crude product as a yellow oil.

### Step 3: Synthesis of 1-(5-fluoro-1-methyl-6-(4-oxocyclohexyl)-1H-indazol-3-yl)dihydropyrimidine-2,4(1H,3H)-dione.

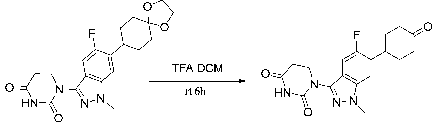

At room temperature, 1-(5-fluoro-1-methyl-6-(1,4-dioxy-aspirin[4.5]dec-8-yl)-1H-indazol-3-yl)dihydropyrimidine-2,4(1H,3H)-dione (1.0 g, 2.70 mmol), DCM (10 mL), and TFA (2 mL) were added to a single-necked flask in sequence, and the mixture was stirred at room temperature for 6 h. The reaction solution was concentrated to afford 0.80 g of a crude product as a yellow solid.

### Step 4: Synthesis of tert-butyl 4-(4-(3-(2,4-dioxotetrahydropyrimidin-1(2H)-yl)-5-fluoro-1-methyl-1H-indazol-6-yl)cyclohexyl)piperazine-1-carboxylate.

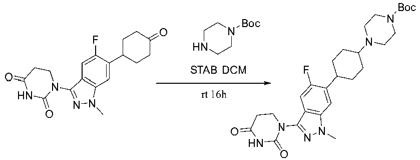

At room temperature, 1-(5-fluoro-1-methyl-6-(4-oxocyclohexyl)-1H-indazol-3-yl)dihydropyrimidine-2,4(1H,3H)-dione (0.80 g, 2.23 mmol), tert-butyl piperazine-1-carboxylate (0.62 g, 3.35 mmol), DCM (20 mL), and STAB (0.95 g, 4.46 mmol) were added to a single-necked flask in sequence. The reaction was carried out at room temperature for 16 h before termination. The reaction mixture was diluted with saturated sodium carbonate aqueous solution, and extracted with DCM. The organic phase was dried over anhydrous Na₂SO₄, filtered, concentrated, and purified by column chromatography (a MeOH/DCM system) to afford 1.05 g of a yellow solid.

### Step 5: Synthesis of 1-(5-fluoro-1-methyl-6-(4-(piperazin-1-yl)cyclohexyl)-1H-indazol-3-yl)dihydropyrimidine-2,4(1H,3H)-dione.

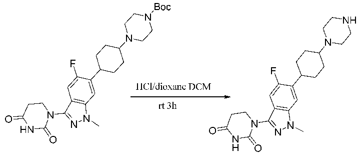

At room temperature, tert-butyl 4-(4-(3-(2,4-dioxotetrahydropyrimidin-1(2H)-yl)-5-fluoro-1-methyl-1H-indazol-6-yl)cyclohexyl)piperazine-1-carboxylate (1.05 g, 2.00 mmol), DCM (10 mL), and hydrochloride in dioxane (10 mL) were added to a single-necked flask in sequence. The reaction was carried out at room temperature for 3 h before termination. Concentration was performed to afford 1.00 g of a crude product as a yellow solid.

### Step 6: Synthesis of 1-(6-(4-(4-(((1R,2R)-2-)(4-(-4-(((3R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)cyclohexyl)-5-fluoro-1-methyl-1H-indazol-3-yl)dihydropyrimidine-2,4(1H,3H)-dione.

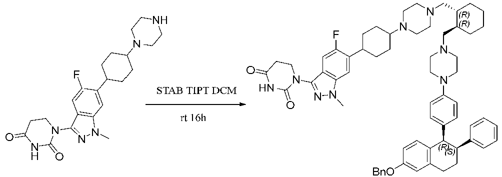

At room temperature, 1-(5-fluoro-1-methyl-6-(4-(piperazin-1-yl)cyclohexyl)-1H-indazol-3-yl)dihydropyrimidine-2,4(1H,3H)-dione (0.10 g, 0.23 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.14 g, 0.23 mmol), DCM (20 mL), and TIPT (1 mL) were added to a single-necked flask in sequence. The reaction was carried out at room temperature for 16 h, and then sodium triacetoxyborohydride (0.07 g, 0.35 mmol) was added and the reaction was carried out for 1 h. Water (1 mL) was added. The mixture was stirred for 5 min, and filtered with celite. The filtrate was concentrated and subjected to column chromatography (a MeOH/DCM system) to afford 0.10 g of a yellow solid.

### Step 7: Synthesis of 1-(5-fluoro-6-(4-(4-((1R,2R)-2-((4-(-4-((1R,2S)-6-hydroxy-2-phenyl-1,2,3,4-tetrahydronaphthalen-1-yl)phenyl)piperazin-1-yl)methyl)cyclohexyl)methyl)piperazin-1-yl)cyclohexyl-1-methyl-1H-indazol-3-yl)dihydropyrimidine-2,4(1H,3H)-dione.

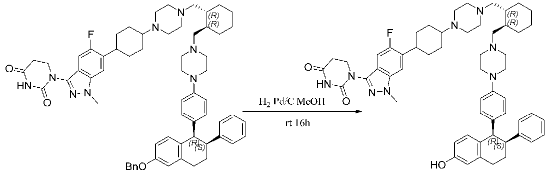

At room temperature, 1-(6-(4-(4-(((1R,2R)-2-)(4-(-4-(((3R,2S)-6-(benzyloxy)-2-phenyl-1,2,3,4-tetrahydronaphthalen-1-yl)phenyl)piperazin-1-yl)methyl)cyclohexyl)methyl)piperazin-1-yl)cyclohexyl)-5-fluoro-1-methyl-1H-indazol-3-yl)dihydropyrimidine-2,4(1H,3H)-dione (0.10 g, 0.10 mmol), Pd/C (0.20 g, 10%), and MeOH (20 mL) were added to a single-necked flask in sequence. The reaction was carried out at room temperature for 16 h under a hydrogen balloon atmosphere. The mixture was filtered through celite, and the filtrate was concentrated and subjected to column chromatography (a MeOH/DCM system) to afford 0.02 g of a white solid. 1H NMR (400 MHz, DMSO-d6) δ 10.56 (s, 1H), 9.14 (s, 1H), 7.42 (d, J = 6.0 Hz, 1H), 7.35 (d, J = 10.9 Hz, 1H), 7.19 - 7.08 (m, 3H), 6.83 (d, J = 7.1 Hz, 2H), 6.67 - 6.58 (m, 2H), 6.56 - 6.44 (m, 3H), 6.21 (d, J = 8.3 Hz, 2H), 4.13 (d, J = 5.0 Hz, 1H), 3.99 (s, 3H), 3.90 (t, J = 6.7 Hz, 2H), 3.04 - 2.89 (m, 8H), 2.75 (t, J = 6.7 Hz, 2H), 2.48 - 2.29 (m, 8H), 2.22 - 2.17 (m, 1H), 2.11 - 2.02 (m, 4H), 1.88 - 1.67 (m, 5H), 1.62 - 1.37 (m, 9H), 1.33 - 1.12 (m, 8H), 1.03 - 0.90 (m, 2H).MS (ESI) m/z:921.9[M+H]⁺.

### Example 34 Synthesis of 1-(6-(4-(cyclopropyl(((1R, 2R)-2-((4-(4-(((1R, 2S)-6-hydroxy-2-phenyl-1, 2, 3,4-tetrahydronaphthalen-1-yl)phenyl)piperazin-1-yl)methyl)cyclohexyl)methyl)amino)cyclohexyl)-5-fluoro-1-methyl-1H-indazol-3-yl)dihydropyrimidine-2,4(1H,3H)-dione (Compound VI-25)

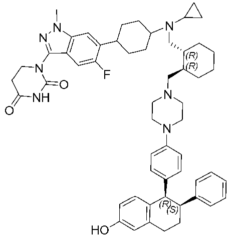

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of 1-(6-(4-(cyclopropylamino)cyclohexyl)-5-fluoro-1-methyl-1H-indazol-3-yl)dihydropyrimidine-2,4(1H,3H)-dione.

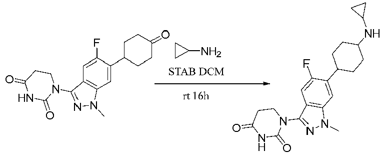

At room temperature, 1-(5-fluoro-1-methyl-6-(4-oxocyclohexyl)-1H-indazol-3-yl)dihydropyrimidine-2,4(1H,3H)-dione (0.10 g, 0.28 mmol), cyclopropylamine (0.06 g, 1.12 mmol), DCM (20 mL), and STAB (0.12 g, 0.56 mmol) were added to a single-necked flask in sequence. The reaction was carried out at room temperature for 16 h before termination. The reaction mixture was diluted with saturated sodium carbonate aqueous solution, and extracted with DCM. The organic phase was dried over anhydrous Na₂SO₄, filtered, concentrated, and purified by column chromatography (a MeOH/DCM system) to afford 0.09 g of a yellow solid.

### Step 2: Synthesis of 1-(6-(4-((((1R,2R)-2-((4-(4-(2R,2S)-6-(benzyloxy)-2-phenyl-1,2,3,4-tetrahydronaphthalen-1-yl)phenyl)piperazin-1-yl)methyl)cyclohexyl)(cyclopropyl)amino)cyclohexyl)-5-fluoro-1-methyl-1H-indazol-3-yl)dihydropyrimidine-2,4(1H,3H)-dione.

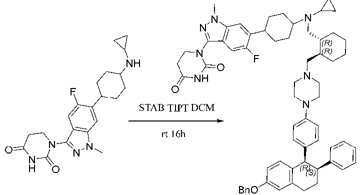

At room temperature, 1-(6-(4-(cyclopropylamino)cyclohexyl)-5-fluoro-1-methyl-1H-indazol-3-yl)dihydropyrimidine-2,4(1H,3H)-dione (0.09 g, 0.23 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.14 g, 0.23 mmol), DCM (20 mL), and TIPT (1 mL) were added to a single-necked flask in sequence. The reaction was carried out at room temperature for 16 h, and then sodium triacetoxyborohydride (0.07 g, 0.35 mmol) was added and the reaction was carried out for 1 h. Water (1 mL) was added. The mixture was stirred for 5 min, and filtered through celite. The filtrate was concentrated and subjected to column chromatography (a MeOH/DCM system) to afford 0.08 g of a yellow solid.

### Step 3: 1-(6-(4-(cyclopropyl(((1R,2R)-2-((4-(4-(((3R,2S)-6-hydroxy-2-phenyl-1,2,3,4-tetrahydronaphthalen-1-yl)phenyl)piperazin-1-yl)methyl)cyclohexyl)methyl)amino)cyclohexyl)-5-fluoro-1-methyl-1H-indazol-3-yl)dihydropyrimidine-2,4(1H,3H)-dione.

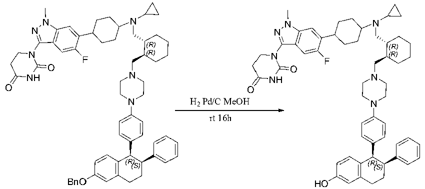

At room temperature, 1-(6-(4-((((1R,2R)-2-((4-(4-(2R,2S)-6-(benzyloxy)-2-phenyl-1,2,3,4-tetrahydronaphthalen-1-yl)phenyl)piperazin-1-yl)methyl)cyclohexyl)(cyclopropyl)amino)cyclohexyl)-5-fluoro-1-methyl-1H-indazol-3-yl)dihydropyrimidine-2,4(1H,3H)-dione (0.08 g, 0.08 mmol), Pd/C (0.20 g, 10%), and MeOH (20 mL) were added to a single-necked flask in sequence. The reaction was carried out at room temperature for 16 h under a hydrogen balloon atmosphere. The mixture was filtered through celite, and the filtrate was concentrated and subjected to column chromatography (a MeOH/DCM system) to afford 0.01 g of a white solid. MS (ESI) m/z:893.1[M+H]+

Example 35 Synthesis of 1-(5-fluoro-6-(6-(((1R, 2R)-2-((4-(4-(((1R, 2S)-6-hydroxy-2-phenyl-1,2,3,4-tetrahydronaphthalen-1-yl)phenyl)piperazin-1-yl)methyl)cyclohexyl)methyl)-2,6-diazaspiro[3.3]heptane-2-yl)-1-methyl-1H-indazol-3-yl)dihydropyrimidine-2,4(1H,3H)-dione (Compound VI-26)

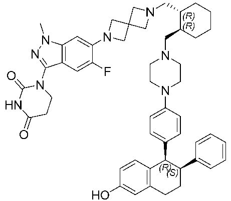

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 6-(3-(2,4-dioxotetrahydropyrimidin-1(2H)-yl)-5-fluoro-1-methyl-1H-indazol-6-yl)-2,6-diazaspiro[3.3]heptane-2-carboxylate.

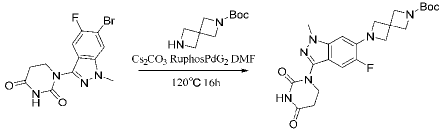

At room temperature, tert-butyl 1-(6-bromo-5-fluoro-1-methyl-1H-indazol-3-yl)dihydropyrimidine-2,4(1H,3H)-dione (1.00 g, 2.93 mmol), 2,6-diazaspiro[3.3]heptane-2-carboxylate (1.16 g, 5.86 mmol), cesium carbonate (1.91 g, 5.86 mmol), Ruphos (0.27 g, 0.58 mmol), RuphosPdG₂ (0.24 g, 0.29 mmol), DMF (100 mL) were added to a single-necked flask in sequence. Under a nitrogen atmosphere, the reaction was carried out at 120°C for 16 h before termination. The reaction mixture was cooled to room temperature, diluted with water, and extracted three times with EA. The combined organic phases were washed with water and brine, and dried over anhydrous Na₂SO₄, filtered, concentrated, and purified by column chromatography (a MeOH/DCM system) to afford 0.80 g of a yellow solid.

### Step 2: Synthesis of 1-(5-fluoro-1-methyl-6-(2,6-diazaspiro[3.3]heptane-2-yl)-1H-indazol-3-yl)dihydropyrimidine-2,4(1H,3H)-dione.

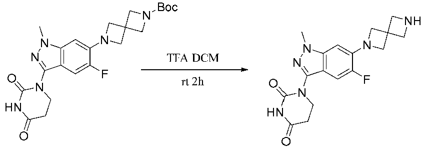

At room temperature, tert-butyl 6-(3-(2,4-dioxotetrahydropyrimidin-1(2H)-yl)-5-fluoro-1-methyl-1H-indazol-6-yl)-2,6-diazaspiro[3.3]heptane-2-carboxylate (0.80 g, 1.75 mmol), TFA (2 mL), and DCM (10 mL) were added to a single-necked flask in sequence. The reaction was carried out at room temperature for 3 h before termination. Concentration was performed to afford 0.70 g of a crude product as a yellow solid.

### Step 3: Synthesis of 1-(6-(6-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-2,6-diazaspiro[3.3]heptane-2-yl)-5-fluoro-1-methyl-1H-indazol-3-yl)dihydropyrimidine-2,4(1H,3H)-dione.

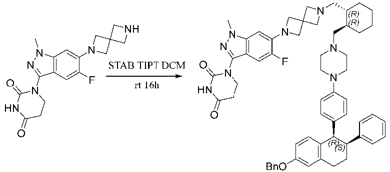

At room temperature, 1-(5-fluoro-1-methyl-6-(2,6-diazaspiro[3.3]heptane-2-yl)-1H-indazol-3-yl)dihydropyrimidine-2,4(1H,3H)-dione (0.20 g, 0.56 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.33 g, 0.56 mmol), DCM (20 mL), and TIPT (1 mL) were added to a single-necked flask in sequence. The reaction was carried out at room temperature for 16 h, and then sodium triacetoxyborohydride (0.18 g, 0.84 mmol) was added and the reaction was carried out for 1 h. Water (1 mL) was added. The mixture was stirred for 5 min, and filtered through celite. The filtrate was concentrated and subjected to column chromatography (a MeOH/DCM system) to afford 0.20 g of a white solid.

### Step 4: Synthesis of 1-(5-fluoro-6-(6-(((1R,2R)-2-((4-(4-(((3R,2S)-6-hydroxy-2-phenyl-1,2,3,4-tetrahydronaphthalen-1-yl)phenyl)piperazin-1-yl)methyl)cyclohexyl)methyl)-2,6-diazaspiro[3.3]heptane-2-yl)-1-methyl-1H-indazol-3-yl)dihydropyrimidine-2,4(1H,3H)-dione.

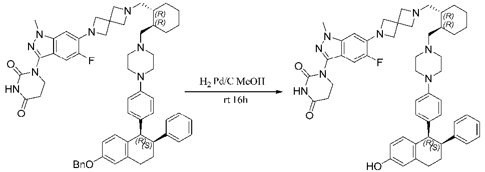

At room temperature, 1-(6-(6-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-2,6-diazaspiro[3.3]heptane-2-yl)-5-fluoro-1-methyl-1H-indazol-3-yl)dihydropyrimidine-2,4(1H,3H)-dione (0.20 g, 0.21 mmol), Pd/C (0.20 g, 10%), and MeOH (20 mL) were added to a single-necked flask in sequence. The reaction was carried out at room temperature for 16 h under a hydrogen balloon atmosphere. The mixture was filtered through celite, and the filtrate was concentrated and subjected to column chromatography (a MeOH/DCM system) to afford 0.08 g of a white solid. MS (ESI) m/z:851.8[M+H]⁺.

Example 36 Synthesis of 1-(5-fluoro-6-(7-(((1R,2R)-2-((4-(4-(((1R,2S)-6-hydroxy-2-phenyl-1,2,3,4-tetrahydronaphthalen-1-yl)phenyl)piperazin-1-yl)methyl)cyclohexyl)methyl)-2,7-diazaspiro[3.5nonan-2-yl)-1-methyl-1H-indazol-3-yl)dihydropyrimidine-2,4(1H,3H)-dione (Compound VI-27)

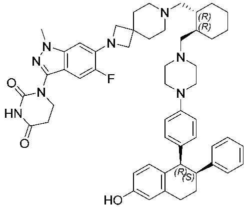

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 2-(3-(2,4-dioxotetrahydropyrimidin-1(2H)-yl)-5-fluoro-1-methyl-1H-indazol-6-yl)-2,7-diazaspiro[3.5]nonan-7-carboxylate.

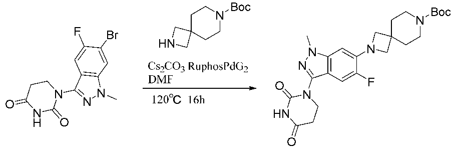

At room temperature, tert-butyl 1-(6-bromo-5-fluoro-1-methyl-1H-indazol-3-yl)dihydropyrimidine-2,4(1H,3H)-dione (1.00 g, 2.93 mmol), 2,7-diazaspiro[3.5]nonane-7-carboxylate (1.20 g, 5.86 mmol), cesium carbonate (1.91 g, 5.86 mmol), Ruphos (0.27 g, 0.58 mmol), RuphosPdG₂ (0.24 g, 0.29 mmol), and DMF (100 mL) were added to a single-necked flask in sequence. Under a nitrogen atmosphere, the reaction was carried out at 120°C for 16 h before termination. The reaction mixture was cooled to room temperature, diluted with water, and extracted three times with EA. The combined organic phases were washed with water and brine, and dried over anhydrous Na₂SO₄, filtered, concentrated, and purified by column chromatography (a MeOH/DCM system) to afford 0.80 g of a yellow solid.

### Step 2: Synthesis of 1-(5-fluoro-1-methyl-6-(2,7-diazaspiro[3.5]nonan-2-yl)-1H-indazol-3-yl)dihydropyrimidine-2,4(1H,3H)-dione.

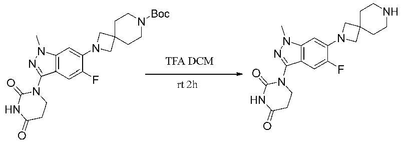

At room temperature, tert-butyl 2-(3-(2,4-dioxotetrahydropyrimidin-1(2H)-yl)-5-fluoro-1-methyl-1H-indazol-6-yl)-2,7-diazaspiro[3.5]nonan-7-carboxylate (0.80 g, 1.74 mmol), TFA (2 mL), and DCM (10 mL) were added to a single-necked flask in sequence. The reaction was carried out at room temperature for 3 h before termination. Concentration was performed to afford 0.70 g of a crude product as a yellow solid.

### Step 3: Synthesis of 1-(6-(7-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-2,7-diazaspiro[3.5]nonan-2-yl)-5-fluoro-1-methyl-1H-indazol-3-yl)dihydropyrimidine-2,4(1H,3H)-dione.

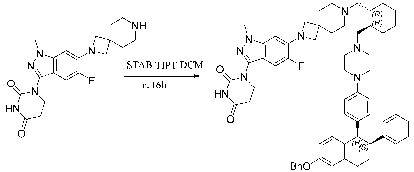

At room temperature, 1-(5-fluoro-1-methyl-6-(2,7-diazaspiro[3.5]nonan-2-yl)-1H-indazol-3-yl)dihydropyrimidine-2,4(1H,3H)-dione (0.20 g, 0.52 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.31 g, 0.52 mmol), DCM (20 mL), and TIPT (1 mL) were added to a single-necked flask in sequence. The reaction was carried out at room temperature for 16 h, and then sodium triacetoxyborohydride (0.16 g, 0.78 mmol) was added and the reaction was carried out for 1 h. Water (1 mL) was added. The mixture was stirred for 5 min, and filtered through celite. The filtrate was concentrated and subjected to column chromatography (a MeOH/DCM system) to afford 0.20 g of a white solid.

### Step 4: Synthesis of 1-(5-fluoro-6-(7-(((1R,2R)-2-((4-(4-(((3R,2S)-6-hydroxy-2-phenyl-1,2,3,4-tetrahydronaphthalen-1-yl)phenyl)piperazin-1-yl)methyl)cyclohexyl)methyl)-2,7-diazaspiro[3.5nonan-2-yl)-1-methyl-1H-indazol-3-yl)dihydropyrimidine-2,4(1H,3H)-dione.

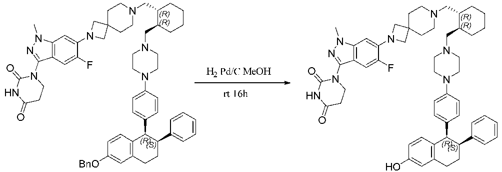

At room temperature, 1-(6-(7-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-2,7-diazaspiro[3.5]nonan-2-yl)-5-fluoro-1-methyl-1H-indazol-3-yl)dihydropyrimidine-2,4(1H,3H)-dione (0.20 g, 0.20 mmol), Pd/C (0.20 g, 10%), and MeOH (20 mL) were added to a single-necked flask in sequence. The reaction was carried out at room temperature for 16 h under a hydrogen balloon atmosphere. The mixture was filtered through celite, and the filtrate was concentrated and subjected to column chromatography (a MeOH/DCM system) to afford 0.06 g of a white solid. ¹H NMR (400 MHz, DMSO-d6) δ 10.52 (s, 1H), 9.15 (s, 1H), 7.28 (d, J = 12.8 Hz, 1H), 7.18 - 7.08 (m, 3H), 6.91 - 6.78 (m, 2H), 6.67 - 6.53 (m, 4H), 6.52 - 6.46 (m, 2H), 6.27 - 6.18 (m, 2H), 4.18 - 4.12 (m, 1H), 3.92 - 3.84 (m, 4H), 3.81 - 3.72 (m, 4H), 3.14 - 2.89 (m, 8H), 2.73 (t, J = 6.7 Hz, 3H), 2.29 - 2.20 (m, 1H), 2.15 - 1.91 (m, 5H), 1.78 - 1.59 (m, 6H), 1.36 (s, 3H), 1.30 - 1.16 (m, 6H), 1.07 - 0.93 (m, 1H), 0.89 - 0.80 (m, 2H). MS (ESI) m/z:879.8[M+H]⁺.

### Example 37 Synthesis of 1-(5-fluoro-6-(1-(((1R, 2R)-2-((4-(4-(((1R, 2S)-6-hydroxy-2-phenyl-1,2,3,4-tetrahydronaphthalen-1-yl)phenyl)piperazin-1-yl)methyl)cyclohexyl)methyl)-4-methoxypiperidin-4-yl)-1-methyl-1H-indazol-3-yl)dihydropyrimidine-2,4(1H,3H)-dione (Compound VI-28)

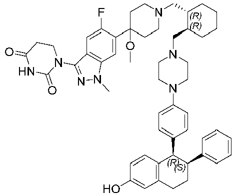

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Synthesis of tert-butyl 2-(3-(2,4-dioxotetrahydropyrimidin-1(2H)-yl)-5-fluoro-1-methyl-1H-indazol-6-yl)-2,7-diazaspiro[3.5]nonan-7-carboxylate.

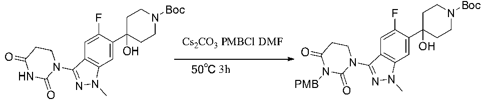

At room temperature, tert-butyl 4-(3-(2,4-dioxotetrahydropyrimidin-1(2H)-yl)-5-fluoro-1-methyl-1H-indazol-6-yl)-4-hydroxypiperidin-1-carboxylate (3.00 g, 6.50 mmol), cesium carbonate (2.12 g, 6.50 mmol), PMBCl (1.22 g, 7.80 mmol), and DMF (50 mL) were added to a single-necked flask in sequence. Under a nitrogen atmosphere, the reaction was carried out at 50°C for 3 h before termination. The reaction mixture was cooled to room temperature, diluted with water, and extracted three times with EA. The combined organic phases were washed with water and brine, and dried over anhydrous Na₂SO₄, filtered, concentrated, and purified by column chromatography (an EA/PE system) to afford 1.90 g of a yellow solid.

### Step 2: Synthesis of tert-butyl 4-(5-fluoro-3-(3-(4-methoxybenzyl)-2,4-dioxotetrahydropyrimidin-1(2H)-yl)-1-methyl-1H-indazol-6-yl)-4-methoxypiperidine-1-carboxylate.

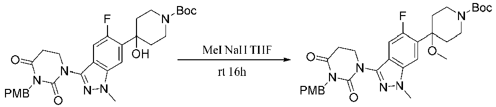

At room temperature, tert-butyl 2-(3-(2,4-dioxotetrahydropyrimidin-1(2H)-yl)-5-fluoro-1-methyl-1H-indazol-6-yl)-2,7-diazaspiro[3.5]nonan-7-carboxylate (1.90 g, 3.24 mmol), THF (20 mL), and NaH (0.26 g, 6.49 mmol) were added to a single-necked flask in sequence. After stirring for 10 min, iodomethane (0.92 g, 6.49 mmol) was added. The reaction was carried out at room temperature for 16 h before termination. The reaction mixture was diluted with saturated ammonium chloride aqueous solution, and extracted three times with EA. The combined organic phases were washed with water and brine, and dried over anhydrous Na₂SO₄, filtered, concentrated, and purified by column chromatography (an EA/PE system) to afford 0.80 g of a yellow solid.

### Step 3: Synthesis of 1-(5-fluoro-6-(4-methoxypiperidin-4-yl)-1-methyl-1H-indazol-3-yl)dihydropyrimidine-2,4(1H,3H)-dione.

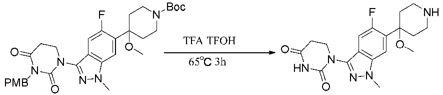

At room temperature, tert-butyl 4-(5-fluoro-3-(3-(4-methoxybenzyl)-2,4-dioxotetrahydropyrimidin-1(2H)-yl)-1-methyl-1H-indazol-6-yl)-4-methoxypiperidine-1-carboxylate (0.15 g, 0.25 mmol), TFA (4 mL), and TFOH (0.15 g, 1.00 mmol) were added to a single-necked flask in sequence. The reaction was carried out at 65°C for 3 h before termination. Concentration was performed to afford 0.10 g of a crude product as a yellow solid.

### Step 4: Synthesis of 1-(6-(1-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-4-methoxypiperidin-4-yl)-5-fluoro-1-methyl-1H-indazol-3-yl)dihydropyrimidine-2,4(1H,3H)-dione.

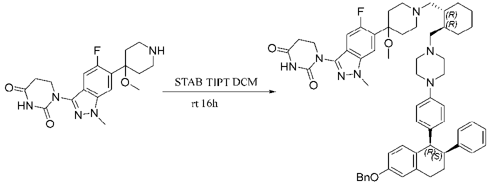

At room temperature, 1-(5-fluoro-6-(4-methoxypiperidin-4-yl)-1-methyl-1H-indazol-3-yl)dihydropyrimidine-2,4(1H,3H)-dione (0.10 g, 0.26 mmol), (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.16 g, 0.26 mmol), DCM (20 mL), and TIPT (1 mL) were added to a single-necked flask in sequence. The reaction was carried out at room temperature for 16 h, and then sodium triacetoxyborohydride (0.08 g, 0.39 mmol) was added and the reaction was carried out for 1 h. Water (1 mL) was added. The mixture was stirred for 5 min, and filtered through celite. The filtrate was concentrated and subjected to column chromatography (a MeOH/DCM system) to afford 0.10 g of a white solid.

### Step 5: Synthesis of 1-(5-fluoro-6-(1-(((1R,2R)-2-((4-(4-(((3R,2S)-6-hydroxy-2-phenyl-1,2,3,4-tetrahydronaphthalen-1-yl)phenyl)piperazin-1-yl)methyl)cyclohexyl)methyl)-4-methoxypiperidin-4-yl)-1-methyl-1H-indazol-3-yl)dihydropyrimidine-2,4(1H,3H)-dione.

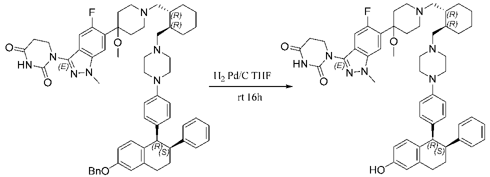

At room temperature, 1-(6-(1-(((1R,2R)-2-((4-(4-(((3R,2S)-6-(benzyloxy)-2-phenyl-1,2,3,4-tetrahydronaphthalen-1-yl)phenyl)piperazin-1-yl)methyl)cyclohexyl)methyl)-4-methoxypiperidin-4-yl)-5-fluoro-1-methyl-1H-indazol-3-yl)dihydropyrimidine-2,4(1H,3H)-dione (0.10 g, 0.10 mmol), Pd/C (0.20 g, 10%), and MeOH (20 mL) were added to a single-necked flask in sequence. The reaction was carried out at room temperature for 16 h under a hydrogen balloon atmosphere. The mixture was filtered through celite, and the filtrate was concentrated and subjected to column chromatography (a MeOH/DCM system) to afford 0.02 g of a white solid. MS (ESI) m/z:868.9[M+H]⁺.

### Example 38 Synthesis of 1-(5-fluoro-6-((2R)-4-hydroxy-1-(((1R, 2R)-2-((4-(4-((1R, 2S)-6-hydroxy-2-phenyl-1,2,3,4-tetrahydronaphthalen-1-yl)phenyl)piperazin-1-yl)methyl)cyclohexyl)methyl)-2-methylpiperidin-4-yl)-1-methyl-1H-indazol-3-yl)dihydropyrimidine-2,4(1H,3H)-dione (Compound VI-29)

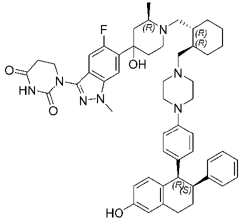

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: tert-butyl (2R)-4-(3-(2,4-dioxotetrahydropyrimidin-1(2H)-yl)-5-fluoro-1-methyl-1H-indazol-6-yl)-4-hydroxy-2-methylpiperidin-1-carboxylate

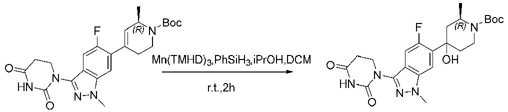

At room temperature, tert-butyl (R)-4-(3-(2,4-dioxotetrahydropyrimidin-1(2H)-yl)-5-fluoro-1-methyl-1H-indazol-6-yl)-6-methyl-3,6-dihydropyridine-1(2H)-carboxylate (0.60 g, 1.31 mmol) was dissolved in 30 mL of dichloromethane and 10 mL of isopropanol. Mn(TMHD)₃ (0.08 g, 0.13 mmol) was added at room temperature, and PhSiH₃ (0.21 g, 1.97 mmol) was added under an ice-water bath. After the addition, oxygen replacement was performed, and the reaction was carried out at room temperature for 16 h. The reaction completion was determined by LC-MS. The reaction solution was concentrated, sanded, and subjected to column chromatography (DCM/MeOH) to give a product. The product was then concentrated to afford 0.22 g of an off-white solid product.

### Step 2: 1-(5-fluoro-6-((2R)-4-hydroxy-2-methylpiperidin-4-yl)-1-methyl-1H-indazol-3-yl)dihydropyrimidine-2,4(1H,3H)-dione hydrochloride

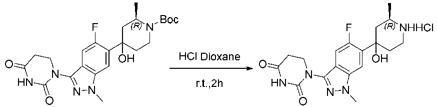

At room temperature, tert-butyl (2R)-4-(3-(2,4-dioxotetrahydropyrimidin-1 (2H)-yl)-5- fluoro-1-methyl-1H-indazol-6-yl)-4-hydroxy-2-methylpiperidin-1-carboxylate (0.22 g, 0.46 mmol) was dissolved in 5 mL of dichloromethane, and then 5 ml of hydrochloride in 1,4-dioxane was added. The reaction was carried out at room temperature for 2 h. The reaction completion was determined by LC-MS. The reaction solution was dried by rotary evaporation to afford 0.13 g of an off-white solid product.

### Step 3: 1-(6-((2R)-1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-4-hydroxy-2-methylpiperidin-4-yl)-5-fluoro-1-methyl-1H-indazol-3-yl)dihydropyrimidine-2,4(1H,3H)-dione

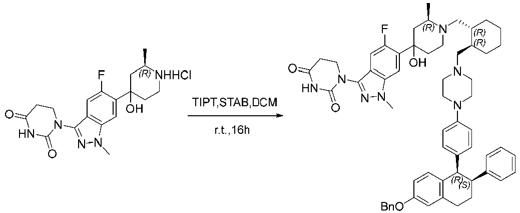

At room temperature, 1-(5-fluoro-6-(4-hydroxypiperidin-4-yl)-1-methyl-1H-indazol-3-yl)dihydropyrimidine-2,4(1H,3H)-dione hydrochloride (0.12 g, 0.32 mmol) and (1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexane-1-carboxaldehyde (0.19 g, 0.32 mmol) were placed in 20 mL of dichloromethane, and 1 mL of tetraisopropyl titanate was added. The reaction was carried out at 20 °C for 16 h. Sodium triacetoxyborohydride (0.10 g, 0.48 mmol) was added. The reaction was continued for 1 h. The reaction completion was determined by LC-MS. 1 mL of water was added to quench the reaction, and then 0.5 mL of ammonia was added. The mixture was stirred for 20 min, and filtered. The filtrate was concentrated, and purified by prep TLC (DCM/MeOH) to afford 0.05 g of a yellow oil.

### Step 4: 1-(5-fluoro-6-((2R)-4-hydroxy-1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)-2-methylpiperidin-4-yl)-1-methyl-1H-indazol-3-yl)dihydropyrimidine-2,4(1H,3H)-dione

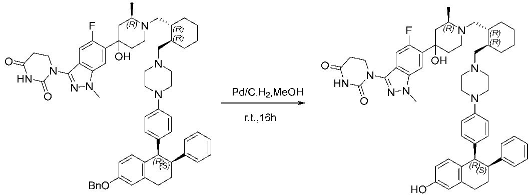

At room temperature, 1-(6-((2R)-1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-4-hydroxy-2-methylpiperidin-4-yl)-5-fluoro-1-methyl-1H-indazol-3-yl)dihydropyrimidine-2,4(1H,3H)-dione (0.05 g, 0.05 mmol) was placed in 10 mL of methanol, and 0.03 g of 5% Pd/C was added. Hydrogen replacement was performed and the reaction was carried out at 20 °C for 16 h. The reaction completion was determined by LC-MS. The reaction solution was filtered, and the filtrate was dried by rotary evaporation and subjected to silica gel chromatography (DCM/MeOH). After filtration, the filtrate was dried by rotary evaporation and lyophilized to afford 0.007 g of an off-white solid product. MS (ESI) m/z:435.4 [M/2+H]⁺.

### Example 39 Synthesis of 1-(5-fluoro-6-((2S)-4-hydroxy-1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)-2-methylpiperidin-4-yl)-1-methyl-1H-indazol-3-yl)dihydropyrimidine-2,4(1H,3H)-dione (Compound VI-30)

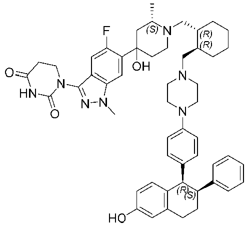

The synthesis method of (1R, 2R)-2-((4-(4-((1R, 2S)-6-(benzyloxy)-2-phenyl-1,2,3,4-tetrahydronaphthalen-1-yl)phenyl)piperazin-1-yl)methyl)cyclohexane-1-carboxaldehyde follows Example 1.

### Step 1: Tert-butyl (2S)-4-(3-(2,4-dioxotetrahydropyrimidin-1(2H)-yl)-5-fluoro-1-methyl-1H-indazol-6-yl)-4-hydroxy-2-methylpiperidin-1-carboxylate

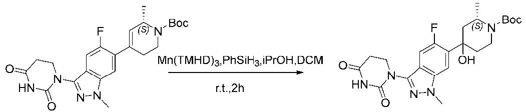

At room temperature, tert-butyl (S)-4-(3-(2,4-dioxotetrahydropyrimidin-1(2H)-yl)-5-fluoro-1-methyl-1H-indazol-6-yl)-6-methyl-3,6-dihydropyridine-1(2H)-carboxylate (0.60 g, 1.31 mmol) was dissolved in 30 mL of dichloromethane and 10 mL of isopropanol. Mn(TMHD)₃ (0.08 g, 0.13 mmol) was added at room temperature, and PhSiH₃ (0.21 g, 1.97 mmol) was added under an ice-water bath. After the addition, oxygen replacement was performed, and the reaction was carried out at room temperature for 16 h. The reaction completion was determined by LC-MS. The reaction solution was concentrated, sanded, and subjected to column chromatography (DCM/MeOH) to give a product. The product was then concentrated to afford 0.22 g of an off-white solid product.

### Step 2: 1-(5-fluoro-6-((2S)-4-hydroxy-2-methylpiperidin-4-yl)-1-methyl-1H-indazol-3-yl)dihydropyrimidine-2,4(1H,3H)-dione hydrochloride

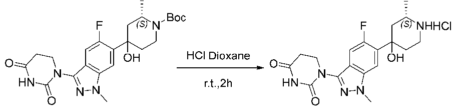

At room temperature, tert-butyl (2S)-4-(3-(2,4-dioxotetrahydropyrimidin-1(2H)-yl)-5-fluoro-1-methyl-1H-indazol-6-yl)-4-hydroxy-2-methylpiperidin-1-carboxylate (0.22 g, 0.46 mmol) was dissolved in 5 mL of dichloromethane, and then 5 ml of hydrochloride in 1,4-dioxane was added. The reaction was carried out at room temperature for 2 h. The reaction completion was determined by LC-MS. The reaction solution was dried by rotary evaporation to afford 0.13 g of an off-white solid product.

### Step 3: 1-(6-((2S)-1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-4-hydroxy-2-methylpiperidin-4-yl)-5-fluoro-1-methyl-1H-indazol-3-yl)dihydropyrimidine-2,4(1H,3H)-dione

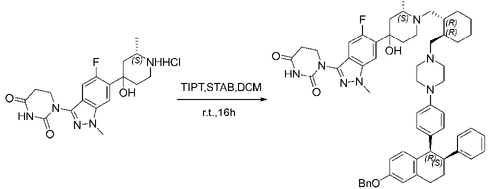

At room temperature, 1-(6-((2S)-1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-4-hydroxy-2-methylpiperidin-4-yl)-5-fluoro-1-methyl-1H-indazol-3-yl)dihydropyrimidine-2,4(1H,3H)-dione (0.13 g, 0.31 mmol) was placed in 20 mL of dichloromethane, and 1 mL of tetraisopropyl titanate was added. The reaction was carried out at 20 °C for 16 h. Sodium triacetoxyborohydride (0.10 g, 0.48 mmol) was added. The reaction was continued for 1 h. The reaction completion was determined by LC-MS. 1 mL of water was added to quench the reaction, and then 0.5 mL of ammonia was added. The mixture was stirred for 20 min, and filtered. The filtrate was concentrated, and purified by prep TLC (DCM/MeOH) to afford 0.03 g of a yellow oil.

### Step 4: 1-(5-fluoro-6-((2S)-4-hydroxy-1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)-2-methylpiperidin-4-yl)-1-methyl-1H-indazol-3-yl)dihydropyrimidine-2,4(1H,3H)-dione

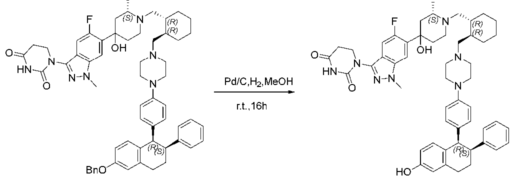

At room temperature, 1-(6-((2S)-1-(((1R,2R)-2-((4-(4-((1R,2S)-6-(benzyloxy)-2-phenyl-1,2,3,4-tetrahydronaphthalen-1-yl)phenyl)piperazin-1-yl)methyl)cyclohexyl)methyl)-4-hydroxy-2-methylpiperidin-4-yl)-5-fluoro-1-methyl-1H-indazol-3-yl)dihydropyrimidine-2,4(1H,3H)-dione (0.03 g, 0.03 mmol) was placed in 10 mL of methanol, and 0.01 g of 5% Pd/C was added. Hydrogen replacement was performed and the reaction was carried out at 20 °C for 16 h. The reaction completion was determined by LC-MS. The reaction solution was filtered, and the filtrate was dried by rotary evaporation and subjected to silica gel chromatography (DCM/MeOH). After filtration, the filtrate was dried by rotary evaporation and lyophilized to afford 0.002 g of an off-white solid product. MS (ESI) m/z:435.2 [M/2+H]⁺.

### Example 40 Synthesis of N-(4-(3-(2,4-dioxotetrahydropyrimidin-1(2H)-yl)-5-fluoro-1-methyl-1H-indazol-6-yl)-1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)acetamide (Compound VI-31)

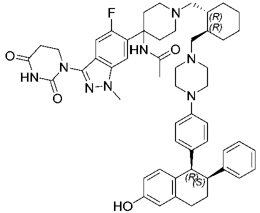

The synthesis method of 1-(5-fluoro-6-(4-hydroxy-1-(((1R, 2R)-2-((4-(4-((1R, 2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)dihydropyrimidine-2,4(1H, 3H)-dione follows Example 32.

### Step 1: Synthesis of N-(4-(3-(2,4-dioxotetrahydropyrimidin-1 (2H)-yl)-5-fluoro-1-methyl-1H-indazol-6-yl)-1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)acetamide.

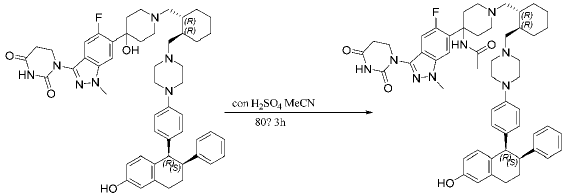

At room temperature, 1-(5-fluoro-6-(4-hydroxy-1-((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-1-methyl-1H-indazol-3-yl)dihydropyrimidine-2,4(1H,3H)-dione (0.20 g, 0.23 mmol), acetonitrile (20 mL), and concentrated sulfuric acid (0.23 g, 2.34 mmol) were added to a single-necked flask in sequence. The reaction was carried out at 80 °C for 3 h. The reaction mixture was cooled to room temperature, treated with ammonia, concentrated, and purified by column chromatography (a MeOH/DCM system) to afford 0.01 g of a white solid. MS (ESI) m/z:895.9[M+H]⁺.

### Example 41 Synthesis of 1-(6-(4-amino-1-(((1R,2R)-2-((4-(4-(((1R,2S)-6-hydroxy-2-phenyl-1,2,3,4-tetrahydronaphthalen-1-yl)phenyl)piperazin-1-yl)methyl)cyclohexyl)methyl)piperidin-4-yl)-5-fluoro-1-methyl-1H-indazol-3-yl)dihydropyrimidine-2,4(1H,3H)-dione (Compound VI-32)

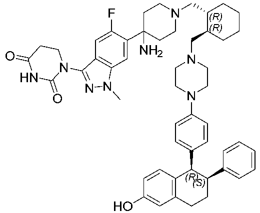

The synthesis method of N-(4-(3-(2,4-dioxotetrahydropyrimidin-1(2H)-yl)-5-fluoro-1-methyl-1H-indazol-6-yl)-1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)acetamide follows Example 40.

### Step 1: Synthesis of 1-(6-(4-amino-1-(((1R,2R)-2-((4-(4-(((3R,2S)-6-hydroxy-2-phenyl-1,2,3,4-tetrahydronaphthalen-1-yl)phenyl)piperazin-1-yl)methyl)cyclohexyl)methyl)piperidin-4-yl)-5-fluoro-1-methyl-1H-indazol-3-yl)dihydropyrimidine-2,4(1H,3H)-dione.

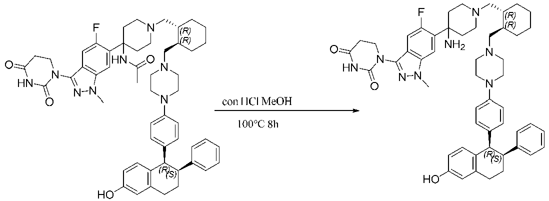

At room temperature, N-(4-(3-(2,4-dioxotetrahydropyrimidin-1(2H)-yl)-5-fluoro-1-methyl-1H-indazol-6-yl)-1-(((1R,2R)-2-((4-(4-((1R,2S)-6-hydroxy-2-phenyl-1,2,3,4-tetrahydronaphthalen-1-yl)phenyl)piperazin-1-yl)methyl)cyclohexyl)methyl)piperidin-4-yl)acetamide (0.10 g, 0.11 mmol), MeOH (10 mL) and concentrated hydrochloric acid (10 mL) were added to a single-necked flask in sequence. The reaction was carried out at 100 °C for 8 h. The reaction mixture was cooled to room temperature, treated with ammonia, concentrated, and purified by column chromatography (a MeOH/DCM system) to afford 0.005 g of a white solid. MS (ESI) m/z:854.0 [M+H]⁺.

Example 34 Synthesis of 1-(6-(4-(cyclopropyl(((1R, 2R)-2-((4-(4-(((1R, 2S)-6-hydroxy-2-phenyl-1,2,3,4-tetrahydronaphthalen-1-yl)phenyl)piperazin-1-yl)methyl)cyclohexyl)methyl)amino)cyclohexyl)-5-fluoro-1-methyl-1H-indazol-3-yl)dihydropyrimidine-2,4(1H,3H)-dione (Compound 1-34)

The following compounds were obtained using the preparation method described above (see Table 8):

**Table 8**

| No. | Structure | Characterization data | No. | Structure | Characterization data |
|---|---|---|---|---|---|
| VI-33 | 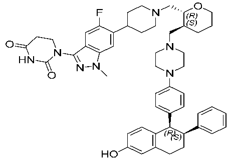 | MS (ESI) m/z: 840.9[M+H]⁺ | VI-34 | 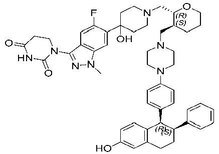 | MS (ESI) m/z: 856.7[M+H]⁺ |
| VI-35 | 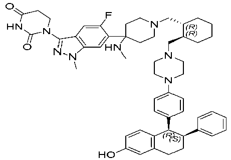 | MS (ESI) m/z: 867.4[M+H ]⁺ | VI-36 | 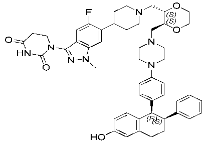 | MS (ESI) m/z: 842.8[M+H]⁺ |
| VI-37 | 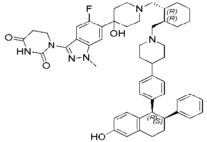 | MS (ESI) m/z: 853.7[M+H ]⁺ | VI-38 | 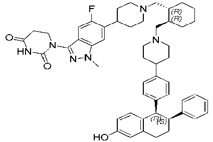 | MS (ESI) m/z: 837.7[M+H]⁺ |
| VI-39 | 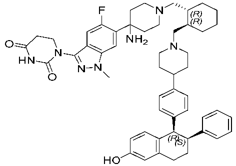 | MS (ESI) m/z: 852.8[M+H ]⁺ | VI-40 | 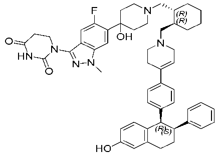 | MS (ESI) m/z: 851.5[M+H]⁺ |
| VI-41 | 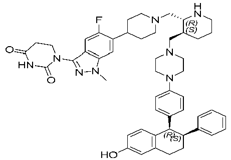 | MS (ESI) m/z: 839.9[M+H ]⁺ | VI-42 | 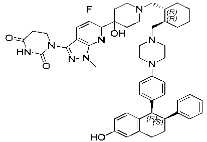 | MS (ESI) m/z: 855.7[M+H]⁺ |
| VI-43 | 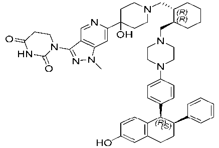 | MS (ESI) m/z: 837.4[M+H ]⁺ | VI-44 | 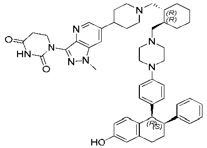 | MS (ESI) m/z: 821.8[M+H]⁺ |
| VI-45 | 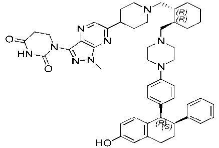 | MS (ESI) m/z: 822.6[M+H ]⁺ | | | |

### Experimental Example 1: MCF7 cell activity test

Cell culture: MCF7 cells (culture conditions: MEM medium + 20% FBS + 1% essential amino acids + +1mg/100ml insulin, 37°C, 5% CO₂). Plating: The cells subcultured into the logarithmic growth phase were collected and transferred into a centrifuge tube, and centrifuged at 800 rpm for 5 min. The cells were resuspended in complete medium and counted. The cells were diluted and adjusted to a final concentration of 30,000 cells/mL. The cells were then seeded into a 96-well plate with 100 µL per well and incubated in an incubator overnight. Administration: Each group of drug solutions was added into the 96-well plate, 100 µL per well, n=3 for each drug concentration. Meanwhile, negative wells (n=6) and blank wells (n=3) were set up. The 96-well plate was incubated in the incubator for further 6 days. CCK8 reagent was added to the 96-well plate, 20 µL per well, and then the plate was placed in the incubator for further 4 h. The absorbance of the solution was measured at a wavelength of 450 nm using an enzyme reader. The proliferation inhibition rate was calculated and the half inhibitory concentration IC₅₀ value was calculated using Graphpad Prism 6.0 software.

### T47D cell activity test

Cell culture: T47D cells (culture conditions: RPMI1640 medium + 10% FBS, 37°C, 5% CO₂). Plating: The cells subcultured into the logarithmic growth phase were collected and transferred into a centrifuge tube, and centrifuged at 800 rpm for 5 min. The cells were resuspended in complete medium and counted. The cells were diluted and adjusted to a final concentration of 30,000 cells/mL. The cells were then seeded into a 96-well plate with 100 µL per well and incubated in an incubator overnight. Administration: Each group of drug solutions was added into the 96-well plate, 100 µL per well, n=3 for each drug concentration. Meanwhile, negative wells (n=6) and blank wells (n=3) were set up. The 96-well plate was incubated in the incubator for further 6 days. CCK8 reagent was added to the 96-well plate, 20 µL per well, and then the plate was placed in the incubator for further 4 h. The absorbance of the solution was measured at a wavelength of 450 nm using an enzyme reader. The proliferation inhibition rate was calculated and the half inhibitory concentration IC₅₀ value was calculated using Graphpad Prism 6.0 software. The results are shown in Table 9.

**Table 9**

| Embodiment | Compound | MCF7 cell IC₅₀ (µM) |
|---|---|---|
| Example 1 | IV-5 | <0.025 |
| Example 2 | VII-1 | <0.050 |
| Example 3 | IV-9 | <0.025 |
| Example 4 | 1-69 | <0.025 |
| Example 5 | IV-3 | <0.025 |
| Example 6 | VII-2 | <0.100 |
| Example 7 | III-1 | <0.025 |
| Example 8 | VII-3 | <0.100 |
| Example 9 | VI-6 | <0.100 |
| Example 10 | I-67 | <0.100 |
| Example 11 | IV-1 | <0.050 |
| Example 12 | VI-5 | <0.050 |
| Example 13 | III-3 | <0.100 |
| Example 14 | VI-8 | <0.050 |
| Example 15 | VI-9 | <0.100 |
| Example 16 | IV-9 | <0.025 |
| Example 17 | III-2 | <0.100 |
| Example 18 | IV-4 | <0.100 |
| Example 19 | V-2 | <0.050 |
| Example 20 | V-5 | <0.100 |
| Example 21 | V-7 | <0.100 |
| Example 22 | V-3 | <0.050 |
| Example 23 | V-1 | <0.100 |
| Example 24 | IV-10 | <0.050 |
| Example 25 | VI-17 | <0.025 |
| Example 26 | VI-18 | <0.025 |
| Example 27 | IV-12 | <0.100 |
| Example 28 | VI-19 | <0.050 |
| Example 29 | VI-20 | <0.025 |
| Example 30 | VI-21 | <0.050 |
| Example 31 | VI-22 | <0.050 |
| Example 32 | VI-23 | <0.025 |
| Example 33 | VI-24 | <0.025 |
| Example 34 | VI-25 | <0.100 |
| Example 35 | VI-26 | <0.050 |
| Example 36 | VI-27 | <0.025 |
| Example 37 | VI-28 | <0.025 |
| Example 38 | VI-29 | <0.025 |
| Example 39 | VI-30 | <0.050 |

Conclusion: The compounds of the present invention have significant inhibitory activity against MCF7 cells or T47D cells.

### Experimental Example 2: ER protein degradation activity test

Cell culture: MCF7 cells (culture conditions: RPMI1640 medium + 10% carbon-adsorbed FBS, 37°C, 5% CO₂), and T47D cells (culture conditions: RPMI1640 medium + 10% FBS, 37°C, 5% CO₂). The cells subcultured into the logarithmic growth phase were collected and transferred into a centrifuge tube, and centrifuged at 800 rpm for 5 min. The cells were resuspended in complete medium and counted to adjust the cell number to 5 × 10⁵ cells/mL. The cells were then evenly plated into a six-well plate, 1 ml per well, and incubated overnight. Drug treatment: The diluted drug solution was added to the six-well plate, 1 ml per well. Meanwhile, two negative control wells were set up, in which 1 ml of empty culture medium containing an equal amount of DMSO was added. The six-well plate was incubated in the incubator for 48 h. Extraction of cell protein: A protein lysis buffer containing inhibitors was prepared in advance for later use, the culture medium was aspirated from the culture flask of adherent cells, and the adherent cells were washed twice with pre-cooled PBS. 100 µl of the pre-cooled protein lysis buffer containing inhibitors was added to the six-well plate and the plate was gently shaken for 5 min. The adherent cells in the well plate were scraped off with a cell scraper, and the cell suspension was transferred to a centrifuge tube and lysed under ice bath for 30 min, with shaking every 5/10 min. The lysate was centrifuged at 12000 rpm for 10 min in a pre-cooled 4° centrifuge. The pellet was discarded, and the supernatant was promptly transferred into a new centrifuge tube and stored for subsequent use. Detection of protein concentration: Following the protocol of the BCA kit, the OD value of the sample in each well was detected by a microplate reader, and the protein concentration in the sample was calculated based on the standard curve. The volume of the lysate was adjusted to equalize the protein concentration across all samples. Protein denaturation: A protein loading buffer (5×) was added to the sample protein supernatant at a ratio of 1:4 and mixed thoroughly. After mixing evenly, the mixture was heated at 100°C for 10 min and then stored in a refrigerator at -80°C. Sample loading and electrophoresis: The pre-prepared SDS-PAGE gel plate was mounted in the electrophoresis box, the electrophoresis buffer was added, and the comb was slowly and gently withdrawn before sample loading. The electrophoresis voltage was set to 150 V until the end. Wet transfer: After cessation of the electrophoresis, the region where the target protein molecular weight was located was determined based on the Maker and cut out with the comb. Meanwhile, a PVDF membrane of similar size was cut, which was soaked in methanol for 15 sec before use. A transfer buffer was poured into the tray, followed by sequential placement of sponge pads and filter papers in the prescribed order. The transfer current was set to 200 mA and the transfer time was set to 1 h. Blocking: A small hole was cut at the lower right corner of the PVDF membrane to record the front and back sides in contact with the gel, and the membrane was submerged in 5% BSA solution and incubated at low speed on a horizontal shaker at room temperature for 1 h. Antibody incubation: After blocking, the membrane was washed three times in TBST solution for 10 min each. The PVDF membrane was directly placed in a primary antibody incubation solution and a secondary antibody incubation solution in sequence for incubation. The incubation with the primary antibody incubation solution should be continued on the horizontal shaker at 4°C for more than 12 h, and the incubation with the secondary antibody incubation solution should be continued on the horizontal shaker at room temperature for 1 h. After each incubation, the membrane was washed three times in TBST solution for 10 min each. Development: The A/B solutions of a developer were mixed in a ratio of 1:1 and used immediately after preparation. The development was performed by a developing instrument according to the procedure.

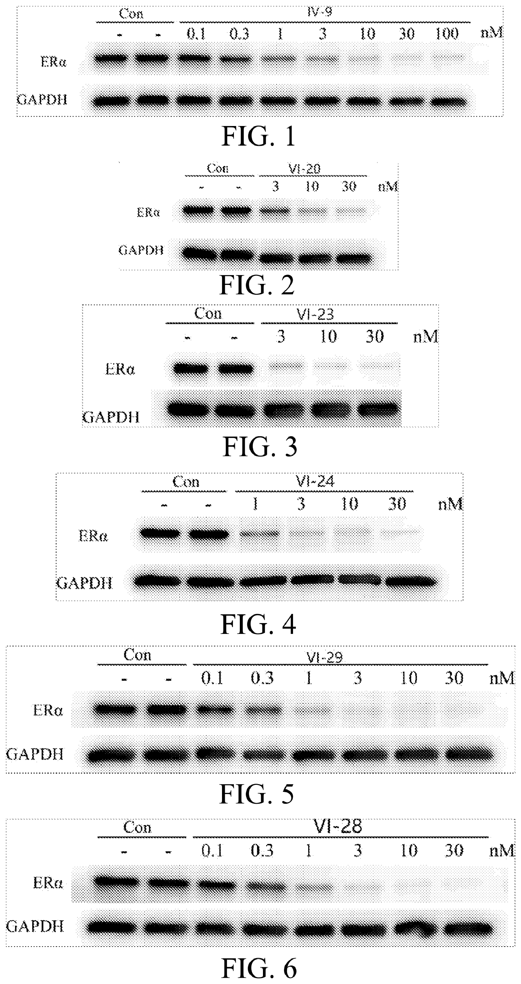

Conclusion: As seen in FIGs. 1-6, the compounds of the present invention can significantly degrade ER protein in tumor cells.

### Experimental Example 3: In vivo tumor inhibitory activity test

MCF-7 cell culture (culture conditions: MEM medium + 20% FBS + 1% essential amino acids + +1mg/100ml insulin, 37°C, 5% CO₂). The cells subcultured into the logarithmic growth phase were collected and transferred into a centrifuge tube, and centrifuged at 800 rpm for 5 min. On the day of inoculation, the cell density was adjusted to 3*10⁷ cells/mL.

Estradiol injection: One day before inoculation, all experimental animals were subcutaneously injected with 8µg/100µL estradiol solution (solvent: 30% propylene glycol + 70% glucose injection), and estradiol was subcutaneously injected at 4µg/50µL/animal every day from the day of inoculation to the end of the experiment.

Cell inoculation: The inoculation site was selected at the right shoulder blade of the experimental animal, and the inoculation density is 3*10⁶ cells/100µL/animal.

Grouped administration: When the average tumor volume reached 80-150 mm³, the animals were randomly grouped according to tumor volume, with the coefficient of variation (CV) of tumor volumes not exceeding 1/3 of the mean volume. The day of grouping was defined as D0, and the administration began on the day of grouping. The body weight of the mice was measured before administration, and the administration was based on the body weight on that day.

Experimental observation and data collection: After cell inoculation, the effect of the tumor on the normal behavior of the animals was routinely monitored every week. Specific contents include the activity, food and water intake, weight gain or loss, eyes, fur and other abnormalities of the experimental animals. After the start of administration, the mice were weighed and dosed daily, and tumor volume was measured twice a week. The tumor volume was calculated as follows: tumor volume (mm³) = 0.52 × tumor long diameter (mm) × tumor short diameter (mm)². At the end of the experiment, the following indicators were analyzed: tumor growth curve, mouse body weight curve, tumor weight, uniform photography of the excised tumors arranged according to groups, and calculation of tumor growth inhibition rate (TGI).

TGI_{TV} (Relative tumor volume inhibition rate) calculation formula: ${RTV}_{n} = \frac{{V}_{nt}}{{V}_{n 0}}$ $TGI = \left(1-\frac{mean {RTV}_{treat}}{mean {RTV}_{vehicle}}\right) \times 100 %$
*Vₙₜ:* Tumor volume of the mouse numbered n on day t;
*V*_{*n*0}*:* Tumor volume of the mouse numbered n on day 0;
*RTVₙ:* Relative tumor volume of the mouse numbered n on day t;
*mean RTVₜᵣₑₐₜ:* RTV mean of the administration group;
*mean RTV_{vehicle}:* RTV mean of the Vehicle group;

The experimental results such as tumor volume and mouse body weight of each group of animals are expressed as mean ± standard error of the mean (Mean ± SEM). All data were analyzed using GraphPad Prism 6.0. The results are shown in Table 10. *P* < 0.05 was considered to be significantly different.

**Table 10 Results of in vivo tumor inhibitory activity**

| Group | Administration dosage | TGI (Day 19) | P value |
|---|---|---|---|
| VI-23 | 5 mg/kg/d (I.V.) | 77.9% | *P* < 0.05 |

Conclusion: The compounds of the present invention have significant tumor growth inhibitory effects in vivo.

The embodiments of the technical solution of the present disclosure are described above by way of examples. It should be understood that the protection scope of the present disclosure is not limited to the above-mentioned embodiments. Any modifications, equivalent substitutions, and improvements, etc. made by those skilled in the art within the spirit and principles of this disclosure should be included in the protection scope of the claims of this application.

## Claims

1. A compound represented by formula (i), or an isomer or pharmaceutically acceptable salt thereof:
A-B-C (i)
wherein,
A is 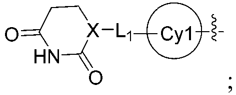
X is selected from N and CH;
Cy1 is selected from 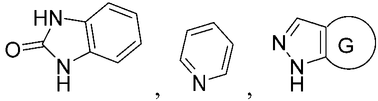 (for example 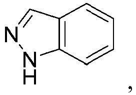 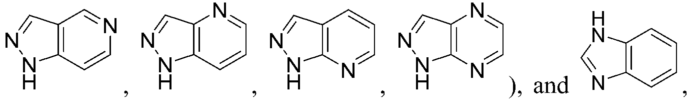 wherein the 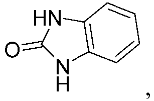 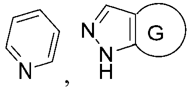 (for example 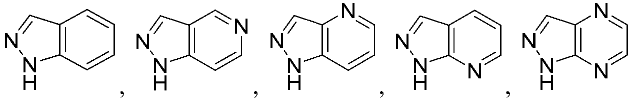 ), and 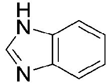
are unsubstituted, or each independently substituted with 1-3 Rₐ₁; G is selected from the following groups that are unsubstituted or optionally substituted with one, two or more Rₐ₁: C₆₋₁₀ aryl or a 5-10 membered heteroaryl group, such as phenyl, pyridyl, and pyrimidinyl;
each Rₐ₁ is independently selected from hydrogen, deuterium, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
B is -L2-Cy2-L3-Cy3-L4-Cy4-L5-;
L1, L2, L3, L4, L5, and L6 are independently selected from a bond, -(CH₂)ₙ-(NH)ₘ-, -O-, -S-, -NR₂, -CR₃₁R₃₂-, -C(O)-, -C(O)O-, -C(O)NR₄-, -NR₅-C(O)-, -S(O)-, -S(O)₂-, -P(O)(R₆)-, C₂₋₈ alkenyl and C₂₋₈ alkynyl;
n and m are independently any integer from zero to six;
R₂, R₃₁, R₃₂, R₄, R₅, and R₆ are independently selected from hydrogen, deuterium, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
Cy2 is selected from a 3-13 membered cycloalkyl group, a 3-13 membered heterocyclyl group, a 3-13 membered cycloalkenyl group, a 3-13 membered cycloalkyl group substituted with 1-3 Rₐ₂, a 3-13 membered heterocyclyl group substituted with 1-3 Rₐ₂, a 3-13 membered cycloalkenyl group substituted with 1-3 Rₐ₂, and Cy21-Cy22, wherein the heteroatom of the 3-13 membered heterocyclyl group is selected from O, N and S; Cy21 and Cy22 are the same or different, and are independently selected from a 3-13 membered cycloalkyl group, a 3-13 membered heterocyclyl group, and a 3-13 membered cycloalkenyl group;
Cy3 is selected from a 3-13 membered cycloalkyl group, a 3-13 membered heterocyclyl group, a 6-13 membered bicyclic condensed group, or a 8-21 membered polycyclic condensed group, wherein the 3-13 membered cycloalkyl group, 3-13 membered heterocyclyl group, 6-13 membered bicyclic condensed group, or 8-21 membered polycyclic condensed group are unsubstituted or are each independently substituted with 1-5 Rₐ₃, preferably a 5-6 membered heterocyclyl group, wherein the heteroatom of the 5-6 membered heterocyclyl group is N, O, S, or Si;
Cy4 is selected from a 3-8 membered cycloalkyl group, a 3-8 membered heterocyclyl group, a 3-8 membered cycloalkenyl group, a 6-13 membered bicyclic condensed cycloalkyl group, a 6-13 membered bicyclic condensed cycloalkenyl group, a 6-13 membered bicyclic condensed heterocyclyl group, a 3-8 membered cycloalkyl group substituted with 1-3 Rₐ₄, a 3-8 membered heterocyclyl group substituted with 1-3 Rₐ₄, a 3-8 membered cycloalkenyl group substituted with 1-3 Rₐ₄, a 6-13 bicyclic condensed cycloalkyl group substituted with 1-3 Rₐ₄, a 6-13 membered bicyclic condensed cycloalkenyl group substituted with 1-3 Rₐ₄, and a 6-13 membered bicyclic condensed heterocyclyl group substituted with 1-3 Rₐ₄, wherein the heteroatom of the 3-8 membered heterocyclyl group or the 6-13 membered bicyclic condensed heterocyclyl group is selected from O, N and S;
each Rₐ₂, Rₐ₃ and Rₐ₄ at each occurrence are independently selected from hydrogen, deuterium, halogen, hydroxy, thiol, silanol, selenol, carboxyl, amino, cyano, nitro, C₁₋₆ alkyl, halogenated C₁₋₆ alkyl, C₁₋₆ alkoxy, halogenated C₁₋₆ alkoxy, C₁₋₆ alkyl-C(O)O-, C₁₋₆ alkylthio, C₁₋₆ alkylseleno, hydroxy C₁₋₆ alkyl, C₁₋₆ alkyl-C(O)NH-, C₁₋₆ alkylamino, (C₁₋₆ alkyl)₂amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, a 3-8 membered cycloalkyl group, a 3-8 membered heterocyclyl group, and a 3-8 membered cycloalkenyl group, wherein the C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, (C₁₋₆ alkyl)₂ aminocarbonyl, 3-8 membered cycloalkyl group, 3-8 membered heterocyclyl group, and 3-8 membered cycloalkenyl group are unsubstituted or optionally substituted with one or more groups selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, halogenated C₁₋₆ alkoxy, a 3-8 membered cycloalkyl group, a 3-8 membered heterocyclyl group, and a 3-8 membered cycloalkenyl group;
C is 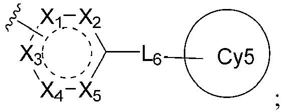
X₁ is N or CR_{b1};
X₂ is N or CR_{b2};
X₃ is N or CR_{b3};
X₄ is N or CR_{b4};
X₅ is N or CR_{b5};
R_{b1}, R_{b2}, R_{b3}, R_{b4} and R_{b5} are independently selected from hydrogen, deuterium, halogen, hydroxy, carboxyl, amino, cyano, nitro, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₂₋₈ alkenyl, C₂₋₈ alkynyl, C₁₋₆ alkylcarbonyl, aminocarbonyl, C₁₋₆ alkylaminocarbonyl, and (C₁₋₆ alkyl)₂ aminocarbonyl;
Cy5 is selected from a 6-13 membered bicyclic condensed group and an 8-21 membered polycyclic condensed group; wherein the 6-13 membered bicyclic condensed group and the 8-21 membered polycyclic condensed group are unsubstituted or substituted with 1-3 Rₐ₅; and
each Rₐ₅ is independently selected from hydrogen, deuterium, hydroxy, amino, carboxyl, cyano, nitro, halogen, C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, a 4-6 membered heterocyclyl group, a 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, a 4-6 membered heterocyclylcarbonyl group and a 5-6 membered heteroaryl-oxy group, wherein the C₁₋₆ alkyl, C₁₋₆ alkoxy, C₁₋₆ alkylamino, (C₁₋₆ alkyl)₂ amino, halogenated C₁₋₆ alkyl, halogenated C₁₋₆ alkoxy, C₂₋₈ alkenyl, C₂₋₈ alkynyl, C₁₋₆ alkylsulfonyl, C₁₋₆ alkylthio, C₃₋₆ cycloalkyl, 4-6 membered heterocyclyl group, 5-6 membered heteroaryl group, aryl, C₁₋₆ alkylcarbonyl, aminocarbonyl, C₁₋₆ alkylaminocarbonyl, (C₁₋₆ alkyl)₂ aminocarbonyl, 4-6 membered heterocyclylcarbonyl group and 5-6 membered heteroaryl-oxy group are unsubstituted or optionally substituted with one or more groups independently selected from hydroxy, amino, carboxyl, cyano, nitro, halogen, C₁₋₆ alkyl, C₁₋₆ alkoxy, C₁₋₆ alkoxy C₁₋₆ alkoxy, C₁₋₆ alkylamino, (C₁₋₆ alkyl)₂ amino, C₁₋₆ alkylcarbonylamino, C₁₋₆ alkylsulfonylamino, C₁₋₆ alkylcarbonyloxy, C₃₋₆ cycloalkyl, C₃₋₆ cycloalkylcarbonyloxy, C₂₋₈ alkynyl, halogenated C₁₋₆ alkyl, C₂₋₈ alkenyl, and halogenated C₁₋₆ alkoxy;
provided that when Cy3 is 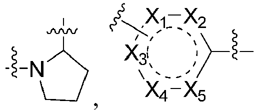
is not

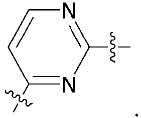

2. The compound represented by formula (i) according to claim 1, or the isomer or pharmaceutically acceptable salt thereof, wherein Cy1 is selected from 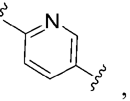 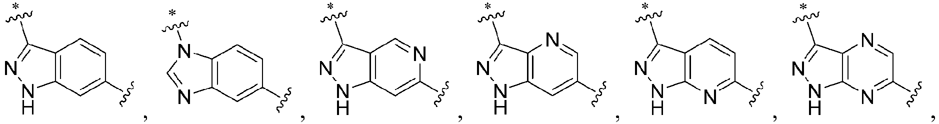 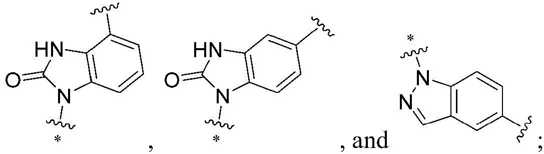 wherein the * end is connected to L1;
preferably, the 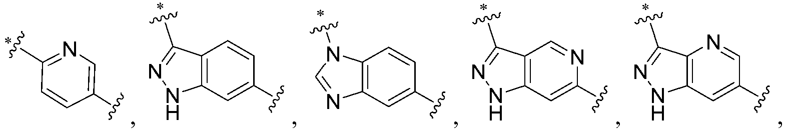 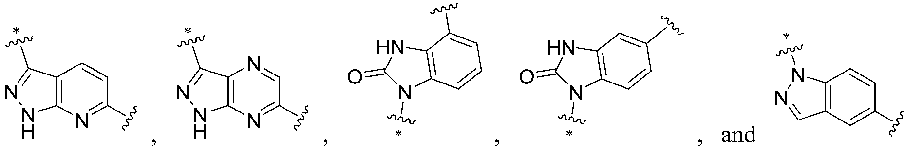 are unsubstituted or each independently substituted with 1-3 Rₐ₁; more preferably, each Rₐ₁ is independently selected from hydrogen, halogen, hydroxyl, carboxyl, amino, C₁₋₆ alkyl, halogenated C₁₋₆ alkyl, C₁₋₆ alkoxy, halogenated C₁₋₆ alkoxy, hydroxy C₁₋₆ alkyl, C₁₋₆ alkylamino, (C₁₋₆ alkyl)₂ amino, C₁₋₆ alkylcarbonyl, aminocarbonyl, C₁₋₆ alkylaminocarbonyl, and (C₁₋₆ alkyl)₂ aminocarbonyl; more preferably, each Rₐ₁ is independently substituted with 1-3 halogen atoms (such as F) or C₁₋₆ alkyl groups (such as methyl);
preferably, Cy1 is selected from 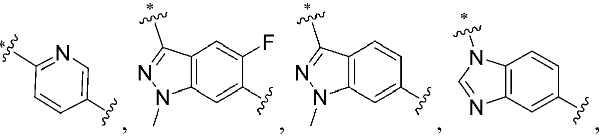 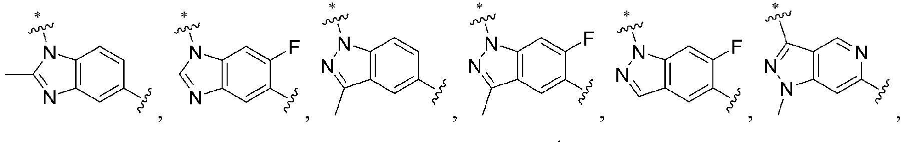 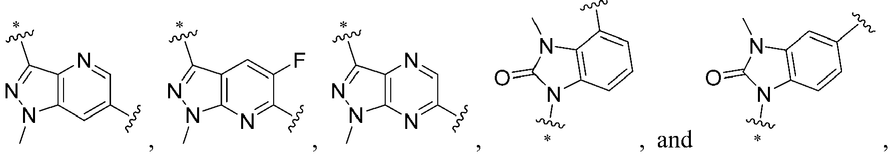 wherein the * end is connected to L1.

3. The compound represented by formula (i) according to claim 1 or 2, or the isomer or pharmaceutically acceptable salt thereof, wherein L1, L2, L3, L4, L5, and L6 are the same or different and are independently selected from a bond, O, NH, C₁₋₆ alkylene, -NHC(O)-, NH(C₁₋₆ alkylene), and -C(O)-;
preferably, L1 is selected from a bond and -NHC(O)-; preferably, when L1 is -NHC(O)-, the C(O) end is connected to Cy1;
preferably, L2 is selected from a bond, NH, and NH(C₁₋₃ alkylene);
preferably, L2 is selected from a bond, NH, and NHCH₂; preferably a bond;
preferably, L3 is selected from C₁₋₃ alkylene, -C(O)-, -CH₂NR₄-, -NR₅-CH₂-, and NH(C₁₋₃ alkylene); wherein R₄ and R₅ are independently selected from C₃₋₆ cycloalkyl;
preferably, L3 is selected from methylene, -C(O)-, and NHCH₂; preferably methylene;
preferably, L4 is selected from a bond, C₁₋₃ alkylene, and -C(O)-;
preferably, L4 is selected from a bond, methylene, and -C(O)-; preferably methylene;
preferably, L5 is selected from a bond and C₁₋₃ alkylene;
preferably, L5 is selected from a bond and methylene; preferably a bond;
preferably, L6 is selected from a bond and -O-; preferably a bond.

4. The compound represented by formula (i) according to any one of claims 1-3, or the isomer or pharmaceutically acceptable salt thereof, wherein Cy2 is selected from a 4-10 membered cycloalkyl group, a 4-10 membered heterocyclyl group, a 4-10 membered cycloalkenyl group, a 4-10 membered cycloalkyl group substituted with 1-3 Rₐ₂, a 4-10 membered heterocyclyl group substituted with 1-3 Rₐ₂, a 4-10 membered cycloalkenyl group substituted with 1-3 Rₐ₂, and Cy21-Cy22, wherein the heteroatom of the 4-10 membered heterocyclyl group is selected from O, N and S; Cy21 and Cy22 are the same or different, and are independently selected from a 4-6 membered cycloalkyl group, a 4-6 membered heterocyclyl group, and a 4-6 membered cycloalkenyl group;
preferably, Cy2 is selected from a 4-6 membered cycloalkyl group, a 4-6 membered heterocyclyl group, a 4-6 membered cycloalkenyl group, a 4-6 membered cycloalkyl group substituted with 1-3 Rₐ₂, a 4-6 membered heterocyclyl group substituted with 1-3 Rₐ₂, and a 4-6 membered cycloalkenyl group substituted with 1-3 Rₐ₂, wherein the heteroatom of the 4-6 membered heterocyclyl group is selected from O, N and S;
preferably, Cy2 is selected from a 6-9 membered heterocyclyl group that is unsubstituted or substituted with 1-3 Rₐ₂, and Cy21-Cy22; wherein Cy21 and Cy22 are the same or different, and are independently selected from cyclohexyl, piperazinyl, and piperidinyl;
preferably, Cy2 is selected from piperazinyl, piperidinyl, 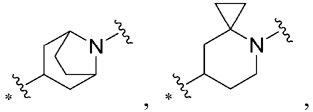 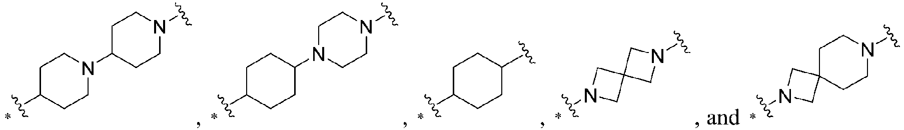 that are unsubstituted or substituted with 1-3 Rₐ₂;
preferably, Cy2 is selected from 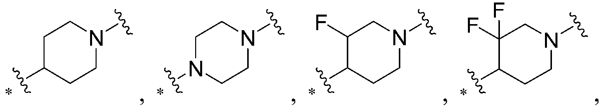 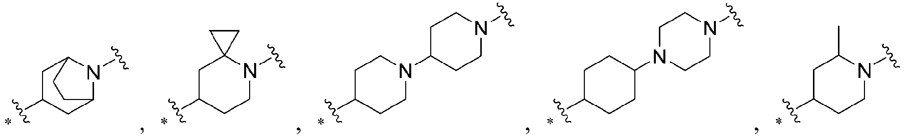 (for example, 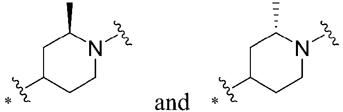 ), 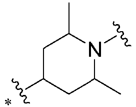 (for example, 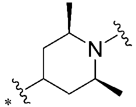 ), 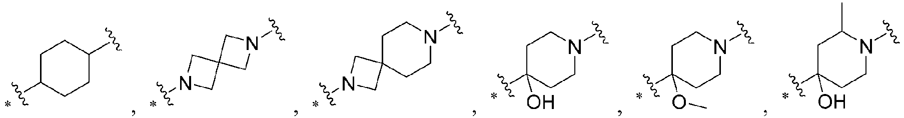 (for example, 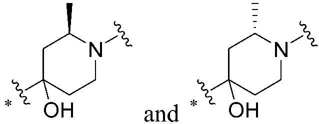 ), 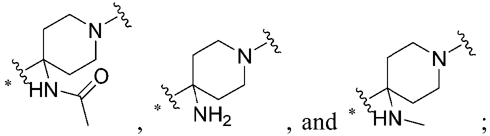 wherein the * end is connected to Cy1;
preferably, Cy3 is selected from a 5-10 membered cycloalkyl group, a 5-10 membered heterocyclyl group, a 6-13 membered bicyclic condensed cycloalkyl group, a 6-13 membered bicyclic condensed cycloalkenyl group, a 6-13 membered bicyclic condensed heterocyclyl group, an 8-13 membered bicyclic condensed heteroaryl group, an 8-13 membered bicyclic condensed aryl group, an 8-21 membered polycyclic condensed cycloalkyl group, an 8-21 membered polycyclic condensed cycloalkenyl group, an 8-21 membered polycyclic condensed heterocyclyl group, an 8-21 membered polycyclic condensed heteroaryl group, and an 8-21 membered polycyclic condensed aryl group;
preferably, Cy3 is selected from a 5-6 membered cycloalkyl group and a 5-6 membered heterocyclyl group; wherein the 5-6 membered cycloalkyl group and 5-6 membered heterocyclyl group are unsubstituted or each independently substituted with 1-3 Rₐ₃;
preferably, Cy3 is selected from cyclohexyl, piperidinyl, tetrahydropyranyl, dioxanyl, and pyrrolidinyl; wherein the cyclohexyl, piperidinyl, tetrahydropyranyl, dioxanyl, and pyrrolidinyl are unsubstituted or each independently substituted with 1-3 Rₐ₃; preferably cyclohexyl;
preferably, Cy3 is selected from 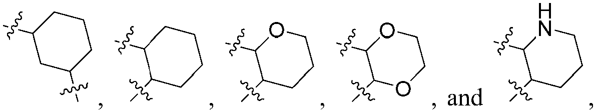 wherein the 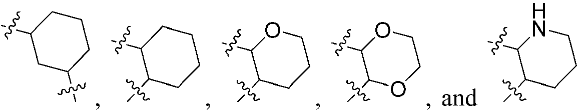
are unsubstituted or each independently substituted with 1-3 Rₐ₃;
preferably, Cy4 is selected from a 5-6 membered heterocyclyl group that is unsubstituted or optionally substituted with one, two or more groups selected from C₁₋₆ alkyl and C₁₋₆ alkoxy;
preferably, Cy4 is selected from piperazinyl, for example, 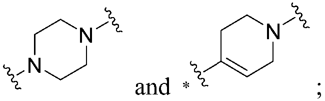 wherein the * end is connected to L5;
preferably, Cy5 is selected from the following groups that are unsubstituted or substituted with 1-3 Rₐ₅: a 6-13 membered bicyclic fused cycloalkenyl group, a 6-13 membered bicyclic fused heteroaryl group, and an 8-21 membered polycyclic fused heterocyclyl group;
preferably, Cy5 is selected from 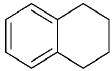
that is unsubstituted or substituted with 1-3 OH or phenyl groups, for example, 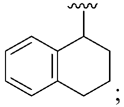
preferably, Cy5 is 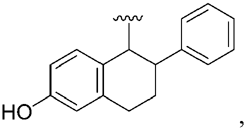
for example

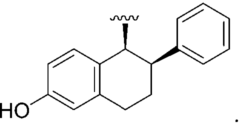

5. The compound represented by formula (i) according to any one of claims 1-4, or the isomer or pharmaceutically acceptable salt thereof, wherein Rₐ₁ is selected from halogen, C₁₋₆ alkyl, and C₁₋₆alkoxy;
preferably, Rₐ₁ is selected from F, methyl, ethyl, and methoxy;
preferably, Rₐ₂, Rₐ₃ and Rₐ₄ are the same or different, and are each independently selected from H, OH, amino, thiol, silanol, selenol, halogen, C₁₋₆ alkyl, C₁₋₆ alkoxy, C₁₋₆ alkyl-NH-, C₃₋₆ cycloalkyl, C₁₋₆ alkyl-C(O)NH-, halogenated C₁₋₆ alkyl, halogenated C₁₋₆ alkoxy, and hydroxy C₁₋₆ alkyl;
preferably, Rₐ₂ is selected from H, OH, amino, halogen, C₁₋₆ alkyl, C₁₋₆ alkoxy, C₁₋₆ alkyl-NH-, C₃₋₆ cycloalkyl, and C₁₋₆ alkyl-C(O)NH-;
preferably, Rₐ₂ is selected from H, F, OH, amino, methylamino, acetamido, methyl, methoxy, and cyclopropyl;
preferably, Rₐ₃ and Rₐ₄ are the same or different and are independently selected from H and halogen;
preferably, Rₐ₃ and Rₐ₄ are the same or different and are independently selected from H and F; preferably, Rₐ₅ is selected from hydroxy and C₆₋₁₀ aryl;
preferably, Rₐ₅ is selected from hydroxy and phenyl;
preferably, Rₐ₅ is selected from hydroxy, C₆₋₁₀ aryl, and C₆₋₁₀ aryl substituted with halogen;
preferably, 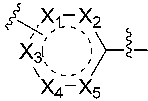 is selected from the following groups that are unsubstituted or optionally substituted with 1 to 4 substituents selected from R_{b1}, R_{b2}, R_{b3}, R_{b4}, and R_{b5}: phenyl and a 6-membered heteroaryl group containing 1 or 2 heteroatoms, for example,

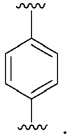

6. The compound represented by formula (i) according to any one of claims 1-5, or the isomer or pharmaceutically acceptable salt thereof, having the structure shown below: 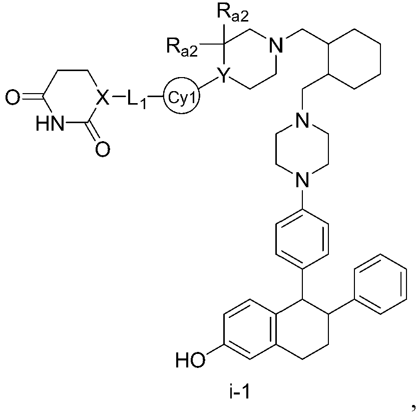 wherein Y is selected from N and CRₐ₂; X, L₁, Cy1, and Rₐ₂ each have the definition described in any one of claims 1-5.

7. The compound represented by formula (i-1) according to claim 6, or the isomer or pharmaceutically acceptable salt thereof, having the structure shown below: 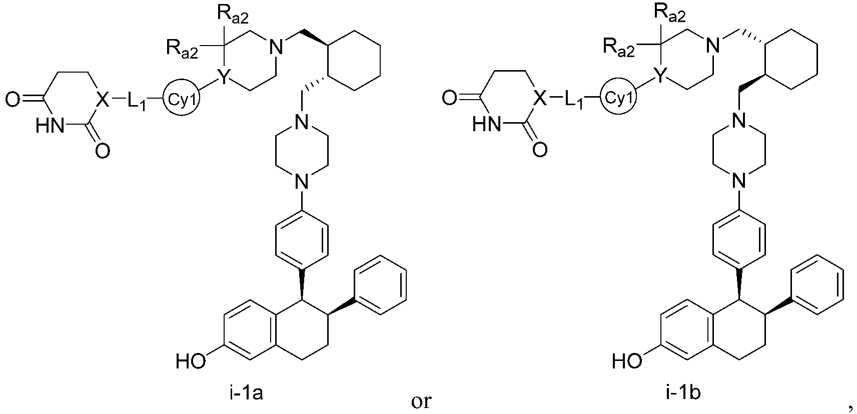 wherein Y is selected from N and CRₐ₂; X, L₁, Cy1, and Rₐ₂ each have the definition described in any one of claims 1-5.

8. The compound represented by formula (i) according to any one of claims 1-5, or the isomer or pharmaceutically acceptable salt thereof, having the structure shown below: 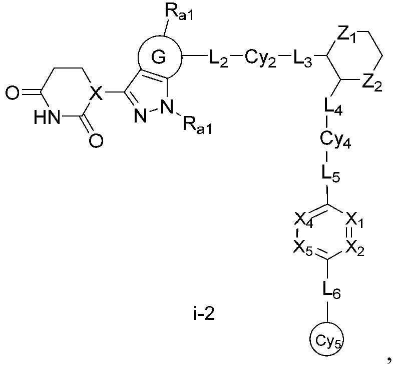 wherein Z₁ and Z₂ are the same or different and are independently selected from C(Rₐ₃)(Rₐ₃), O, N(Rₐ₃), S, and Si(Rₐ₃)(Rₐ₃); wherein X, G, L2, L₃, L4, L₅, L₆, Cy2, Cy4, Cy5, Rₐ₁, Rₐ₃, X₁, X₂, X₄, and X₅ each have the definition described in any one of claims 1-5.

9. The compound represented by formula (i-2) according to claim 8, or the isomer or pharmaceutically acceptable salt thereof, having the structure shown below: 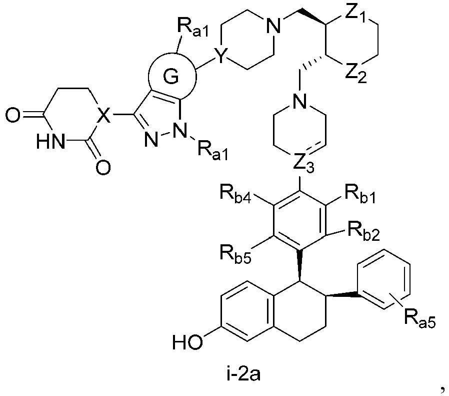 wherein Z₃ is selected from N, C, and CRₐ₄; 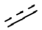 indicates a single or double bond; G, Y, Zi, Z₂, R_{b1}, R_{b2}, R_{b4}, R_{b5}, Rₐ₁, and Rₐ₅ each have the definition described in any one of claims 1-5.

10. The compound according to any one of claims 1-9, or the isomer or pharmaceutically acceptable salt thereof, selected from the compounds shown below:
| Serial No. | Structure | Serial No. | Structure |
|---|---|---|---|
| III-1 | 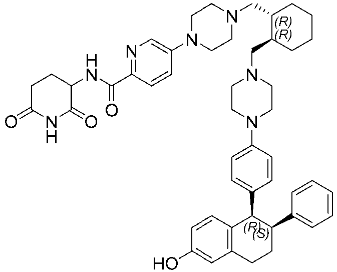 | III-2 | 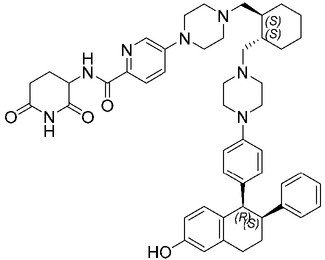 |
| III-3 | 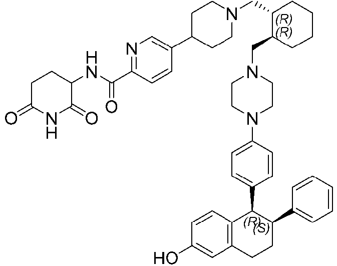 | III-4 | 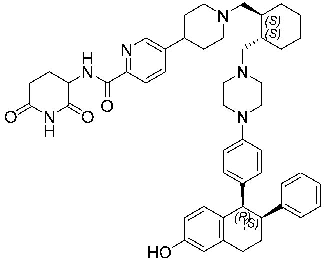 |
| Serial No. | Structure | Serial No. | Structure |
|---|---|---|---|
| IV-1 | 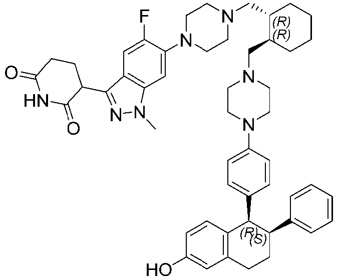 | IV-2 | 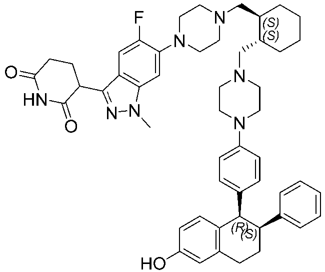 |
| IV-3 | 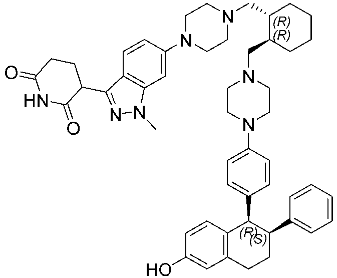 | IV-4 | 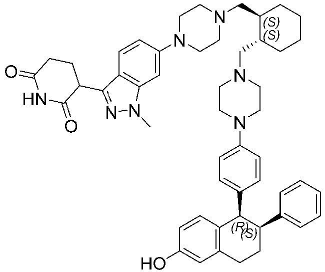 |
| IV-5 | 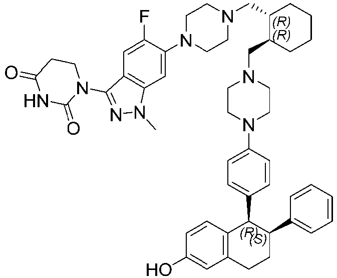 | IV-6 | 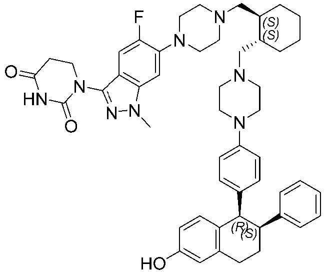 |
| IV-7 | 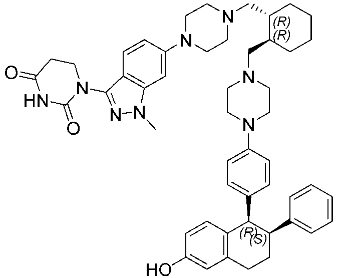 | IV-8 | 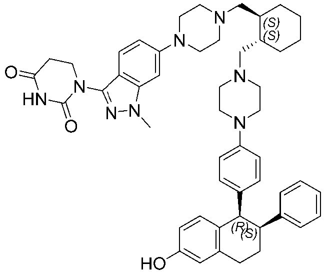 |
| IV-9 | 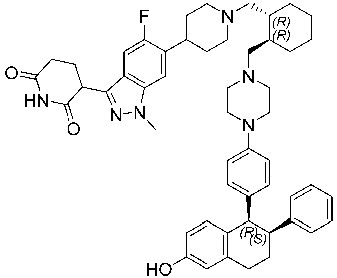 | IV-10 | 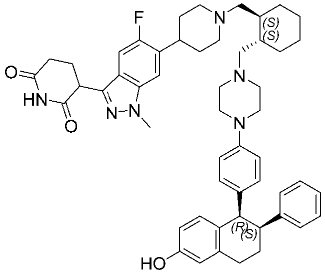 |
| IV-11 | 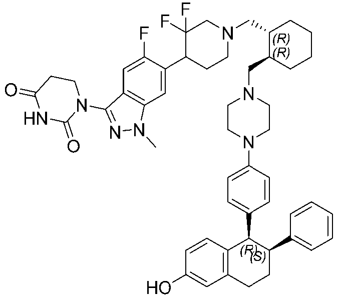 | IV-12 | 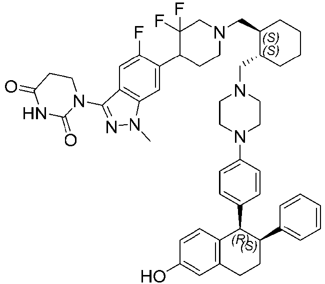 |
| IV-13 | 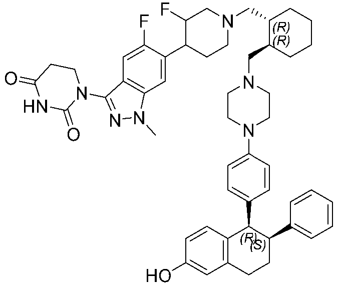 | IV-14 | 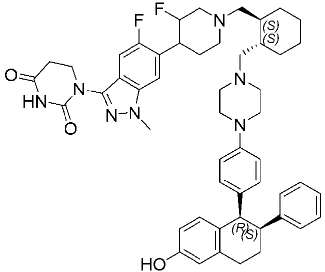 |
| IV-15 | 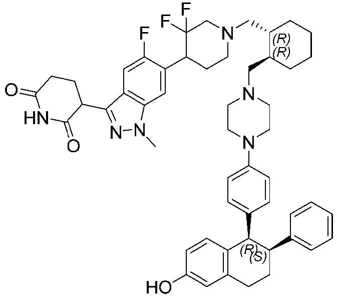 | IV-16 | 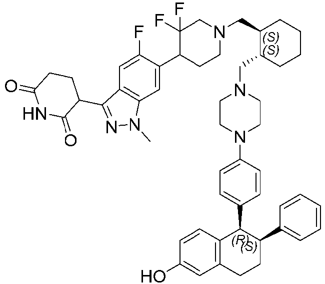 |
| IV-17 | 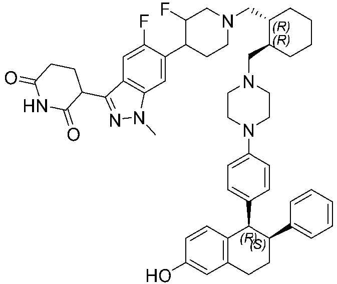 | IV-18 | 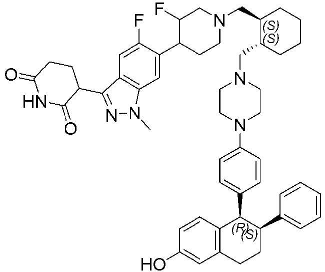 |
| V-1 | 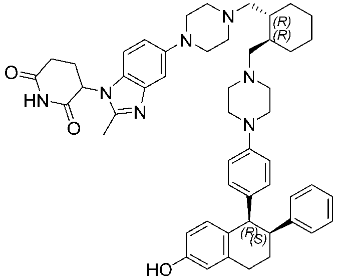 | V-2 | 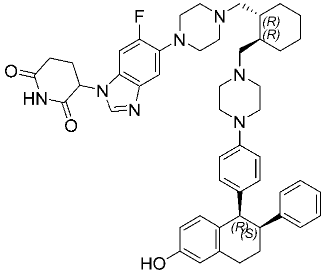 |
| V-3 | 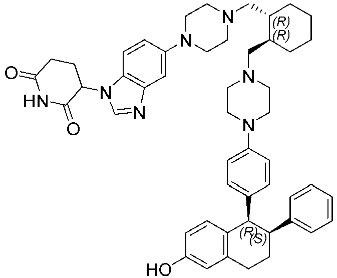 | V-4 | 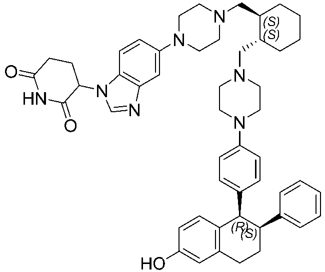 |
| V-5 | 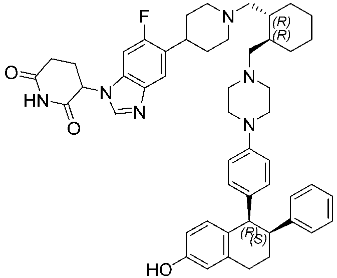 | V-6 | 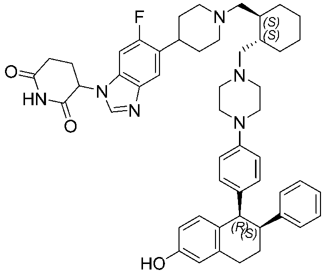 |
| V-7 | 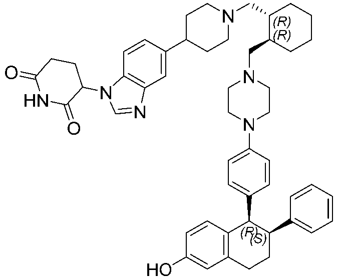 | V-8 | 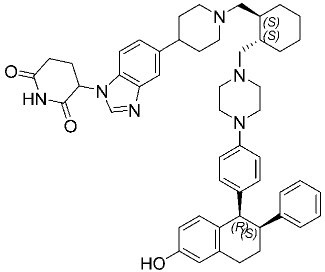 |
| V-9 | 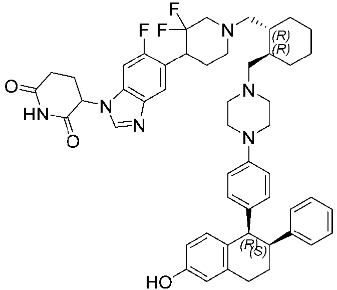 | V-10 | 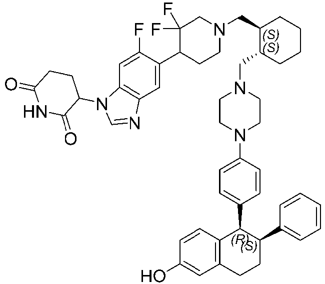 |
| V-11 | 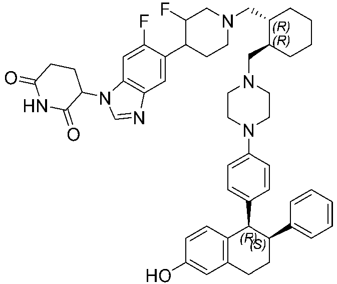 | V-12 | 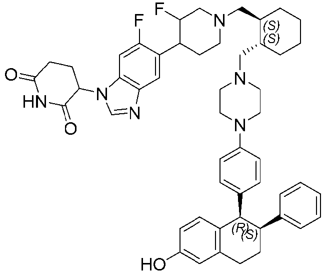 |
| Seria 1 No. | Structure | Serial No. | Structure |
|---|---|---|---|
| VI-1 | 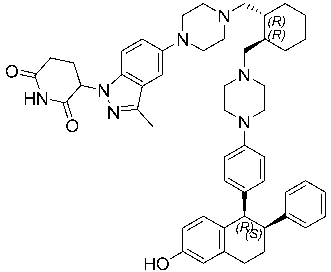 | VI-2 | 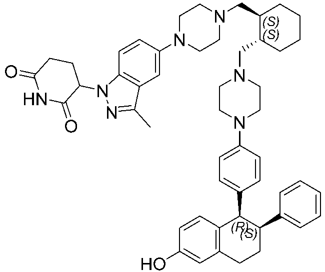 |
| VI-3 | 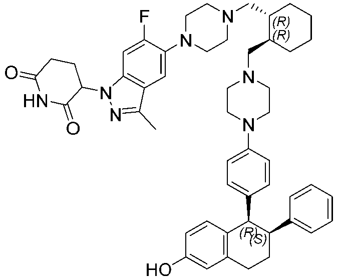 | VI-4 | 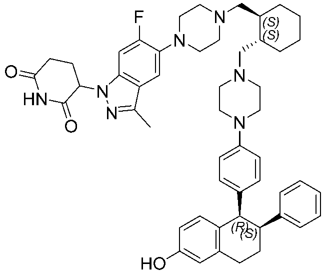 |
| VI-5 | 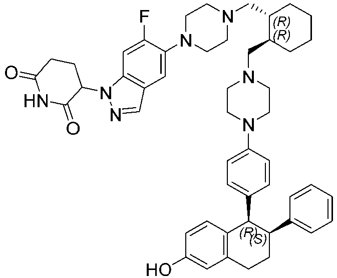 | VI-6 | 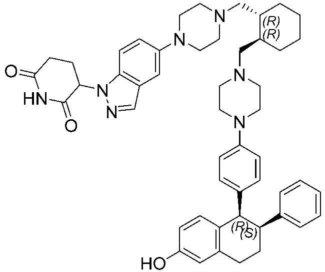 |
| VI-7 | 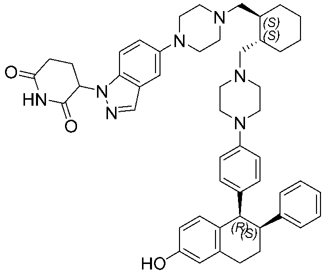 | VI-8 | 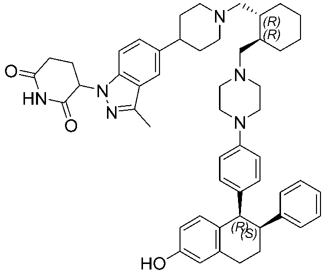 |
| VI-9 | 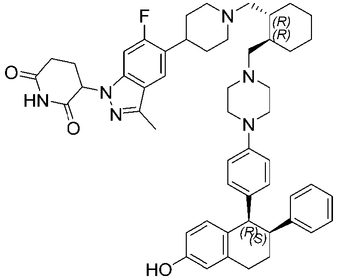 | VI-10 | 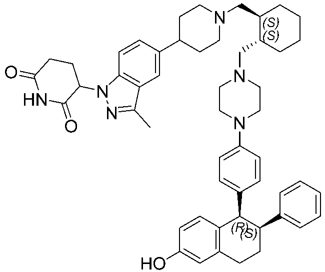 |
| VI-11 | 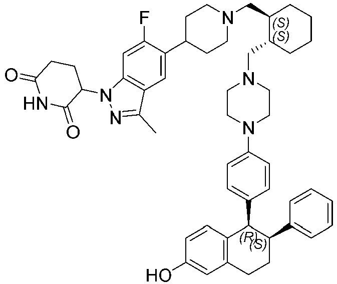 | VI-12 | 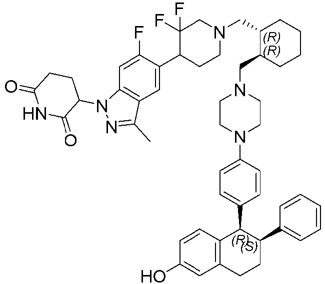 |
| VI-13 | 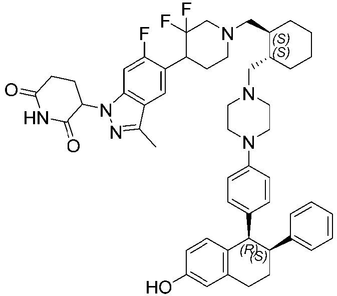 | VI-14 | 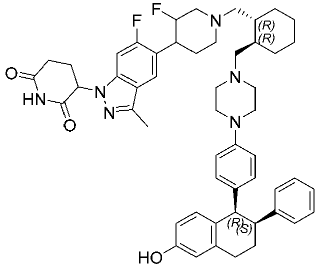 |
| VI-15 | 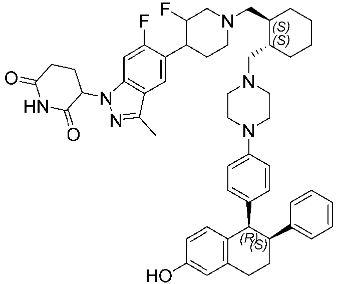 | VI-16 | 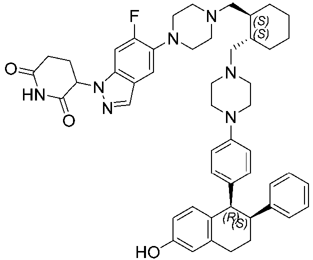 |
| VI-17 | 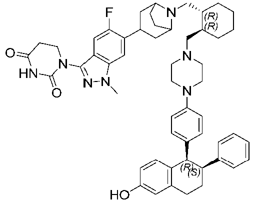 | VI-18 | 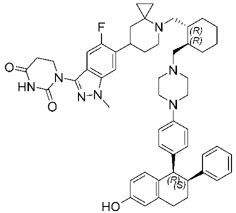 |
| VI-19 | 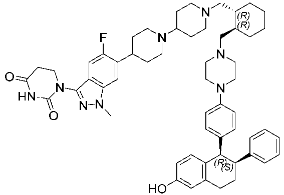 | VI-20 | 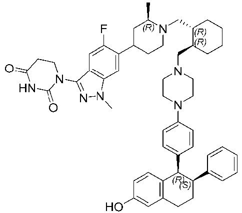 |
| VI-21 | 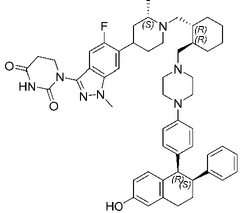 | VI-22 | 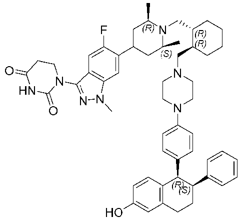 |
| VI-23 | 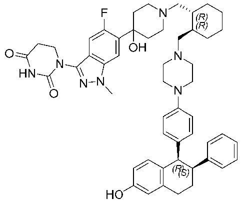 | VI-24 | 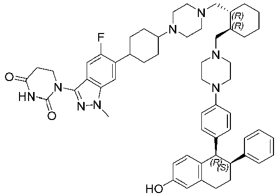 |
| VI-25 | 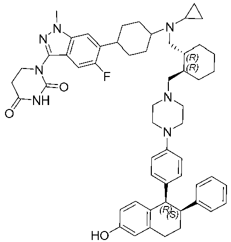 | VI-26 | 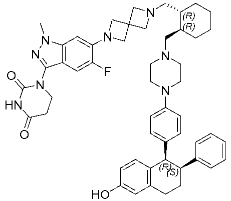 |
| VI-27 | 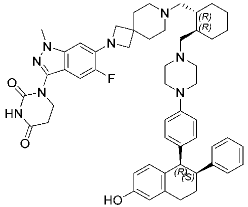 | VI-28 | 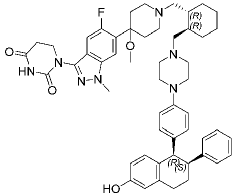 |
| VI-29 | 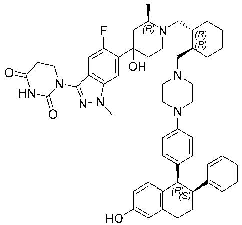 | VI-30 | 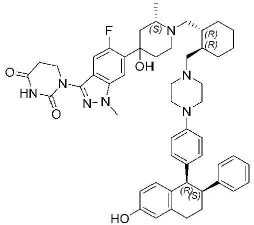 |
| VI-31 | 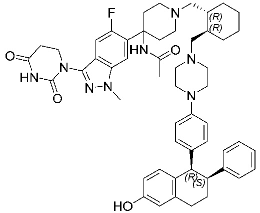 | VI-32 | 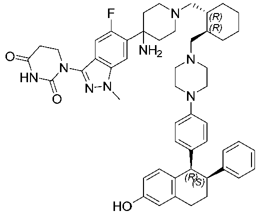 |
| VI-33 | 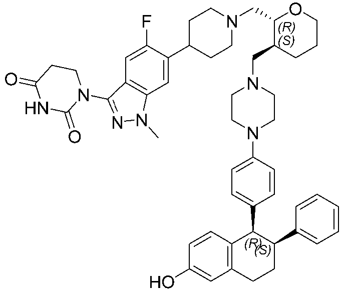 | VI-34 | 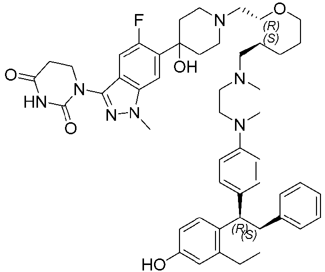 |
| VI-35 | 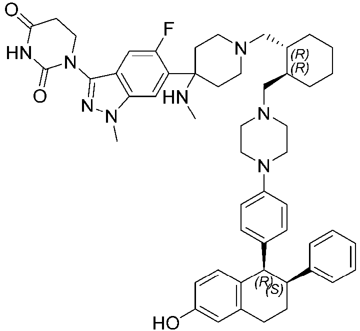 | VI-36 | 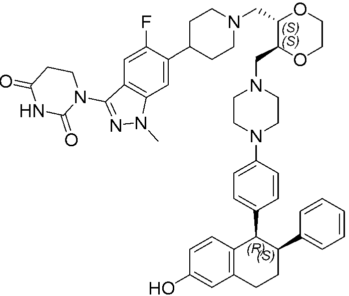 |
| VI-37 | 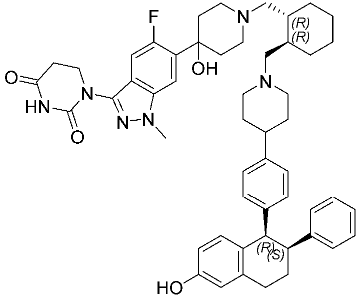 | VI-38 | 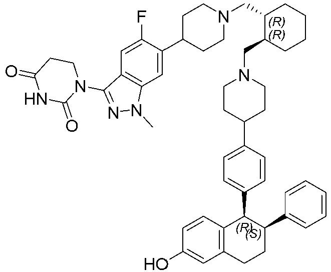 |
| VI-39 | 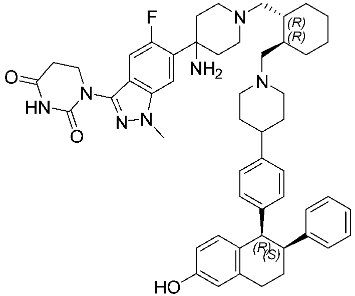 | VI-40 | 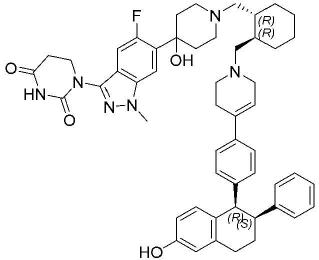 |
| VI-41 | 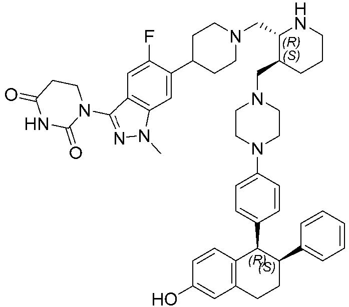 | VI-42 | 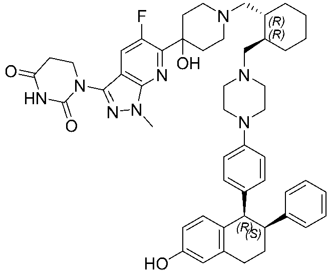 |
| VI-43 | 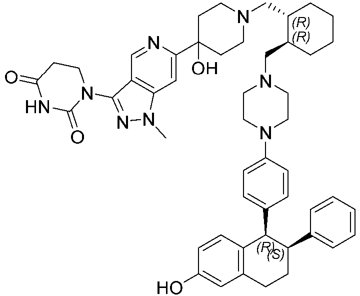 | VI-44 | 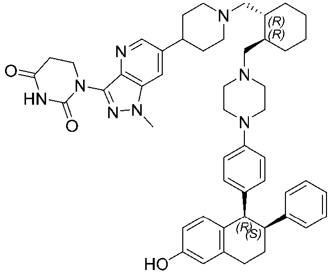 |
| VI-45 | 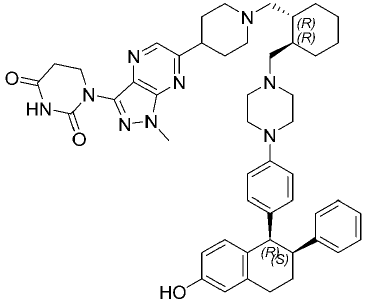 | | |
| Serial No. | Structure | Serial No. | Structure |
|---|---|---|---|
| VII-1 | 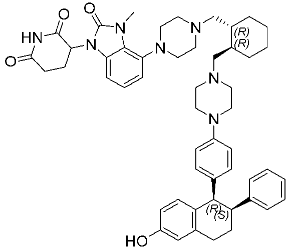 | VII-2 | 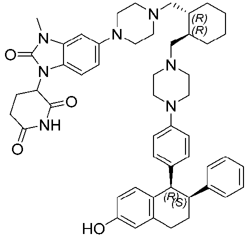 |
| VII-3 | 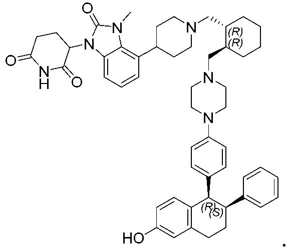 | | |

11. A pharmaceutical composition, comprising the compound according to any one of claims 1-10, or the isomer or pharmaceutically acceptable salt thereof, and one or more pharmaceutically acceptable carriers, diluents or excipients.

12. Use of the compound according to any one of claims 1-10, or the isomer or pharmaceutically acceptable salt thereof, or the pharmaceutical composition according to claim 11 in the preparation of a medicament for treating and/or preventing a disease or condition by degrading a target protein;
preferably, the disease or condition is selected from abnormal cell proliferation, tumors, immune diseases, diabetes, cardiovascular diseases, infectious diseases and inflammatory diseases; and preferably tumors and infectious diseases;
preferably, the tumor is cancer; preferably selected from breast cancer, endometrial cancer, testicular cancer, cervical cancer, prostate cancer, ovarian cancer, fallopian tube tumors, leukemia, skin cancer, squamous cell carcinoma, basal cell carcinoma, bladder cancer, colorectal cancer, esophageal cancer, head and neck cancer, kidney cancer, liver cancer, lung cancer, pancreatic cancer, gastric cancer, lymphoma, melanoma, sarcoma, peripheral neuroepithelioma, glioma, astrocytoma, ependymoma, glioblastoma, neuroblastoma, gangliocytoma, medulloblastoma, pineal cell tumor, meningioma, neurofibroma, neurilemoma, thyroid cancer, Wilms' tumor and teratoma; and more preferably selected from breast cancer, endometrial cancer, testicular cancer, cervical cancer, prostate cancer, ovarian cancer and fallopian tube tumors;
preferably, the infectious disease is selected from viral pneumonia, influenza, avian influenza, meningitis, gonorrhea, or a disease from infection with HIV, HBV, HCV, HSV, HPV, RSV, CMV, Ebola virus, flavivirus, trace virus, rotavirus, coronavirus, EBV, drug-resistant virus, RNA virus, DNA virus, adenovirus, poxvirus, picornavirus, togavirus, orthomyxovirus, retrovirus, hepadnavirus, Gram-negative bacteria, Gram-positive bacteria, atypical bacteria, Staphylococcus, Streptococcus, Escherichia coli, Salmonella, Helicobacter pylori, Chlamydiaceae, Mycoplasmaceae, fungi, protozoa, intestinal worms, worms, prions or parasites.

13. Use of the compound according to any one of claims 1-10, or the isomer or pharmaceutically acceptable salt thereof, or the pharmaceutical composition according to claim 11 in the preparation of a medicament for treating and/or preventing an estrogen-receptor mediated or dependent disease or condition, wherein the estrogen-receptor mediated or dependent disease or condition is a tumor, preferably cancer, more preferably breast cancer, endometrial cancer, testicular cancer, cervical cancer, prostate cancer, ovarian cancer and fallopian tube tumor, and most preferably breast cancer.
